# EUROPEAN PATENT APPLICATION

(11) **EP 4 410 307 A1**
(43) Date of publication of application: **07.08.2024**
(21) Application number: 22875194.7
(22) Date of filing: 30.09.2022
(51) Int. Cl.: A61K 39/395, C07K 16/46, C12N 5/10, A61K 47/68, C12N 15/13, A61P 35/00

(54) **PYRROLO BENZODIAZEPINE DERIVATIVE, AND CONJUGATE, PREPARATION METHOD AND USE THEREOF**

(30) Priority: 30.09.2021 CN 202111164628; 19.11.2021 CN 202111399183; 07.04.2022 CN 202210363759; 15.07.2022 CN 202210836645
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN); Suncadia Pharmaceuticals Co., Ltd, Chengdu, Sichuan 610000 (CN)
(72) Inventor: XU, Jianyan, Shanghai 200245 (CN); CAI, Xiaofeng, Shanghai 200245 (CN); QU, Bolei, Shanghai 200245 (CN); ZHANG, Ying, Shanghai 200245 (CN); CHEN, Xiaoxi, Shanghai 200245 (CN); HE, Feng, Shanghai 200245 (CN); TAO, Weikang, Shanghai 200245 (CN); ZHU, Lingjian, Lianyungang, Jiangsu 222047 (CN); HONG, Min, Lianyungang, Jiangsu 222047 (CN); HUANG, Jian, Lianyungang, Jiangsu 222047 (CN); LI, Yanbing, Chengdu, Sichuan 610000 (CN); XU, Xiaolang, Chengdu, Sichuan 610000 (CN); HU, Zhipeng, Chengdu, Sichuan 610000 (CN); XUE, Zhouyang, Chengdu, Sichuan 610000 (CN); YOU, Lingfeng, Shanghai 200245 (CN)
(74) Representative: Dragotti & Associati S.R.L.
(86) International application number: PCT/CN2022/123462
(87) International publication number: WO 2023/051814

(57) **Abstract**

The present disclosure relates to a pyrrolo benzodiazepine derivative and a conjugate, preparation method and use thereof. In particular, the present disclosure relates to a compound of formula (DL) and a antibody-drug conjugate thereof, a pharmaceutical composition containing same, and a use thereof in the preparation of a medicament for the treatment of cancer. The definitions of groups in the general formula (DL) are as defined in the description.

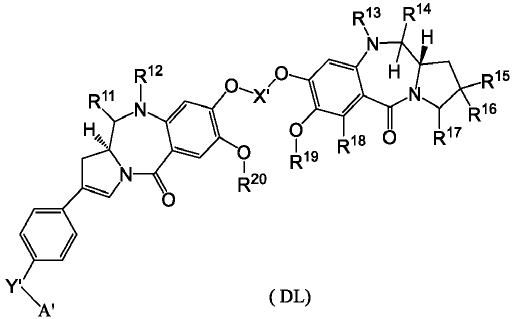

## Description

### TECHNICAL FIELD

The present disclosure relates to pyrrolo benzodiazepine derivatives, antibody-drug conjugates thereof, and pharmaceutical use thereof.

### BACKGROUND

Pyrrolo benzodiazepines (PBDs) are a class of sequence-selective DNA minor groove binding agents capable of preventing DNA from binding to transcription factors, thus causing cell replication arrest and ultimately cell death. They act independently of the replication cycles of cells and cause DNA damage that is difficult to repair, showing relatively high levels of cytotoxicity. The naturally occurring anti-tumor antibiotic anthramycin, the first example of PBD monomers, was discovered in the 1960s.

The cytotoxicity of PBDs is greatly increased when they form dimeric structures (PBD dimers). There are many reports of their use in antibody-drug conjugates (WO2014057074, WO2014057119, WO2015052321, WO2016166307, WO2017004026, WO2019046859, WO2019065964, etc.). However, there is still an urgent clinical need for safer and more effective PBD ADCs.

### SUMMARY

The present disclosure provides a pyrrolo benzodiazepine derivative and an antibody-drug conjugate thereof.

The present disclosure provides an antibody-drug conjugate or a pharmaceutically acceptable salt thereof, represented by the following structure: 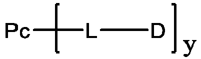
wherein Pc is an antibody, and a glycan chain of the antibody may be optionally remodeled;
L is a linker; Pc binds to L through an amino acid thereof or the glycan chain;
y is a drug loading of about 1 to about 10;
D is represented by formula (D): 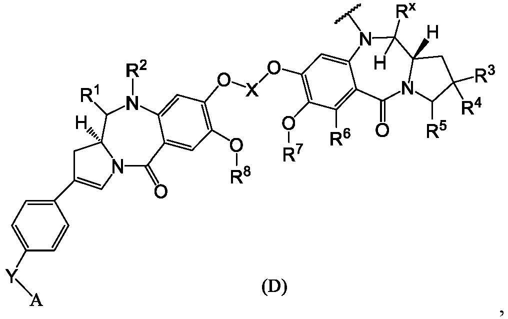
wherein:
   R¹ is H, oxo, halogen, or alkyl;
   R² is H, alkyl, or 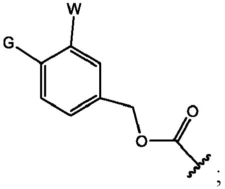 W is H, -C(O)R^{t}, or -C(O)NH-R^{t}; G is a glucuronide group or galactoside group; R^{t} is alkyl or alkoxyalkyl;
   R³ is H or alkyl;
   R⁴ is H or alkyl;
   R⁵ is H, halogen, or alkyl;
   or R³ and R⁴, together with the atom to which they are attached, form a ring, or R⁴ and
   R⁵, together with the atoms to which they are attached, form a ring;
   R⁶ is H or alkyl;
   R⁷ is alkyl or cycloalkyl; or R⁶ and R⁷, together with the atoms to which they are attached, form a ring;
   R⁸ is alkyl or cycloalkyl;
   R^{x} is H or hydroxy;
   X is alkylene or -(CH₂)ₘ-X₁-(CH₂)ₙ-;
   X₁ is a nitrogen atom, an oxygen atom, a sulfur atom, cycloalkyl, heterocyclyl, heteroaryl, or aryl; the cycloalkyl, heterocyclyl, heteroaryl, or aryl is optionally substituted with one or more substituents, wherein each of the substituents is independently selected from the group consisting of amino, hydroxy, hydroxyalkyl, alkyl, and alkoxy;
   Y is a bond, a nitrogen atom, an oxygen atom, or a sulfur atom;
   A is alkyl, cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R⁹, cycloalkyl, cycloalkylalkyl, haloalkyl, aryl, and heterocyclyl;
   R⁹ is H, alkyl, and hydroxyalkyl;
   m is 1, 2, or 3; n is 1, 2, or 3;
   provided that when X is alkylene, 1) R¹ is oxo, or 2) R^{x} is H, or 3) R⁴ and R⁵, together with the atoms to which they are attached, form a ring, or 4) R⁶ and R⁷, together with the atoms to which they are attached, form a ring, or 5) R⁷ and/or R⁸ are cycloalkyl; or 6) A is not alkyl.

In some embodiments, the aforementioned antibody-drug conjugate or the pharmaceutically acceptable salt thereof is represented by the following structure: 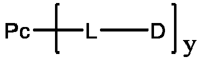
wherein Pc is an antibody, and a glycan chain of the antibody may be optionally remodeled;
L is a linker; Pc binds to L through an amino acid thereof or the glycan chain;
y is a drug loading of about 1 to about 10;
D is represented by formula (D): 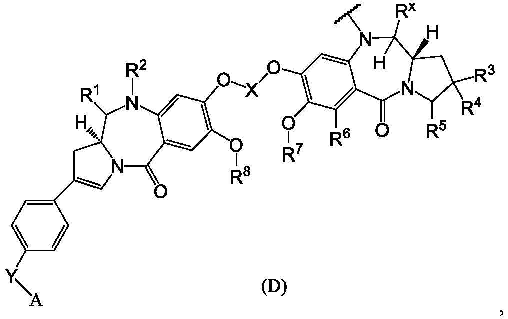
wherein:
   R¹ is H, oxo, halogen, or alkyl;
   R² is H, alkyl, or 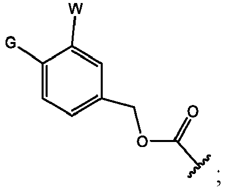 W is H, -C(O)R^{t}, or -C(O)NH-R^{t}; G is a glucuronide group or galactoside group; R^{t} is alkyl or alkoxyalkyl;
   R³ is H or alkyl;
   R⁴ is H or alkyl;
   R⁵ is H, halogen, or alkyl;
   or R³ and R⁴, together with the atom to which they are attached, form a ring, or R⁴ and
   R⁵, together with the atoms to which they are attached, form a ring;
   R⁶ is H or alkyl;
   R⁷ is alkyl or cycloalkyl; or R⁶ and R⁷, together with the atoms to which they are attached, form a ring;
   R⁸ is alkyl or cycloalkyl;
   R^{x} is H or hydroxy;
   X is alkylene or -(CH₂)ₘ-X₁-(CH₂)ₙ-;
   X₁ is a nitrogen atom, an oxygen atom, a sulfur atom, cycloalkyl, heterocyclyl, heteroaryl, or aryl; the cycloalkyl, heterocyclyl, heteroaryl, or aryl is optionally substituted with one or more substituents, wherein each of the substituents is independently selected from the group consisting of amino, hydroxy, hydroxyalkyl, alkyl, and alkoxy;
   Y is a bond, a nitrogen atom, an oxygen atom, or a sulfur atom;
   A is alkyl, cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R⁹, cycloalkyl, aryl, and heterocyclyl;
   R⁹ is H, alkyl, and hydroxyalkyl;
   m is 1, 2, or 3; n is 1, 2, or 3;
   provided that when X is alkylene, 1) R¹ is oxo, or 2) R^{x} is H, or 3) R⁴ and R⁵, together with the atoms to which they are attached, form a ring, or 4) R⁶ and R⁷, together with the atoms to which they are attached, form a ring, or 5) R⁷ and/or R⁸ are cycloalkyl; or 6) A is not alkyl.

In some embodiments, in the aforementioned antibody-drug conjugate or the pharmaceutically acceptable salt thereof, the R¹ is H or oxo; preferably, R¹ is H.

In some embodiments, in the aforementioned antibody-drug conjugate or the pharmaceutically acceptable salt thereof, the R² is H or C₁₋₆ alkyl; preferably, R² is H.

In some embodiments, in the aforementioned antibody-drug conjugate or the pharmaceutically acceptable salt thereof, the R² is represented by the following structure: 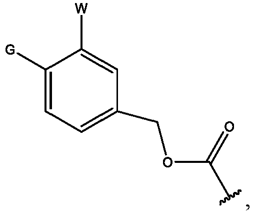 wherein W is H or -C(O)NH-R, and G is a glucuronide group; R^{t} is C₁₋₆ alkyl or C₁₋₆ alkoxy C₁₋₆ alkyl.

In some embodiments, in the aforementioned antibody-drug conjugate or the pharmaceutically acceptable salt thereof, the R^{x} is hydroxy.

In some embodiments, in the aforementioned antibody-drug conjugate or the pharmaceutically acceptable salt thereof,
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form 3-membered cycloalkyl (a 3-membered carbocycle);
R⁵ is H; R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is alkylene; preferably, X is C₁₋₁₂ alkylene; more preferably, X is pentylene;
Y is selected from the group consisting of a bond, a nitrogen atom, an oxygen atom, and a sulfur atom; preferably, Y is a bond or a sulfur atom; more preferably, Y is a bond;
A is selected from the group consisting of cycloalkyl, heterocyclyl, aryl, and heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R⁹, cycloalkyl, cycloalkylalkyl, haloalkyl, aryl, and heterocyclyl; preferably, A is selected from the group consisting of C₃₋₆ cycloalkyl, 5- to 6-membered heterocyclyl, phenyl, and 5- to 6-membered heteroaryl; the cycloalkyl, heterocyclyl, aryl, or heteroaryl is optionally substituted with one or more substituents, wherein each of the substituents is independently selected from the group consisting of C₁₋₆ hydroxyalkyl, C₁₋₆ alkyl, C₃₋₆ cycloalkyl C₁₋₆ alkyl, C₁₋₆ haloalkyl, and -C(O)R⁹;
R⁹ is selected from the group consisting of H, alkyl, and hydroxyalkyl; preferably, R⁹ is C₁₋₆ alkyl or C₁₋₆ hydroxyalkyl.

In some embodiments, in the aforementioned antibody-drug conjugate or the pharmaceutically acceptable salt thereof,
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form a 3-membered carbocycle;
R⁵ is H; R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is alkylene; preferably, X is C₁₋₁₂ alkylene; more preferably, X is pentylene;
Y is selected from the group consisting of a bond, a nitrogen atom, an oxygen atom, and a sulfur atom; preferably, Y is a bond or a sulfur atom; more preferably, Y is a bond;
A is selected from the group consisting of cycloalkyl, heterocyclyl, aryl, and heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R⁹, cycloalkyl, aryl, and heterocyclyl; preferably, A is selected from the group consisting of C₃₋₆ cycloalkyl, 5- to 6-membered heterocyclyl, phenyl, and 5- to 6-membered heteroaryl; the cycloalkyl, heterocyclyl, aryl, or heteroaryl is optionally substituted with one or more substituents, wherein each of the substituents is independently selected from the group consisting of C₁₋₆ hydroxyalkyl, C₁₋₆ alkyl, and -C(O)R⁹;
R⁹ is selected from the group consisting of H, alkyl, and hydroxyalkyl; preferably, R⁹ is C₁₋₆ alkyl or C₁₋₆ hydroxyalkyl.

In some embodiments, in the aforementioned antibody-drug conjugate or the pharmaceutically acceptable salt thereof,
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form a 3-membered carbocycle;
R⁵ is H; R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is -(CH₂)ₘ-X₁-(CH₂)ₙ-;
X₁ is an oxygen atom, cycloalkyl, heteroaryl, or aryl; the cycloalkyl, heteroaryl, or aryl is optionally substituted with one or more substituents, wherein each of the substituents is independently selected from the group consisting of amino, hydroxy, hydroxyalkyl, alkyl, and alkoxy; preferably, X₁ is an oxygen atom, C₃₋₆ cycloalkyl, pyridinyl, or phenyl; the C₃₋₆ cycloalkyl or phenyl is optionally substituted with one or more amino or C₁₋₆ alkyl;
m is selected from the group consisting of 1, 2, and 3; n is selected from the group consisting of 1, 2, and 3.

In some embodiments, in the aforementioned antibody-drug conjugate or the pharmaceutically acceptable salt thereof,
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form 3-membered cycloalkyl (a 3-membered carbocycle);
X is alkylene; preferably, X is C₁₋₆ alkylene; more preferably, X is pentylene;
R⁶ is H or alkyl; preferably, R⁶ is H or C₁₋₆ alkyl;
R⁷ is alkyl or cycloalkyl; preferably, R⁷ is C₁₋₆ alkyl or C₃₋₆ cycloalkyl;
or R⁶ and R⁷, together with the atoms to which they are attached, form a ring; preferably, R⁶ and R⁷, together with the atoms to which they are attached, form a 4-6-membered ring;
R⁸ is alkyl or cycloalkyl; preferably, R⁸ is C₁₋₆ alkyl or C₃₋₆ cycloalkyl;
provided that R⁷ and R⁸ are not simultaneously alkyl.

In some embodiments, in the aforementioned antibody-drug conjugate or the pharmaceutically acceptable salt thereof,
R¹ is H or alkyl; preferably, R¹ is H or C₁₋₆ alkyl; more preferably, R¹ is H;
R³ is H;
R⁴ and R⁵, together with the atoms to which they are attached, form a ring; preferably,
R⁴ and R⁵, together with the atoms to which they are attached, form 3-membered cycloalkyl (a 3-membered carbocycle);
R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is alkylene; preferably, X is C₁₋₁₂ alkylene; more preferably, X is pentylene.

In some embodiments, in the aforementioned antibody-drug conjugate or the pharmaceutically acceptable salt thereof, L is -L^{a}-L^{b}-L^{c}-L^{d}-;
L^{a} is selected from the group consisting of: 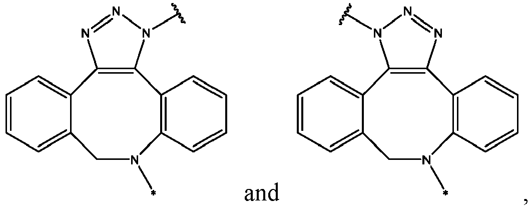 wherein the asterisk * indicates binding to L^{b}, and the wavy line 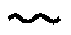 indicates binding to the remodeled glycan chain of Pc;
L^{b} is selected from the group consisting of -C(O)-CH₂CH₂-C(O)-, -C(O)-(CH₂CH₂)₂-C(O)-, -C(O)-CH₂CH₂-C(O)-NH-(CH₂CH₂)₂-C(O)-, -C(O)-CH₂CH₂-C(O)-NH-(CH₂CH₂O)₂-CH₂-C(O)-, -C(O)-CH₂CH₂-NH-C(O)-(CH₂CH₂O)₄-CH₂CH₂-C(O)-, -CH₂-OC(O)-, and -OC(O)-;
L^{c} is a peptide residue consisting of 2 to 7 amino acid residues, wherein the amino acid residues are residues formed from phenylalanine (F), alanine (A), proline (P), isoleucine (I), glycine (G), valine (V), lysine (K), citrulline (Cit), serine (S), glutamic acid (E), or aspartic acid (D); the amino acid residues are unsubstituted or are each independently substituted with one or more substituents, wherein each of the substituents is independently selected from the group consisting of halogen, hydroxy, cyano, amino, C₁₋₆ alkyl, C₁₋₆ haloalkyl, C₁₋₆ alkoxy, and C₃₋₇ cycloalkyl;
L^{d} is -NH-R^{a}-CH₂O-C(O)-, -NH-CH₂O-R^{b}-C(O)-, or a bond;
R^{a} is phenyl or 5-6 membered heterocyclyl; the phenyl and 5-6 membered heterocyclyl are unsubstituted or are each independently substituted with one or more substituents, wherein each of the substituents is independently selected from the group consisting of halogen, oxo, hydroxy, cyano, amino, C₁₋₆ alkyl, C₁₋₆ haloalkyl, and C₁₋₆ alkoxy;
R^{b} is C₁₋₆ alkyl or C₃₋₇ cycloalkyl; the C₁₋₆ alkyl and C₃₋₇ cycloalkyl are unsubstituted or are each independently substituted with one or more substituents, wherein each of the substituents is independently selected from the group consisting of halogen, oxo, hydroxy, cyano, amino, C₁₋₆ alkyl, C₃₋₇ cycloalkyl, 5-6 membered heterocyclyl, C₁₋₆ haloalkyl, and C₁₋₆ alkoxy.

In some embodiments, in the aforementioned antibody-drug conjugate or the pharmaceutically acceptable salt thereof, L^{b} is -C(O)-CH₂CH₂-C(O)-.

In some embodiments, in the aforementioned antibody-drug conjugate or the pharmaceutically acceptable salt thereof, L^{c} is -GGVA-, -VA-, -GGFG-, -GGPI-, -GGVCit-, -GGVK-, and -GGPL-, preferably -GGVA-.

In some embodiments, in the aforementioned antibody-drug conjugate or the pharmaceutically acceptable salt thereof,
L^{d} is -NH-R^{a}-CH₂O-C(O)-, and R^{a} is phenyl.

In some embodiments, in the aforementioned antibody-drug conjugate or the pharmaceutically acceptable salt thereof, the antibody Pc is an antibody that binds to tumor cells and can be taken in by tumor cells and is preferably selected from the group consisting of an anti-HER2 (ErbB2) antibody, an anti-EGFR antibody, an anti-B7H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-Claudin 18.2 antibody, an anti-ROR1 antibody, an anti-CD19 antibody, an anti-Trop2 antibody, an anti-CD79b antibody, an anti-SLC44A4 antibody, and an anti-Mesothelin antibody.

In some embodiments, in the aforementioned antibody-drug conjugate or the pharmaceutically acceptable salt thereof, the y is 1-8.

In some embodiments, in the aforementioned antibody-drug conjugate or the pharmaceutically acceptable salt thereof, the y is 1-4; preferably, y is selected from the group consisting of about 1, about 2, about 3, and about 4; more preferably, y is selected from about 2. y is an average antibody-to-drug ratio, which may be a decimal or integer. In some embodiments, in the aforementioned antibody-drug conjugate or the pharmaceutically acceptable salt thereof, the antibody binds, from a glycan chain that binds to Asn297 of the antibody, to L, and the glycan chain is an N297 glycan chain.

In some embodiments, the aforementioned antibody-drug conjugate or the pharmaceutically acceptable salt thereof is of the following structure:

Pc-(N297 glycan chain)-[L-D]y

wherein the N297 glycan chain is a remodeled glycan chain that binds to an Asn at position 297 of a heavy chain of Pc; the antibody Pc has two heavy chains and thus has two position 297 Asns; there may be two said N297 glycan chains binding to the two N297s of the heavy chains of Pc, respectively, or only one said N297 glycan chain binding to one N297 of the heavy chains of Pc;
y is 1-4, preferably 1-2;
Pc, L, and D are as defined above.

In some embodiments, in the aforementioned antibody-drug conjugate or the pharmaceutically acceptable salt thereof, the glycan chain structure is selected from the group consisting of N297-(Fuc)SG1, N297-(Fuc)SG2, N297-(Fuc)SG3, N297-(Fuc)SG4, and N297-(Fuc)SG5: 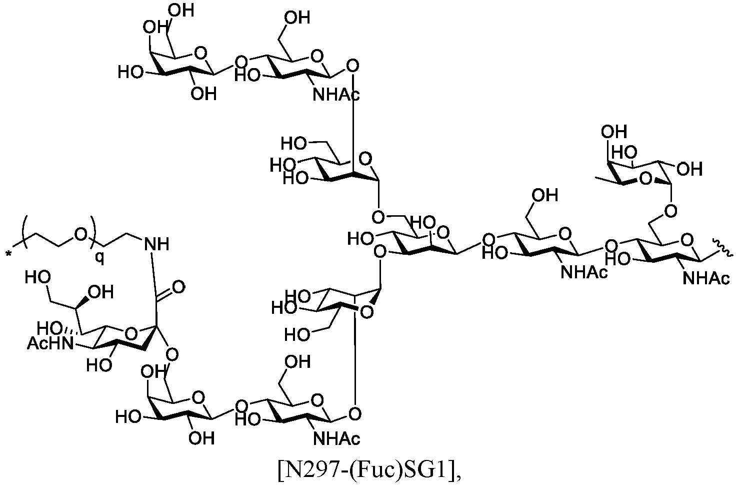 which is simplified as: 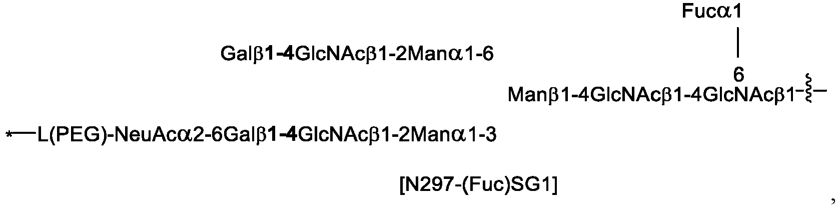 or 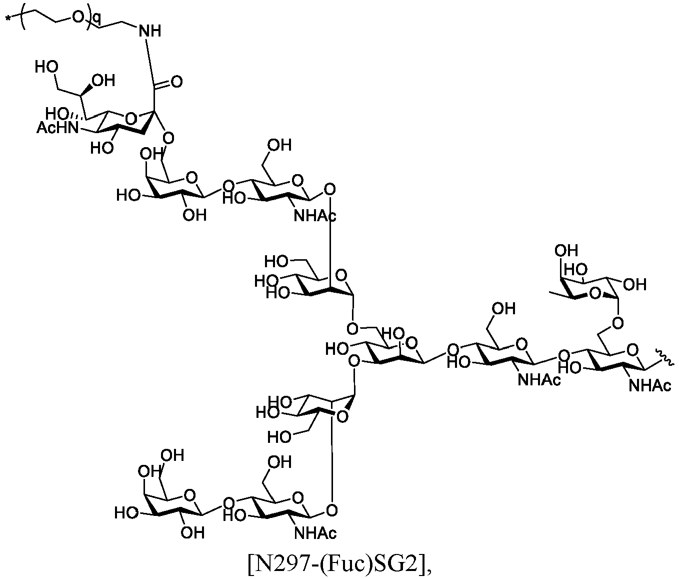 which is simplified as: 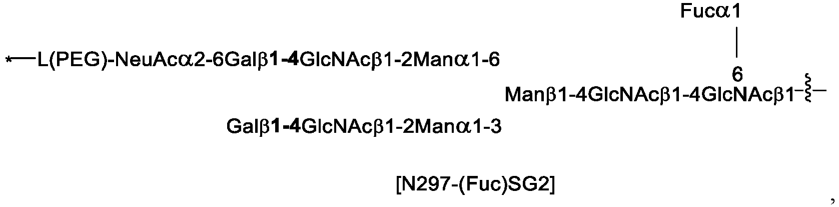 or 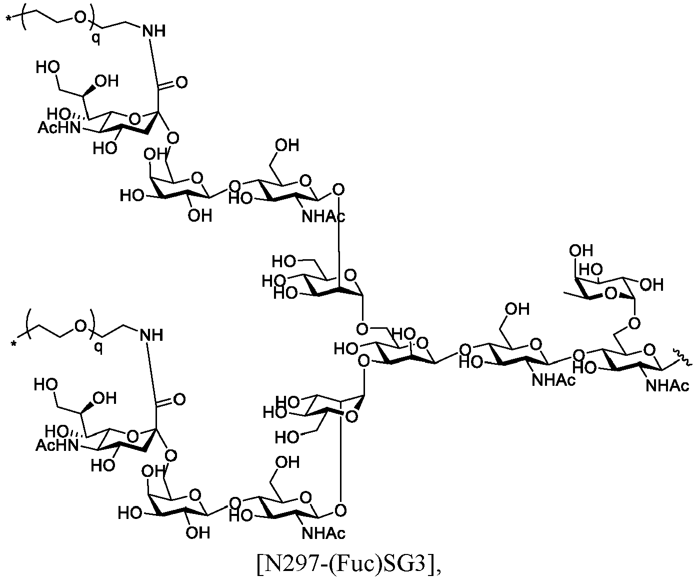 which is simplified as: 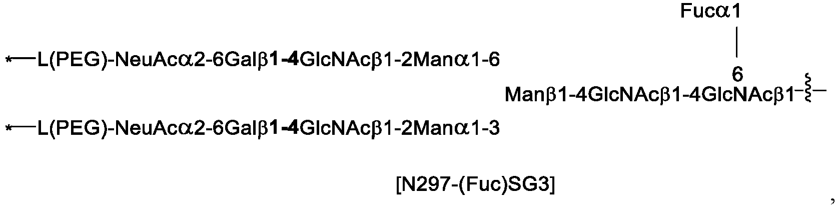 or 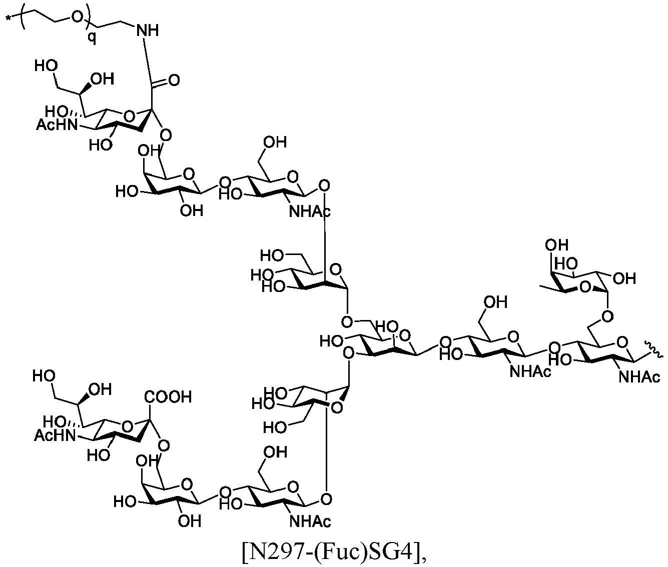 which is simplified as: 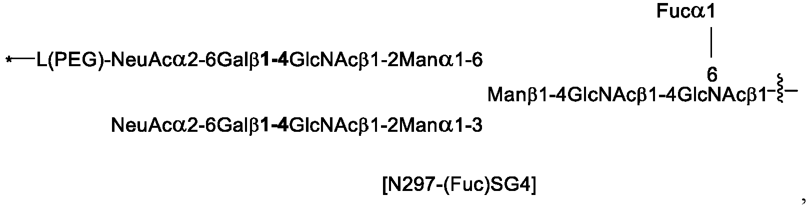 or 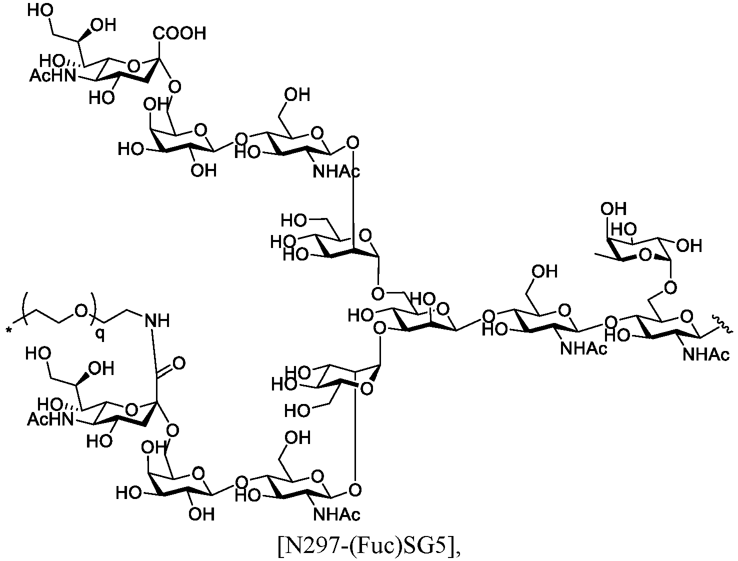 which is simplified as: 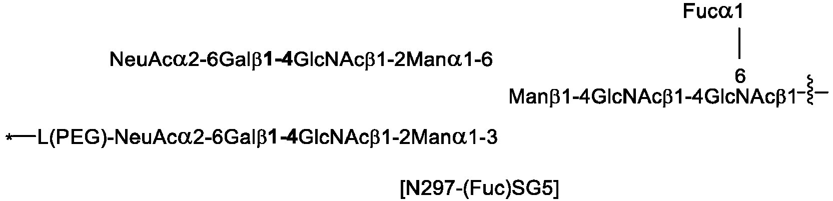
wherein the wavy line 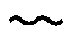 indicates binding to an Asn at position 297 of a heavy chain of Pc;
L(PEG) indicates -(CH₂CH₂O)qCH₂CH₂-NH-, wherein q is selected from the group consisting of 0-20, preferably 1-10; the amino group at the right end indicates amide bonding with the carboxylic acid at locant 2 of the N-acetylneuraminic acid (NeuAc) at the non-reducing terminus on the 1-3 chain side or/and 1-6 chain side of a β-Man branched chain of the N297 glycan chain;
the asterisk * indicates binding to the linker L; preferably, the asterisk * indicates binding to the nitrogen atom at locant 1 or 3 on the triazole ring in each structural formula linker;
Gal represents galactose; GlcNAc represents N-acetylgalactose; Man represents mannose; Fuc represents fucose; NeuAc represents N-acetylneuraminic acid.

In some embodiments, in the aforementioned antibody-drug conjugate, the glycan chain structure is selected from the group consisting of: 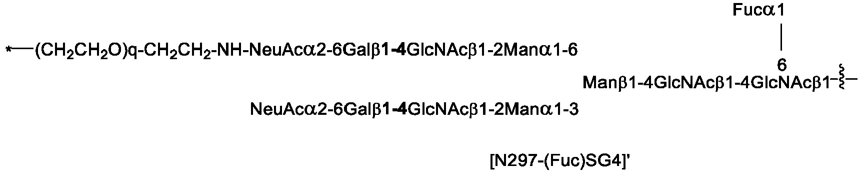 and 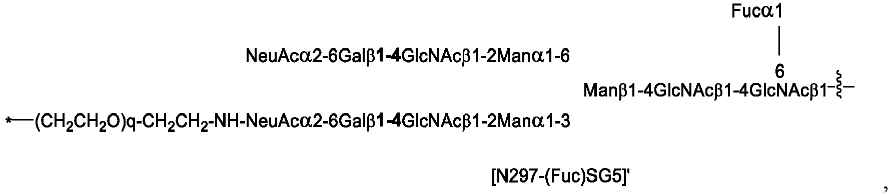
wherein q is selected from the group consisting of 2 and 3;
Gal, GlcNAc, Man, Fuc, and NeuAc are as defined above.

In some embodiments, in the aforementioned antibody-drug conjugate, the glycan chain structure is: 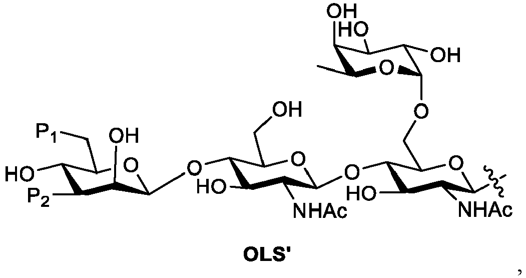
wherein the wavy line 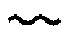 indicates binding to an Asn at position 297 of a heavy chain of Pc;
P₁ and P₂ are each independently selected from the group consisting of hydroxy, *-(CH₂CH₂O)s¹-, and *-(CH₂CH₂O)s²-(CH₂CH₂CH₂O)s³-(CH₂CH₂O)s⁴-; the *-(CH₂CH₂O)s¹- and *-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy;
s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5; provided that P₁ and P₂ are not simultaneously hydroxy or *-(CH₂CH₂O)s¹-;
the asterisk * indicates binding to the linker L; preferably, the asterisk * indicates binding to the nitrogen atom at locant 1 or 3 on the triazole ring in each structural formula linker.

In some embodiments, in the aforementioned antibody-drug conjugate, the N297 glycan chain structure is selected from the group consisting of: 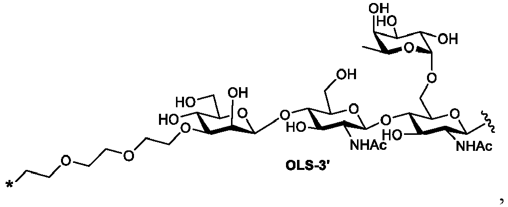 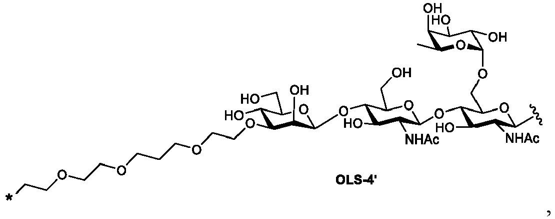 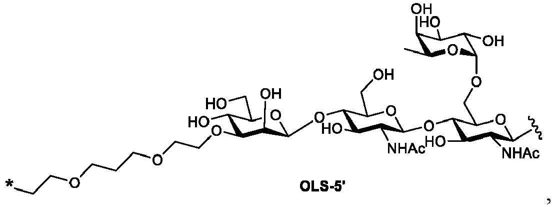 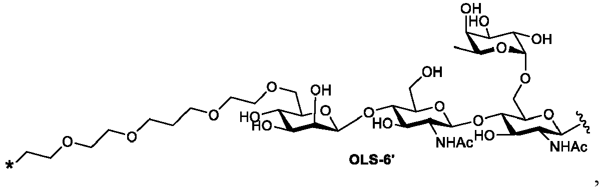 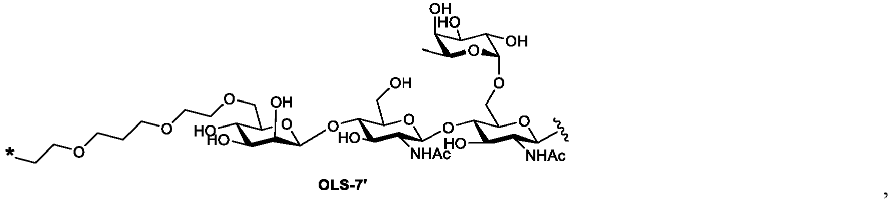 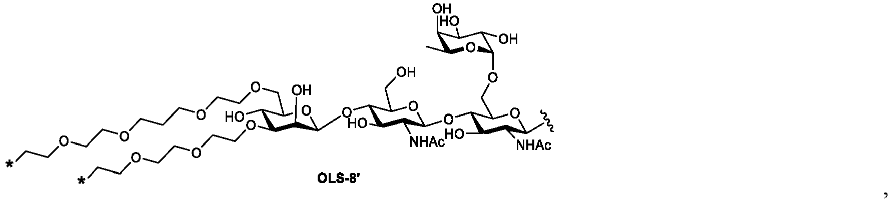 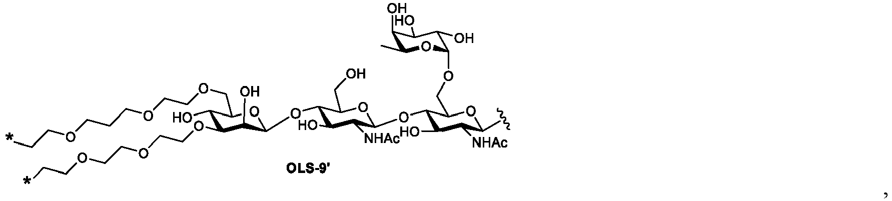 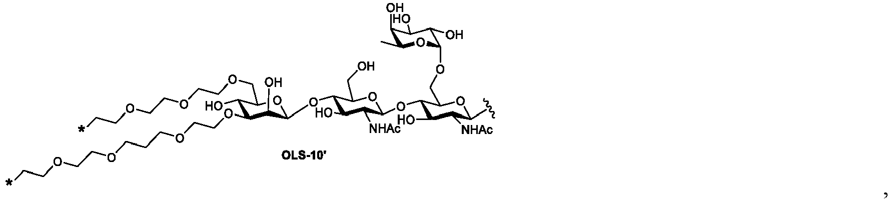 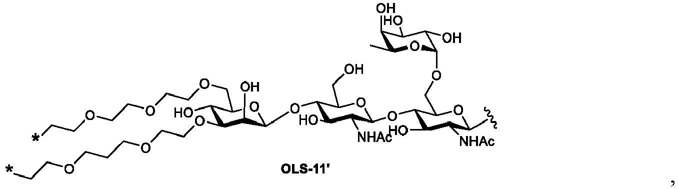 and

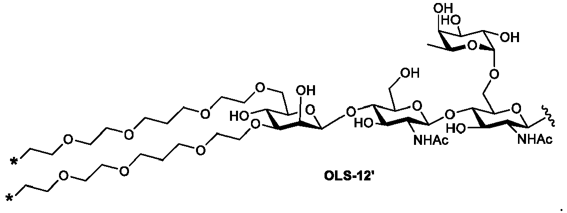

In some embodiments, the aforementioned antibody-drug conjugate or the pharmaceutically acceptable salt thereof is selected from the group consisting of: 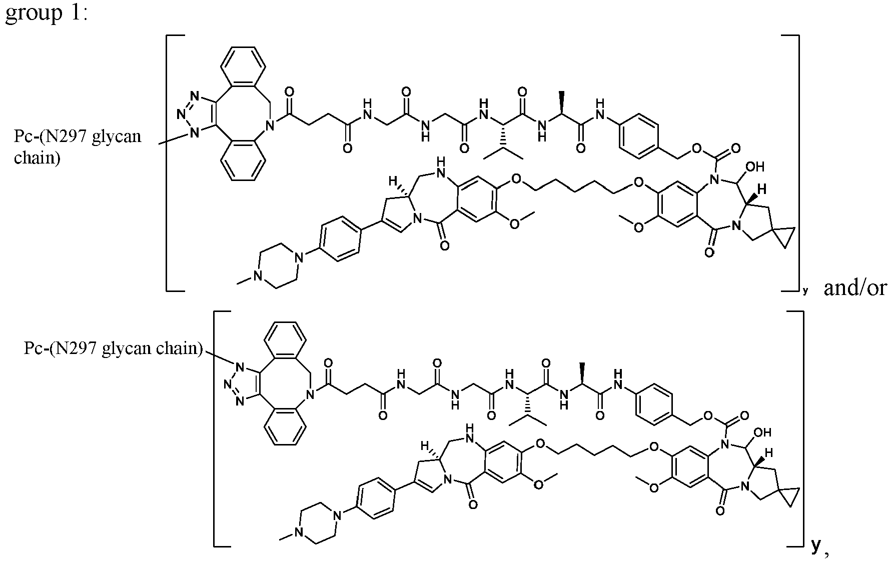 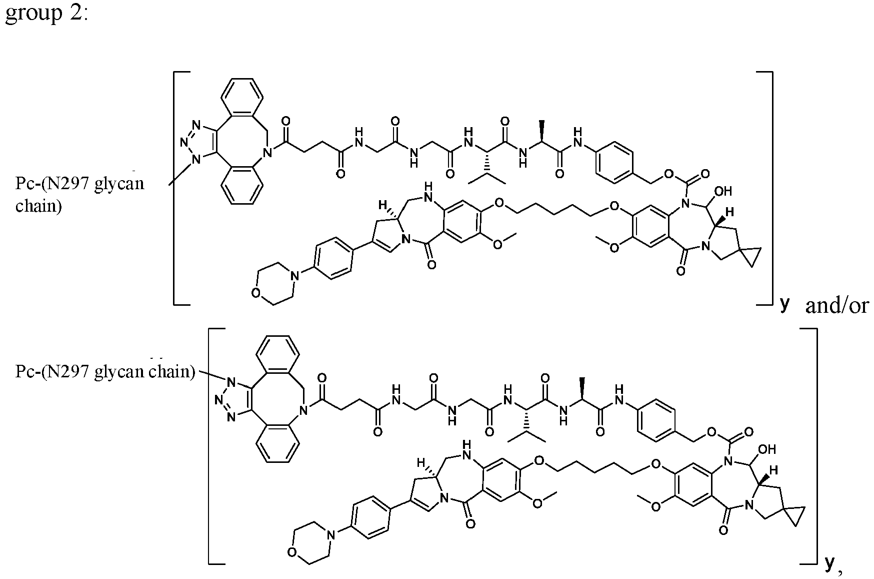 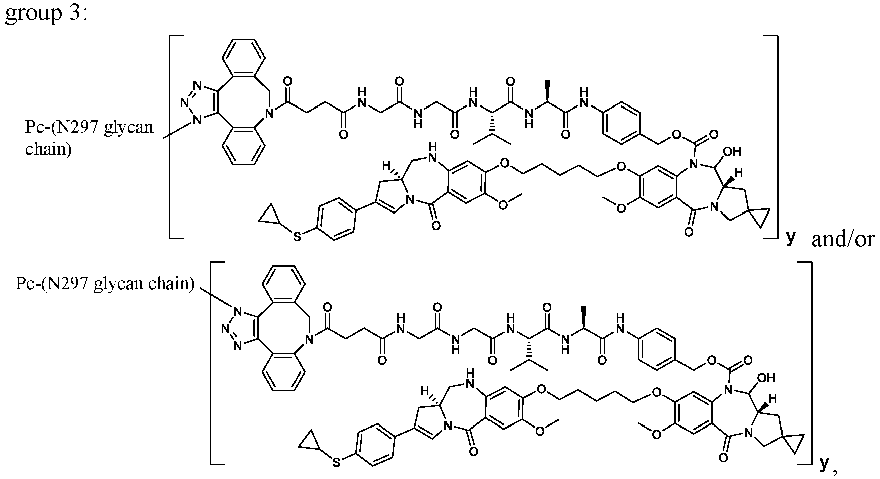 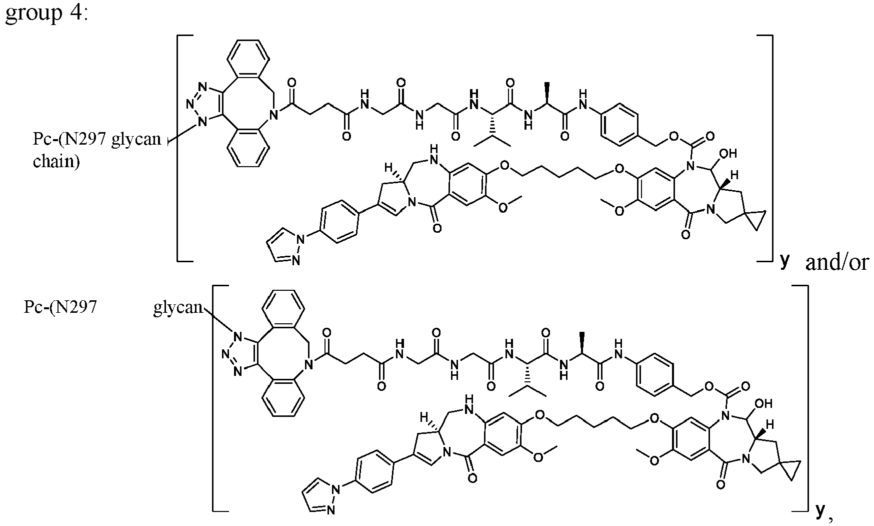 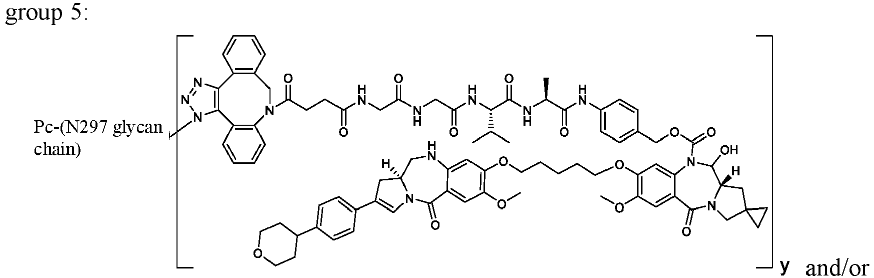 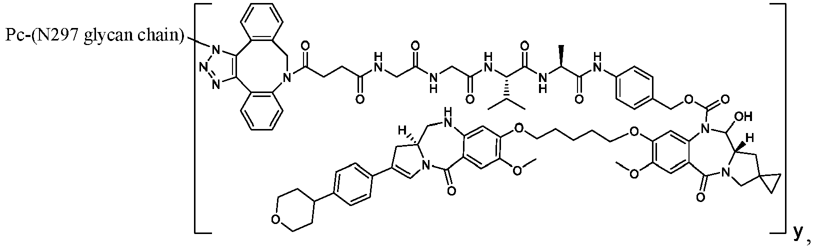 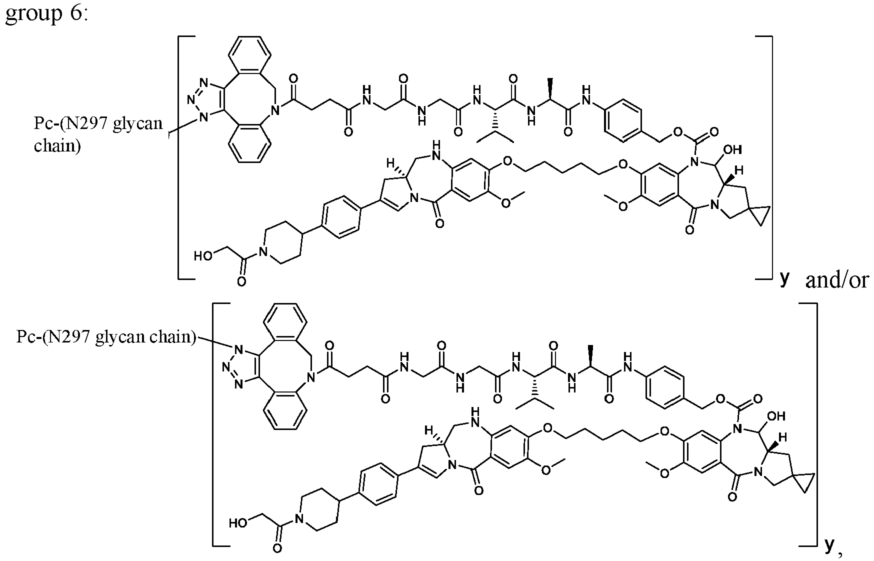 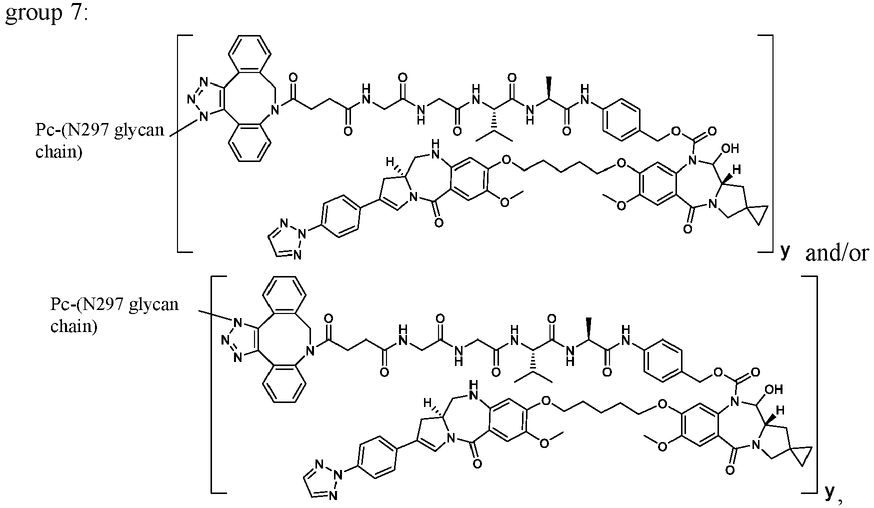 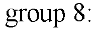 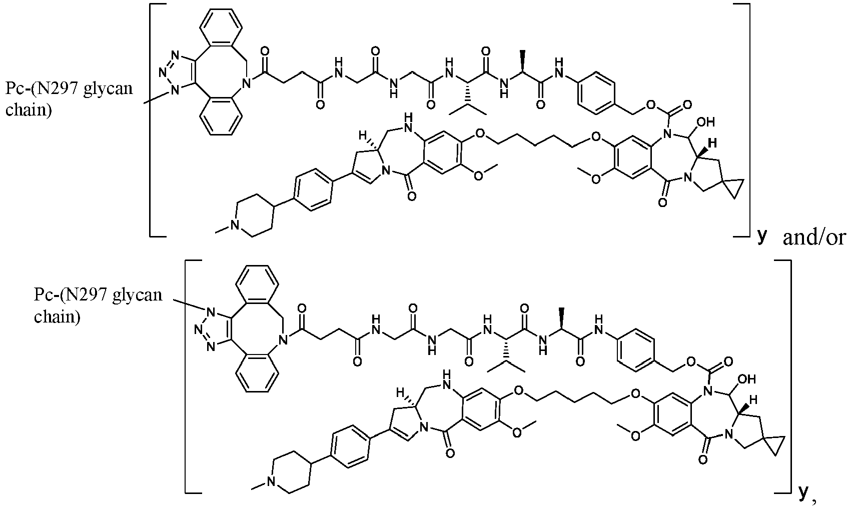 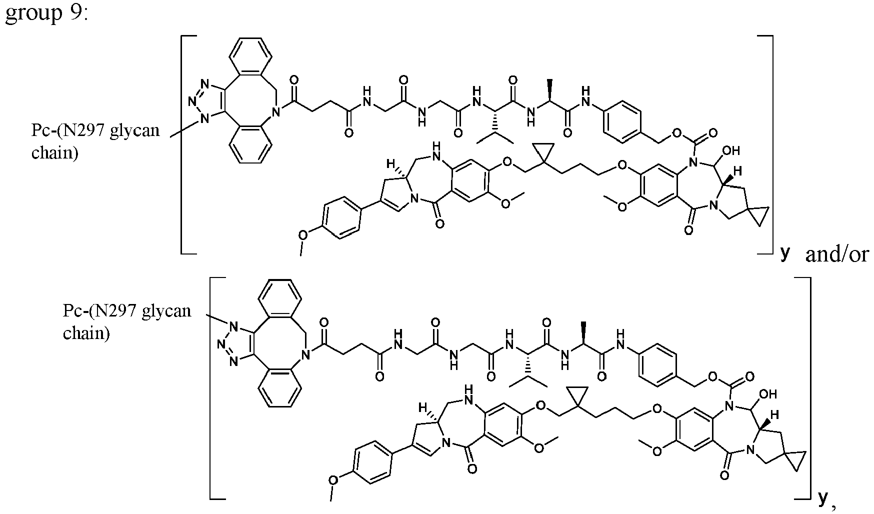 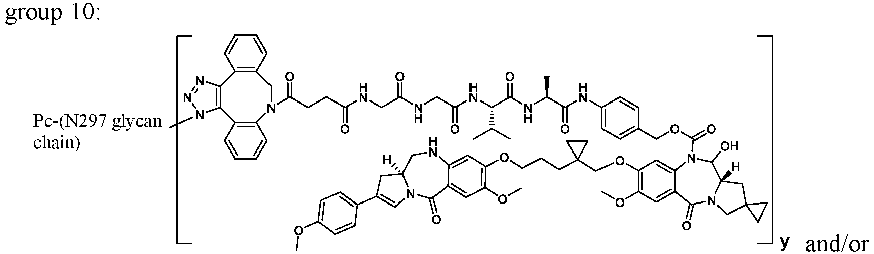 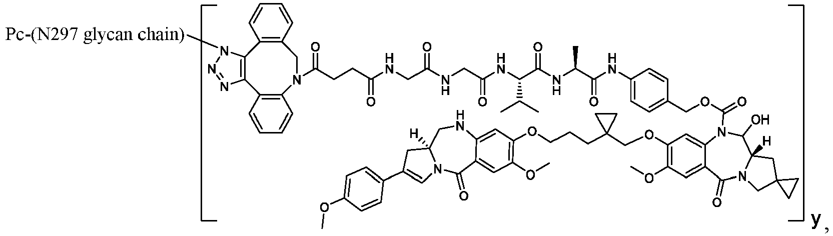 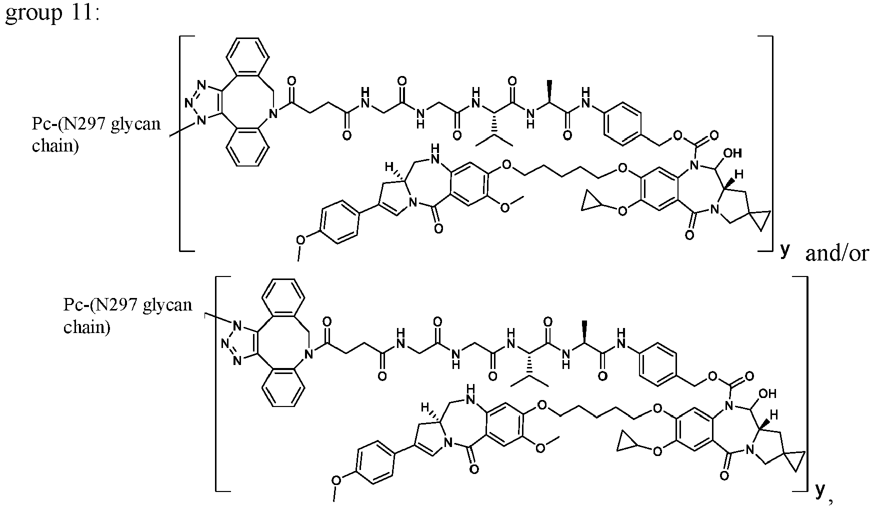 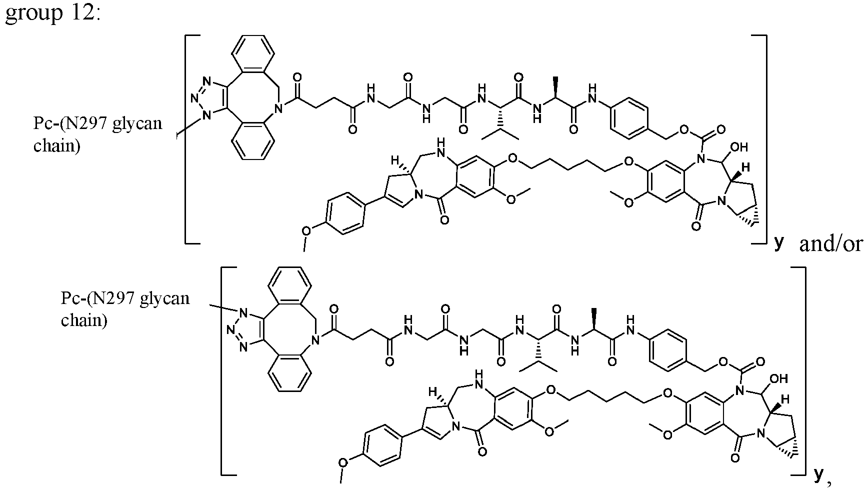 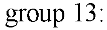 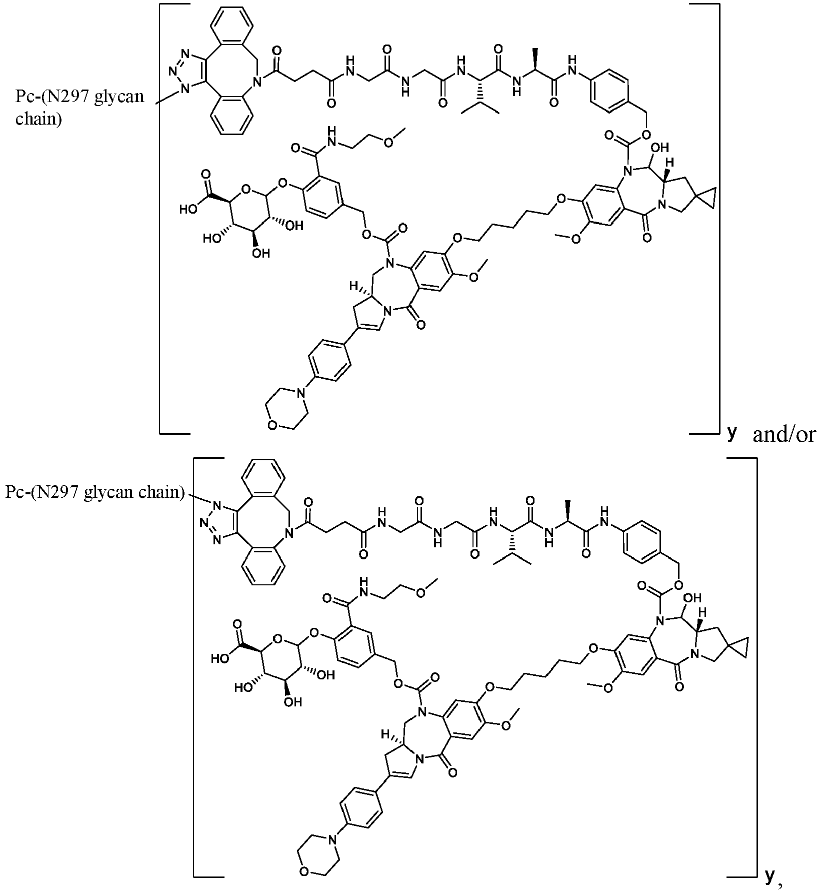 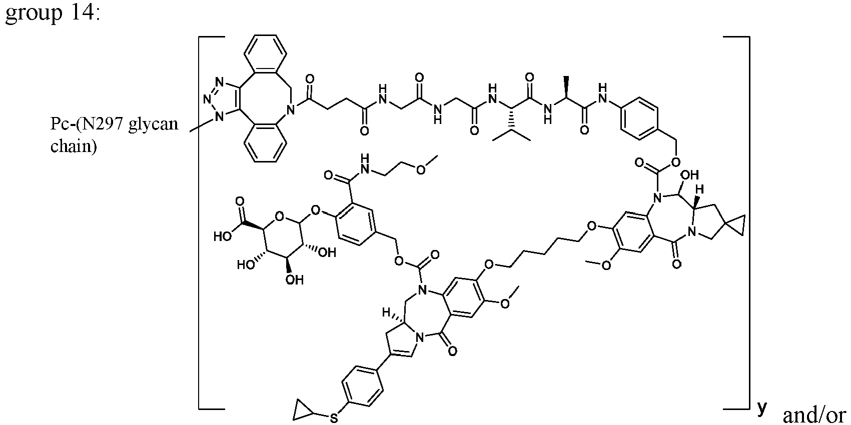 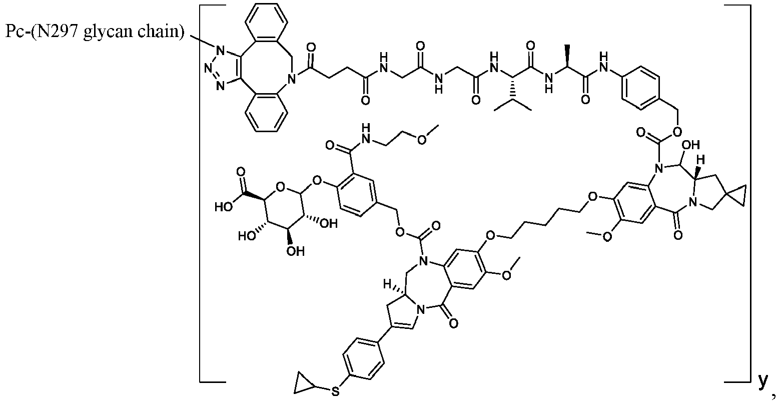 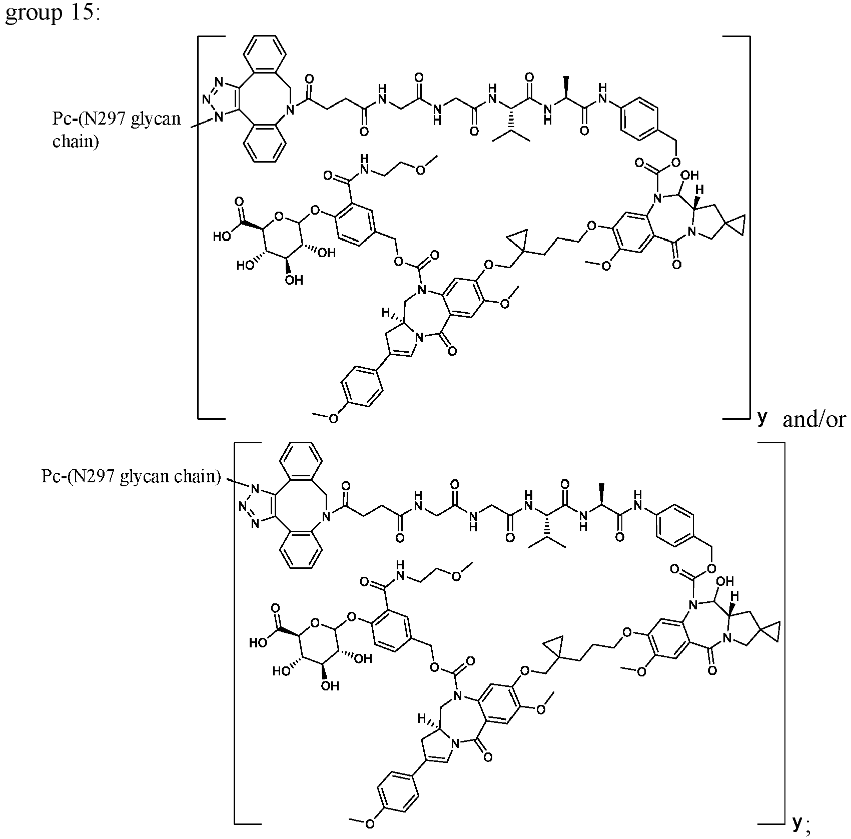 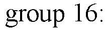 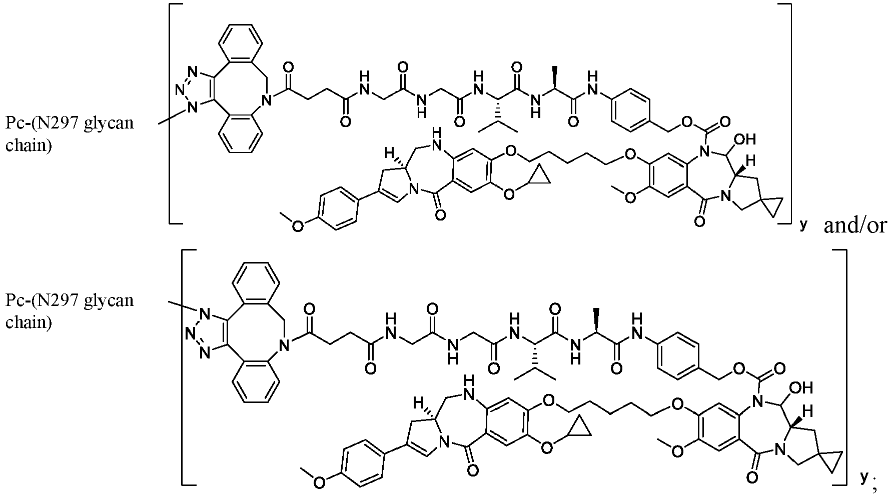 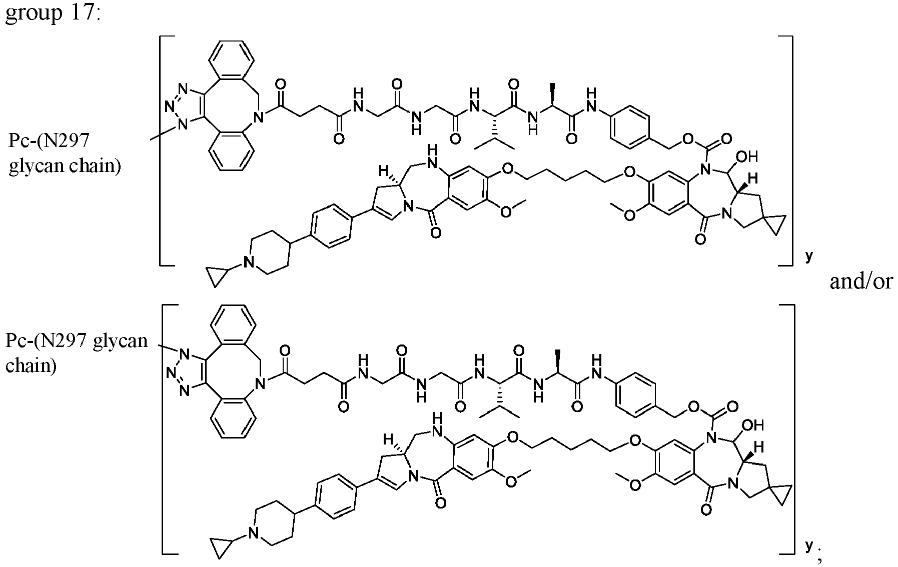 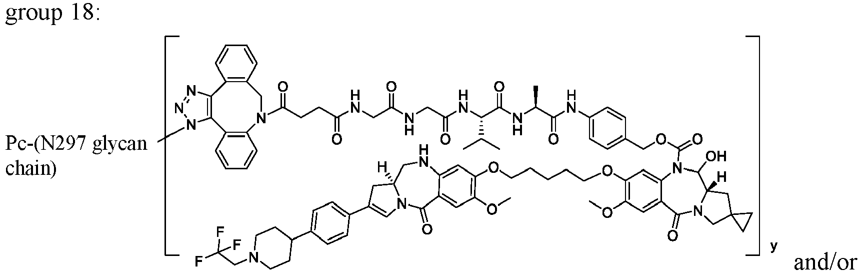 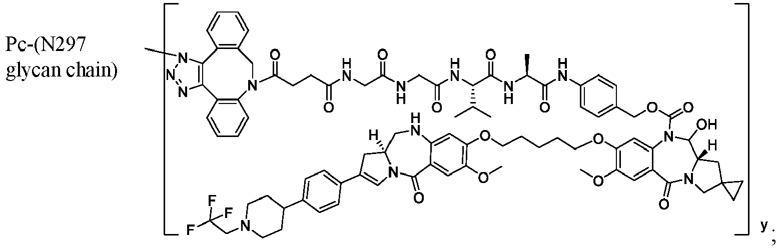
wherein y is selected from the group consisting of 1-4, preferably 1-2;
the N297 glycan chain structure is selected from the group consisting of: 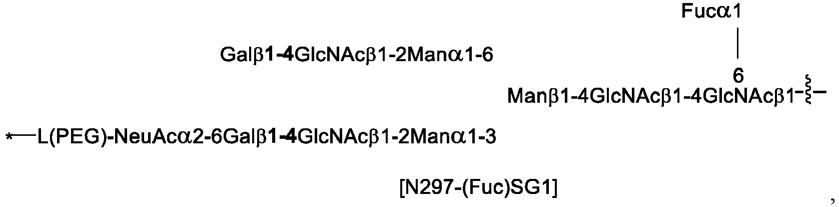 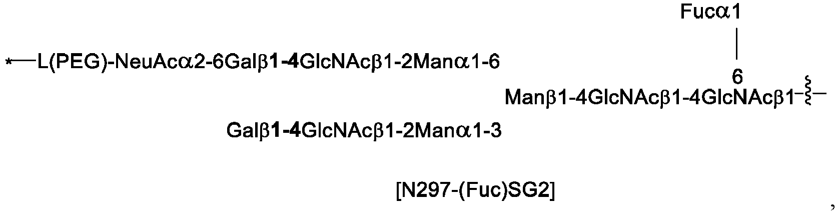 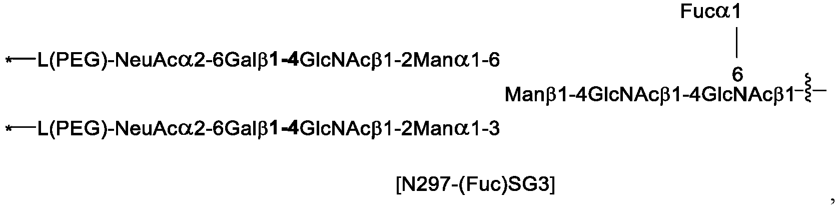 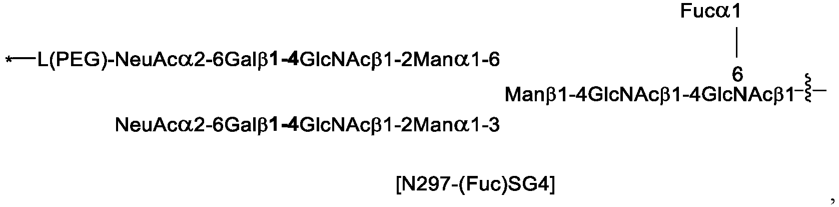 and 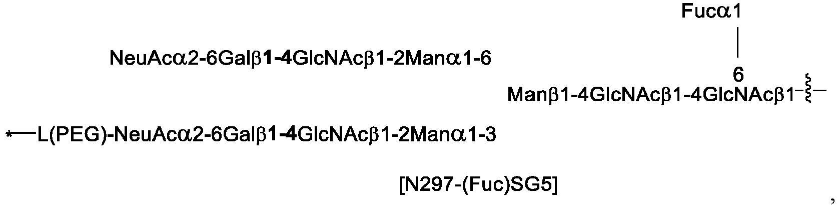
wherein the wavy line 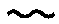 indicates binding to an Asn at position 297 of a heavy chain of Pc;
L(PEG) indicates -(CH₂CH₂O)qCH₂CH₂-NH-, wherein q is selected from the group consisting of 0-20, preferably 1-10; the amino group at the right end indicates amide bonding with the carboxylic acid at locant 2 of the N-acetylneuraminic acid at the non-reducing terminus on the 1-3 chain side or/and 1-6 chain side of a β-Man branched chain of the N297 glycan chain;
the asterisk * indicates binding to the linker L; preferably, the asterisk * indicates binding to the nitrogen atom at locant 1 or 3 on the triazole ring in each structural formula linker;
or the glycan chain is an N297 glycan chain, and the glycan chain structure is selected from: 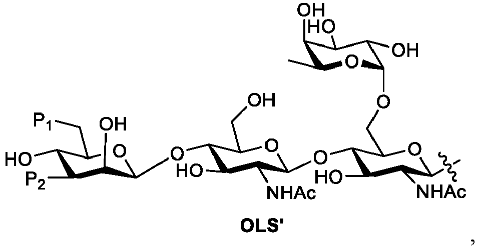
wherein the wavy line 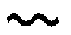 indicates binding to an Asn at position 297 of a heavy chain of the antibody Pc, which may be binding to two position 297 Asn sites or one position 297 Asn site;
P₁ and P₂ are each independently selected from the group consisting of hydroxy, *-(CH₂CH₂O)s¹-, and *-(CH₂CH₂O)s²-(CH₂CH₂CH₂O)s³-(CH₂CH₂O)s⁴-; the *-(CH₂CH₂O)s¹- and *-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy;
the asterisk * indicates binding to the linker L; preferably, the asterisk * indicates binding to the nitrogen atom at locant 1 or 3 on the triazole ring in each structural formula linker;
s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5; s¹, s², s³, and s⁴ are conventionally understood to be integers;
provided that P₁ and P₂ are not simultaneously hydroxy or *-(CH₂CH₂O)s¹-;
preferably, the glycan chain is an N297 glycan chain, and the glycan chain structure is selected from the group consisting of: 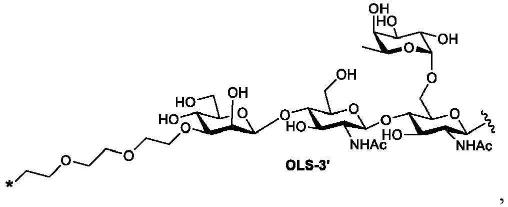 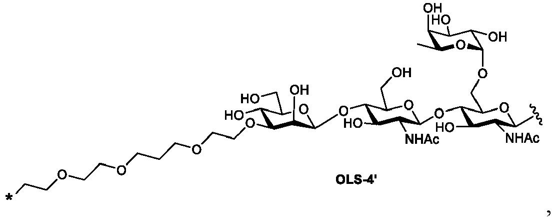 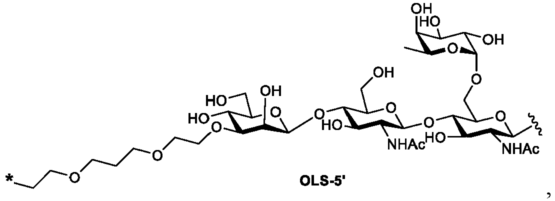 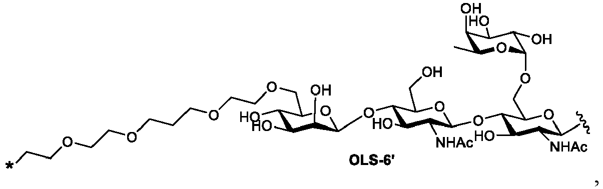 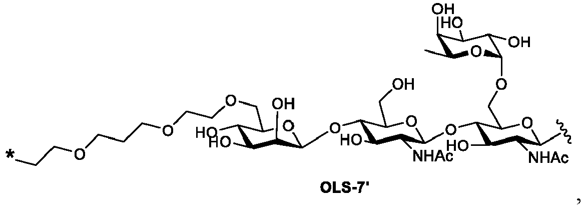 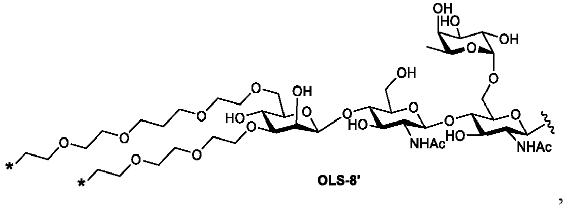 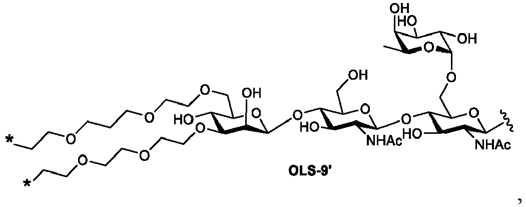 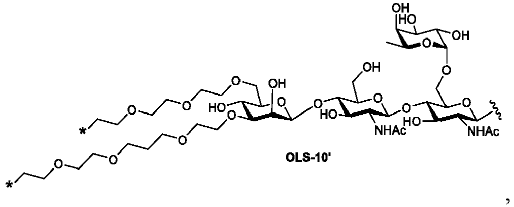 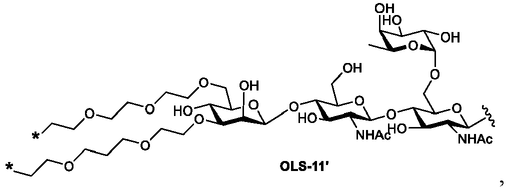 and 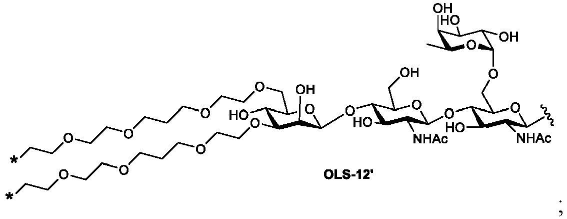
the Pc is as defined above; preferably, the antibody is selected from the group consisting of an anti-HER2 antibody, an anti-HER3 antibody, an anti-B7H3 antibody, an anti-Claudin18.2 antibody, and an anti-CD19 antibody;
more preferably, the anti-HER2 antibody comprises a light chain, the sequence of which is set forth in SEQ ID NO: 1, and a heavy chain, the sequence of which is set forth in SEQ ID NO: 2; the anti-Claudin 18.2 antibody comprises a light chain, the sequence of which is set forth in SEQ ID NO: 3, and a heavy chain, the sequence of which is set forth in SEQ ID NO: 4; the anti-HER3 antibody comprises a light chain, the sequence of which is set forth in SEQ ID NO: 5, and a heavy chain, the sequence of which is set forth in SEQ ID NO: 6.

In some embodiments, the aforementioned antibody-drug conjugate or the pharmaceutically acceptable salt thereof is selected from the group consisting of: 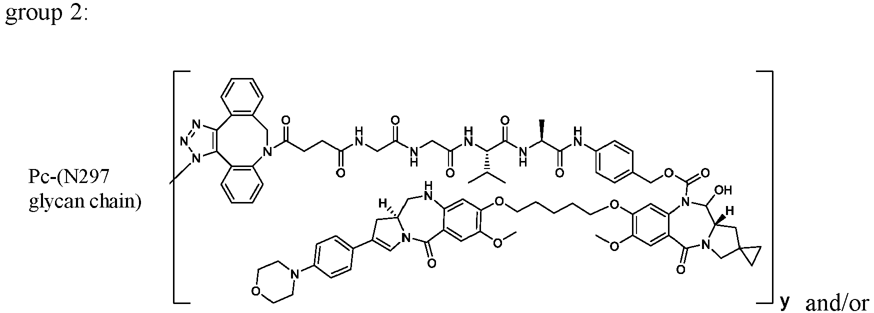 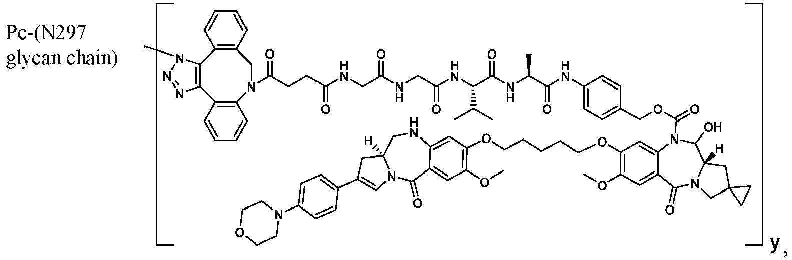 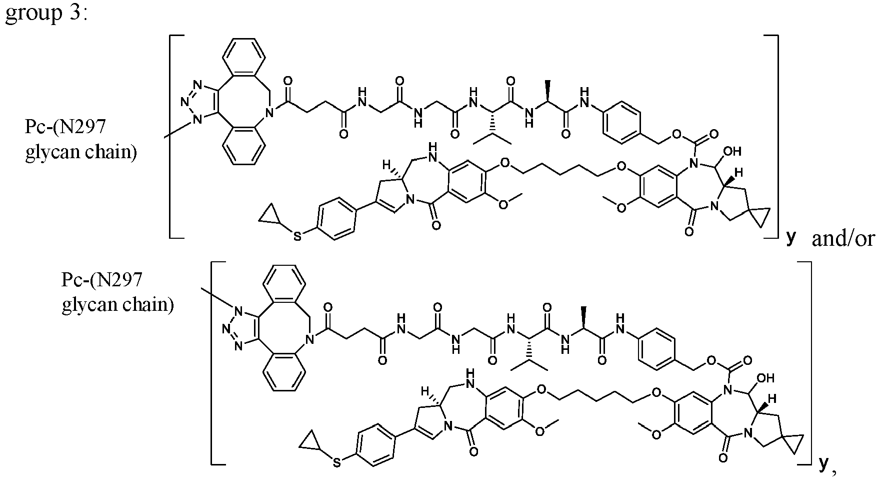 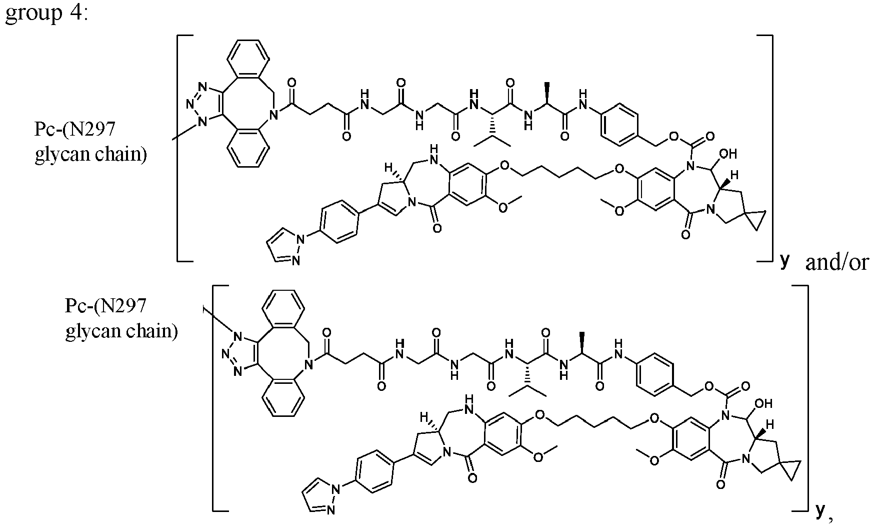 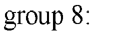 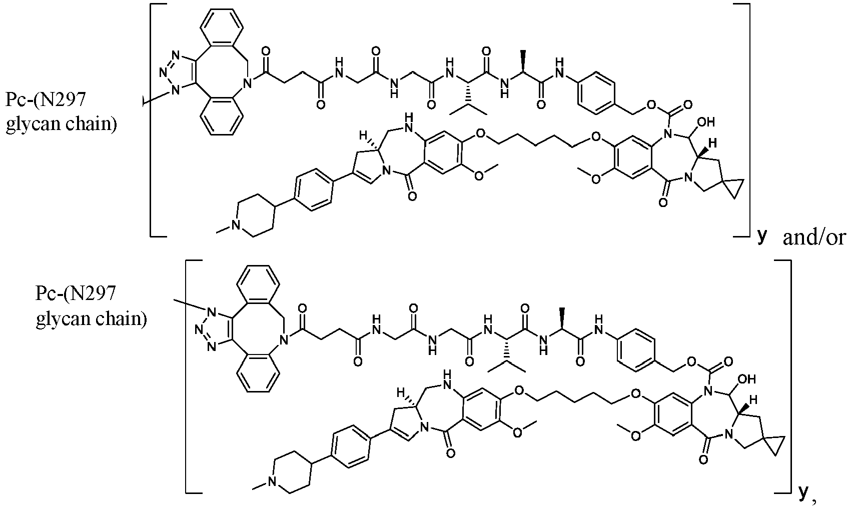 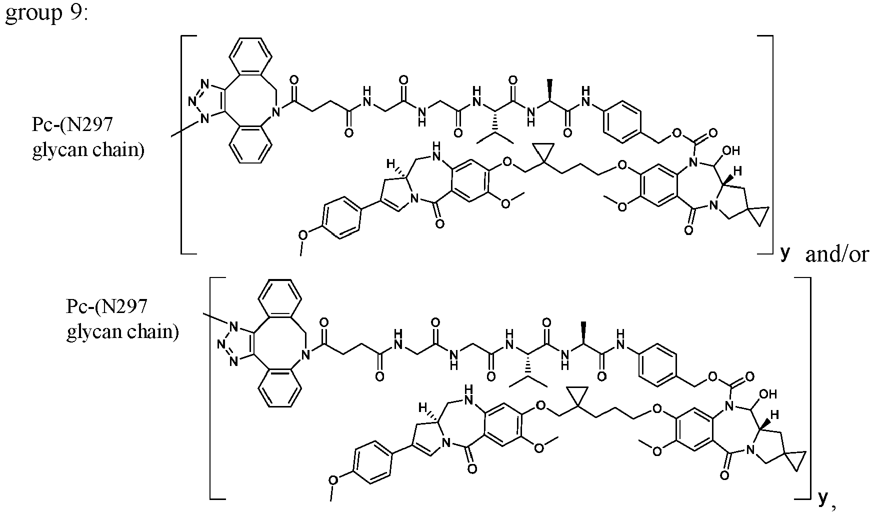 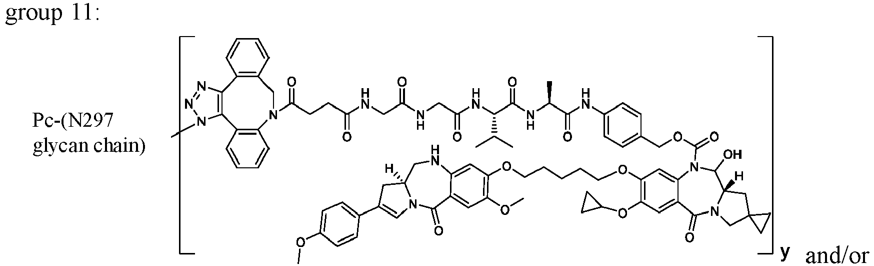 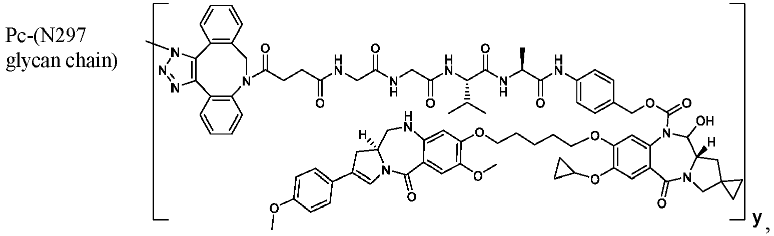
the Pc is as defined above; preferably, the antibody Pc is selected from the group consisting of an anti-HER2 antibody, an anti-HER3 antibody, an anti-Claudin18.2 antibody, and an anti-CD19 antibody;
more preferably, the anti-HER2 antibody comprises a light chain, the sequence of which is set forth in SEQ ID NO: 1, and a heavy chain, the sequence of which is set forth in SEQ ID NO: 2; the anti-Claudin 18.2 antibody comprises a light chain, the sequence of which is set forth in SEQ ID NO: 3, and a heavy chain, the sequence of which is set forth in SEQ ID NO: 4; the anti-HER3 antibody comprises a light chain, the sequence of which is set forth in SEQ ID NO: 5, and a heavy chain, the sequence of which is set forth in SEQ ID NO: 6;
wherein y is 1-2;
the N297 glycan chain structure is selected from the group consisting of: 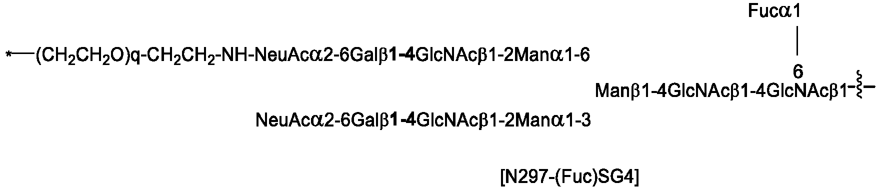 and 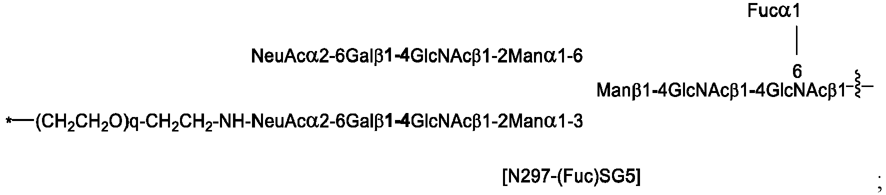
wherein q is selected from the group consisting of 2 and 3;
wherein the wavy line 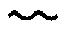 indicates binding to an Asn at position 297 of a heavy chain of Pc, which may be binding to two position 297 Asn sites or one position 297 Asn site;
the amino group at the right end indicates amide bonding with the carboxylic acid at locant 2 of the N-acetylneuraminic acid at the non-reducing terminus on the 1-3 chain side or/and 1-6 chain side of a β-Man branched chain of the N297 glycan chain;
the asterisk * indicates binding to the linker L; specifically, the asterisk * indicates binding to the nitrogen atom at locant 1 or 3 on the triazole ring in each structural formula linker;
or the N297 glycan chain structure is selected from the group consisting of: 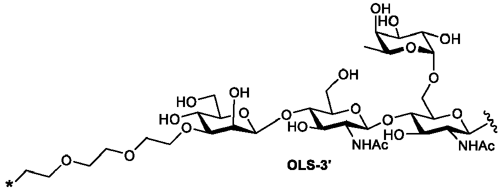 and 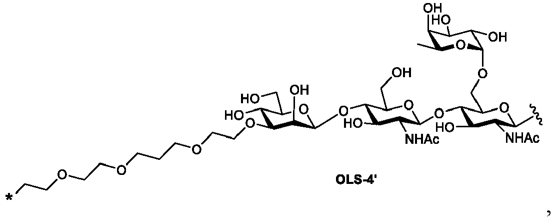 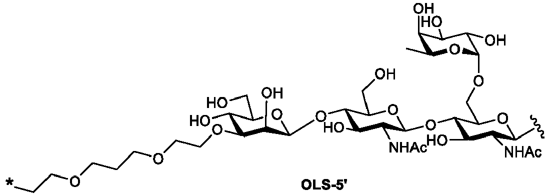
wherein the wavy line 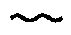 indicates binding to an Asn at position 297 of a heavy chain of Pc, which may be binding to two position 297 Asn sites or one position 297 Asn site; the asterisk * indicates binding to the linker L; specifically, the asterisk * indicates binding to the nitrogen atom at locant 1 or 3 on the triazole ring in each structural formula linker.

In another aspect, the present disclosure provides a glycan chain structure selected from the group consisting of: 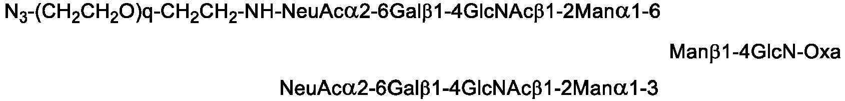 and 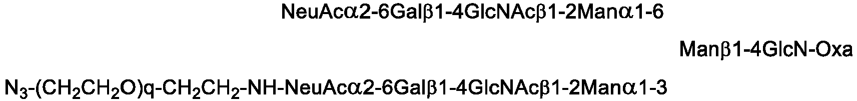 wherein q is selected from the group consisting of 0-20; preferably, q is selected from the group consisting of 1-10; more preferably, q is selected from the group consisting of 2 and 3; wherein Oxa represents an oxazoline ring.

In another aspect, the present disclosure provides a method for preparing the aforementioned glycan chain, comprising the following steps: 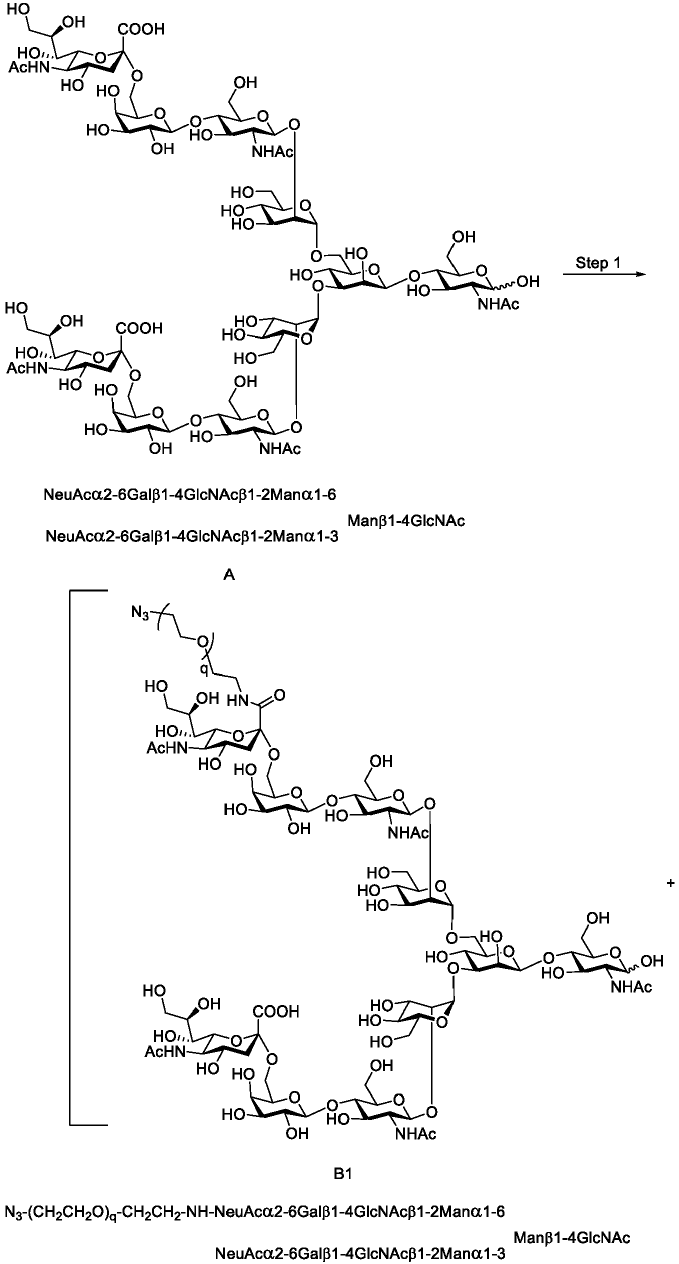 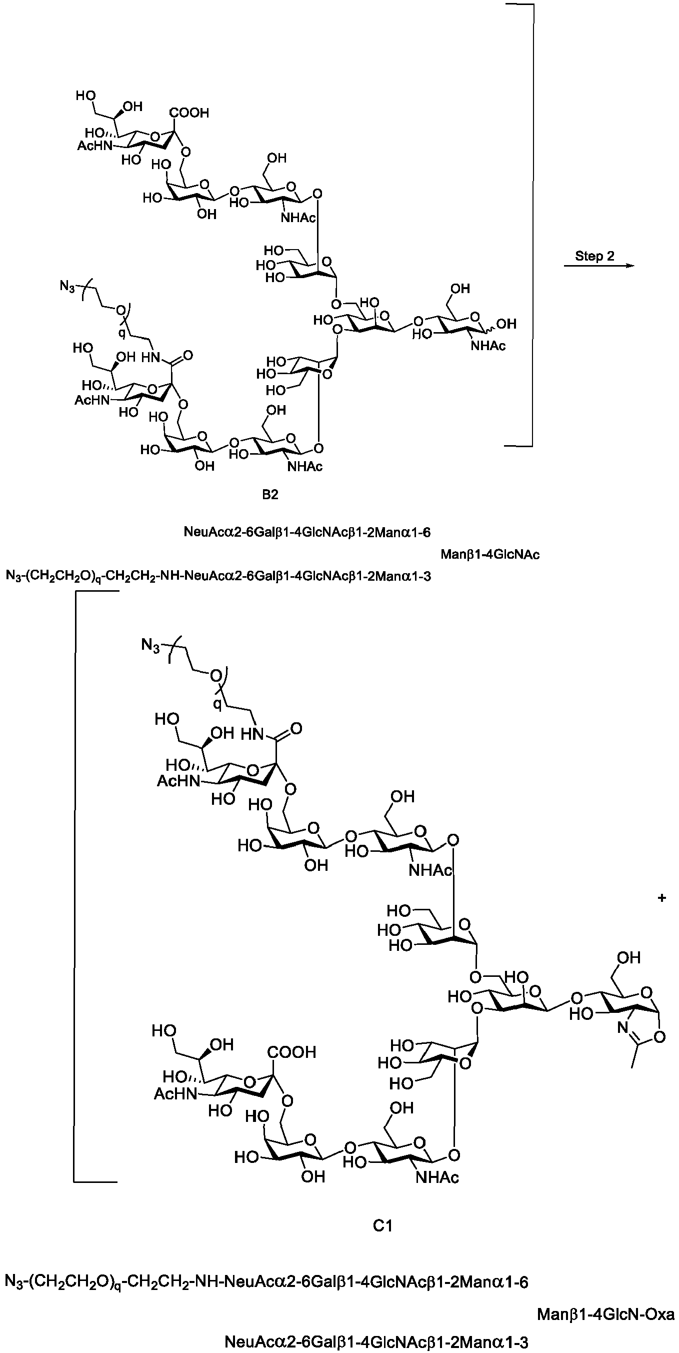 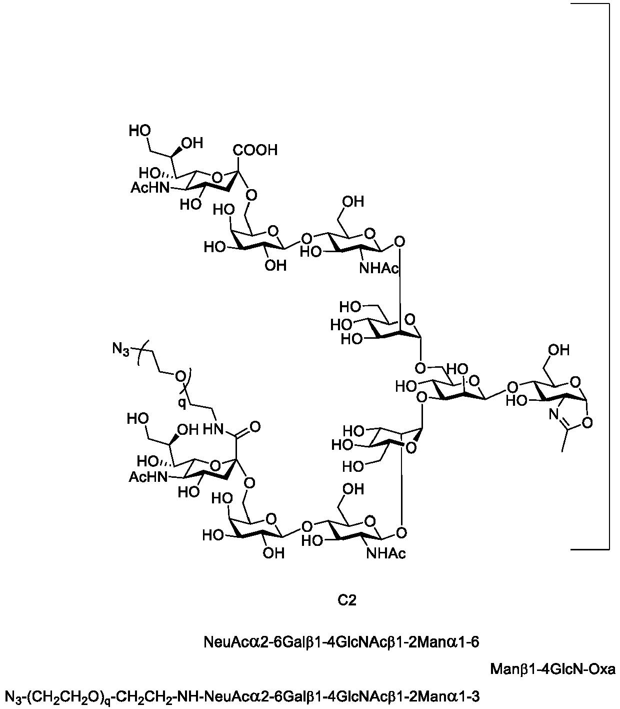 which are simplified as: 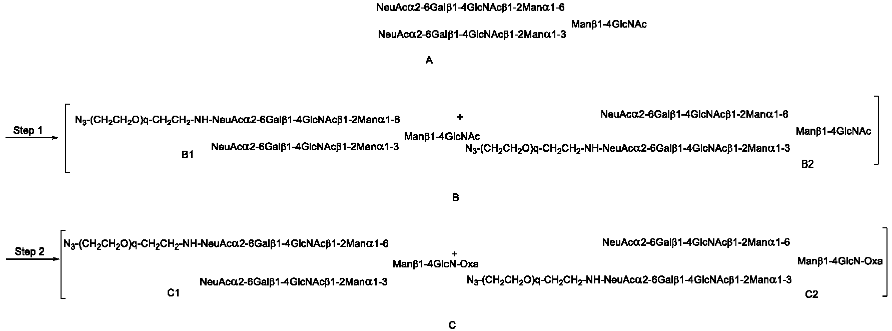
wherein q is selected from the group consisting of 0-20, preferably 1-10;
wherein,
in step 1, compound A reacts with a compound containing an azido functional group to give compound B; the compound B is a mixture of compounds B1 and B2;
in step 2, compound B undergoes a reaction to give compound C; the compound C is a mixture of compounds C1 and C2.

In some embodiments, the aforementioned reaction of step 1 uses a coupling agent, and the coupling agent is selected from the group consisting of HATU and DMTMM; the pH value of the reaction ranges from about 4.5 to about 8.0; the temperature of the reaction is about 20 °C to about 60 °C; the compound containing an azido functional group is selected from the group consisting of 11-azido-3,6,9-trioxaundecan-1-amine and 2-(2-(2-azidoethoxy)ethoxy)ethan-1-amine;
the aforementioned reaction of step 2 is conducted under alkaline conditions in the presence of DMC or CDBMI.

In some embodiments, the coupling agent used in the aforementioned reaction of step 1 is HATU; the pH value of the reaction ranges from about 7.0 to about 8.5; the temperature of the reaction is 20 °C to about 30 °C; the compound containing an azido functional group is 11-azido-3,6,9-trioxaundecan-1-amine;
the aforementioned reaction of step 2 is conducted in the presence of triethylamine and DMC.

In some embodiments, the coupling agent used in the aforementioned reaction of step 1 is DMTMM; the pH value of the reaction ranges from about 4.5 to about 6.5; the temperature of the reaction is 45 °C to about 65 °C; the compound containing an azido functional group is 2-(2-(2-azidoethoxy)ethoxy)ethan-1-amine;
the aforementioned reaction of step 2 is conducted in the presence of a potassium phosphate buffer and CDBMI.

In another aspect, the present disclosure provides a disaccharide compound represented by formula (OLS): 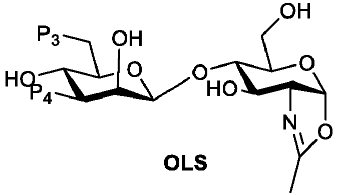
P₃ and P₄ are each independently selected from the group consisting of hydroxy, N₃-(CH₂CH₂O)s¹-, and N₃-(CH₂CH₂O)s²-(CH₂ CH₂CH₂O)s³-(CH₂CH₂O)s⁴-; the N₃-(CH₂CH₂O)s¹- and N₃-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy;
s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5;
provided that P₃ and P₄ are not simultaneously hydroxy or N₃-(CH₂CH₂O)s¹-;
preferably, the disaccharide compound represented by formula (OLS) is selected from the group consisting of: 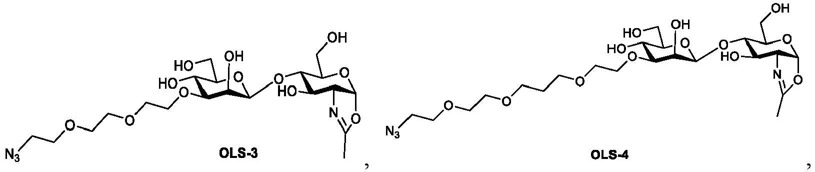 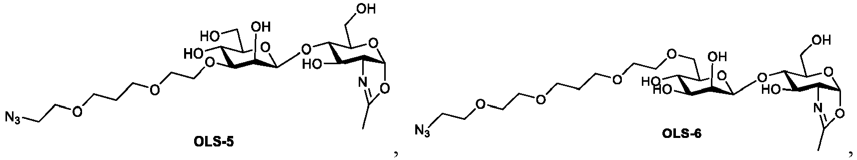 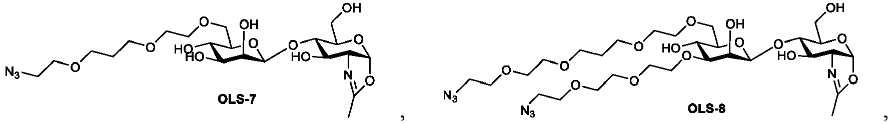 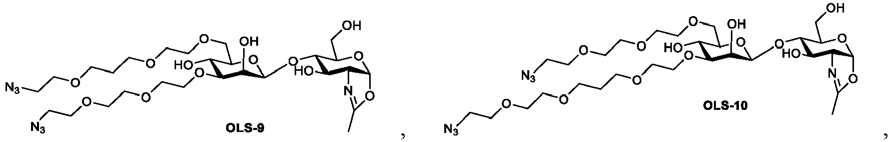 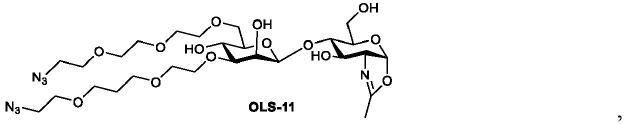 and

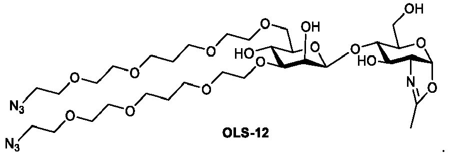

In another aspect, the present disclosure provides a deglycosylated antibody Pc -GlcNAc(Fucα1,6), of which the structure is: 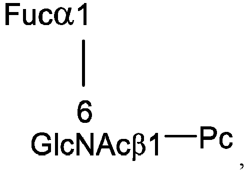 and the molecular formula is 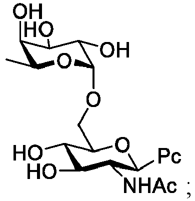 Pc is as defined above.

In another aspect, the present disclosure provides a Pc-(glycan chain N297), the structure of which is: 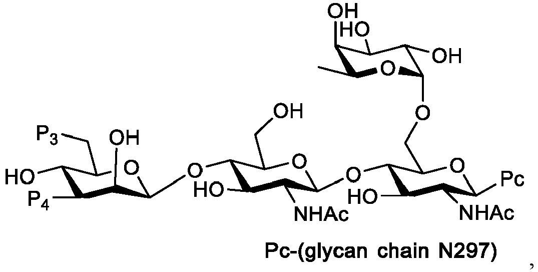
wherein Pc is as defined above;
P₃ and P₄ are each independently selected from the group consisting of hydroxy, N₃-(CH₂CH₂O)s¹-, and N₃-(CH₂CH₂O)s²-(CH₂CH₂CH₂O)s³-(CH₂CH₂O)s⁴-; the N₃-(CH₂CH₂O)s¹- and N₃-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy;
s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5; provided that P₃ and P₄ are not simultaneously hydroxy or N₃-(CH₂CH₂O)s¹-.

In another aspect, the present disclosure provides a method for preparing a glycan chain-remodeled antibody, comprising:

Pc(WT) → Pc -GlcNAc(Fucα1,6) → Pc-(glycan chain N297)

step 1, deglycosylation of an antibody Pc(WT): adding a glycosyltransferase under acidic conditions for a reaction to give a deglycosylated antibody, wherein the deglycosylated antibody is Pc -GlcNAc(Fucα1,6); and
step 2, remodeling of a glycan chain: reacting Pc -GlcNAc(Fucα1,6) with a disaccharide compound represented by formula (OLS) under acidic conditions in the presence of the glycosyltransferase to introduce an azido-containing disaccharide glycan chain to N297 to give a glycan chain-remodeled antibody Pc-(glycan chain N297);
preferably, the acidic conditions are a pH 6.5 PBS buffer; the glycosyltransferase is the enzyme Endo S.

In another aspect, the present disclosure provides a method for preparing an antibody-drug conjugate, comprising:

Pc(WT) → Pc -GlcNAc(Fucα1,6) → Pc-(glycan chain N297) → Pc-(N297 glycan chain)-[L-D]y

step 1, deglycosylation of an antibody Pc(WT): adding a glycosyltransferase under acidic conditions for a reaction to give a deglycosylated antibody, wherein the deglycosylated antibody is Pc -GlcNAc(Fucα1,6); preferably, the acidic conditions are a pH 6.5 PBS buffer; the glycosyltransferase is the enzyme Endo S;
step 2, remodeling of a glycan chain: reacting Pc -GlcNAc(Fucα1,6) with a disaccharide compound represented by formula (OLS) under acidic conditions in the presence of the glycosyltransferase to introduce an azido-containing disaccharide glycan chain to N297 to give a glycan chain-remodeled antibody Pc-(glycan chain N297), wherein preferably, the acidic conditions are a pH 6.5 PBS buffer; the glycosyltransferase is the enzyme Endo S; and
step 3: reacting the glycan chain-remodeled antibody Pc-(glycan chain N297) with a compound represented by formula (DL) under alkaline conditions to give an antibody-drug conjugate Pc-(N297 glycan chain)-[L-D]y, wherein preferably, the alkaline conditions are a pH 7.4 PBS buffer.

In another aspect, the present disclosure provides a compound represented by formula (DL) or a pharmaceutically acceptable salt thereof: 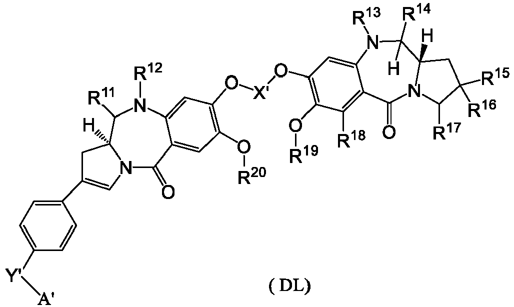 wherein:
R¹¹ is H, oxo, halogen, or alkyl;
R¹² is H, alkyl, or 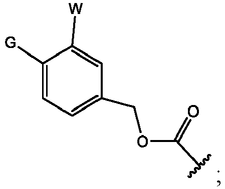 W is H, -C(O)R^{t1}, or -C(O)NH-R^{t1}; G is selected from the group consisting of a glucuronide group and galactoside group; R^{t1} is selected from the group consisting of alkyl and alkoxyalkyl;
R¹³ is L^{a'}-L^{b}-L^{c}-L^{d}-:
   L^{a'} is 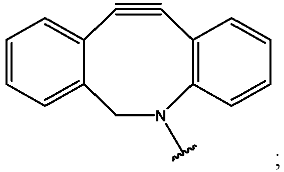
   L^{b} is selected from the group consisting of -C(O)-CH₂CH₂-C(O)-, -C(O)-(CH₂CH₂)₂-C(O)-, -C(O)-CH₂CH₂-C(O)-NH-(CH₂CH₂)₂-C(O)-, -C(O)-CH₂CH₂-C(O)-NH-(CH₂CH₂O)₂-CH₂-C(O)-, -C(O)-CH₂CH₂-NH-C(O)-(CH₂CH₂ O)₄-CH₂CH₂-C(O)-, -CH₂-OC(O)-, and -OC(O)-;
   L^{c} is a peptide residue consisting of 2 to 7 amino acid residues, wherein the amino acid residues are residues formed from phenylalanine, alanine, proline, isoleucine, glycine, valine, lysine, citrulline, serine, glutamic acid, or aspartic acid; the amino acid residues are unsubstituted or are each independently substituted with one or more substituents, wherein each of the substituents is independently selected from the group consisting of halogen, hydroxy, cyano, amino, C₁₋₆ alkyl, C₁₋₆ haloalkyl, C₁₋₆ alkoxy, and C₃₋₇ cycloalkyl;
   L^{d} is -NH-R^{a}-CH₂O-C(O)-, -NH-CH₂O-R^{b}-C(O)-, or a bond;
   R^{a} is phenyl or 5-6 membered heterocyclyl; the phenyl and 5-6 membered heterocyclyl are unsubstituted or are each independently substituted with one or more substituents, wherein each of the substituents is independently selected from the group consisting of halogen, oxo, hydroxy, cyano, amino, C₁₋₆ alkyl, C₁₋₆ haloalkyl, and C₁₋₆ alkoxy;
   R^{b} is C₁₋₆ alkyl or C₃₋₇ cycloalkyl; the C₁₋₆ alkyl and C₃₋₇ cycloalkyl are unsubstituted or are each independently substituted with one or more substituents, wherein each of the substituents is independently selected from the group consisting of halogen, oxo, hydroxy, cyano, amino, C₁₋₆ alkyl, C₃₋₇ cycloalkyl, 5-6 membered heterocyclyl, C₁₋₆ haloalkyl, and C₁₋₆ alkoxy;
   R¹⁴ is H, hydroxy, or alkoxy;
   or R¹³ and R¹⁴, together with the atoms to which they are attached, form imine bonds (N=C);
   R¹⁵ is H or alkyl;
   R¹⁶ is H or alkyl;
   R¹⁷ is H, halogen, or alkyl;
   or R¹⁵ and R¹⁶, together with the atom to which they are attached, form a ring, or R¹⁶ and R¹⁷, together with the atoms to which they are attached, form a ring;
   R¹⁸ is H or alkyl;
   R¹⁹ is alkyl or cycloalkyl; or R¹⁸ and R¹⁹, together with the atoms to which they are attached, form a ring;
   R²⁰ is alkyl or cycloalkyl;
   X' is alkylene or -(CH₂)_{m'}-X'₁-(CH₂)_{n'}-;
   X'₁ is a nitrogen atom, an oxygen atom, a sulfur atom, cycloalkyl, heterocyclyl, heteroaryl, or aryl; the cycloalkyl, heterocyclyl, heteroaryl, or aryl is optionally substituted with one or more substituents, wherein each of the substituents is independently selected from the group consisting of amino, hydroxy, hydroxyalkyl, alkyl, and alkoxy;
   Y' is a bond, a nitrogen atom, an oxygen atom, or a sulfur atom;
   A' is alkyl, cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R²¹, cycloalkyl, cycloalkylalkyl, haloalkyl, aryl, and heterocyclyl;
   R²¹ is H, alkyl, and hydroxyalkyl;
   m' is 1, 2, or 3; n' is 1, 2, or 3;
   provided that when X' is alkylene, 1) R¹¹ is oxo, or 2) R¹⁴ is H, or 3) R¹⁶ and R¹⁷, together with the atoms to which they are attached, form a ring, or 4) R¹⁸ and R¹⁹, together with the atoms to which they are attached, form a ring, or 5) R¹⁹ and/or R²⁰ are cycloalkyl; or 6) A' is not alkyl.

In some embodiments, in the aforementioned compound or the pharmaceutically acceptable salt thereof, R¹³ and R¹⁴, together with the atoms to which they are attached, form imine bonds (N=C).

In some embodiments, in the aforementioned compound or the pharmaceutically acceptable salt thereof,
R¹³ is L^{a'}-L^{b}-L^{c}-L^{d}-:
L^{a'} is 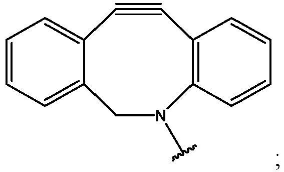
L^{b} is selected from the group consisting of -C(O)-CH₂CH₂-C(O)-, -C(O)-(CH₂CH₂)₂-C(O)-, -C(O)-CH₂CH₂-C(O)-NH-(CH₂CH₂)₂-C(O)-, -C(O)-CH₂CH₂-C(O)-NH-(CH₂CH₂O)₂-CH₂-C(O)-, -C(O)-CH₂CH₂-NH-C(O)-(CH₂CH₂O)₄-CH₂CH₂-C(O)-, -CH₂-OC(O)-, and -OC(O)-;
L^{c} is a peptide residue consisting of 2 to 7 amino acid residues, wherein the amino acid residues are residues formed from phenylalanine, alanine, proline, isoleucine, glycine, valine, lysine, citrulline, serine, glutamic acid, or aspartic acid; the amino acid residues are unsubstituted or are each independently substituted with one or more substituents, wherein each of the substituents is independently selected from the group consisting of halogen, hydroxy, cyano, amino, C₁₋₆ alkyl, C₁₋₆ haloalkyl, C₁₋₆ alkoxy, and C₃₋₇ cycloalkyl;
L^{d} is -NH-R^{a}-CH₂O-C(O)-, -NH-CH₂O-R^{b}-C(O)-, or a bond;
R^{a} is phenyl or 5-6 membered heterocyclyl; the phenyl and 5-6 membered heterocyclyl are unsubstituted or are each independently substituted with one or more substituents, wherein each of the substituents is independently selected from the group consisting of halogen, oxo, hydroxy, cyano, amino, C₁₋₆ alkyl, C₁₋₆ haloalkyl, and C₁₋₆ alkoxy;
R^{b} is C₁₋₆ alkyl or C₃₋₇ cycloalkyl; the C₁₋₆ alkyl and C₃₋₇ cycloalkyl are unsubstituted or are each independently substituted with one or more substituents, wherein each of the substituents is independently selected from the group consisting of halogen, oxo, hydroxy, cyano, amino, C₁₋₆ alkyl, C₃₋₇ cycloalkyl, 5-6 membered heterocyclyl, C₁₋₆ haloalkyl, and C₁₋₆ alkoxy.

In some embodiments, in the aforementioned compound or the pharmaceutically acceptable salt thereof, the R¹⁴ is H or hydroxy; preferably, R¹⁴ is hydroxy.

In some embodiments, in the aforementioned compound or the pharmaceutically acceptable salt thereof, the R¹¹ is H or oxo; preferably, R¹¹ is H.

In some embodiments, in the aforementioned compound or the pharmaceutically acceptable salt thereof, the R¹² is H, oxo, or C₁₋₆ alkyl; preferably, R¹² is H.

In some embodiments, in the aforementioned compound or the pharmaceutically acceptable salt thereof, the R¹² is represented by the following structure: 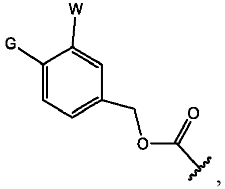 wherein W is H or -C(O)NH-R^{t1}, and G is a glucuronide group; R^{t1} is C₁₋₆ alkyl or C₁₋₆ alkoxy C₁₋₆ alkyl.

In some embodiments, in the aforementioned compound or the pharmaceutically acceptable salt thereof,
R¹¹ is H or alkyl; preferably, R¹¹ is H or C₁₋₆ alkyl;
R¹⁵ and R¹⁶, together with the atom to which they are attached, form a 3-6 membered carbocycle;
R¹⁷ is H; R¹⁸ is H;
R¹⁹ is alkyl; preferably, R¹⁹ is C₁₋₆ alkyl;
R²⁰ is alkyl; preferably, R²⁰ is C₁₋₆ alkyl;
X' is alkylene; preferably, X' is C₁₋₆ alkylene; more preferably, X' is pentylene;
Y' is selected from the group consisting of a bond, a nitrogen atom, an oxygen atom, and a sulfur atom; preferably, Y' is a bond or a sulfur atom; more preferably, Y' is a bond;
A' is cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R²¹, cycloalkyl, cycloalkylalkyl, haloalkyl, aryl, and heterocyclyl; preferably, A' is selected from the group consisting of C₃₋₆ cycloalkyl, 5-to 6-membered heterocyclyl, phenyl, and 5- to 6-membered heteroaryl; the C₃₋₆ cycloalkyl, 5- to 6-membered heterocyclyl, phenyl, or 5- to 6-membered heteroaryl is optionally substituted with one or more substituents, wherein each of the substituents is independently selected from the group consisting of C₁₋₆ hydroxyalkyl, C₁₋₆ alkyl, C₃₋₆ cycloalkyl C₁₋₆ alkyl, C₁₋₆ haloalkyl, and -C(O)R²¹;
R²¹ is selected from the group consisting of H, alkyl, and hydroxyalkyl; preferably, R²¹ is C₁₋₆ alkyl or C₁₋₆ hydroxyalkyl.

In some embodiments, in the aforementioned compound or the pharmaceutically acceptable salt thereof,
R¹¹ is H or alkyl; preferably, R¹¹ is H or C₁₋₆ alkyl;
R¹⁵ and R¹⁶, together with the atom to which they are attached, form a 3-6 membered carbocycle;
R¹⁷ is H; R¹⁸ is H;
R¹⁹ is alkyl; preferably, R¹⁹ is C₁₋₆ alkyl;
R²⁰ is alkyl; preferably, R²⁰ is C₁₋₆ alkyl;
X' is -(CH₂)_{m'}-X'₁-(CH₂)_{n'}-;
X'₁ is a nitrogen atom, an oxygen atom, a sulfur atom, cycloalkyl, heteroaryl, or aryl; the cycloalkyl, heteroaryl, or aryl is optionally substituted with one or more substituents, wherein each of the substituents is independently selected from the group consisting of amino, hydroxy, hydroxyalkyl, alkyl, and alkoxy; preferably, X'₁ is an oxygen atom, C₃₋₆ cycloalkyl, pyridinyl, or phenyl; the C₃₋₆ cycloalkyl, pyridinyl, or phenyl is optionally substituted with one or more amino or C₁₋₆ alkyl;
Y' is an oxygen atom;
A' is alkyl; preferably, A' is C₁₋₆ alkyl;
m' is selected from the group consisting of 1, 2, and 3; n' is selected from the group consisting of 1, 2, and 3.

In some embodiments, in the aforementioned compound or the pharmaceutically acceptable salt thereof,
R¹¹ is H or alkyl; preferably, R¹¹ is H or C₁₋₆ alkyl;
R¹⁵ and R¹⁶, together with the atom to which they are attached, form a 3-6 membered carbocycle;
R¹⁷ is H;
R¹⁸ is H or alkyl; preferably, R¹⁸ is H or C₁₋₆ alkyl;
R¹⁹ is alkyl or cycloalkyl; preferably, R¹⁹ is C₁₋₆ alkyl or C₁₋₆ hydroxyalkyl;
or R¹⁸ and R¹⁹, together with the atoms to which they are attached, form a ring; preferably, R¹⁸ and R¹⁹, together with the atoms to which they are attached, form a 3-6-membered carbocycle;
R²⁰ is alkyl or cycloalkyl; preferably, R²⁰ is C₁₋₆ alkyl or C₁₋₆ hydroxyalkyl;
X' is alkylene; preferably, X' is C₁₋₆ alkylene; more preferably, X' is pentylene;
Y' is an oxygen atom;
A' is alkyl; preferably, A' is C₁₋₆ alkyl;
provided that R¹⁹ and R²⁰ are not simultaneously alkyl.

In some embodiments, in the aforementioned compound or the pharmaceutically acceptable salt thereof,
R¹¹ is H or C₁₋₆ alkyl; preferably, R¹¹ is H;
R¹⁵ is H;
R¹⁶ and R¹⁷, together with the atoms to which they are attached, form a 3-6 membered carbocycle;
R¹⁸ is H;
R¹⁹ is alkyl; preferably, R¹⁹ is C₁₋₆ alkyl;
R²⁰ is alkyl; preferably, R²⁰ is C₁₋₆ alkyl;
X' is alkylene; preferably, X' is C₁₋₆ alkylene; more preferably, X' is pentylene;
Y' is an oxygen atom;
A' is alkyl; preferably, A' is C₁₋₆ alkyl.

Typical compounds disclosed in the present disclosure include, but are not limited to:

| E xa m pl e N o. | Structures and names of compounds |
|---|---|
| 2-1 | 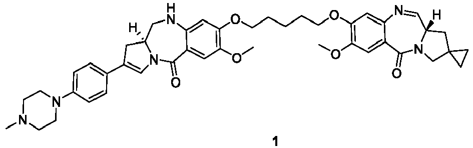 |
| | (*S*)-7-Methoxy-8-((5-(((*S*)-7-methoxy-2-(4-(4-methylpiperazin-1-yl)phenyl)-5-oxo-5,10,11,11*a*-tetrahydro-1*H*-benzo[*e*]pyrrolo[1,2-*a*][1,4]diazepin-8-yl)oxy)pentyl)ox y)-1,11*a*-dihydro-3*H*,5*H*-spiro[benzo[*e*]pyrrolo[1,2-*a*][1,4]diazepine-2,1'-cyclopro pan]-5-one **1** |
| 2-2 | 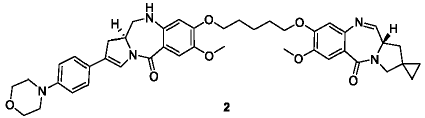 |
| | (*S*)-7-Methoxy-8-((5-(((*S*)-7-methoxy-2-(4-morpholinylphenyl)-5-oxo-5,10,11,11*a-*tetrahydro-1*H*-benzo[*e*]pyrrolo[1,2-*a*][1,4]diazepin-8-yl)oxy)pentyl)oxy)-1,11*a*-dih ydro-3*H*,5*H*-spiro[benzo[*e*]pyrrolo[1,2-*a*][1,4]diazepine-2,1'-cyclopropan]-5-one **2** |
| 2-3 | 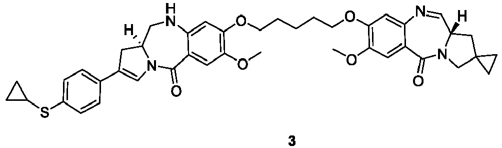 |
| | (*S*)-8-((5-(((*S*)-2-(4-(Cyclopropylthio)phenyl-7-methoxy-5-oxo-5,10,11,1*a*-tetrahy dro-1*H*-benzo[*e*]pyrrolo[1,2-*a*][1,4]diazepin-8-yl)oxy)pentyl)oxy)-7-methoxy-1,11 *a*-dihydro-3*H*,5*H*-spiro[benzo[*e*]pyrrolo[1,2-*a*][1,4]diazepine-2,1'-cyclopropan]-5-one **3** |
| 2-4 | 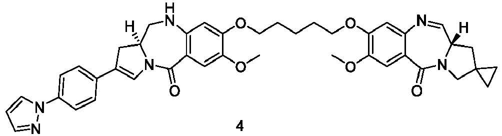 |
| | (*S*)-8-((5-(((S*)*-2-(4-(1*H*-Pyrazol-1-yl)phenyl)-7-methoxy-5-oxo-5,10,11,11*a*-tetrah ydro-1*H*-benzo[*e*]pyrrolo[1,2-*a*][1,4]diazepin-8-yl)oxy)pentyl)oxy)-7-methoxy-1,1 1*a*-dihydro-3*H*,5*H*-spiro[benzo[*e*]pyrrolo[1,2-*a*][1,4]diazepine-2,1'-cyclopropan]-5 -one **4** |
| 2-5 | 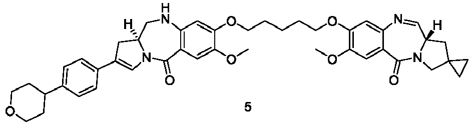 |
| | (*S*)-7-Methoxy-8-((5-(((*S*)-7-methoxy-5-oxo-2-(4-(tetrahydro-2*H*-pyran-4-yl)pheny l)-5,10,11,11*a*-tetrahydro-1*H*-benzo[*e*]pyrrolo[1,2-*a*][1,4]diazepin-8-yl)oxy)pentyl) oxy)-1,11*a*-dihydro-3*H*,5*H*-spiro[benzo[*e*]pyrrolo[1,2-*a*][1,4]diazepine-2,1'-cyclop ropan]-5-one **5** |
| 2-6 | 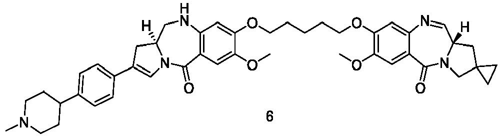 |
| | (*S*)-7-Methoxy-8-((5-(((*S*)-7-methoxy-2-(4-(1-methylpiperidin-4-yl)phenyl)-5-oxo-5,10,11,11*a*-tetrahydro-1*H*-benzo[*e*]pyrrolo[1,2-*a*][1,4]diazepin-8-yl)oxy)pentyl)ox y)-1,11*a*-dihydro-3*H*,5*H*-spiro[benzo[*e*]pyrrolo[1,2-*a*][1,4]diazepine-2,1'-cyclopro pan]-5-one **6** |
| 2-7 | 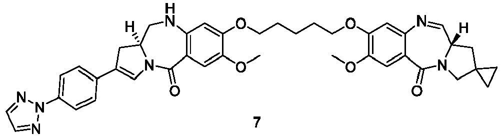 |
| | (*S*)-8-((5-(((*S*)-2-(4-(2*H*-1,2,3-Triazol-2-yl)phenyl)-7-methoxy-5-oxo-5,10,11,11*a*-t etrahydro-1*H*-benzo[*e*]pyrrolo[1,2-*a*][1,4]diazepin-8-yl)oxy)pentyl)oxy)-7-methox y-1,11*a*-dihydro-3*H*,5*H*-spiro[benzo[*e*]pyrrolo[1,2-*a*][1,4]diazepine-2,1'-cycloprop an]-5-one 7 |
| 2-8 | 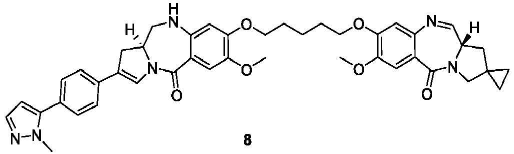 |
| | (*S*)-7-Methoxy-8-((5-(((*S*)-7-methoxy-2-(4-(1-methyl-1*H*-pyrazol-5-yl)phenyl)-5-o xo-5,10,11,11*a*-tetrahydro-1*H*-benzo[*e*]pyrrolo[1,2-*a*][1,4]diazepin-8-yl)oxy)pentyl )oxy)-1,11a-dihydro-3*H*,5*H*-spiro[benzo[*e*]pyrrolo[1,2-*a*][1,4]diazepine-2,1'-cyclo propan]-5-one **8** |
| 2-9 | 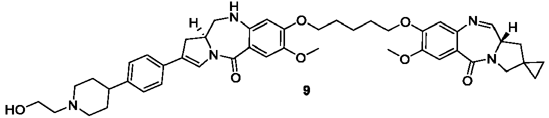 |
| | (*S*)-8-((5-(((*S*)-2-(4-(1-(2-Hydroxyethyl)piperidin-4-yl)phenyl)-7-methoxy-5-oxo-5 ,10,11,11*a*-tetrahydro-1*H*-benzo[*e*]pyrrolo[1,2-*a*][1,4]diazepin-8-yl)oxy)pentyl)oxy )-7-methoxy-1,11*a*-dihydro-3*H*,5*H*-spiro[benzo[*e*]pyrrolo[1,2-*a*][1,4]diazepine-2,1' -cyclopropan]-5-one **9** |
| 2-10 | 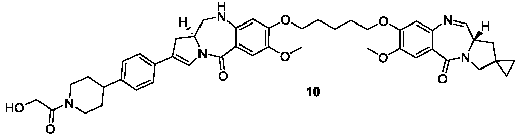 |
| | (*S*)-8-((5-(((*S*)-2-(4-(1-(2-Hydroxyacetyl)piperidin-4-yl)phenyl)-7-methoxy-5-oxo-5,10,11,11*a*-tetrahydro-1*H*-benzo[*e*]pyrrolo[1,2-*a*][1,4]diazepin-8-yl)oxy)pentyl)ox y)-7-methoxy-1,11*a*-dihydro-3*H*,5*H*-spiro[benzo[*e*]pyrrolo[1,2-*a*][1,4]diazepine-2, 1'-cyclopropan]-5-one **10** |
| 2-11 | 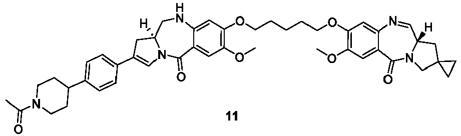 |
| | (*S*)-8-((5-(((*S*)-2-(4-(1-Acetylpiperidin-4-yl)phenyl)-7-methoxy-5-oxo-5,10,11,11*a-*tetrahydro-1*H*-benzo[*e*]pyrrolo[1,2-*a*][1,4]diazepin-8-yl)oxy)pentyl)oxy)-7-methox y-1,11*a*-dihydro-3*H*,5*H*-spiro[benzo[*e*]pyrrolo[1,2-*a*][1,4]diazepine-2,1'-cycloprop an]-5-one **11** |
| 2-12 | 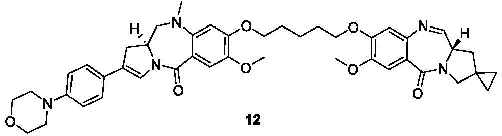 |
| | (*S*)-7-Methoxy-8-((5-(((*S*)-7-methoxy-10-methyl-2-(4-morpholinylphenyl)-5-oxo-5 ,10,11,11*a*-tetrahydro-1*H*-benzo[*e*]pyrrolo[1,2-*a*][1,4]diazepin-8-yl)oxy)pentyl)oxy )-1,11*a*-dihydro-3*H*,5*H*-spiro[benzo[*e*]pyrrolo[1,2-*a*][1,4]diazepine-2,1'-cycloprop an]-5-one **12** |
| 2-13 | 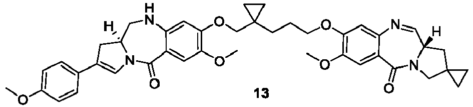 |
| | (*S*)-7-Methoxy-8-(3-(1-((((*S*)-7-methoxy-2-(4-methoxyphenyl)-5-oxo-5,10,11,11*a*-t etrahydro-1*H*-benzo[*e*]pyrrolo[1,2-*a*][1,4]diazepin-8-yl)oxy)methyl)cyclopropyl)pr opoxy)-1,11*a*-dihydro-3*H*,5*H*-spiro[benzo[*e*]pyrrolo[1,2-*a*][1,4]diazepine-2,1'-cycl opropan]-5-one **13** |
| 2-14 | 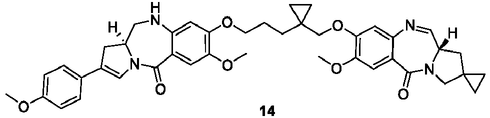 |
| | (*S*)-7-Methoxy-8-((1-(3-(((*S*)-7-methoxy-2-(4-methoxyphenyl)-5-oxo-5,10,11,11*a*-t etrahydro-1*H*-benzo[*e*]pyrrolo[1,2-*a*][1,4]diazepin-8-yl)oxy)propyl)cyclopropyl)m ethoxy)-1,11*a*-dihydro-3*H*,5*H*-spiro[benzo[*e*]pyrrolo[1,2-*a*][1,4]diazepine-2,1'-cycl opropan]-5-one **14** |
| 2-15 | 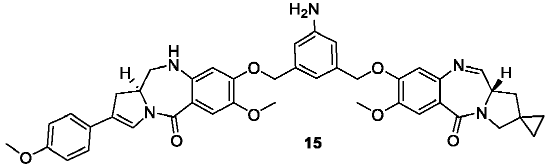 |
| | (*S*)-8-((3-Amino-5-((((*S*)-7-methoxy-2-(4-methoxyphenyl)-5-oxo-5,10,11,11*a*-tetra hydro-1*H*-benzo[*e*]pyrrolo[1,2-*a*][1,4]diazepin-8-yl)oxy)methyl)benzyl)oxy)-7-met hoxy-1,11*a*-dihydro-3*H*,5*H*-spiro[benzo[*e*]pyrrolo[1,2-*a*][1,4]diazepine-2,1'-cyclop ropan]-5-one **15** |
| 2-16 | 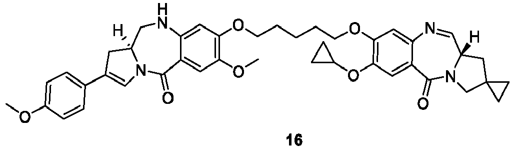 |
| | (*S*)-7-Cyclopropoxy-8-((5-(((*S*)-7-methoxy-2-(4-methoxyphenyl)-5-oxo-5,10,11,11 *a*-tetrahydro-1*H*-benzo[*e*]pyrrolo[1,2-*a*][1,4]diazepin-8-yl)oxy)pentyl)oxy)-1,11*a-*dihydro-3*H*,5*H*-spiro[benzo[*e*]pyrrolo[1,2-*a*][1,4]diazepine-2,1'-cyclopropan]-5-on e **16** |
| 2-17 | 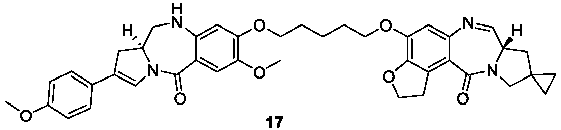 |
| | (*S*)-4-((5-(((*S*)-7-Methoxy-2-(4-methoxyphenyl)-5-oxo-5,10,11,1*a*-tetrahydro-1*H-*benzo[*e*]pyrrolo[1,2-*a*][1,4]diazepin-8-yl)oxy)pentyl)oxy)-1,2,7*a*,8-tetrahydro-10*H* ,12*H*-spiro[benzofuro[5,4-*e*]pyrrolo[1,2-*a*][1,4]diazepine-9,1'-cyclopropan]-12-one **17** |
| 2-18 | 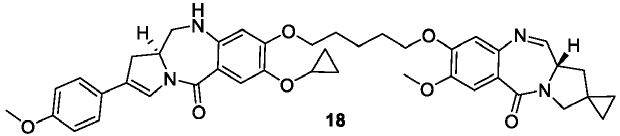 |
| | (*S*)-8-((5-(((*S*)-7-Cyclopropoxy-2-(4-methoxyphenyl)-5-oxo-5,10,11,11*a*-tetrahydr o-1*H*-benzo[*e*]pyrrolo[1,2-α][1,4]diazepin-8-yl)oxy)pentyl)oxy)-7-methoxy-1,11*a-*dihydro-3*H*,5*H*-spiro[benzo[*e*]pyrrolo[1,2-*a*][1,4]diazepine-2,1'-cyclopropan]-5-on |
| | e **18** |
| 2-19 | 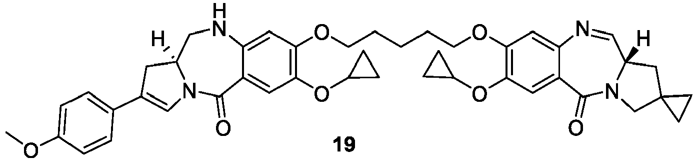 |
| | (*S*)-7-Cyclopropoxy-8-((5-(((*S*)-7-cyclopropoxy-2-(4-methoxyphenyl)-5-oxo-5,10, 11,1*a*-tetrahydro-1*H*-benzo[*e*]pyrrolo[1,2-*a*][1,4]diazepin-8-yl)oxy)pentyl)oxy)-1, 11*a*-dihydro-3*H*,5*H*-spiro[benzo[*e*]pyrrolo[1,2-*a*][1,4]diazepine-2,1'-cyclopropan]-5-one **19** |
| 2-20 | 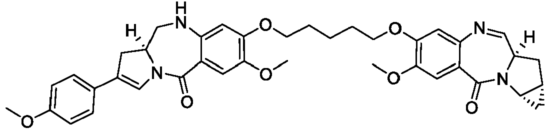 |
| | (1*aS*,9*aR*,10*aS*)-5-Methoxy-6-((5-(((*S*)-7-methoxy-2-(4-methoxyphenyl)-5-oxo-5,1 0,11,11*a*-tetrahydro-1*H*-benzo[*e*]pyrrolo[1,2-*a*][1,4]diazepin-8-yl)oxy)pentyl)oxy)-1*a*,9*a*,10,10*a*-tetrahydrobenzo[*e*]cyclopropa[4,5]pyrrolo[1,2-*a*][1,4]diazepin-3(1*H*) -one |
| | 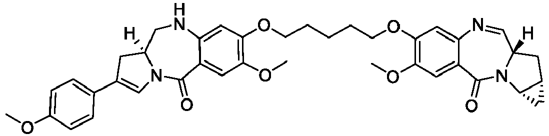 |
| | (1*aS*,9*aS*,10*aS*)-5-Methoxy-6-((5-(((*S*)-7-methoxy-2-(4-methoxyphenyl)-5-oxo-5,1 0,11,11*a*-tetrahydro-1*H*-benzo[*e*]pyrrolo[1,2-*a*][1,4]diazepin-8-yl)oxy)pentyl)oxy)-1*a*,9*a*,10,10*a*-tetrahydrobenzo[*e*]cyclopropa[4,5]pyrrolo[1,2-*a*][1,4]diazepin-3(1*H*) -one |
| 2-22 | 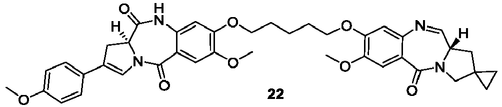 |
| | (*S*)-7-Methoxy-8-((5-(((*S*)-7-methoxy-5-oxo-5,11*a*-dihydro-1*H*,3*H*-spiro[benzo[*e*]p yrrolo[1,2-*a*][1,4]diazepine-2,1'-cyclopropan] -8-yl)oxy)pentyl)oxy)-2-(4-methoxyp henyl)-1,11*a*-dihydro-5*H*-benzo[*e*]pyrrolo[1,2-*a*][1,4]diazepine-5,11(10*H*)-dione **22** |
| 2-26 | 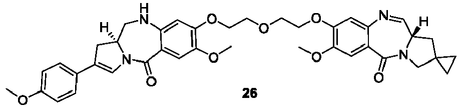 |
| | (*S*)-7-Methoxy-8-(2-(2-(((*S*)-7-methoxy-2-(4-methoxyphenyl)-5-oxo-5,10,11,11*a*-t etrahydro-1*H*-benzo[*e*]pyrrolo[1,2-*a*][1,4]diazepin-8-yl)oxy)ethoxy)ethoxy)-1,11*a-*dihydro-3*H*,5*H*-spiro[benzo[*e*]pyrrolo[1,2-*a*][1,4]diazepine-2,1'-cyclopropan]-5-on e **26** |
| 2-27 | 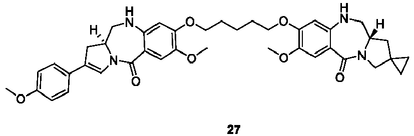 |
| | (*S*)-7-Methoxy-8-((5-(((*S*)-7-methoxy-2-(4-methoxyphenyl)-5-oxo-5,10,11,1*a*-tetr ahydro-1*H*-benzo[*e*]pyrrolo[1,2-*a*][1,4]diazepin-8-yl)oxy)pentyl)oxy)-1,10,11,11*a-*tetrahydro-3*H*,5*H*-spiro[benzo[*e*]pyrrolo[1,2-*a*][1,4]diazepine-2,1'-cyclopropan]-5-one 27 |
| 2-29 | 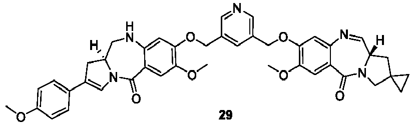 |
| | (*S*)-7-Methoxy-8-((5-((((*S*)-7-methoxy-2-(4-(methoxyphenyl)-5-oxo-5,10,11,11*a*-te trahydro-1*H*-benzo[*e*]pyrrolo[1,2-*a*][1,4]diazepin-8-yl)oxy)methyl)pyridin-3-yl)me thoxy)-1,11*a*-dihydro-3*H*,5*H*-spiro[benzo[*e*]pyrrolo[1,2-*a*][1,4]diazepine-2,1'-cycl opropan]-5-one **29** |
| 2-30 | 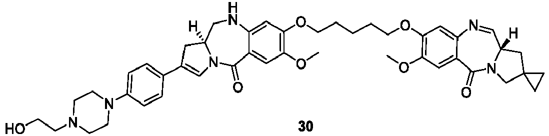 |
| | (*S*)-8-((5-(((*S*)-2-(4-(4-(2-Hydroxyethyl)piperazin-1-yl)phenyl)-7-methoxy-5-oxo-5 ,10,11,11*a*-tetrahydro-1*H*-benzo[*e*]pyrrolo[1,2-*a*][1,4]diazepin-8-yl)oxy)pentyl)oxy )-7-methoxy-1,11*a*-dihydro-3*H*,5*H*-spiro[benzo[*e*]pyrrolo[1,2-*a*][1,4]diazepine-2,1' -cyclopropan]-5-one **30** |
| 2-31 | 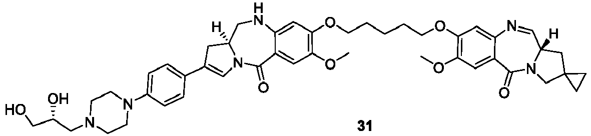 |
| | (*S*)-8-((5-(((*S*)-2-(4-(4-((*R*)-2,3-Dihydroxypropyl)piperazin-1-yl)phenyl)-7-methox y-5-oxo-5,10,11,11*a*-tetrahydro-1*H*-benzo[*e*]pyrrolo[1,2-*a*][1,4]diazepin-8-yl)oxy) pentyl)oxy)-7-methoxy-1,11*a*-dihydro-3*H*,5*H*-spiro[benzo[*e*]pyrrolo[1,2-*a*][1,4]dia zepine-2,1'-cyclopropan]-5-one **31** |
| 2-32 | 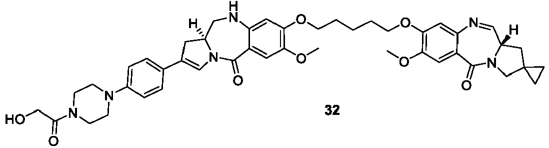 |
| | (*S*)-8-((5-(((*S*)-2-(4-(4-(2-Hydroxyacetyl)piperazin-1-yl)phenyl)-7-methoxy-5-oxo-5,10,11,11*a*-tetrahydro-1*H*-benzo[*e*]pyrrolo[1,2-*a*][1,4]diazepin-8-yl)oxy)pentyl)ox y)-7-methoxy-1,11*a*-dihydro-3*H*,5*H*-spiro[benzo[*e*]pyrrolo[1,2-*a*][1,4]diazepine-2, 1'-cyclopropan]-5-one **32** |
| 2-33 | 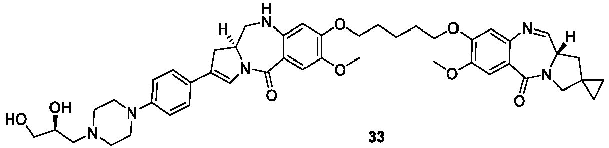 |
| | (*S*)-8-((5-(((*S*)-2-(4-(4-((*S*)-2,3-Dihydroxypropyl)piperazin-1-yl)phenyl)-7-methox y-5-oxo-5,10,11,11*a*-tetrahydro-1*H*-benzo[*e*]pyrrolo[1,2-*a*][1,4]diazepin-8-yl)oxy) pentyl)oxy)-7-methoxy-1,11*a*-dihydro-3*H*,5*H*-spiro[benzo[*e*]pyrrolo[1,2-*a*][1,4]dia zepine-2,1'-cyclopropan]-5-one **33** |
| 2-34 | 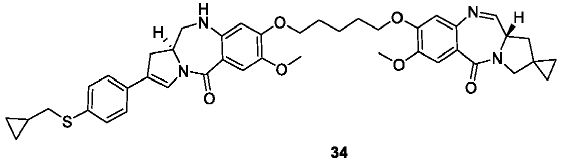 |
| | (*S*)-8-((5-(((*S*)-2-(4-((Cyclopropylmethyl)thio)phenyl)-7-methoxy-5-oxo-5,10,11,1 1*a*-tetrahydro-1*H*-benzo[*e*]pyrrolo[1,2-*a*][1,4]diazepin-8-yl)oxy)pentyl)oxy)-7-met hoxy-1,11*a*-dihydro-3*H*,5*H*-spiro[benzo[*e*]pyrrolo[1,2-*a*][1,4]diazepine-2,1'-cyclop ropan]-5-one **34** |
| 2-35 | 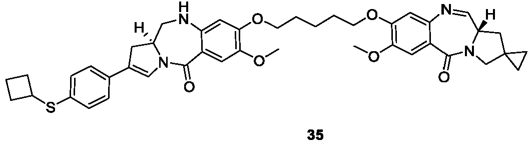 |
| | (*S*)-8-((5-(((*S*)-2-(4-(Cyclobutylthio)phenyl)-7-methoxy-5-oxo-5,10,11,11*a*-tetrahy dro-1*H*-benzo[*e*]pyrrolo[1,2-*a*][1,4]diazepin-8-yl)oxy)pentyl)oxy)-7-methoxy-1,11 *a*-dihydro-3*H*,5*H*-spiro[benzo[*e*]pyrrolo[1,2-*a*][1,4]diazepine-2,1'-cyclopropan]-5-one **35** |
| 2-36 | 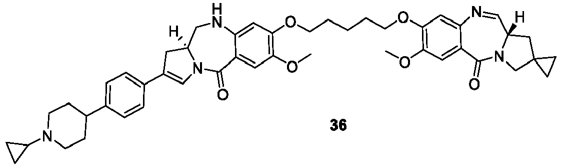 |
| | (*S*)-8-((5-(((*S*)-2-(4-(1-Cyclopropylpiperidin-4-yl)phenyl)-7-methoxy-5-oxo-5,10,1 1,11*a*-tetrahydro-1*H*-benzo[*e*]pyrrolo[1,2-*a*][1,4]diazepin-8-yl)oxy)pentyl)oxy)-7-methoxy-1,11*a*-dihydro-3*H*,5*H*-spiro[benzo[*e*]pyrrolo[1,2-*a*][1,4]diazepine-2,1'-cy clopropan]-5-one **36** |
| 2-37 | 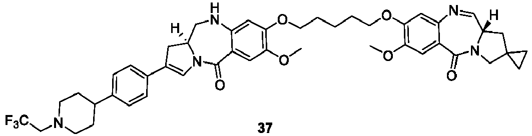 |
| | (*S*)-7-Methoxy-8-((5-(((*S*)-7-methoxy-5-oxo-2-(4-(1-(2,2,2-trifluoroethyl)piperidin -4-yl)phenyl)-5,10,11,11*a*-tetrahydro-1*H*-benzo[*e*]pyrrolo[1,2-*a*][1,4]diazepin-8-yl) |
| | oxy)pentyl)oxy)-1,11*a*-dihydro-3*H*,5*H*-spiro[benzo[*e*]pyrrolo[1,2-*a*][1,4]diazepine-2,1'-cyclopropan]-5-one **37** |
| 2-38 | 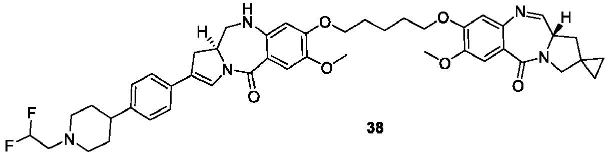 |
| | (*S*)-8-((5-(((*S*)-2-(4-(1-(2,2-Difluoroethyl)piperidin-4-yl)phenyl)-7-methoxy-5-oxo -5,10,11,11*a*-tetrahydro-1*H*-benzo[*e*]pyrrolo[1,2-*a*][1,4]diazepin-8-yl)oxy)pentyl)o xy)-7-methoxy-1,11*a*-dihydro-3*H*,5*H*-spiro[benzo[*e*]pyrrolo[1,2-*a*][1,4]diazepine-2 ,1'-cyclopropan]-5-one **38** |
| 2-39 | 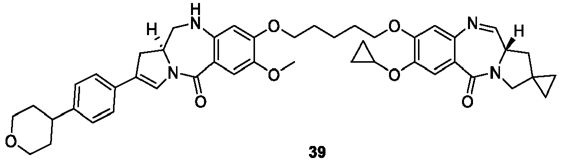 |
| | (S)-7-Cyclopropoxy-8-((5-(((S)-7-methoxy-5-oxo-2-(4-(tetrahydro-2H-pyran-4-yl) phenyl)-5,10,11,11a-tetrahydro-1H-benzo[e]pyrrolo[1,2-a][1,4]diazepin-8-yl)oxy)p entyl)oxy)-1,11a-dihydro-3H,5H-spiro[benzo[e]pyrrolo[1,2-a][1,4]diazepine-2,1'-c yclopropan]-5-one |

In some embodiments, the aforementioned compound or the pharmaceutically acceptable salt thereof is selected from the group consisting of: 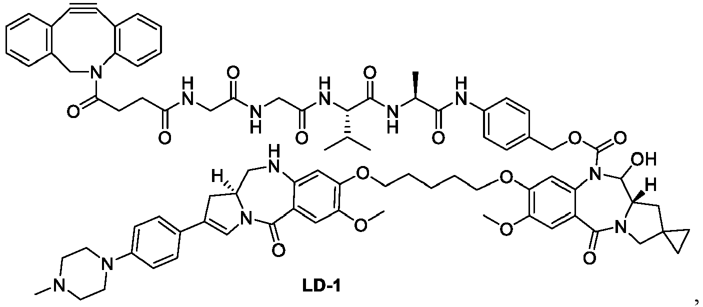 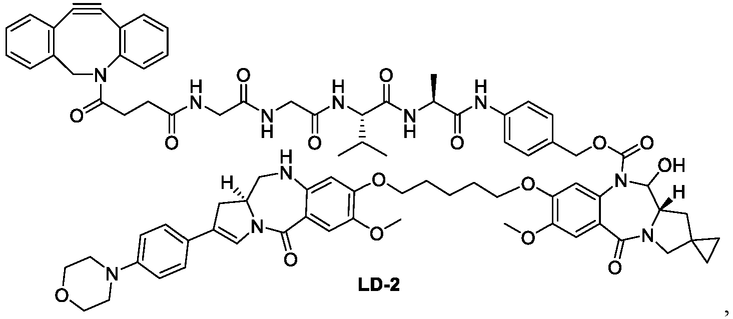 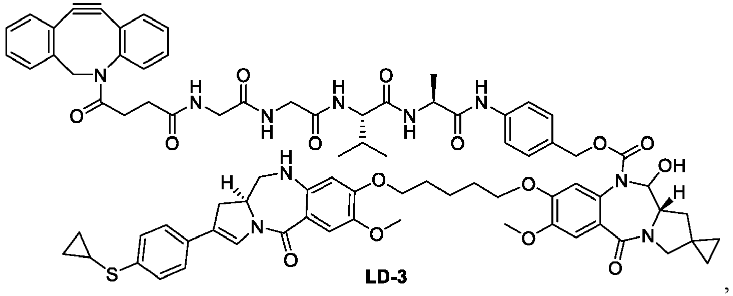 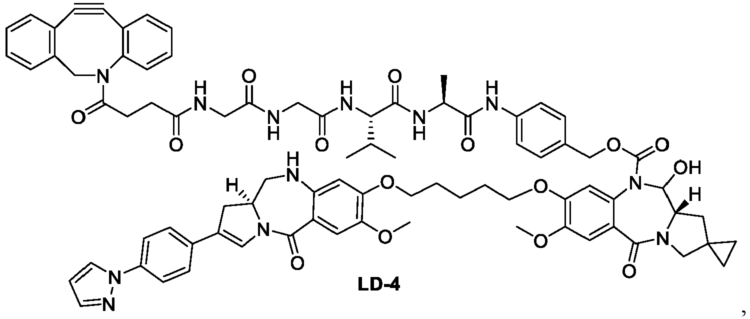 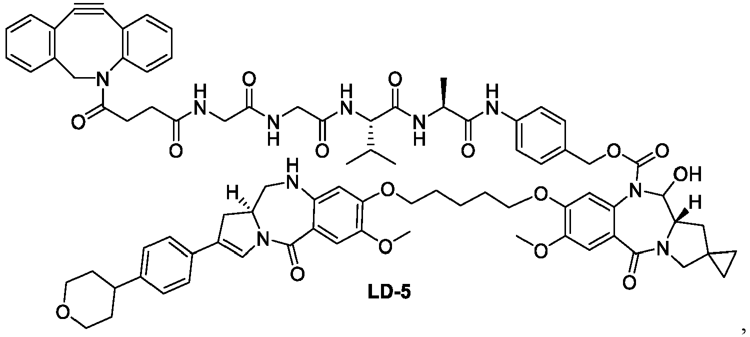 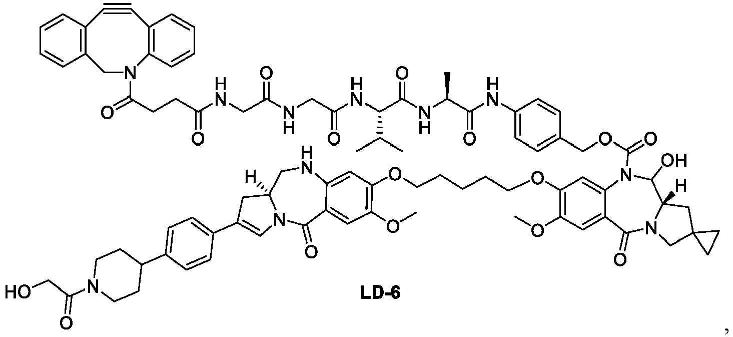 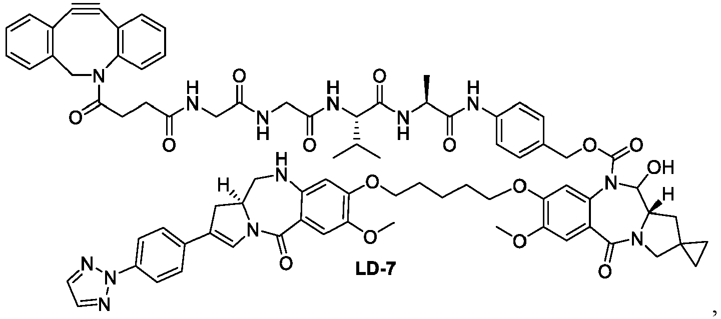 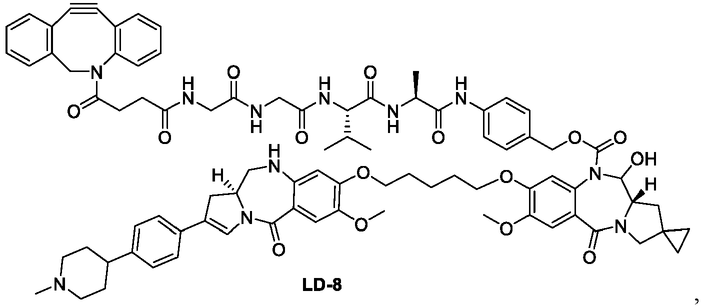 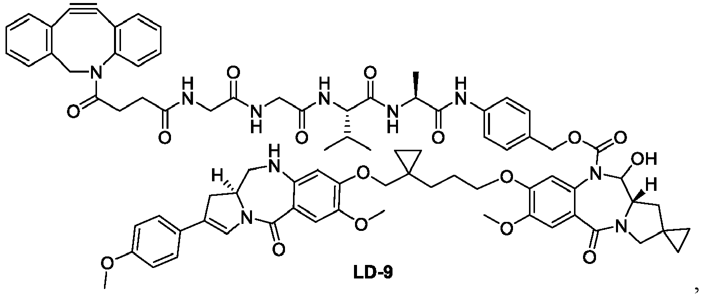 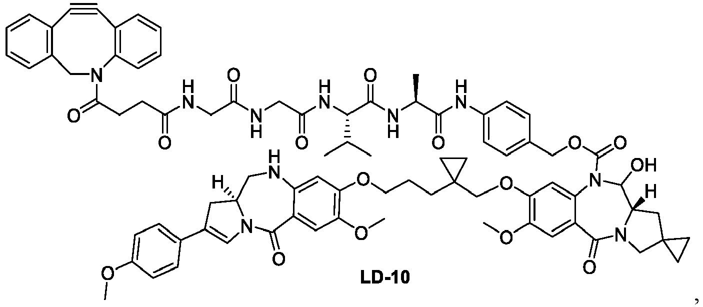 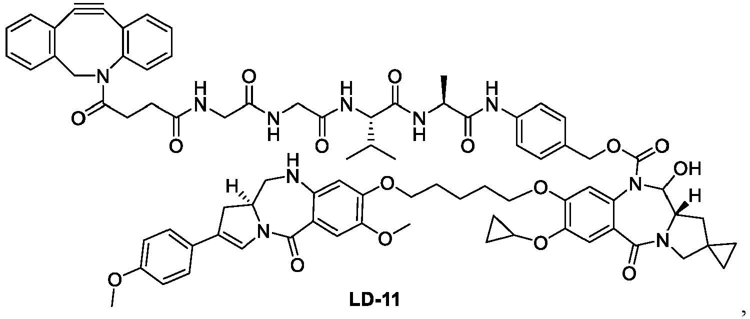 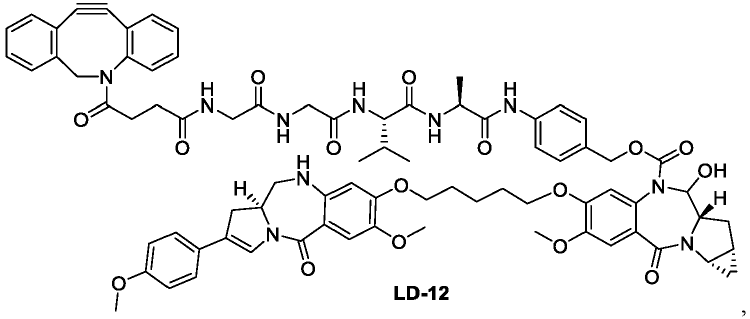 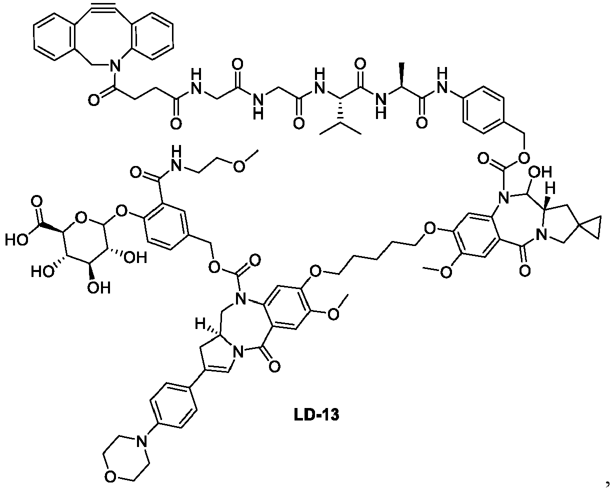 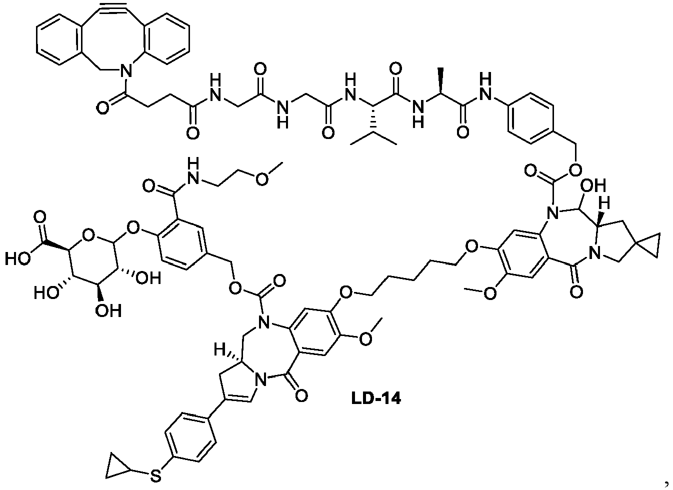 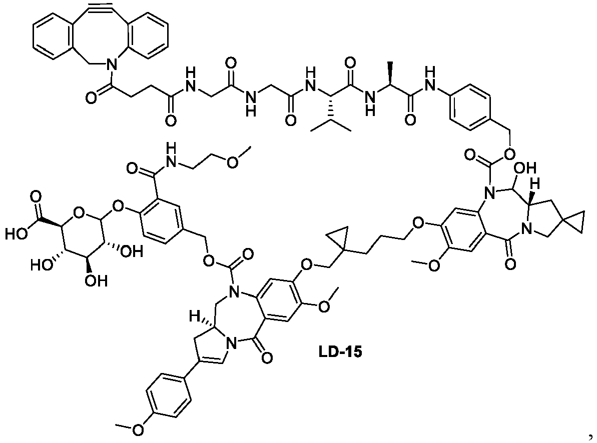 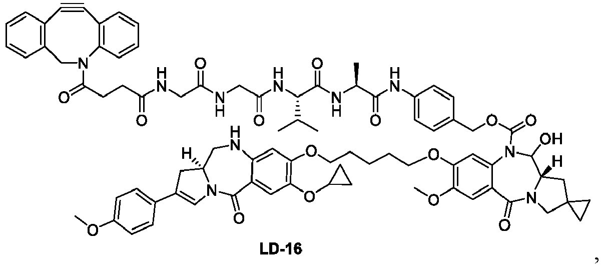 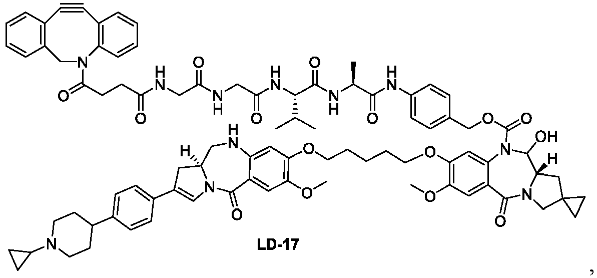 and

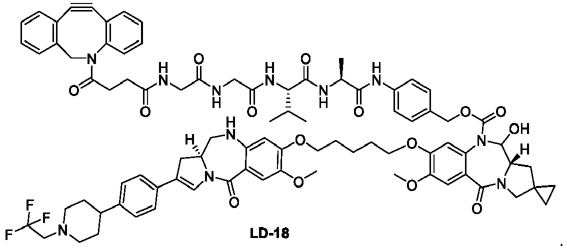

In another aspect, the present disclosure provides an antibody-drug conjugate or a pharmaceutically acceptable salt thereof, comprising the compound represented by formula (DL) or the pharmaceutically acceptable salt thereof according to any of the above.

In another aspect, the present disclosure provides a pharmaceutical composition, comprising a therapeutically effective amount of the antibody-drug conjugate or the pharmaceutically acceptable salt thereof according to any of the above, or the compound represented by formula (DL) or the pharmaceutically acceptable salt thereof according to any of the above, and a pharmaceutically acceptable carrier, diluent, or excipient.

In another aspect, the present disclosure provides the antibody-drug conjugate or the pharmaceutically acceptable salt thereof according to any of the above, or the compound represented by formula (DL) or the pharmaceutically acceptable salt thereof according to any of the above, or the aforementioned pharmaceutical composition for use as a medicament.

In another aspect, the present disclosure provides use of the antibody-drug conjugate or the pharmaceutically acceptable salt thereof according to any of the above, or the compound represented by formula (DL) or the pharmaceutically acceptable salt thereof according to any of the above, or the aforementioned pharmaceutical composition for use in a medicament for treating or preventing a tumor;
preferably, the tumor is selected from the group consisting of ovarian cancer, lung cancer, gastric cancer, endometrial cancer, testicular cancer, cervical cancer, placental choriocarcinoma, kidney cancer, urothelial cancer, colorectal cancer, prostate cancer, glioblastoma multiforme, brain tumor, pancreatic cancer, breast cancer, melanoma, liver cancer, bladder cancer, and esophageal cancer.

In another aspect, the present disclosure provides use of the antibody-drug conjugate or the pharmaceutically acceptable salt thereof according to any of the above, or the compound represented by formula (DL) or the pharmaceutically acceptable salt thereof according to any of the above, or the aforementioned pharmaceutical composition for use in the preparation of a medicament for treating or preventing a tumor;
preferably, the tumor is selected from the group consisting of ovarian cancer, lung cancer, gastric cancer, endometrial cancer, testicular cancer, cervical cancer, placental choriocarcinoma, kidney cancer, urothelial cancer, colorectal cancer, prostate cancer, glioblastoma multiforme, brain tumor, pancreatic cancer, breast cancer, melanoma, liver cancer, bladder cancer, and esophageal cancer.

In another aspect, the present disclosure further relates to a method for treating and/or preventing a tumor or cancer, the method comprising administering to a subject in need thereof a therapeutically effective dose of the antibody-drug conjugate or the pharmaceutically acceptable salt thereof according to any of the above, or the compound represented by formula (DL) or the pharmaceutically acceptable salt thereof according to any of the above, or the aforementioned pharmaceutical composition;
preferably, the tumor or cancer is selected from the group consisting of ovarian cancer, lung cancer, gastric cancer, endometrial cancer, testicular cancer, cervical cancer, placental choriocarcinoma, kidney cancer, urothelial cancer, colorectal cancer, prostate cancer, glioblastoma multiforme, brain tumor, pancreatic cancer, breast cancer, melanoma, liver cancer, bladder cancer, and esophageal cancer.

Active compounds (including compounds or antibody-drug conjugates) may be formulated into forms suitable for administration by any proper route. The composition of the present disclosure is formulated with one or more pharmaceutically acceptable carriers using a conventional method. Thus, the active compound of the present disclosure may be formulated into a variety of dosage forms for oral administration, administration by injection (e.g., intravenous, intramuscular, or subcutaneous), or administration by inhalation or insufflation. The compound of the present disclosure may also be formulated into a sustained-release dosage form, such as tablets, hard or soft capsules, aqueous or oily suspensions, emulsions, injections, dispersible powders or granules, suppositories, lozenges or syrups.

As a general guide, the active compound is preferably in the form of a unit dose, or in the form of a single dose that can be administered by the patient. The unit dose of the compound or composition of the present disclosure may be expressed as a tablet, a capsule, a cachet, a vial, a powder, a granule, a lozenge, a suppository, a regenerating powder, or a liquid formulation. A suitable unit dose may be 0.1 mg-1000 mg.

The pharmaceutical composition of the present disclosure may comprise, in addition to the active compound, one or more auxiliary materials selected from the group consisting of a filler, a diluent, a binder, a wetting agent, a disintegrant, an excipient, and the like. Depending on the method of administration, the composition may comprise 0.1 to 99 wt.% of the active compound.

The pharmaceutical composition comprising the active ingredient may be in a form suitable for oral administration, for example, in the form of a tablet, a dragee, a lozenge, an aqueous or oil suspension, a dispersible powder or granule, an emulsion, a hard or soft capsule, or a syrup or elixir. Oral compositions can be prepared according to any method known in the art for preparing pharmaceutical compositions. Such oral compositions may comprise a binder, a filler, a lubricant, a disintegrant, or a pharmaceutically acceptable wetting agent, and may also comprise one or more ingredients selected from the group consisting of sweetener, corrigent, colorant, and preservative, so as to provide a pleasant-to-eye and palatable pharmaceutical formulation.

An aqueous suspension comprises an active substance and an excipient that is used for mixing and suitable for the preparation of the aqueous suspension. The aqueous suspension may also comprise one or more preservatives, one or more colorants, one or more corrigents, and one or more sweeteners.

The oil suspension may be formulated by suspending the active ingredient in a vegetable oil. The oil suspension may comprise a thickening agent. The sweeteners and corrigents described above may be added to provide a palatable formulation.

The pharmaceutical compositions may also be prepared as follows: dispersible powders or granules for preparing aqueous suspensions provide the active ingredient, and water is added to mix the active ingredient with one or more of dispersants, wetting agents, suspending agents, or preservatives. Other excipients, such as sweeteners, corrigents, and colorants, may also be added. Antioxidants such as ascorbic acid are added to preserve these compositions.

The pharmaceutical composition of the present disclosure may also be in the form of an oil-in-water emulsion.

The pharmaceutical composition may be in the form of a sterile injectable aqueous solution. Acceptable vehicles or solvents that can be used include water, Ringer's solution, and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug. Such materials include cocoa butter, glycerogelatin, hydrogenated vegetable oils, polyethylene glycols of various molecular weights and mixtures of fatty acid esters of polyethylene glycols.

As is well known to those skilled in the art, the dose of the drug administered depends on a variety of factors, including, but not limited to, the activity of the particular compound used, the age of the subject, the body weight of the subject, the health condition of the subject, the behavior of the subject, the diet of the subject, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

The present disclosure relates to conjugates comprising a targeting (binding) moiety and a therapeutic moiety that is a drug, with improved ability to target different cancer cells, infectious disease organisms, and/or for use in the treatment of autoimmune diseases. The antibody targeting moiety is linked to the compound therapeutic moiety via an intracellularly cleavable linkage that increases the therapeutic efficacy.

### DETAILED DESCRIPTION

### Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to". Unless otherwise specified, "comprise" includes "consist of'.

The "antibody" described in the present disclosure is used in the broadest sense herein and includes different antibody structures, including but not limited to, a monoclonal antibody, a polyclonal antibody, a murine antibody, a chimeric antibody, a humanized antibody, a multispecific antibody (e.g., a bispecific antibody), and antigen-binding fragments, as long as they exhibit the desired antigen-binding activity and specificity. Preferably, the "antibody" described in the present disclosure refers to one that is substantially similar to natural antibodies in structure, which comprises two intact light chains and two intact heavy chains.

The term antibody-drug conjugate (ADC) means that a monoclonal antibody or an antibody fragment is linked to a biologically active toxic drug by a linker unit. The antibody or the antibody fragment described in the present disclosure may be conjugated to the effector molecule by any means. For example, the antibody or the antibody fragment may be chemically or recombinantly attached to the cytotoxic drug. Chemical means for preparing fusions or conjugates are known in the art. The method for conjugating the antibody or the antibody fragment and the drug must be capable of linking the antibody to the cytotoxic drug without interfering with the ability of the antibody or the antibody fragment to bind to the target molecule.

The term "medicament" or "drug" refers to a substance with biological activity, e.g., a cytotoxic agent or an immunomodulatory agent. The cytotoxic agent is a chemical molecule capable of greatly disrupting the normal growth of tumor cells. In some embodiments of the present disclosure, a drug may be represented by D.

A method for remodeling glycoproteins of a heterogeneous antibody by an enzymatic reaction or the like and homogeneously introducing a glycan chain having a functional group has been reported in recent years (ACS Chemical Biology 2012, 7, 110; ACS Medicinal Chemistry Letters 2016, 7, 1005). An attempt has been made to site-specifically introduce a drug using this glycan chain remodeling technique to synthesize a homogeneous ADC (Bioconjugate Chemistry 2015, 26, 2233; Angew. Chem. Int. Ed. 2016, 55, 2361-2367; US2016361436).

In the glycan chain remodeling of the present disclosure, heterogeneous glycan chains attached to a protein (an antibody or the like) are removed using a hydrolase with only the GlcNAc at the termini kept to prepare a homogenous protein moiety with GlcNAc (hereinafter referred to as an "acceptor"). Subsequently, an arbitrary glycan chain separately prepared (hereinafter referred to as a "donor") is provided, and the acceptor and the donor are linked using a glycosyltransferase. Thus, a homogeneous glycoprotein with an arbitrary glycan chain structure can be synthesized.

In the present disclosure, a "glycan chain" refers to a structural unit of two or more monosaccharides linked by glycosidic bonds. Specific monosaccharides or glycan chains are sometimes abbreviated, for example, as "GlcNAc-" or "MSG-". When any of these abbreviations is used in a structural formula, an oxygen atom or nitrogen atom of a glycosidic bond at a reducing terminus belonging to another structural unit is not included in the abbreviation indicating the glycan chain, unless otherwise specified.

In the present disclosure, for convenience, in the description of a monosaccharide as a basic unit of a glycan chain, in the ring structure, the position of a carbon atom binding to an oxygen atom constituting the ring and directly binding to a hydroxy group (or an oxygen atom belonging to a glycosidic bond) is defined as locant 1 (locant 2 only for sialic acid), unless otherwise specified. The names of the example compounds are provided in view of the chemical structure as a whole, and that rule does not necessarily apply.

When a glycan chain is indicated by a symbol (e.g., GLY, SG, MSG, or GlcNAc) in the present disclosure, the symbol includes the carbon atom at the reducing terminus unless particularly defined but does not include the N or O belonging to an N- or O-glycosidic bond.

In the present invention, unless particularly stated, a partial structure when the side chain of an amino acid is linked to a glycan chain is indicated in such a manner that the side chain portion is shown in parentheses, for example, "(SG-)Asn".

The antibody or a functional fragment thereof may bind, from the side chain of an amino acid residue thereof (e.g., cysteine or lysine), directly to L or bind, from a glycan chain or remodeled glycan chain of the antibody, to L; preferably bind, from a glycan chain or remodeled glycan chain of the antibody, to L; and more preferably bind, from a remodeled glycan chain of the antibody, to L.

The glycan chain of the antibody of the present disclosure is a N-linked glycan chain or an O-linked glycan chain.

The N-linked glycan chain binds to an amino acid side chain of the antibody through an N-glycosidic bond, and the O-linked glycan chain binds to an amino acid side chain of the antibody through an O-glycosidic bond.

IgG is known to have a highly conserved N-linked glycan chain in asparagine residue number 297 (hereinafter referred to as "Asn297 or N297") in the Fc region of its heavy chain, which contributes to the activity, dynamics, or the like of the antibody molecule (Biotechnol. Prog., 2012, 28, 608-622; Sanglier-Cianferani, S., Anal. Chem., 2013, 85, 715-736). The amino acid sequence in the IgG constant region is highly conserved. In Edelman et al., (Proc. Natl. Acad. Sci. U.S.A., Vol. 63, No. 1 (May 15, 1969), pp. 78-85), each amino acid is determined by Eu numbering (Eu INDEX). For example, Asn297, to which an N-linked glycan chain is added in the Fc region, corresponds to position 297 in Eu numbering, and each amino acid can be uniquely determined by Eu numbering even if the actual position of the amino acid has changed due to molecule fragmentation or region deletion.

In the antibody-drug conjugate of the present disclosure, it is more preferable for the antibody or a functional fragment thereof to bind, from a glycan chain binding to the side chain of Asn297 thereof (hereinafter referred to as "N297 glycan chain"), to L, and it is more preferable for the antibody or a functional fragment thereof to bind, from the N297 glycan chain, to L, wherein the N297 glycan chain is a remodeled glycan chain. The antibody having the remodeled glycan chain of the present disclosure is referred to as a glycan chain-remodeled antibody.

SGP, an abbreviation for sialyl glyco peptide (or N-acetylneuraminic acid), is a representative of N-linked complex glycan chains. SGP can be separated or purified from the yolk of a hen egg using the method described in, for example, WO2011/0278681. It is also commercially available.

In the present disclosure, a glycan chain structure formed by deleting a N-acetylneuraminic acid from the non-reducing terminus of any of the β-Man branched chains of SG(10) is referred to MSG(9); a glycan chain having a sialic acid only in the 1-3 branched-chain glycan chain is referred to as MSG1; a glycan chain having a N-acetylneuraminic acid only in the 1-6 branched-chain glycan chain is referred to as MSG2.

The term "linker unit", "linking fragment", or "linking unit" refers to a chemical structure fragment or bond, which is linked to an antibody or antibody fragment at one end and to a drug at the other end, and may also be linked to a drug after being linked to another linker. A linker unit may comprise one or more linker components. Exemplary linker components include 6-maleimidocaproyl, maleimidopropionyl, valine-citrulline, valine-alanine, alanine-phenylalanine, p-aminobenzyloxycarbonyl, N-succinimidyl 4-(2-pyridylthio)pentanoate, N-succinimidyl 4-(N-maleimidomethyl)cyclohexane-1 carboxylate, and N-succinimidyl(4-iodo-acetyl)aminobenzoate. A linker may comprise a stretcher, a spacer, and an amino acid unit, which may be synthesized using methods known in the art, such as those described in US20050238649A1. A linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers may be used (Chari et al., Cancer Research, 52: 127-131(1992); U.S. Patent No. 5208020).

The term "peptide" refers to a molecule formed by linking 2 or more amino acid molecules by peptide bonds.

The term "sugar" includes O-glycoside, N-glycoside, S-glycoside, and C-glycoside (C-glycoslyl) carbohydrate derivatives of the monosaccharide and disaccharide classes and may originate from naturally-occurring sources or may be synthetic in origin. For example, "sugar" includes derivatives such as (but not limited to) glucuronic acid, galacturonic acid, galactose, and glucose, and derivatives thereof.

The glucuronide group in the present disclosure has the following structure:

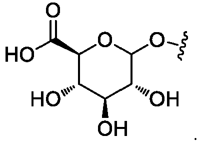

The galactoside group in the present disclosure has the following structure:

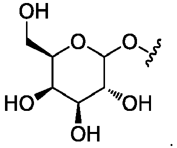

The term "drug loading" or "average number of bound drugs" is also referred to as drug-to-antibody ratio (DAR), i.e., the average number of drugs conjugated per antibody in an antibody-drug conjugate. In the antibody-drug conjugate of the present disclosure, the number of bound drugs per antibody molecule is an important factor that affects its efficacy and safety. Production of the antibody-drug conjugate is performed by defining reaction conditions including the amounts of starting materials and reagents used for the reaction so as to render the number of bound drugs constant. However, unlike chemical reactions of low-molecular-weight compounds, the reaction generally yields a mixture consisting of different numbers of bound drugs. The number of bound drugs per antibody molecule is determined as an average value, i.e., the average number of bound drugs or drug loading (DAR: drug-to-antibody ratio). Drug loading may range, for example, from about 1 to about 10 conjugated drugs per antibody. In some embodiments, the drug loading ranges from about 1 to about 8, preferably 2-8, 2-7, 2-6, 2-5, 2-4, 3-4, 3-5, 5-6, 5-7, 5-8, and 6-8, conjugated drugs per antibody. For example, the drug loading may be 1, 2, 3, 4, 5, 6, 7, 8, 9, or 10. The number of pyrrolo benzodiazepine derivatives bound by an antibody molecule is controllable. The average number of bound drugs per antibody (DAR) may be 1-10, preferably 1-8, and more preferably 1-4, pyrrolo benzodiazepine derivatives. In an embodiment of the present disclosure, the drug loading may be represented by y. Drug loading can be determined by conventional methods such as UV/visible spectroscopy, mass spectrometry, ELISA assays, and RP-HPLC. In an embodiment of the present disclosure, the antibody binds to the linker L through an N297 glycan chain thereof, wherein the N297 glycan chain binds to an Asn at position 297 of a heavy chain of the antibody. When the antibody has two heavy chains, the heavy chains of the antibody have two position 297 Asn-binding sites, i.e., N297 sites. The N297 glycan chain may bind to the two position 297 Asns of the heavy chains of the antibody or one of the position 297 Asns of the heavy chains of the antibody. The average number of drugs conjugated through the N297 glycan chain in the antibody-drug conjugate is 1-4, preferably 1-3, or preferably 1-2. In some embodiments, the DAR ranges from 1 to 2 when the N297 glycan chain of the present disclosure is selected from the group consisting of OLS-1, OLS-2, OLS-3, OLS-4, OLS-5, OLS-6, and OLS-7. In some embodiments, the DAR ranges from 1 to 4 (preferably from 1.5 to 3.9, more preferably from 2.8 to 3.9, and most preferably from 1 to 2) when the N297 glycan chain is selected from the group consisting of OLS-8, OLS-9, OLS-10, OLS-11, and OLS-12.

Two glycan chains or one glycan chain may be remodeled at the two N297 sites of the heavy chains of the antibody in the present disclosure. In the present disclosure, y¹ represents the average number of glycan chains remodeled to the N297 sites of the heavy chains of the antibody, i.e., the average number of remodeled glycan chains per antibody, y¹ may be 1-2 and may be about 1.5, 1.6, 1.7, 1.8, 1.85, 1.9, 1.95, 1.98, and 2.

Drug loading can be controlled by the following non-limiting methods, including:
(1) controlling the molar ratio of the linker arm drug to the mAb,
(2) controlling reaction time and temperature, and
(3) selecting different reaction reagents.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl is preferably an alkyl group having 1 to 12 carbon atoms (i.e., C₁₋₁₂ alkyl), more preferably an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched-chain isomers thereof, and the like. Alkyl may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; it has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). The alkylene is preferably an alkylene group having 1 to 12 carbon atoms (i.e., C₁₋₁₂ alkylene), more preferably an alkylene group having 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, and the like. Alkylene may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl is as defined above, and it has 2 to 12 (e.g., 2, 3, 4, 5, 6, 7, 8, 9, 10, 11, or 12) carbon atoms (i.e., C₂₋₁₂ alkenyl). The alkenyl is preferably an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl is as defined above, and it has 2 to 12 (e.g., 2, 3, 4, 5, 6, 7, 8, 9, 10, 11, or 12) carbon atoms (i.e., C₂₋₁₂ alkynyl). The alkynyl is preferably an alkynyl group having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e., 3- to 12-membered cycloalkyl), more preferably a cycloalkyl group having 3 to 8 ring atoms (i.e., 3- to 8-membered cycloalkyl, e.g., C₃₋₇ cycloalkyl), and most preferably a cycloalkyl group having 3 to 6 ring atoms (i.e., 3- to 6-membered cycloalkyl, e.g., C₃₋₆ cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, which may contain one or more double bonds in the rings or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that the system contains at least one all-carbon ring and the point of attachment is on the all-carbon ring; the system has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl is preferably a spirocycloalkyl group having 6 to 14 ring atoms (i.e., 6- to 14-membered spirocycloalkyl), more preferably a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl), preferably monospirocycloalkyl or bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples include: 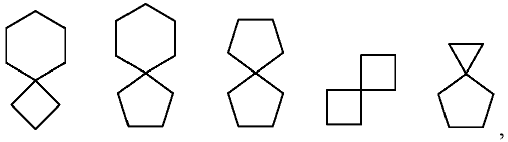 the points of attachment of which may be any positions; 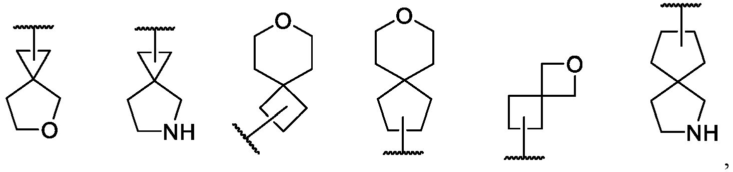 and the like.

The term "fused cycloalkyl" refers to a polycyclic system in which two adjacent carbon atoms are shared between rings, which is formed by fusing a monocyclic cycloalkyl group with one or more monocyclic cycloalkyl groups or fusing a monocyclic cycloalkyl group with one or more of heterocyclyl, aryl, or heteroaryl, wherein the point of attachment is on the monocyclic cycloalkyl group; the system may contain one or more double bonds in the rings and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl is preferably a fused cycloalkyl group having 6 to 14 ring atoms (i.e., 6- to 14-membered fused cycloalkyl), more preferably a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5 -membered/5 -membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples include: 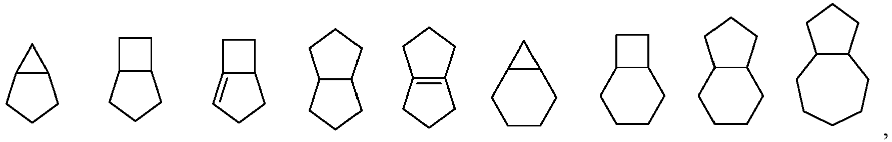 the points of attachment of which may be any positions; 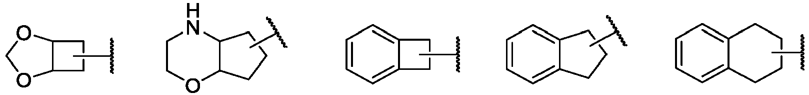 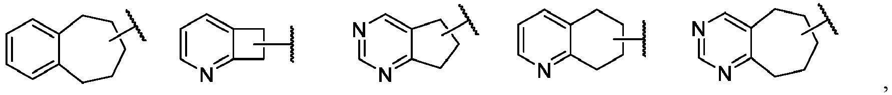 and the like.

The term "bridged cycloalkyl" refers to an all-carbon polycyclic system in which two carbon atoms not directly linked are shared between rings, which may contain one or more double bonds in the rings and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). The bridged cycloalkyl is preferably a bridged cycloalkyl group having 6 to 14 carbon atoms (i.e., 6- to 14-membered bridged cycloalkyl), more preferably a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl or tetracyclic bridged cycloalkyl), preferably bicyclic bridged cycloalkyl or tricyclic bridged cycloalkyl. Non-limiting examples include: 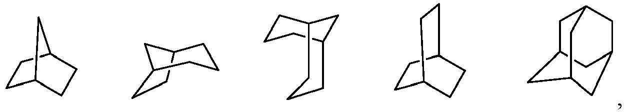 the points of attachment of which may be any positions.

Cycloalkyl may be substituted or unsubstituted, and when it is substituted, it may be substituted at any accessible point of attachment, and the substituent is preferably selected from one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl is preferably a heterocyclyl group having 3 to 12 ring atoms (i.e., 3- to 12-membered heterocyclyl), further preferably a heterocyclyl group having 3 to 8 ring atoms (i.e., 3- to 8-membered heterocyclyl), more preferably a heterocyclyl group having 3 to 6 ring atoms (i.e., 3- to 6-membered heterocyclyl), and most preferably a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, which may contain one or more double bonds in the rings and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that the system contains at least one monocyclic heterocyclyl group and the point of attachment is on the monocyclic heterocyclyl group; the system has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). The spiroheterocyclyl is preferably a spiroheterocyclyl group having 6 to 14 ring atoms (i.e., 6- to 14-membered spiroheterocyclyl), more preferably a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl), preferably monospiroheterocyclyl or bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples include: 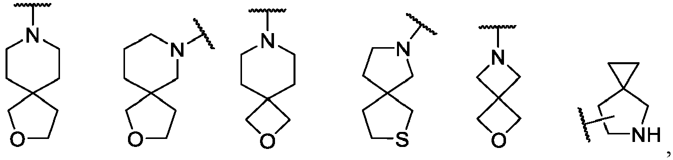 and the like.

The term "fused heterocyclyl" refers to a polycyclic heterocyclic system in which two adjacent atoms are shared between rings, which may contain one or more double bonds in the rings and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); the system is formed by fusing a monocyclic heterocyclyl group with one or more monocyclic heterocyclyl groups or fusing a monocyclic heterocyclyl group with one or more of cycloalkyl, aryl, or heteroaryl, wherein the point of attachment is on the monocyclic heterocyclyl group, and the system has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl is preferably a fused heterocyclyl group having 6 to 14 ring atoms (i.e., 6-to 14-membered fused heterocyclyl), more preferably a fused heterocyclyl group having 7 to 10 ring atoms (i.e., 7- to 10-membered fused heterocyclyl). The fused heterocyclyl includes bicyclic heterocyclyl and polycyclic fused heterocyclyl (e.g., tricyclic fused heterocyclyl and tetracyclic fused heterocyclyl), preferably bicyclic fused heterocyclyl or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples include: 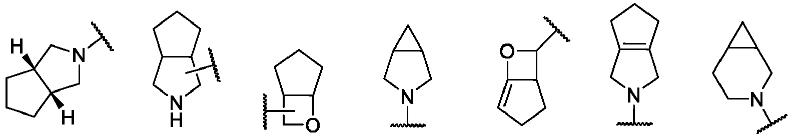 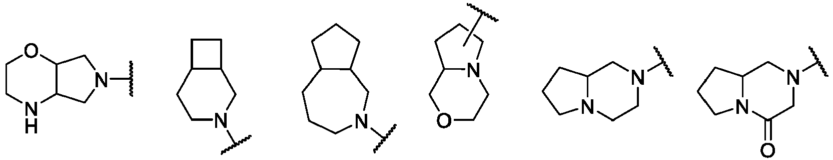 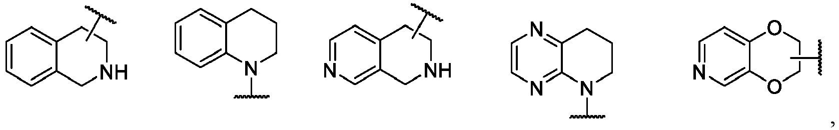 and the like.

The term "bridged heterocyclyl" refers to a polycyclic heterocyclic system in which two atoms not directly linked are shared between rings, which may contain one or more double bonds in the rings and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); the system has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl is preferably a bridged heterocyclyl group having 6 to 14 ring atoms (i.e., 6- to 14-membered bridged heterocyclyl), more preferably a bridged heterocyclyl group having 7 to 10 ring atoms (i.e., 7- to 10-membered bridged heterocyclyl). According to the number of constituent rings, the bridged heterocyclyl may be bicyclic bridged heterocyclyl or polycyclic bridged heterocyclyl (e.g., tricyclic bridged heterocyclyl or tetracyclic bridged heterocyclyl), preferably bicyclic bridged heterocyclyl or tricyclic bridged heterocyclyl. Non-limiting examples include: 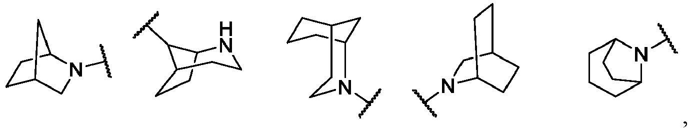 and the like.

Heterocyclyl may be substituted or unsubstituted, and when it is substituted, it may be substituted at any accessible point of attachment, and the substituent is preferably selected from one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, which has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). The monocyclic aryl is, for example, phenyl. Non-limiting examples of the polycyclic aryl include: naphthyl, anthracenyl, phenanthrenyl, and the like. The polycyclic aryl further includes those formed by fusing phenyl with one or more of heterocyclyl or cycloalkyl or fusing naphthyl with one or more of heterocyclyl or cycloalkyl, wherein the point of attachment is on the phenyl or naphthyl, and in the circumstances the number of ring atoms continues to represent the number of ring atoms in the polycyclic aromatic ring system; non-limiting examples include: 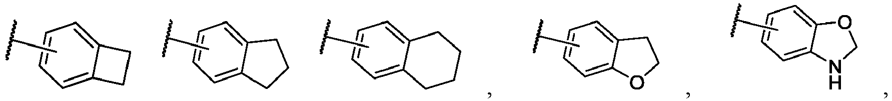 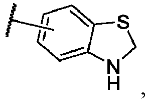 and the like.

Aryl may be substituted or unsubstituted, and when it is substituted, it may be substituted at any accessible point of attachment, and the substituent is preferably selected from one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 5 to 14 (e.g., 5, 6, 7, 8, 9, 10, 11, 12, 13, or 14) ring atoms (i.e., 5- to 14-membered heteroaryl). The heteroaryl is preferably a heteroaryl group having 5 to 10 ring atoms (i.e., 5- to 10-membered heteroaryl), more preferably a heteroaryl group having 5 or 6 ring atoms (i.e., 5-or 6-membered heteroaryl).

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridinonyl, N-alkylpyridinone (e.g., 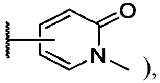 pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl further includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl further includes those formed by fusing monocyclic heteroaryl with one or more of cycloalkyl or heterocyclyl, wherein the point of attachment is on the monocyclic heteroaromatic ring, and in the circumstances the number of ring atoms continues to represent the number of ring atoms in the polycyclic heteroaromatic ring system. Non-limiting examples include: 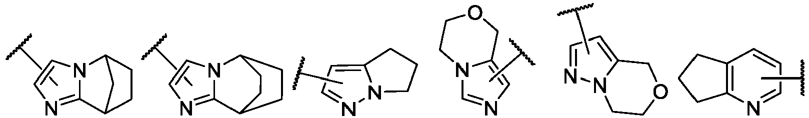 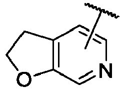 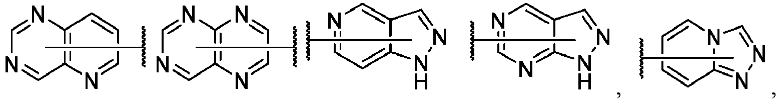 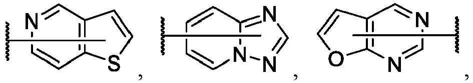 and the like.

Heteroaryl may be substituted or unsubstituted, and when it is substituted, it may be substituted at any accessible point of attachment, and the substituent is preferably selected from one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group in order for the amino group to remain unchanged when reactions take place on other parts of the molecule. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), trifluoroacetyl (Tfa), trityl (Trt), 2,4-dimethoxybenzyl (DMB), acetyl, benzyl, allyl, p-methoxybenzyl, and the like.

The term "hydroxy protecting group" refers to an easily removable group that is introduced onto a hydroxy group to block or protect the hydroxy group such that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to an alkyl group substituted with one or more halogens, wherein the alkyl is as defined above.

The term "haloalkoxy" refers to an alkoxy group substituted with one or more halogens, wherein the alkoxy is as defined above.

The term "deuterated alkyl" refers to an alkyl group substituted with one or more deuterium atoms, wherein the alkyl is as defined above.

The term "hydroxyalkyl" refers to an alkyl group substituted with one or more hydroxy groups, wherein the alkyl is as defined above.

The term "methylidene" refers to =CH₂.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

In the chemical structure of the compound of the present disclosure, a bond " 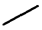 " indicates an unspecified configuration; that is, if chiral isomers exist in the chemical structure, the bond " 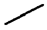 " may be " 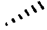 ",, or " 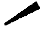 ", or includes both the configurations of " 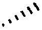 " and " 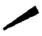 ". For all carbon-carbon double bonds, both *Z*- and *E*-forms are included, even if only one configuration is named.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. An example of a lactam-lactim equilibrium is present between A and B as shown below:

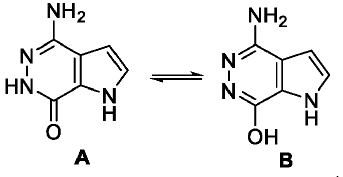

For example, reference to pyrazolyl is understood to include any one of the following two structures or a mixture of the two tautomers:

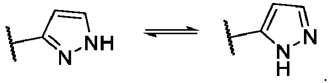

All tautomeric forms fall within the scope of the present disclosure, and the nomenclature of the compounds does not exclude any tautomers.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (deuterium, T), ¹¹C, ¹³C, ¹⁴C, ¹⁵N, ¹⁷O, ¹⁸O, ³²p, ³³p, ³³S , ³⁴S, ³⁵S, ³⁶S, ¹⁸F, ³⁶Cl, ⁸²Br, ¹²³I, ¹²⁴I, ¹²⁵I, ¹²⁹I, and ¹³¹I, deuterium is preferred.

Compared with non-deuterated drugs, deuterated drugs have the advantages of reduced toxic and side effects, increased drug stability, enhanced curative effect, prolonged biological half-lives, and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen atom with at least one deuterium atom.

When a position is specifically assigned deuterium (D), the position should be understood as deuterium with an abundance that is at least 1000 times greater than the natural abundance of deuterium (which is 0.015%) (i.e., at least 15% deuterium incorporation). The deuterium of the compounds in the examples with an abundance greater than the natural abundance of deuterium may be deuterium with an abundance that is at least 1000 times greater (i.e., at least 15% deuterium incorporation), at least 2000 times greater (i.e., at least 30% deuterium incorporation), at least 3000 times greater (i.e., at least 45% deuterium incorporation), at least 3340 times greater (i.e., at least 50.1% deuterium incorporation), at least 3500 times greater (i.e., at least 52.5% deuterium incorporation), at least 4000 times greater (i.e., at least 60% deuterium incorporation), at least 4500 times greater (i.e., at least 67.5% deuterium incorporation), at least 5000 times greater (i.e., at least 75% deuterium incorporation), at least 5500 times greater (i.e., at least 82.5% deuterium incorporation), at least 6000 times greater (i.e., at least 90% deuterium incorporation), at least 6333.3 times greater (i.e., at least 95% deuterium incorporation), at least 6466.7 times greater (i.e., at least 97% deuterium incorporation), at least 6600 times greater (i.e., at least 99% deuterium incorporation), or at least 6633.3 times greater (i.e., at least 99.5% deuterium incorporation), or deuterium with a higher abundance.

"Optional" or "optionally" means that the event or circumstance subsequently described may, but does not necessarily, occur and includes an instance where the event or circumstance occurs and an instance where it does not. For example, "C₁₋₆ alkyl that is optionally substituted with a halogen or cyano" includes an instance where the alkyl is substituted with the halogen or cyano and an instance where the alkyl is not substituted with the halogen or cyano.

"Substitution" or "substituted" means that one or more, preferably 1-6, and more preferably 1-3 hydrogen atoms, in the group are independently substituted with a corresponding number of substituents. Those skilled in the art can determine (experimentally or theoretically) possible or impossible substitutions without undue effort. For example, it may be unstable when an amino or hydroxy group with free hydrogen binds to a carbon atom with an unsaturated bond (e.g., an alkene).

"Pharmaceutical composition" refers to a mixture of one or more of the compounds described herein or physiologically acceptable salts thereof and other chemical components, as well as other components such as physiologically acceptable carriers and excipients. The pharmaceutical composition is intended to promote administration to an organism and facilitate the absorption of the active ingredient so that it can exert its biological activity.

"Pharmaceutically acceptable salt" refers to a salt of the compound of the present disclosure, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" or "inhibitory effective amount" refers to an amount of a drug or an agent that is non-toxic and sufficient to provide the desired effect. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

### Examples and Test Examples

The present disclosure is further described below with reference to examples, which are not intended to limit the scope of the present disclosure.

Experimental procedures without conditions specified in the examples of the present disclosure were generally conducted according to conventional conditions or according to conditions recommended by the manufacturers of the starting materials or commercial products. Reagents without specific origins indicated are commercially available conventional reagents.

### I. Antibody Examples

The following antibodies can be prepared using a conventional preparation method for antibodies, including, for example, vector construction, transfection of eukaryotic cells such as HEK293 cells (Life Technologies Cat. No. 11625019), expression, and purification.

The sequences of the anti-HER2 antibody trastuzumab (trastuzumab of WO2020/063676A1) are as follows:
The light chain of Ab1 
DIQMTQSPSSLSASVGDRVTITCRASQDVNTAVAWYQQKPGKAPKLLIYSASFLY
SGVPSRFSGSRSGTDFTLTISSLQPEDFATYYCQQHYTTPPTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 1
The heavy chain of Ab1

EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 2

The sequences of the anti-Claudin18.2 antibody (the anti-Claudin18.2 antibody h1902-5 of WO 2021/115426 A1) are as follows:
The light chain of Ab2 
DIVLTQSPDSLAVSLGERATINCKSSQSLLNSGNQKNYLTWYQQKPGQPPKLLIY
WASTRESGVPDRFSGSGSGTDFTLTISSLQAEDVAVYYCQNAYTYPFTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
SEQ ID NO: 3
The heavy chain of Ab2

EVQLVQSGAEVKKPGASVKVSCKASGYTFTSYWMHWVRQAPGQRLEWMGMI
HPNSGSTNYNEKFKGRVTITRDTSASTAYMELSSLRSEDTAVYYCARLKTGNSF
DYWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 4

The sequences of the anti-HER3 antibody (the anti-HER3 antibody HER3-29 of PCT/CN2021/123733) are as follows:
The light chain of Ab3: 
DIQMTQSPSSLSASIGDRATITCRASQHVGTYLNWYQQKPGKTPKLLISGAANL
QSGVPSRFSGSGSGTDFTLTISSLQPEDFATYYCQQSYNTPPFSFGQGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
SEQ ID NO: 5
The heavy chain of Ab3:

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
SEQ ID NO: 6

The sequence of a claudin18.2 full-length plasmid for constructing NUGC4 cell strains highly and moderately expressing Claudin18.2 is as follows:

MAVTACQGLGFVVSLIGIAGIIAATCMDQWSTQDLYNNPVTAVFNYQGLWRSCV
RESSGFTECRGYFTLLGLPAMLQAVRALMIVGIVLGAIGLLVSIFALKCIRIGSME
DSAKANMTLTSGIMFIVSGLCAIAGVSVFANMLVTNFWMSTANMYTGMGGMV
QTVQTRYTFGAALFVGWVAGGLTLIGGVMMCIACRGLAPEETNYKAVSYHASG
HSVAYKPGGFKASTGFGSNTKNKKIYDGGARTEDEVQSYPSKHDYV,
SEQ ID NO: 7

### II. Compound Examples

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument or Bruker AVANCE NEO 500M, with dimethyl sulfoxide-D6 (DMSO-d₆), chloroform-D (CDCl₃), and methanol-D4 (CD₃OD) as solvents and tetramethylsilane (TMS) as an internal standard.

The MS analyses were performed using Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters, MS model: waters ACQuity Qda Detector/waters SQ Detector), and THERMO Ultimate 3000-Q Exactive (manufacturer: THERMO, MS model: THERMO Q Exactive).

The high performance liquid chromatography (HPLC) analyses were performed on Agilent HPLC 1200DAD, Agilent HPLC 1200VWD, and Waters HPLC e2695-2489 high-pressure liquid chromatographs.

The chiral HPLC analyses were performed on an Agilent 1260 DAD high performance liquid chromatograph.

The preparative high-performance liquid chromatography purifications were performed using Waters 2545-2767, Waters 2767-SQ Detecor2, Shimadzu LC-20AP, and Gilson GX-281 preparative chromatographs.

The preparative chiral chromatography purifications were performed on a Shimadzu LC-20AP preparative chromatograph.

The CombiFlash preparative flash chromatograph used was Combiflash Rf200 (TELEDYNE ISCO).

In the thin-layer chromatography (TLC) analyses, Yantai Huanghai HSGF254 or Qingdao GF254 silica gel plates with 0.15 mm-0.2 mm layer thickness were used, and in the TLC separations and purifications, plates with 0.4 mm-0.5 mm layer thickness were used.

In the silica gel column chromatography purifications, 200-300 mesh silica gels (Huanghai, Yantai) were generally used as carriers.

The kinase mean inhibition rates and the IC₅₀ values were measured with a NovoStar microplate reader (BMG, Germany).

Known starting materials in the present disclosure may be synthesized by using or following methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator or an HC2-SS hydrogenator.

The hydrogenation reactions generally involved 3 vacuumization and hydrogen filling cycles.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The reaction progress was monitored by thin-layer chromatography (TLC) in the examples. The developing solvents used in the reactions, the eluent systems used in the column chromatography purifications, and the developing solvent systems used in the thin-layer chromatography include: A: dichloromethane/methanol systems, and B: n-hexane/ethyl acetate systems; the volume ratio of the solvents was adjusted depending on the polarity of a compound or by adding a small amount of a basic or acidic reagent such as triethylamine and acetic acid.

### Example 2-1 1

### (S)-7-Methoxy-8-((5-(((S)-7-methoxy-2-(4-(4-methylpiperazin-1-yl)phenyl)-5-oxo-5,10, 11,11a-tetrahydro-1H-benzo[e]pyrrolo[1,2-a][1,4]diazepin-8-yl)oxy)pentyl)oxy)-1,11a-dihydro-3H,5H-spiro[benzo[e]pyrrolo[1,2-a][1,4]diazepine-2,1'-cyclopropan] -5-one 1

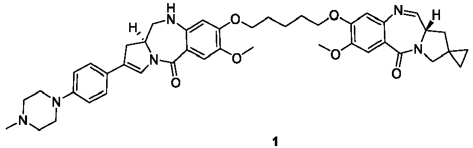

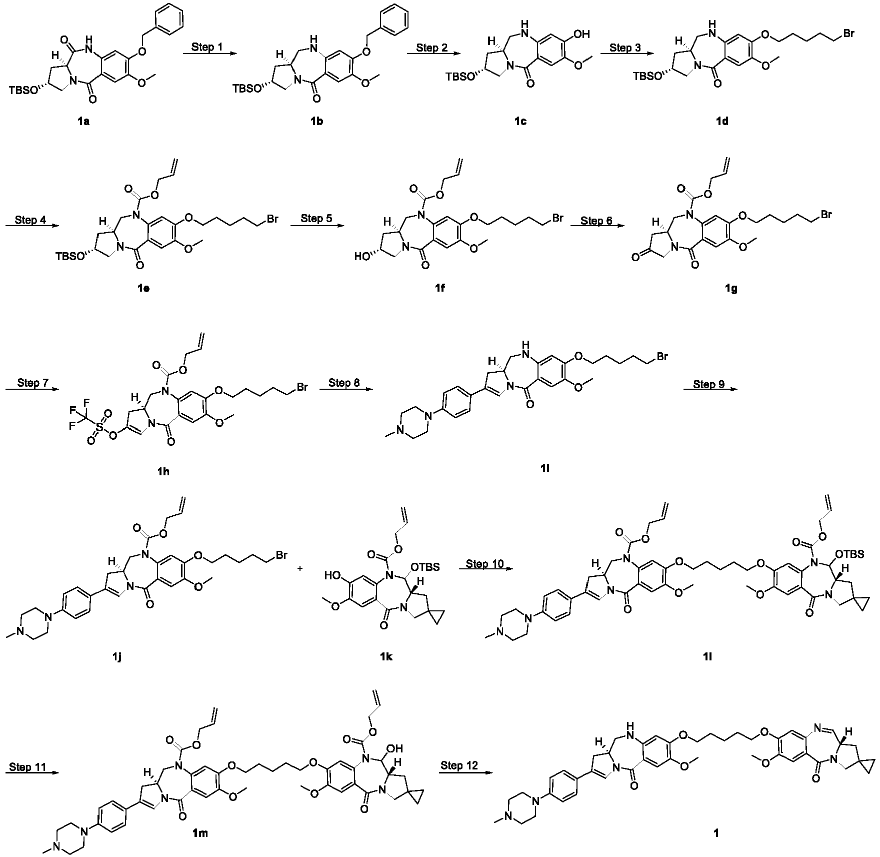

### Step 1

### (2R,11aS)-8-(Benzyloxy)-2-((tert-butyldimethylsilyl)oxy)-7-methoxy-1,2,3,10,11,11a-h exahydro-5H-benzo[e]pyrrolo[1,2-a][1,4]diazepin-5-one 1b

(2*R*,11*aS*)-8-(Benzyloxy)-2-((tert-butyldimethylsilyl)oxy)-7-methoxy-1,2,3,11*a*-tetrahy dro-5*H*-benzo[*e*]pyrrolo[1,2*-a*][1,4]diazepine-5,11(10*H*)-dione **1a** (8.0 g, 16.58 mmol, prepared by the method disclosed for compound 16 of Example 3 on page 75 of the specification of the patent application "WO2016/149546 A1") was placed in a three-necked reaction flask and dissolved with anhydrous tetrahydrofuran (50 mL). The reaction system was purged with nitrogen gas 3 times and cooled in an ice-water bath to 0-5 °C, and borane-tetrahydrofuran complex (1 M, 163 mL, 163 mmol) was added dropwise with vigorous stirring. After the addition, the ice-water bath was removed, and the mixture was warmed to room temperature and stirred for 2 h. The mixture was cooled in an ice-water bath to 0-5 °C, and absolute methanol (200 mL) was slowly added dropwise with stirring to quench the reaction. After the addition, the ice-water bath was removed, and the mixture was warmed to room temperature and stirred for another 2 h. The organic solvent was removed by concentration under reduced pressure, and water (100 mL) was added to the residue. Extraction was performed with dichloromethane (60 mL × 3). The organic phases were combined, washed with saturated sodium chloride solution (80 mL), dried over anhydrous sodium sulfate, and filtered. Then the filtrate was concentrated under reduced pressure, and the resulting residue was purified by silica gel column chromatography with eluent system B to give the title product **1b** (5.6 g, yield: 72.1%).

MS m/z (ESI): 469.2 [M+1].

### Step 2

### (2R,11aS)-2-((tert-Butyldimethylsilyl)oxy)-8-hydroxy-7-methoxy-1,2,3,10,11,11a-hexa hydro-5H-benzo[e]pyrrolo[1,2-a][1,4]diazepin-5-one 1c

**1b** (4.6 g, 9.81 mmol) was dissolved in anhydrous tetrahydrofuran (100 mL), and palladium on carbon (920 mg, content 10%, dry) was added. The reaction system was purged with hydrogen gas three times, heated to 45 °C, and stirred for 3 h. The reaction mixture was filtered. The filter cake was rinsed with anhydrous tetrahydrofuran (50 mL), and the filtrate was concentrated under reduced pressure and dried *in vacuo* to give a crude product of the title product **1c** (3.6 g, yield: 96.9%). The product was directly used in the next step without purification.

MS m/z (ESI): 379.2 [M+1].

### Step 3

### (2R,11a,S)-8-((5-Bromopentyl)oxy)-2-((tert-butyldimethylsilyl)oxy)-7-methoxy-1,2,3,10 ,11,11a-hexahydro-5H-benzo[e]pyrrolo[1,2-a][1,4]diazepin-5-one 1d

Crude **1c** (1.7 g, 4.50 mmol) was dissolved in *N,N-*dimethylformamide (20 mL), and 1,5-dibromopentane (5.2 g, 22.62 mmol) and anhydrous potassium carbonate (744 mg, 5.39 mmol) were added. The mixture was stirred at room temperature for 5 h. Water (100 mL) was added to the reaction mixture, and extraction was performed with ethyl acetate (60 mL × 3). The organic phases were combined, washed with water (50 mL × 3) and saturated sodium chloride solution (50 mL) in sequence, dried over anhydrous sodium sulfate, and filtered. Then the filtrate was concentrated under reduced pressure, and the resulting residue was purified by silica gel column chromatography with eluent system B to give the title product **1d** (1.7 g, yield: 71.7%).

MS m/z (ESI): 527.2 [M+1].

### Step 4

### Allyl

### (2R,11aS)-8-((5-bromopentyl)oxy)-2-((tert-butyldimethylsilyl)oxy)-7-methoxy-5-oxo-2, 3,11,11a-tetrahydro-1H-benzo[e]pyrrolo[1,2-a][1,4]diazepine-10(5H)-carboxylate 1e

**1d** (1.70 g, 3.23 mmol) was dissolved in anhydrous dichloromethane (30 mL), and the solution was cooled in an ice-water bath. Pyridine (440 mg, 5.56 mmol) and allyl chloroformate (1.34 g, 11.12 mmol) were sequentially added, and the mixture was stirred for 20 min in the ice bath. The reaction mixture was diluted with dichloromethane (30 mL), and a 10% aqueous citric acid solution (20 mL) was then added. After two minutes of stirring, the mixture was left to stand, and it separated into layers. The organic phase was kept. The organic phase was washed with saturated sodium chloride solution (20 mL), dried over anhydrous sodium sulfate, and filtered. Then the filtrate was concentrated under reduced pressure, and the resulting residue was purified by silica gel column chromatography with eluent system B to give the title product **1e** (1.89 g, yield: 95.8%).

MS m/z (ESI): 611.3 [M+1].

### Step 5

### Allyl

### (2R,11aS)-8-((5-bromopentyl)oxy)-2-hydroxy-7-methoxy-5-oxo-2,3,11,11a-tetrahydro-1H-benzo[e]pyrrolo[1,2-a][1,4]diazepine-10(5H)-carboxylate 1f

**1e** (1.89 g, 3.10 mmol) was dissolved in anhydrous tetrahydrofuran (30 mL), and the solution was cooled in an ice-water bath. A solution of tetrabutylammonium fluoride in tetrahydrofuran (1 M, 6.2 mL, 6.20 mmol) was added. After the addition, the ice-water bath was removed, and the mixture was stirred at room temperature for 20 min. The reaction mixture was diluted with ethyl acetate (60 mL) and washed with saturated aqueous ammonium chloride solution (30 mL × 5) and saturated aqueous sodium chloride solution (30 mL × 2) in sequence. The organic phase was dried over anhydrous sodium sulfate and filtered. Then the filtrate was concentrated under reduced pressure, and the resulting residue was purified by silica gel column chromatography with eluent system A to give the title product **1f** (1.39 g, yield: 90.4%).

MS m/z (ESI): 497.1 [M+1].

### Step 6

### Allyl

### (S)-8-((5-bromopentyl)oxy)-7-methoxy-2,5-dioxo-2,3,11,11a-tetrahydro-1H-benzo[e]py rrolo[1,2-a][1,4]diazepine-10(5H)-carboxylate 1g

**1f** (1.39 g, 2.80 mmol) was dissolved in anhydrous dichloromethane (30 mL), and the solution was cooled in an ice-water bath. Sodium bicarbonate (1.41 g, 16.78 mmol) and the Dess-Martin oxidant (3.56 g, 8.39 mmol) were sequentially added. After the addition, the ice-water bath was removed, and the mixture was warmed to room temperature and stirred for 4 h. The reaction mixture was quenched with saturated aqueous sodium bicarbonate (30 mL) and extracted with dichloromethane (30 mL × 3). The organic phases were combined, washed with saturated sodium chloride solution (30 mL), dried over anhydrous sodium sulfate, and filtered. Then the filtrate was concentrated under reduced pressure, and the resulting residue was purified by silica gel column chromatography with eluent system B to give the title product **1g** (1.14 g, yield: 82.4%).

MS m/z (ESI): 495.1 [M+1].

### Step 7

### Allyl

### (S)-8-((5-bromopentyl)oxy)-7-methoxy-5-oxo-2-(((trifluoromethyl)sulfonyl)oxy)-11,11 a-dihydro-1H-benzo[e]pyrrolo[1,2-a][1,4]diazepine-10(5H)-carboxylate 1h

**1g** (960 mg, 1.94 mmol) was dissolved in anhydrous dichloromethane (20 mL), and the solution was cooled in an acetonitrile-dry ice bath to -40 °C. 2,6-Lutidine (624 mg, 5.83 mmol) was added dropwise, and the mixture was stirred for 5 min. Trifluoromethanesulfonic anhydride (1.09 g, 3.88 mmol) was then added. After the addition, the mixture was stirred at -40 °C for another 1 h. The reaction mixture was quenched with saturated aqueous ammonium chloride (20 mL) and extracted with dichloromethane (30 mL × 3). The organic phases were combined, washed with saturated sodium chloride solution (30 mL), dried over anhydrous sodium sulfate, and filtered. Then the filtrate was concentrated under reduced pressure, and the resulting residue was purified by silica gel column chromatography with eluent system B to give the title product **1h** (764 mg, yield: 62.9%).

MS m/z (ESI): 627.0 [M+1].

### Step 8

### (S)-8-((5-Bromopentyl)oxy)-7-methoxy-2-(4-(4-methylpiperazin-1-yl)phenyl)-1,10,11,1 1a-tetrahydro-5H-benzo[e]pyrrolo[1,2-a][1,4]diazepin-5-one 1i

**1h** (188 mg, 0.30 mmol) was dissolved in tetrahydrofuran (5 mL), and 4-(4-methyl-1-piperazinyl)phenylboronic acid (198 mg, 0.90 mmol, Accela ChemBio), [1,1-bis(diphenylphosphino)ferrocene]palladium(II) dichloride (66 mg, 0.09 mmol, adamas), potassium carbonate (207 mg, 1.50 mmol), and water (0.5 mL) were sequentially added. The reaction system was purged with nitrogen gas three times and stirred at room temperature for 12 h. The reaction mixture was diluted with dichloromethane (50 mL), dried over anhydrous sodium sulfate, and filtered. Then the filtrate was concentrated under reduced pressure, and the resulting residue was purified by silica gel column chromatography with eluent system A to give a mixture containing the title product **1i.** The resulting mixture was dissolved in anhydrous dichloromethane (5 mL), and tetrakis(triphenylphosphine)palladium(0) (104 mg, 0.09 mmol) and p-toluenesulfonic acid (155 mg, 0.90 mmol) were added. The reaction system was purged with nitrogen gas three times and stirred at room temperature for 12 h. The reaction mixture was concentrated under reduced pressure, and the resulting residue was purified by silica gel column chromatography with eluent system A to give the title product **1i** (120 mg, yield: 70.3%).

MS m/z (ESI): 569.2 [M+1].

### Step 9

### Allyl

### (S)-8-((5-bromopentyl)oxy)-7-methoxy-2-(4-(4-methylpiperazin-1-yl)phenyl)-5-oxo-11, 11a-dihydro-1H-benzo[e]pyrrolo[1,2-a][1,4]diazepine-10(5H)-carboxylate 1j

**1i** (120 mg, 0.21 mmol) was dissolved in anhydrous dichloromethane (3 mL), and the solution was cooled in an ice-water bath. Pyridine (50 mg, 0.63 mmol) and allyl chloroformate (102 mg, 0.85 mmol) were sequentially added, and the mixture was stirred for 20 min in the ice bath. Dichloromethane (30 mL) was added for dilution, and saturated aqueous ammonium chloride (20 mL) was then added. After two minutes of stirring, the mixture was left to stand, and it separated into layers. The organic phase was kept, and the aqueous phase was extracted with dichloromethane (30 mL × 3). The organic phases were combined. The organic phase was washed with saturated sodium chloride solution (20 mL), dried over anhydrous sodium sulfate, and filtered. Then the filtrate was concentrated under reduced pressure, and the resulting residue was purified by silica gel column chromatography with eluent system A to give the title product **1j** (66 mg, yield: 47.9%).

MS m/z (ESI): 653.4 [M+1].

### Step 10

### Allyl

### (11aS)-8-((5-(((S)-10-((allyloxy)carbonyl)-7-methoxy-2-(4-(4-methylpiperazin-1-yl)phe nyl)-5-oxo-5,10,11,11a-tetrahydro-1H-benzo[e]pyrrolo[1,2-a][1,4]diazepin-8-yl)oxy)pe ntyl)oxy)-11-((tert-butyldimethylsilyl)oxy)-7-methoxy-5-oxo-11,11a-dihydro-1H,3H-sp iro[benzo[e]pyrrolo[1,2-a][1,4]diazepine-2,1'-cyclopropane]-10(5H)-carboxylate 1l

**1j** (66 mg, 0.10 mmol) was dissolved in N,N-dimethylformamide (3 mL), and allyl (11*aS*)-11-((tert-butyldimethylsilyl)oxy)-8-hydroxy-7-methoxy-5-oxo-11,11*a*-dihydro-1 *H*,3*H*-spiro[benzo[*c*]pyrrolo[1,2-*a*][1,4]diazepine-2,1'-cyclopropane]-10(5*H*)-carboxylat e **1k** (66 mg, 0.12 mmol, prepared by the method disclosed in Example 45 on page 223 of the specification of the patent application "CN 111164208 A") was added. Then anhydrous potassium carbonate (56 mg, 0.41 mmol) was added, and the mixture was stirred at room temperature for 12 h. Water (20 mL) was added to the reaction mixture, and extraction was performed with ethyl acetate (20 mL × 3). The organic phases were combined, washed with water (20 mL × 3) and saturated sodium chloride solution (20 mL) in sequence, dried over anhydrous sodium sulfate, and filtered. Then the filtrate was concentrated under reduced pressure, and the resulting residue was purified by thin-layer chromatography with developing solvent system A to give the title product **1l** (50 mg, yield: 46.7%).

MS m/z (ESI): 1061.7 [M+1].

### Step 11

### Allyl

### (11aS)-8-((5-(((S)-10-((allyloxy)carbonyl)-7-methoxy-2-(4-(4-methylpiperazin-1-yl)phe nyl)-5-oxo-5,10,11,11a-tetrahydro-1H-benzo[e]pyrrolo[1,2-a][1,4]diazepin-8-yl)oxy)pe ntyl)oxy)-11-hydroxy-7-methoxy-5-oxo-11,11a-dihydro-1H,3H-spiro[benzo[e]pyrrolo[ 1,2-a][1,4]diazepine-2,1'-cyclopropane]-10(5H)-carboxylate 1m

**1l** (50 mg, 0.047 mmol) was dissolved in anhydrous tetrahydrofuran (3 mL), and glacial acetic acid (17 mg, 0.28 mmol) and a solution of tetrabutylammonium fluoride in tetrahydrofuran (1 M, 0.24 mL, 0.24 mmol) were sequentially added. After the addition, the mixture was stirred at room temperature for 2 h. The reaction mixture was diluted with ethyl acetate (30 mL) and washed with saturated aqueous sodium bicarbonate (20 mL × 2) and saturated aqueous sodium chloride (20 mL × 2) in sequence. The organic phase was dried over anhydrous sodium sulfate and filtered. Then the filtrate was concentrated under reduced pressure, and the resulting residue was purified by thin-layer chromatography with developing solvent system A to give the title product **1m** (15 mg, yield: 33.6%).

MS m/z (ESI): 947.9 [M+1].

### Step 12

(*S*)-7-Methoxy-8-((5-(((*S*)-7-methoxy-2-(4-(4-methylpiperazin-1-yl)phenyl)-5-oxo-5,10, 11,11*a*-tetrahydro-1*H*-benzo[*e*]pyrrolo[1,2-*a*][1,4]diazepin-8-yl)oxy)pentyl)oxy)-1,11*a-*dihydro-3*H*,5*H*-spiro[benzo[*e*]pyrrolo[1,2-*a*][1,4]diazepine-2,1'-cyclopropan]-5-one **1 1m** (15 mg, 0.016 mmol) was dissolved in anhydrous dichloromethane (2 mL), and pyrrolidine (11.5 mg, 0.16 mmol) and tetrakis(triphenylphosphine)palladium(0) (1.85 mg, 0.0016 mmol) were sequentially added. The mixture was stirred for 30 min in a nitrogen atmosphere. The organic solvent was removed by concentration under reduced pressure, and the resulting residue was purified by thin-layer chromatography with developing solvent system A to give the title product **1** (10 mg, yield: 83.0%).

MS m/z (ESI): 761.4 [M+1].

¹H NMR (500 MHz, DMSO-*d*₆) δ 7.92 (d, 1H), 7.42-7.40 (m, 1H), 7.36-7.29 (m, 4H), 7.22-7.18 (m, 2H), 6.94 (d, 2H), 6.85 (s, 1H), 6.64 (d, 1H), 6.52(d, 1H), 6.32 (s, 1H), 5.32 (t, 1H), 4.20-4.17 (m, 1H), 4.14-4.09 (m, 2H), 4.04-4.00 (m, 1H), 3.97-3.93 (m, 2H), 3.84-3.78 (m, 4H), 3.67 (s, 3H), 3.58-3.48 (m, 5H), 2.78-2.75 (m, 1H), 2.74-2.72 (m, 1H), 2.66-2.63 (m, 2H), 2.38-2.36 (m, 2H), 1.85-1.79 (m, 4H), 1.61-1.58 (m, 2H), 1.49-1.44 (m, 3H), 0.88-0.84 (m, 4H).

### Example 2-2 2

### (S)-7-Methoxy-8-((5-(((S)-7-methoxy-2-(4-morpholinylphenyl)-5-oxo-5,10,11,11a-tetra hydro-1H-benzo[e]pyrrolo[1,2-α][1,4]diazepin-8-yl)oxy)pentyl)oxy)-1,11a-dihydro-3H, 5H-spiro[benzo[e]pyrrolo[1,2-a][1,4]diazepine-2,1'-cyclopropan]-5-one 2

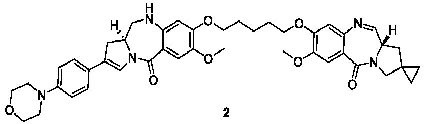

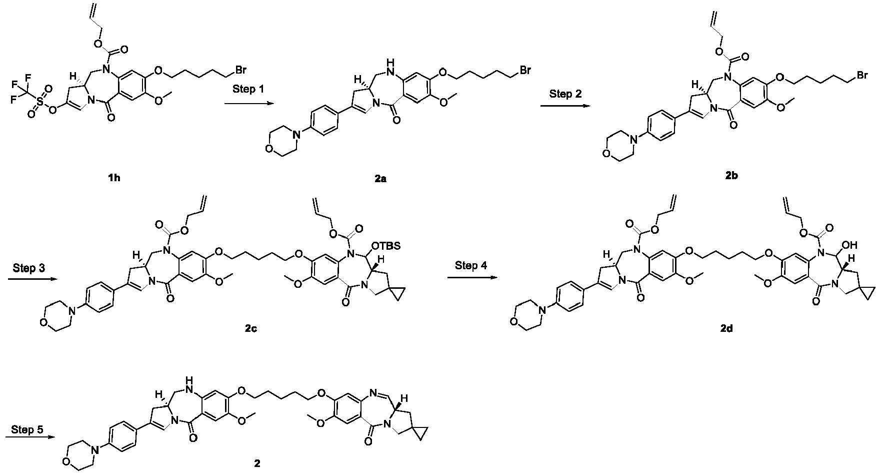

### Step 1

### (S)-8-((5-Bromopentyl)oxy)-7-methoxy-2-(4-morpholinylphenyl)-1,10,11,11a-tetrahydr o-5H-benzo[e]pyrrolo[1,2-a][1,4]diazepin-5-one 2a

The title product **2a** (42 mg, yield: 23.7%) was obtained by replacing the starting material 4-(4-methyl-1-piperazinyl)phenylboronic acid of step 8 of Example 2-1 1 with (4-morpholinylphenyl)phenylboronic acid and using the same conditions.

MS m/z (ESI): 556.4 [M+1].

### Step 2

### Allyl

### (S)-8-((5-bromopentyl)oxy)-7-methoxy-2-(4-morpholinylphenyl)-5-oxo-11,11a-dihydro -1H-benzo[e]pyrrolo[1,2-a][1,4]diazepine-10(5H)-carboxylate 2b

The title product **2b** (45 mg, yield: 93.1%) was obtained by replacing the starting material **1i** of step 9 of Example 2-1 1 with **2a** (42 mg, 0.075 mmol) and using the same reaction conditions.

MS m/z (ESI): 640.2 [M+1].

### Step 3

### Allyl

### (11aS)-8-((5-(((S)-10-((allyloxy)carbonyl)-7-methoxy-2-(4-morpholinylphenyl)-5-oxo-5 ,10,11,11a-tetrahydro-1H-benzo[e]pyrrolo[1,2-a][1,4]diazepin-8-yl)oxy)pentyl)oxy)-11-((tert-butyldimethylsilyl)oxy)-7-methoxy-5-oxo-11,11a-dihydro-1H,3H-spiro[benzo[e]p yrrolo[1,2-a][1,4]diazepine-2,1'-cyclopropane]-10(5H)-carboxylate 2c

A crude product of the title product **2c** (89 mg) was obtained by replacing the starting material **1j** of step 10 of Example 2-1 1 with **2b** (45 mg, 0.07 mmol) and using the same reaction conditions without purification, and the product was directly used in the next step without purification.

MS m/z (ESI): 1048.4 [M+1].

### Step 4

### Allyl

### (11aS)-8-((5-(((S)-10-((allyloxy)carbonyl)-7-methoxy-2-(4-morpholinylphenyl)-5-oxo-5 ,10,11,11a-tetrahydro-1H-benzo[e]pyrrolo[1,2-a][1,4]diazepin-8-yl)oxy)pentyl)oxy)-11-hydroxy-7-methoxy-5-oxo-11,11a-dihydro-1H,3H-spiro[benzo[e]pyrrolo[1,2-a][1,4]dia zepine-2,1'-cyclopropane]-10(5H)-carboxylate 2d

The title product **2d** (45 mg, yield: 68.4%) was obtained by replacing the starting material **1l** of step 11 of Example 2-1 1 with crude **2c** (89 mg) and using the same reaction conditions.

MS m/z (ESI): 934.4 [M+1].

### Step 5

### (S)-7-Methoxy-8-((5-(((S)-7-methoxy-2-(4-morpholinylphenyl)-5-oxo-5,10,11,11a-tetra hydro-1H-benzo[e]pyrrolo[1,2-a][1,4]diazepin-8-yl)oxy)pentyl)oxy)-1,11a-dihydro-3H, 5H-spiro[benzo[e]pyrrolo[1,2-a][1,4]diazepine-2,1'-cyclopropan]-5-one 2

The title product **2** (22 mg, yield: 91.6%) was obtained by replacing the starting material **1m** of step 12 of Example 2-1 1 with **2d** (30 mg, 0.032 mmol) and using the same reaction conditions.

MS m/z (ESI): 748.7 [M+1].

1H NMR (500 MHz, DMSO-d6) δ 7.92 (d, 1H), 7.41 (s, 1H), 7.34-7.31 (m, 2H), 7.07-7.05 (m, 1H), 6.94-6.90 (m, 2H), 6.86-6.83 (m, 1H), 6.59-6.56 (m, 1H), 6.33-6.30 (m, 1H), 4.21-4.15 (m, 2H), 4.14-4.09 (m, 1H), 4.04-4.01 (m, 1H), 3.96-3.91 (m, 2H), 3.82 (s, 3H), 3.75-3.73 (m, 4H), 3.70 (s, 3H), 3.63-3.59 (m, 2H), 3.57-3.50 (m, 2H), 3.27-3.23 (m, 2H), 3.13-3.10 (m, 4H), 2.77-2.72 (m, 1H), 2.44-2.42 (m, 1H), 2.03-1.98 (m, 2H), 1.84-1.81 (m, 2H), 1.60-1.56 (m, 2H), 0.88-0.83 (m, 2H), 0.69-0.64 (m, 2H).

### Example 2-3 3

### (S)-8-((5-(((S)-2-(4-(Cyclopropylthio)phenyl-7-methoxy-5-oxo-5,10,11,11a-tetrahydro-1H-benzo[e]pyrrolo[1,2-a][1,4]diazepin-8-yl)oxy)pentyl)oxy)-7-methoxy-1,11a-dihydr o-3H,5H-spiro[benzo[e]pyrrolo[1,2-a][1,4]diazepine-2,1'-cyclopropan]-5-one 3

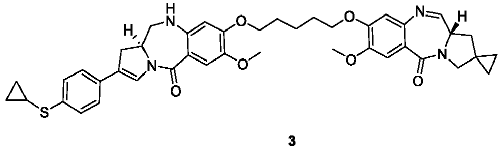

### Step 1

### ((4-Cyclopropylthio)phenyl)boronic acid

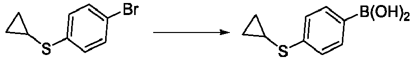

Tetrahydrofuran (1 mL) was placed in a reaction flask. The reaction system was purged with nitrogen gas three times and cooled in a dry ice-acetone bath to -78 °C, and a 2.5 M n-butyllithium solution (1.24 mL, 3.11 mmol) was added dropwise. After the dropwise addition, the mixture was stirred for 10 min. A solution of (4-bromophenyl)(cyclopropyl)sulfane (437 mg, 1.91 mmol, prepared by the well-known method "Bioorganic and Medicinal Chemistry, 2012, 20(14), 2982-2991") in 2 mL of tetrahydrofuran was added dropwise. After the dropwise addition, the mixture was stirred at -78 °C for another 1 h, and a solution of triisopropyl borate (488 mg, 2.59 mmol) in 2 mL of tetrahydrofuran was added dropwise. After the dropwise addition, the reaction mixture was warmed to room temperature and stirred for another 3 h. 10 mL of water was added to the reaction mixture, and the mixture was stirred for 30 min. Then 10 mL of 1 M hydrochloric acid was added, and the mixture was stirred for 30 min. The reaction mixture was concentrated under reduced pressure, extracted with ethyl acetate (10 mL × 3), and washed with saturated sodium chloride solution (20 mL). The organic phase was dried over anhydrous sodium sulfate and filtered. Then the filtrate was concentrated under reduced pressure, and the resulting residue was purified on a CombiFlash preparative flash chromatograph with eluent system B to give ((4-cyclopropylthio)phenyl)boronic acid (178 mg, yield: 48.1%).

MS m/z (ESI): 193.1 [M-1].

The title product 3 (20 mg, overall yield: 22.5%) was prepared by using the synthesis scheme of Example 2-1 1 and replacing the reagent 4-(4-methyl-1-piperazinyl)phenylboronic acid of step 8 with ((4-cyclopropylthio)phenyl)boronic acid.

MS m/z (ESI): 735.2 [M+1].

¹H NMR (500 MHz, DMSO-d6) δ 7.91 (d, 1H), 7.56 (s, 1H),7.42-7.37 (m, 2H), 7.34-7.28 (m, 3H), 7.05 (s, 1H), 6.85-6.81 (m, 1H), 6.59-6.54 (m, 1H), 6.34-6.30 (m, 1H), 4.24-4.08 (m, 2H), 4.05-3.99 (m, 1H), 3.97-3.91 (m, 2H), 3.83-3.77 (m, 2H), 3.72-3.63 (m, 4H), 3.61-3.45 (m, 3H), 3.28-3.21 (m, 2H), 2.76 (dd, 1H), 2.45-2.39 (m, 1H), 2.33-2.24 (m, 2H), 2.07-1.95 (m, 2H), 1.86-1.76 (m, 3H), 1.63-1.52 (m, 2H), 1.35-1.32 (m, 1H), 1.10-1.05 (m, 2H), 0.87-0.81 (m, 1H), 0.70-0.53 (m, 4H).

### Example 2-4 4

### (S)-8-((5-(((S)-2-(4-1H-Pyrazol-1-yl)phenyl)-7-methoxy-5-oxo-5,10,11,11a-tetrahydro-1H-benzo[e]pyrrolo[1,2-a][1,4]diazepin-8-yl)oxy)pentyl)oxy)-7-methoxy-1,11a-dihydr o-3H,5H-spiro[benzo[e]pyrrolo[1,2-a][1,4]diazepine-2,1'-cyclopropan]-5-one 4

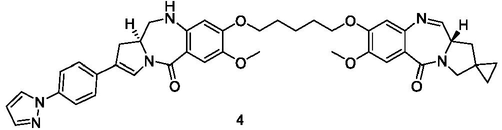

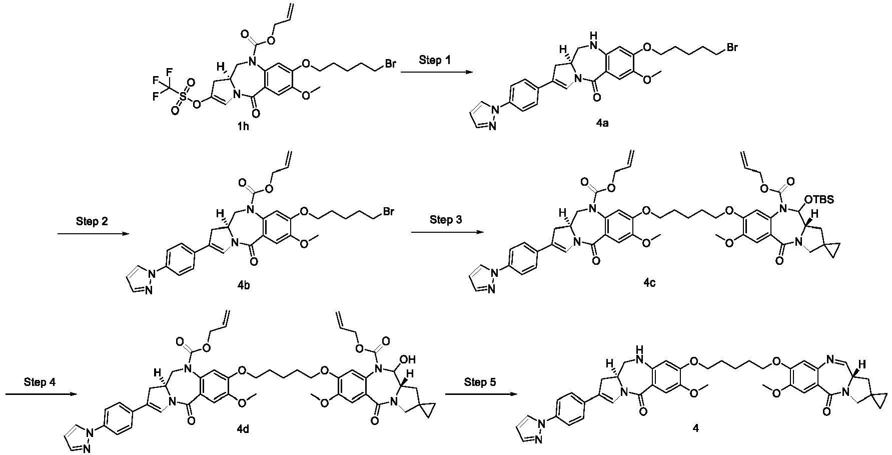

### Step 1

### (S)-2-(4-(1H-Pyrazol-1-yl)phenyl)-8-((5-bromopentyl)oxy)-7-methoxy-1,10,11,11a-tetr ahydro-5H-benzo[e]pyrrolo[1,2-a][1,4]diazepin-5-one 4a

The title product **4a** (80 mg, yield: 49.8%) was obtained by replacing the starting material 4-(4-methyl-1-piperazinyl)phenylboronic acid of step 8 of Example 2-1 1 with (4-(1*H*-pyrazol-1-yl)phenyl)boronic acid and using the same reaction conditions.

MS m/z (ESI): 537.1 [M+1].

### Step 2

### Allyl

### (S)-2-(4-(1H-pyrazol-1-yl)phenyl)-8-((5-bromopentyl)oxy)-7-methoxy-5-oxo-11,11a-di hydro-1H-benzo[e]pyrrolo[1,2-a][1,4]diazepine-10(5H)-carboxylate 4b

The title product **4b** (50 mg, yield: 80%) was obtained by replacing the starting material **1i** of step 9 of Example 2-1 1 with **4a** (54 mg, 0.10 mmol) and using the same reaction conditions.

MS m/z (ESI): 621.2 [M+1].

### Step 3

### Allyl

### (11aS)-8-((5-(((S)-2-(4-(1H-pyrazol-1-yl)phenyl)-10-((allyloxy)carbonyl)-7-methoxy-5-oxo-5,10,11,11a-tetrahydro-1H-benzo[c]pyrrolo[1,2-a][1,4]diazepin-8-yl)oxy)pentyl)ox y)-11-((tert-butyldimethylsilyl)oxy)-7-methoxy-5-oxo-11,11a-dihydro-1H,3H-spiro[ben zo[e]pyrrolo[1,2-a][1,4]diazepine-2,1'-cyclopropane]-10(5H)-carboxylate 4c

The title product **4c** (51 mg, yield: 74.6%) was obtained by replacing the starting material **1j** of step 10 of Example 2-1 1 with **4b** (50 mg, 0.08 mmol) and using the same reaction conditions.

MS m/z (ESI): 1029.5 [M+1].

### Step 4

### Allyl

### (11aS)-8-((5-(((S)-2-(4-(1H-pyrazol-1-yl)phenyl)-10-((allyloxy)carbonyl)-7-methoxy-5-oxo-5,10,11,11a-tetrahydro-1H-benzo[e]pyrrolo[1,2-a][1,4]diazepin-8-yl)oxy)pentyl)ox y)-11-hydroxy-7-methoxy-5-oxo-11,11a-dihydro-1H,3H-spiro[benzo[e]pyrrolo[1,2-a][1 ,4]diazepine-2,1'-cyclopropane]-10(5H)-carboxylate 4d

The title product **4d** (40 mg, yield: 87.5%) was obtained by replacing the starting material **1l** of step 11 of Example 2-1 1 with **4c** (51 mg, 0.05 mmol) and using the same reaction conditions.

MS m/z (ESI): 915.4 [M+1].

### Step 5

### (S)-8-((5-(((S)-2-(4-(1H-Pyrazol-1-yl)phenyl)-7-methoxy-5-oxo-5,10,11,11a-tetrahydro-1H-benzo[e]pyrrolo[1,2-a][1,4]diazepin-8-yl)oxy)pentyl)oxy)-7-methoxy-1,11a-dihydr o-3H,5H-spiro[benzo[e]pyrrolo[1,2-a][1,4]diazepine-2,1'-cyclopropan]-5-one 4

The title product **4** (25 mg, yield: 85.8%) was obtained by replacing the starting material **1m** of step 12 of Example 2-1 1 with **4d** (37 mg, 0.04 mmol) and using the same reaction conditions.

MS m/z (ESI): 729.3 [M+1].

¹H NMR (500 MHz, DMSO-d6) δ 8.08 (d, 1H), 7.97 (d, 2H), 7.92 (d, 1H), 7.73-7.67 (m, 2H), 7.64 (d, 2H), 7.33-7.32 (m, 1H), 6.86 (s, 1H), 6.64-6.59 (m, 1H), 6.49-6.41 (m, 2H), 5.34 (t, 1H), 4.28-4.24 (m, 2H), 4.15-4.11 (m, 1H), 4.05-4.01 (m, 2H), 3.97-3.91 (m, 4H), 3.82 (s, 3H), 3.66 (s, 3H), 3.61-3.54 (m, 1H), 2.86-2.82 (m, 1H), 2.66-2.64 (m, 1H), 2.38-2.35 (m, 1H), 2.06-1.96 (m, 1H), 1.87-1.81 (m, 4H), 1.63-1.59 (m, 2H), 0.72-0.59 (m, 4H).

### Example 2-5 5

### (S)-7-Methoxy-8-((5-(((S)-7-methoxy-5-oxo-2-(4-(tetrahydro-2H-pyran-4-yl)phenyl)-5, 10,11,11a-tetrahydro-1H-benzo[e]pyrrolo[1,2-a][1,4]diazepin-8-yl)oxy)pentyl)oxy)-1,1 1a-dihydro-3H,5H-spiro[benzo[e]pyrrolo[1,2-a][1,4]diazepine-2,1'-cyclopropan]-5-one 5

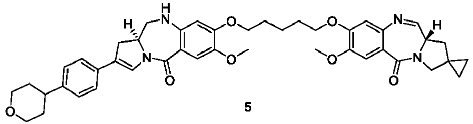

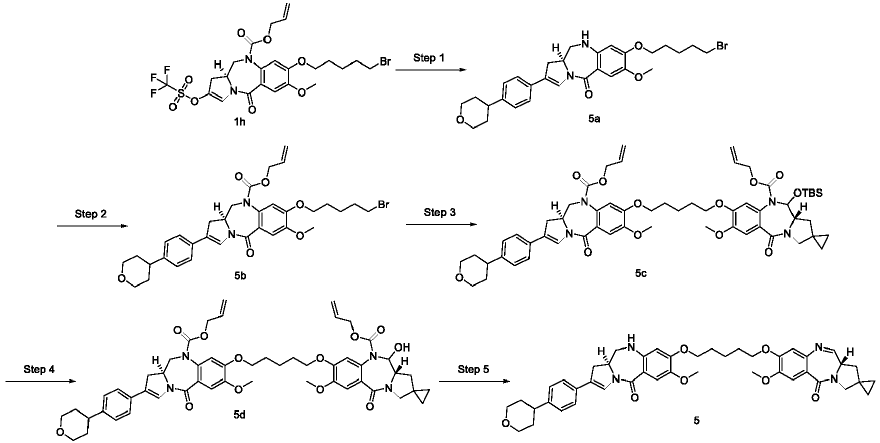

### Step 1

### (S)-8-((5-Bromopentyl)oxy)-7-methoxy-2-(4-(tetrahydro-2H-pyran-4-yl)phenyl)-1,10,11,11a-tetrahydro-5H-benzo[e]pyrrolo[1,2-a][1,4]diazepin-5-one 5a

The title product **5a** (116 mg, yield: 70%) was obtained by replacing the starting material 4-(4-methyl-1-piperazinyl)phenylboronic acid of step 8 of Example **2-1 1** with 4-(4-tetrahydropyranyl)phenylboronic acid pinacol ester and using the same reaction conditions.

MS m/z: 555.2 [M+1].

### Step 2

### Allyl (S)-8-((5-bromopentyl)oxy)-7-methoxy-5-oxo-2-(4-(tetrahydro-2H-pyran-4-yl)phenyl)-11,11a-dihydro-1H-benzo[c]pyrrolo[1,2-a][1,4]diazepine-10(5H)-carboxylate 5b

The title product **5b** (58 mg, yield: 90.1%) was obtained by replacing the starting material **1i** of step 9 of Example **2-1 1** with **5a** and using the same reaction conditions. MS m/z: 639.2 [M+1].

### Step 3

### Allyl

### (11aS)-8-((5-(((S)-10-((allyloxy)carbonyl)-7-methoxy-5-oxo-2-(4-(tetrahydro-2H-pyran -4-yl)phenyl)-5,10,11,1 1a-tetrahydro-1H-benzo[e]pyrrolo[1,2-a][1,4] diazepin-8-yl)oxy)pentyl)oxy)-11-((tert-butyldimethylsilyl)oxy)-7-methoxy-5-oxo-11,11a-dihydro-1H,3H-spiro[benzo[e]pyrrolo[1,2-a][1,4]diazepine-2,1'-cyclopropane]-10(5H)-carboxylate 5c

The title product 5c (57 mg, yield: 60%) was obtained by replacing the starting material **1j** of step 10 of Example **2-1 1** with **5b** and using the same reaction conditions.

MS m/z: 1047.5 [M+1].

### Step 4

### Allyl (11aS)-8-((5-(((S)-10-((allyloxy)carbonyl)-7-methoxy-5-oxo-2-(4-(tetrahydro-2H-pyran-4-yl)phenyl)-5,10,11,11a-tetrahydro-1H-benzo[e]pyrrolo[1,2-a] [1,4]diazepin-8-yl)oxy)pentyl)oxy)-11-hydroxy-7-methoxy-5-oxo-11,11a-dihydro-1H,3H-spiro[benzo[e]pyrrolo[1,2-a][1,4]diazepine-2,1'-cyclopropane]-10(5H)-carboxylate 5d

The title product **5d** (37 mg, yield: 80%) was obtained by replacing the starting material **1l** of step 11 of Example **2-1 1** with **5c** and using the same reaction conditions.

MS m/z: 933.4 [M+1].

### Step 5

### (S)-7-Methoxy-8-((5-(((S)-7-methoxy-5-oxo-2-(4-(tetrahydro-2H-pyran-4-yl)phenyl)-5, 10,11,11a-tetrahydro-1H-benzo[e]pyrrolo[1,2-a][1,4]diazepin-8-yl)oxy)pentyl)oxy)-1,1 1a-dihydro-3H,5H-spiro[benzo[e]pyrrolo[1,2-a][1,4]diazepine-2,1'-cyclopropan]-5-one 5

The title product **5** (24 mg, yield: 80%) was obtained by replacing the starting material **1m** of step 12 of Example **2-1 1** with **5d** and using the same reaction conditions.

MS m/z: 747.4 [M+1].

¹H NMR (500 MHz, DMSO-d6) δ 7.90 (d, 1H), 7.52 (s, 1H), 7.36 (d, 2H), 7.32 (s, 1H), 7.28 (s, 1H), 7.21 (d, 2H), 6.83 (s, 1H), 6.31 (s, 1H), 5.31 (t, 1H), 4.22-4.17 (m, 2H), 4.13-4.08 (m, 1H), 4.03-3.99 (m, 2H), 3.97-3.90 (m, 5H), 3.81 (s, 3H), 3.65 (s, 3H), 3.56-3.46 (m, 4H), 2.80-2.70 (m, 2H), 2.65-2.62 (m, 1H), 2.36-2.34 (m, 1H), 2.07-1.93 (m, 5H), 1.84-1.78 (m, 4H), 1.61-1.53 (m, 2H), 0.80-0.61 (m, 4H).

### Example 2-6 6

### (S)-7-Methoxy-8-((5-(((S)-7-methoxy-2-(4-(1-methylpiperidin-4-yl)phenyl)-5-oxo-5,10, 11,11a-tetrahydro-1H-benzo[e]pyrrolo[1,2-a][1,4]diazepin-8-yl)oxy)pentyl)oxy)-1,11a-dihydro-3H,5H-spiro[benzo[e]pyrrolo[1,2-a][1,4]diazepine-2,1'-cyclopropan]-5-one 6

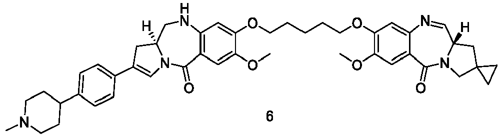

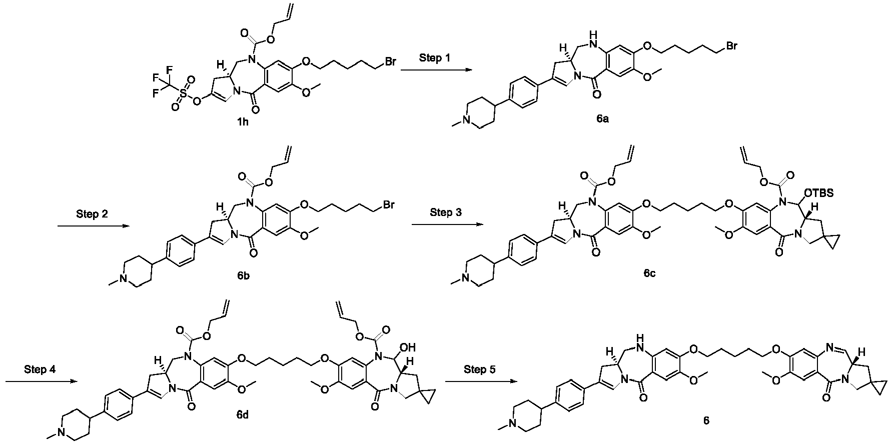

### Step 1

### (S)-8-((5-Bromopentyl)oxy)-7-methoxy-2-(4-(1-methylpiperidin-4-yl)phenyl)-1,10,11,1 1a-tetrahydro-5H-benzo[e]pyrrolo[1,2-a][1,4]diazepin-5-one 6a

The title product **6a** (51 mg, yield: 30%) was obtained by replacing the starting material 4-(4-methyl-1-piperazinyl)phenylboronic acid of step 8 of Example 2-1 1 with (4-(1-methylpiperidin-4-yl)phenyl)boronic acid and using the same reaction conditions. MS m/z (ESI): 568.2 [M+1].

### Step 2

### Allyl

### (S)-8-((5-bromopentyl)oxy)-7-methoxy-2-(4-(1-methylpiperidin-4-yl)phenyl)-5-oxo-11, 1 1a-dihydro-1H-benzo[e]pyrrolo[1,2-a][1,4]diazepine-10(5H)-carboxylate 6b

The title product **6b** (52 mg, yield: 89%) was obtained by replacing the starting material 1i of step 9 of Example 2-1 1 with **6a** (51 mg, 0.09 mmol) and using the same reaction conditions.

MS m/z (ESI): 652.2 [M+1].

### Step 3

### Allyl

### (11aS)-8-((5-(((S)-10-((allyloxy)carbonyl)-7-methoxy-2-(4-(1-methylpiperidin-4-yl)phe nyl)-5-oxo-5,10,11,11a-tetrahydro-1H-benzo[e]pyrrolo[1,2-a][1,4]diazepin-8-yl)oxy)pe ntyl)oxy)-11-((tert-butyldimethylsilyl)oxy)-7-methoxy-5-oxo-11,11a-dihydro-1H,3H-sp iro[benzo[e]pyrrolo[1,2-a][1,4]diazepine-2,1'-cyclopropane]-10(5H)-carboxylate 6c

The title product **6c** (42 mg, yield: 50%) was obtained by replacing the starting material 1j of step 10 of Example 2-1 1 with **6b** (52 mg, 0.08 mmol) and using the same reaction conditions.

MS m/z (ESI): 1060.5 [M+1].

### Step 4

### Allyl

### (11aS)-8-((5-(((S)-10-((allyloxy)carbonyl)-7-methoxy-2-(4-(1-methylpiperidin-4-yl)phe nyl)-5-oxo-5,10,11,11a-tetrahydro-1H-benzo[e]pyrrolo[1,2-a][1,4]diazepin-8-yl)oxy)pe ntyl)oxy)-11-hydroxy-7-methoxy-5-oxo-11,11a-dihydro-1H,3H-spiro[benzo[e]pyrrolo[ 1,2-a][1,4]diazepine-2,1'-cyclopropane]-10(5H)-carboxylate 6d

The title product **6d** (30 mg, yield: 80%) was obtained by replacing the starting material **1l** of step 11 of Example 2-1 1 with **6c** (42 mg, 0.04 mmol) and using the same reaction conditions.

MS m/z (ESI): 946.5 [M+1].

### Step 5

### (S)-7-Methoxy-8-((5-(((S)-7-methoxy-2-(4-(1-methylpiperidin-4-yl)phenyl)-5-oxo-5,10, 11,11a-tetrahydro-1H-benzo[e]pyrrolo[1,2-a][1,4]diazepin-8-yl)oxy)pentyl)oxy)-1,11a-dihydro-3H,5H-spiro[benzo[e]pyrrolo[1,2-a][1,4]diazepine-2,1'-cyclopropan] -5-one 6 The title product 6 (19 mg, yield: 80%) was obtained by replacing the starting material 1m of step 12 of Example 2-1 1 with 6d (30 mg, 0.032 mmol) and using the same reaction conditions.

MS m/z (ESI): 760.4 [M+1].

¹H NMR (500 MHz, DMSO-d6) δ 7.92 (d, 1H), 7.55 (s, 1H), 7.39 (d, 2H), 7.34 (s, 1H), 7.30 (s, 1H), 7.21 (d, 2H), 6.85 (s, 1H), 6.33 (s, 1H), 5.33 (t, 1H), 4.25-4.19 (m, 2H), 4.17-4.10 (m, 1H), 4.05-4.01 (m, 2H), 3.97-3.92 (m, 5H), 3.83 (s, 3H), 3.67 (s, 3H), 3.62-3.48 (m, 4H), 2.80-2.70 (m, 2H), 2.69-2.63 (m, 1H), 2.38-2.36 (m, 1H), 2.03-1.97 (m, 8H), 1.87-1.80 (m, 4H), 1.61-1.53 (m, 2H), 0.82-0.58 (m, 4H).

### Example 2-7 7

### (S)-8-((5-(((S)-2-(4-(2H-1,2,3-Triazol-2-yl)phenyl)-7-methoxy-5-oxo-5,10,11,11a-tetrah ydro-1H-benzo[e]pyrrolo[1,2-a][1,4]diazepin-8-yl)oxy)pentyl)oxy)-7-methoxy-1,11a-di hydro-3H,5H-spiro[benzo[e]pyrrolo[1,2-a][1,4]diazepine-2,1'-cyclopropan]-5-one 7

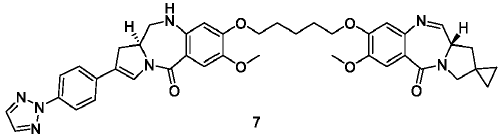

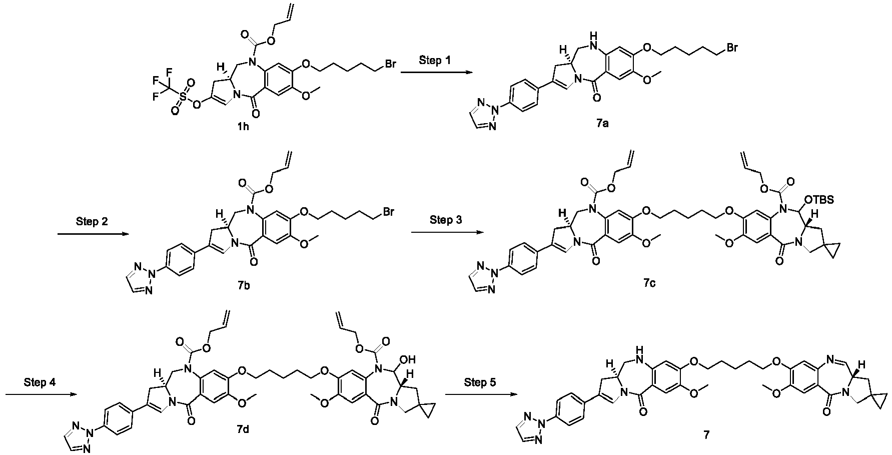

### Step 1

### (S)-2-(4-(2H-1,2,3-Triazol-2-yl)phenyl)-8-((5-bromopentyl)oxy)-7-methoxy-1,10,11,11 a-tetrahydro-5H-benzo[e]pyrrolo[1,2-a][1,4]diazepin-5-one 7a

The title product **7a** (80 mg, yield: 49.7%) was obtained by replacing the starting material 4-(4-methyl-1-piperazinyl)phenylboronic acid of step 8 of Example 2-1 1 with (4-(2*H*-1,2,3-triazol-2-yl)phenyl)boronic acid and using the same reaction conditions. MS m/z (ESI): 538.1 [M+1].

### Step 2

### Allyl

### (S)-2-(4-(2H-1,2,3-triazol-2-yl)phenyl)-8-((5-bromopentyl)oxy)-7-methoxy-5-oxo-11,11 a-dihydro-1H-benzo[e]pyrrolo[1,2-a][1,4]diazepine-10(5H)-carboxylate 7b

The title product **7b** (50 mg, yield: 80%) was obtained by replacing the starting material **1i** of step 9 of Example 2-1 1 with **7a** (54 mg, 0.10 mmol) and using the same reaction conditions.

MS m/z (ESI): 622.2 [M+1].

### Step 3

### Allyl

### (11aS)-8-((5-(((S)-2-(4-(2H-1,2,3-triazol-2-yl)phenyl)-10-((allyloxy)carbonyl)-7-metho xy-5-oxo-5,10,11,11a-tetrahydro-1H-benzo[e]pyrrolo[1,2-a][1,4]diazepin-8-yl)oxy)pent yl)oxy)-11-((tert-butyldimethylsilyl)oxy)-7-methoxy-5-oxo-11,11a-dihydro-1H,3H-spir o[benzo[e]pyrrolo[1,2-a][1,4]diazepine-2,1'-cyclopropane]-10(5H)-carboxylate 7c

The title product **7c** (51 mg, yield: 63%) was obtained by replacing the starting material **1j** of step 10 of Example 2-1 1 with **7b** (50 mg, 0.08 mmol) and using the same reaction conditions.

MS m/z (ESI): 1030.5 [M+1].

### Step 4

### Allyl

### (11aS)-8-((5-(((S)-2-(4-(2H-1,2,3-triazol-2-yl)phenyl)-10-((allyloxy)carbonyl)-7-metho xy-5-oxo-5,10,11,11a-tetrahydro-1H-benzo[e]pyrrolo[1,2-a][1,4]diazepin-8-yl)oxy)pent yl)oxy)-11-hydroxy-7-methoxy-5-oxo-11,11a-dihydro-1H,3H-spiro[benzo[e]pyrrolo[1,2 -a][1,4]diazepine-2,1'-cyclopropane]-10(5H)-carboxylate 7d

The title product **7d** (37 mg, yield: 80.8%) was obtained by replacing the starting material **1l** of step 11 of Example 2-1 1 with **7c** (51 mg, 0.05 mmol) and using the same reaction conditions.

MS m/z (ESI): 916.4 [M+1].

### Step 5

### (S)-8-((5-(((S)-2-(4-(2H-1,2,3-Triazol-2-yl)phenyl)-7-methoxy-5-oxo-5,10,11,11a-tetrah ydro-1H-benzo[e]pyrrolo[1,2-a][1,4]diazepin-8-yl)oxy)pentyl)oxy)-7-methoxy-1,11a-di hydro-3H,5H-spiro[benzo[e]pyrrolo[1,2-a][1,4]diazepine-2,1'-cyclopropan]-5-one 7

The title product 7 (25 mg, yield: 85.7%) was obtained by replacing the starting material **1m** of step 12 of Example 2-1 1 with **7d** (37 mg, 0.04 mmol) and using the same reaction conditions.

MS m/z (ESI): 730.3 [M+1].

¹H NMR (500 MHz, DMSO-d6) δ 8.13 (s, 2H), 7.99 (d, 2H), 7.92 (d, 1H), 7.70-7.69 (m, 1H), 7.64 (d, 2H), 7.33-7.32 (m, 1H), 6.86 (s, 1H), 6.64-6.59 (m, 1H), 6.33 (s, 1H), 5.33 (t, 1H), 4.29-4.24 (m, 2H), 4.15-4.12 (m, 1H), 4.05-4.02 (m, 2H), 3.97-3.92 (m, 4H), 3.83 (s, 3H), 3.68 (s, 3H), 3.62-3.53 (m, 1H), 2.87-2.83 (m, 1H), 2.66-2.64 (m, 1H), 2.37-2.35 (m, 1H), 2.06-1.95 (m, 1H), 1.87-1.80 (m, 4H), 1.63-1.58 (m, 2H), 0.71-0.55 (m, 4H).

### Example 2-8 8

### (S)-7-Methoxy-8-((5-(((S)-7-methoxy-2-(4-(1-methyl-1H-pyrazol-5-yl)phenyl)-5-oxo-5, 10,11,11a-tetrahydro-1H-benzo[e]pyrrolo[1,2-a][1,4]diazepin-8-yl)oxy)pentyl)oxy)-1,1 1a-dihydro-3H,5H-spiro[benzo[e]pyrrolo[1,2-a][1,4]diazepine-2,1 '-cyclopropan] -5-one 8

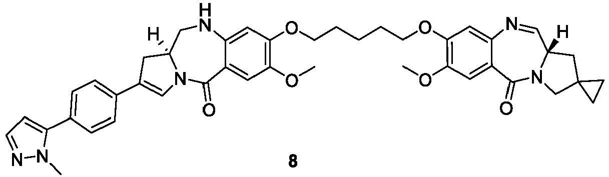

### Example 2-9 9

### (S)-8-((5-(((S)-2-(4-(1-(2-Hydroxyethyl)piperidin-4-yl)phenyl)-7-methoxy-5-oxo-5,10,1 1,11a-tetrahydro-1H-benzo[e]pyrrolo[1,2-a][1,4]diazepin-8-yl)oxy)pentyl)oxy)-7-meth oxy-1,11a-dihydro-3H,5H-spiro[benzo[e]pyrrolo[1,2-a][1,4]diazepine-2,1'-cyclopropan ]-5-one 9

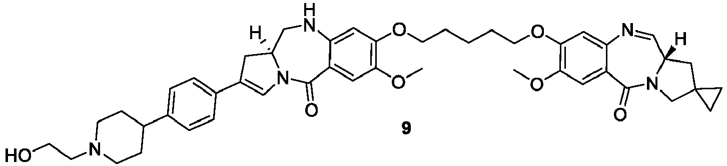

### Example 2-10 10

### (S)-8-((5-(((S)-2-(4-(1-(2-Hydroxyacetyl)piperidin-4-yl)phenyl)-7-methoxy-5-oxo-5,10, 11,11a-tetrahydro-1H-benzo[e]pyrrolo[1,2-a][1,4]diazepin-8-yl)oxy)pentyl)oxy)-7-met hoxy-1,11a-dihydro-3H,5H-spiro[benzo[e]pyrrolo[1,2-a][1,4]diazepine-2,1 '-cyclopropa n]-5-one 10

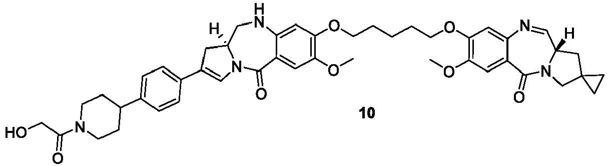

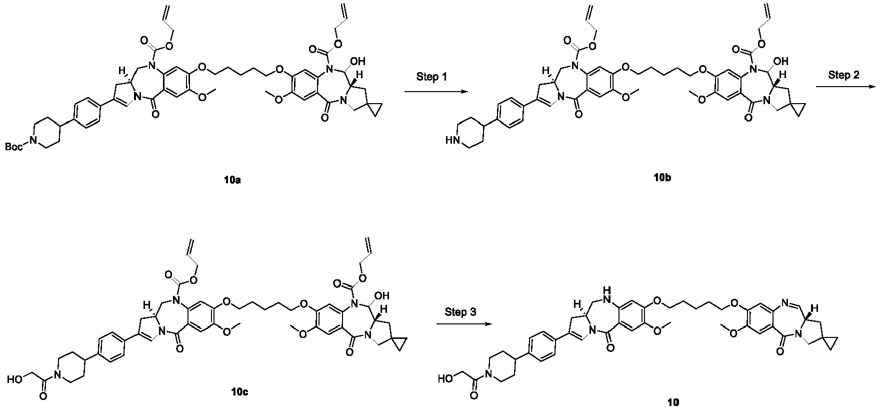

### Step 1

### Allyl

### (11aS)-8-((5-(((S)-10-((allyloxy)carbonyl)-7-methoxy-5-oxo-2-(4-(piperidin-4-yl)pheny l)-5,10,11,11a-tetrahydro-1H-benzo[e]pyrrolo[1,2-a][1,4]diazepin-8-yl)oxy)pentyl)oxy) -11-hydroxy-7-methoxy-5-oxo-11,11a-dihydro-1H,3H-spiro[benzo[e]pyrrolo[1,2-a][1,4 ]diazepine-2,1'-cyclopropane]-10(5H)-carboxylate 10b

### Allyl

(11aS)-8-((5-(((S)-10-((allyloxy)carbonyl)-2-(4-(1-(tert-butoxycarbonyl)piperidin-4-yl) phenyl)-7-methoxy-5-oxo-5,10,11,11a-tetrahydro-1*H*-benzo[*e*]pyrrolo[1,2-*a*][1,4]diazep in-8-yl)oxy)pentyl)oxy)-11-hydroxy-7-methoxy-5-oxo-11,11a-dihydro-1H,3H-spiro[ben zo[e]pyrrol o[1,2-*a*][1,4]diazepine-2,1'-cyclopropane]-10(5H)-carboxylate **10a** was obtained by using the synthesis scheme of Example 2-1 1l and replacing the reagent 4-(4-methyl-1-piperazinyl)phenylboronic acid of step 8 with tert-butyl 4-(4-(4,4,5,5-tetramethyl-1,3,2-dioxaborolan-2-yl)phenyl)piperidine-1-carboxylate. **10a** (25 mg, 0.022 mmol) was placed in a round-bottom flask, and dichloromethane (1 mL) was added. After 5 min of stirring in an ice bath, 1 N hydrochloric acid (1 mL) was added. After half an hour of reaction at room temperature, the reaction mixture was quenched with saturated ammonium bicarbonate solution (10 mL) and extracted with water (10 mL × 2) and dichloromethane (20 mL × 2). The resulting organic phase was dried and concentrated by rotary evaporation to give the title product **10b** (20 mg, 98%).

### Step 2

### Allyl

### (11aS)-8-((5-(((S)-10-((allyloxy)carbonyl)-2-(4-(1-(2-hydroxyacetyl)piperidin-4-yl)phe nyl)-7-methoxy-5-oxo-5,10,11,11a-tetrahydro-1H-benzo[e]pyrrolo[1,2-a][1,4]diazepin-8-yl)oxy)pentyl)oxy)-11-hydroxy-7-methoxy-5-oxo-11,11a-dihydro-1H,3H-spiro[benzo [e]pyrrolo[1,2-a][1,4]diazepine-2,1'-cyclopropane]-10(5H)-carboxylate 10c

**10b** (20 mg, 0.021 mmol) and hydroxyacetic acid (1.8 mg, 0.023 mmol) were dissolved in tetrahydrofuran (1.5 mL), and 2-(7-azabenzotriazol)-*N,N,N',N*'-tetramethyluronium hexafluorophosphate (16.3 mg, 0.042 mmol) and N,N-diisopropylethylamine (8.1 mg, 0.062 mmol) were added. After the addition, the reaction system was purged with nitrogen gas three times and stirred at 25 °C for 3 h. The reaction mixture was concentrated under reduced pressure, and the resulting residue was purified using a CombiFlash preparative flash chromatograph with eluent system A to give the title product **10c** (10 mg, yield: 47.1%).

MS m/z (ESI): 990.8 [M+1].

### Step 3

### (S)-8-((5-(((S)-2-(4-(1-(2-Hydroxyacetyl)piperidin-4-yl)phenyl)-7-methoxy-5-oxo-5,10, 11,11a-tetrahydro-1H-benzo[e]pyrrolo[1,2-a][1,4]diazepin-8-yl)oxy)pentyl)oxy)-7-met hoxy-1,11a-dihydro-3H,5H-spiro[benzo[e]pyrrolo[1,2-a][1,4]diazepine-2,1'-cyclopropa n]-5-one 10

The title product **10** (8 mg, yield: 98.5%) was obtained by replacing the starting material **1m** of step 12 of Example 2-1 1 with **10c** (10 mg, 0.01 mmol) and using the same reaction conditions.

MS m/z (ESI): 804.7 [M+1].

¹H NMR (500 MHz, DMSO-d6) δ 7.91 (d, 1H), 7.54 (s, 1H), 7.38 (d, 2H), 7.34 (s, 1H), 7.30 (s, 1H), 7.21 (d, 2H), 6.85 (s, 1H), 6.33 (s, 1H), 5.33 (t, 1H), 4.25-4.19 (m, 2H), 4.17-4.10 (m, 1H), 4.05-4.01 (m, 2H), 3.98-3.92 (m, 2H), 3.83 (s, 2H), 3.67 (s, 2H), 3.58-3.49 (m, 7H), 2.77-2.68 (m, 4H), 2.03-1.95 (m, 5H), 1.86-1.79 (m, 3H), 1.61-1.53 (m, 3H), 1.50-1.44 (m, 3H), 0.89-0.80 (m, 4H), 0.69-0.65 (m, 2H).

### Example 2-11 11

### (S)-8-((5-(((S)-2-(4-(1-Acetylpiperidin-4-yl)phenyl)-7-methoxy-5-oxo-5,10,11,11a-tetra hydro-1H-benzo[e]pyrrolo[1,2-a][1,4]diazepin-8-yl)oxy)pentyl)oxy)-7-methoxy-1,11a-dihydro-3H,5H-spiro[benzo[e]pyrrolo[1,2-a][1,4]diazepine-2,1'-cyclopropan]-5-one 11

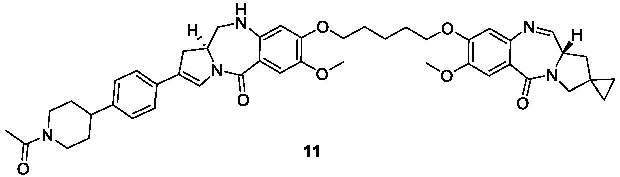

The title product **11** (18 mg, overall yield: 76.3%) was prepared by using the synthesis scheme of Example 2-10 and replacing the reagent hydroxyacetic acid of step 2 with glacial acetic acid.

MS m/z (ESI): 788.6 [M+1].

¹H NMR (500 MHz, DMSO-d6) δ 7.91 (d, 1H), 7.53 (s, 1H), 7.37 (d, 2H), 7.33 (s, 1H), 7.30 (s, 1H), 7.21 (d, 2H), 6.85 (s, 1H), 6.56 (d, 1H), 6.32 (s, 1H), 4.52 (d, 1H), 4.25-4.17 (m, 1H), 4.15-4.09 (m, 1H), 4.06-3.99 (m, 1H), 3.99-3.86 (m, 4H), 3.82 (s, 3H), 3.67 (s, 3H), 3.59-3.52 (m, 1H), 3.49 (d, 1H), 3.12 (t, 1H), 2.81-2.70 (m, 2H), 2.58 (t, 1H), 2.06-2.02 (m, 4H), 1.87-1.72 (m, 7H), 1.61-1.54 (m, 3H), 1.49-1.39 (m, 2H), 0.89-0.81 (m, 2H), 0.81-0.76 (m, 1H), 0.72-0.62 (m, 3H).

### Example 2-12 12

### (S)-7-Methoxy-8-((5-(((S)-7-methoxy-10-methyl-2-(4-morpholinylphenyl)-5-oxo-5,10,1 1,11a-tetrahydro-1H-benzo[e]pyrrolo[1,2-a][1,4]diazepin-8-yl)oxy)pentyl)oxy)-1,11a-d ihydro-3H,5H-spiro[benzo[e]pyrrolo[1,2-a][1,4]diazepine-2,1'-cyclopropan]-5-one 12

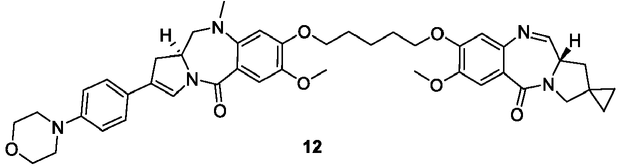

### Example 2-13 13

### (S)-7-Methoxy-8-(3-(1-((((S)-7-methoxy-2-(4-methoxyphenyl)-5-oxo-5,10,11,11a-tetra hydro-1H-benzo[e]pyrrolo[1,2-a][1,4]diazepin-8-yl)oxy)methyl)cyclopropyl)propoxy)-1,11a-dihydro-3H,5H-spiro[benzo[e]pyrrolo[1,2-a][1,4]diazepine-2,1'-cyclopropan]-5-o ne 13

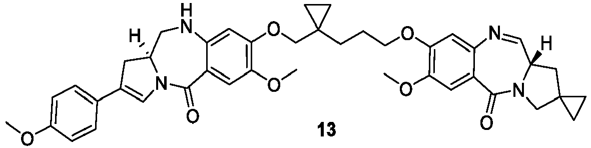

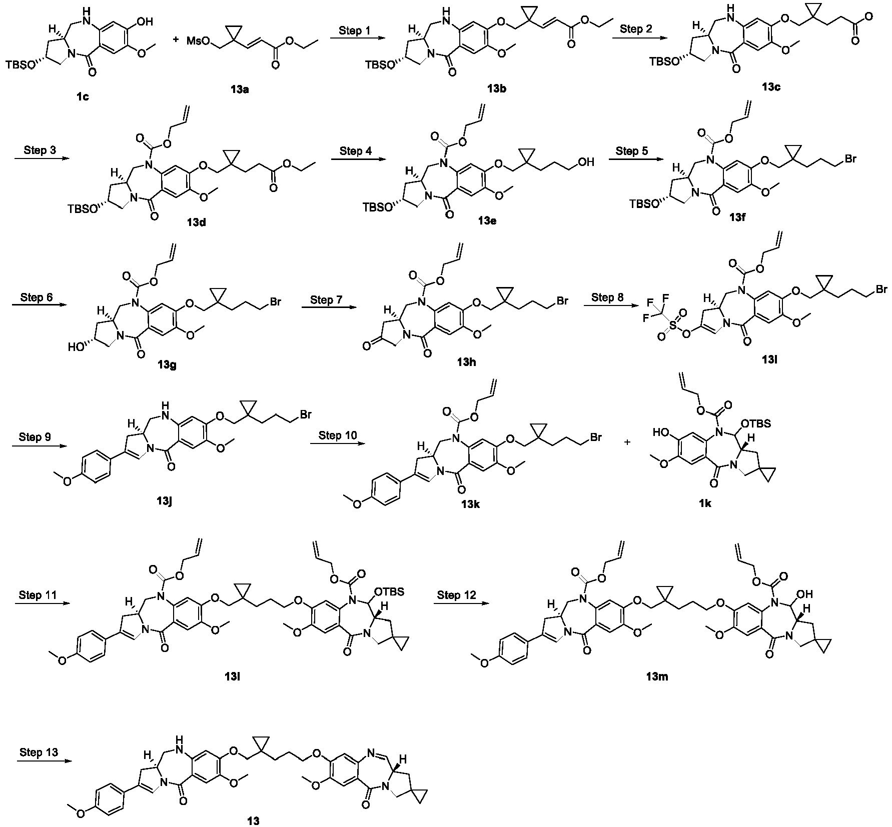

### Step 1

### Ethyl

### (E)-3-(1-((((2R,11aS)-2-((tert-butyldimethylsilyl)oxy)-7-methoxy-5-oxo-2,3,5,10,11,11 a-hexahydro-1H-benzo[e]pyrrolo[1,2-a][1,4]diazepin-8-yl)oxy)methyl)cyclopropyl)acr ylate 13b

**1c** (300 mg, 0.79 mmol) and ethyl (*E*)-3-(1-(((methylsulfonyl)oxy)methyl)cyclopropyl)acrylate **13a** (378 mg, 1.52 mmol, prepared by the method disclosed for intermediate 2 on page 32 of the specification of the patent application "CN104395312 B") were dissolved in *N*,*N*dimethylformamide (4 mL), and potassium carbonate (548 mg, 3.97 mmol) was added. After the addition, the reaction system was purged with nitrogen gas three times, heated to 90 °C, and stirred for 3 h. The reaction mixture was cooled to room temperature. Water (50 mL) was added, and extraction was performed with ethyl acetate (10 mL × 3). The organic phases were combined, washed with saturated sodium chloride solution (30 mL), dried over anhydrous sodium sulfate, and filtered. Then the filtrate was concentrated under reduced pressure, and the resulting residue was purified using a CombiFlash preparative flash chromatograph with eluent system B to give the title product **13b** (354 mg, yield: 84.1%).

MS m/z (ESI): 531.2 [M+1].

### Step 2

### Ethyl

### 3-(1-((((2R,11aS)-2-((tert-butyldimethylsilyl)oxy)-7-methoxy-5-oxo-2,3,5,10,11,11a-he xahydro-1H-benzo[e]pyrrolo[1,2-a][1,4]diazepin-8-yl)oxy)methyl)cyclopropyl)propion ate 13c

**13b** (354 mg, 0.67 mmol) was dissolved in methanol (6 mL), and cobalt chloride hexahydrate (32 mg, 0.13 mmol) was added. After the addition, the reaction system was purged with nitrogen gas three times and stirred for 30 min, and 2 mL of sodium borohydride (126 mg, 3.33 mmol) in *N,N*-dimethylformamide was added dropwise. After the dropwise addition, the mixture was stirred for 2 h, and another 1 mL of sodium borohydride (76 mg, 2.01 mmol) in *N*,*N*-dimethylformamide was added dropwise. After the dropwise addition, the mixture was stirred for 2.5 h. Water (60 mL) was added to the reaction mixture in an ice bath, and extraction was performed with ethyl acetate (10 mL × 3). The organic phases were combined, washed with saturated sodium chloride solution (30 mL), dried over anhydrous sodium sulfate, and filtered. Then the filtrate was concentrated under reduced pressure to give a crude product of the title product **13c** (336 mg), and the product was directly used in the next step without purification.

MS m/z (ESI): 533.3 [M+1].

### Step 3

### Allyl

### (2R,11a,S)-2-((tert-butyldimethylsilyl)oxy)-8-((1-(3-ethoxy-3-oxopropyl)cyclopropyl)m ethoxy)-7-methoxy-5-oxo-2,3,11,11a-tetrahydro-1H-benzo[e]pyrrolo[1,2-a][1,4]diazepi ne-10(5H)-carboxylate 13d

**13c** (336 mg, 0.63 mmol) was dissolved in dichloromethane (8 mL), and pyridine (100 mg, 1.26 mmol) was added under ice-bath conditions. Allyl chloroformate (304 mg, 2.52 mmol) was added dropwise. After the addition, the mixture was stirred for 15 min. An ice-cold 10% citric acid solution (10 mL) was added to the reaction mixture, and extraction was performed with dichloromethane (10 mL × 3). The organic phases were combined, washed with saturated sodium chloride solution (20 mL), dried over anhydrous sodium sulfate, and filtered. Then the filtrate was concentrated under reduced pressure, and the resulting residue was purified using a CombiFlash preparative flash chromatograph with eluent system B to give the title product **13d** (313 mg, yield: 80.4%).

MS m/z (ESI): 617.3 [M+1].

### Step 4

### Allyl

### (2R,11aS)-2-((tert-butyldimethylsilyl)oxy)-8-((1-(3-hydroxypropyl)cyclopropyl)methox y)-7-methoxy-5-oxo-2,3,11,11a-tetrahydro-1H-benzo[e]pyrrolo[1,2-a][1,4]diazepine-10 (5H)-carboxylate 13e

**13d** (300 mg, 0.49 mmol) was dissolved in 16 mL of a mixed solvent of tetrahydrofuran and ethanol (V/V = 1:1), and sodium borohydride (110 mg, 2.91 mmol) and lithium chloride (134 mg, 3.16 mmol) were added. After the addition, the reaction system was purged with nitrogen gas three times and stirred for 1.5 h. Sodium borohydride (55 mg, 1.46 mmol) and lithium chloride (67 mg, 1.58 mmol) were added to the reaction mixture, and the mixture was stirred for another 4 h. An ammonium chloride solution (30 mL) and water (20 mL) were added to the reaction mixture, and extraction was performed with ethyl acetate (10 mL × 3). The organic phases were combined, washed with saturated sodium chloride solution (30 mL), dried over anhydrous sodium sulfate, and filtered. Then the filtrate was concentrated under reduced pressure, and the resulting residue was purified using a CombiFlash preparative flash chromatograph with eluent system B to give the title product **13e** (204 mg, yield: 72.9%).

MS m/z (ESI): 575.3 [M+1].

### Step 5

### Allyl

### (2R,11aS)-8-((1-(3-bromopropyl)cyclopropyl)methoxy)-2-((tert-butyldimethylsilyl)oxy) -7-methoxy-5-oxo-2,3,11,11a-tetrahydro-1H-benzo[e]pyrrolo[1,2-a][1,4]diazepine-10(5 H)-carboxylate 13f

**13e** (187 mg, 0.32 mmol) was dissolved in dichloromethane (5 mL), and triethylamine (164 mg, 1.63 mmol) was added. 4-Dimethylaminopyridine (12 mg, 0.10 mmol) was added under ice-bath conditions, and methanesulfonyl chloride (112 mg, 0.98 mmol) was added dropwise. After the addition, the mixture was warmed to room temperature and stirred for 1.5 h. Water (15 mL) was added to the reaction mixture, and extraction was performed with dichloromethane (6 mL × 3). The organic phases were combined, washed with saturated sodium chloride solution (20 mL), dried over anhydrous sodium sulfate, and filtered. Then the filtrate was concentrated under reduced pressure, and the resulting residue was dissolved in tetrahydrofuran (5 mL). Lithium bromide (339 mg, 3.91 mmol) was added, and the mixture was stirred for 16 h. Water (20 mL) was added to the reaction mixture, and extraction was performed with ethyl acetate (10 mL × 3). The organic phases were combined, washed with saturated sodium chloride solution (30 mL), dried over anhydrous sodium sulfate, and filtered. Then the filtrate was concentrated under reduced pressure to give a crude product of the title product **13f** (207 mg), and the product was directly used in the next step without purification.

MS m/z (ESI): 637.2 [M+1].

### Step 6

### Allyl

### (2R,11aS)-8-((1-(3-bromopropyl)cyclopropyl)methoxy)-2-hydroxy-7-methoxy-5-oxo-2, 3,11,11a-tetrahydro-1H-benzo[e]pyrrolo[1,2-a][1,4]diazepine-10(5H)-carboxylate 13g 13f (207 mg, 0.32 mmol) was dissolved in tetrahydrofuran (5 mL), and a solution of tetrabutylammonium fluoride in tetrahydrofuran (1 M, 0.96 mL, 0.96 mmol) was added under ice-bath conditions. After the addition, the ice bath was removed, and the mixture was warmed to room temperature and stirred for 1.5 h. Saturated ammonium chloride solution (20 mL) and water (10 mL) were added to the reaction mixture, and extraction was performed with ethyl acetate (8 mL × 3). The organic phases were combined, washed with saturated sodium chloride solution (15 mL), dried over anhydrous sodium sulfate, and filtered. Then the filtrate was concentrated under reduced pressure, and the resulting residue was purified using a CombiFlash preparative flash chromatograph with eluent system A to give the title product 13g (160 mg, yield: 94.1%).

MS m/z (ESI): 523.1 [M+1].

### Step 7

### Allyl

### (S)-8-((1-(3-bromopropyl)cyclopropyl)methoxy)-7-methoxy-2,5-dioxo-2,3,11,11a-tetra hydro-1H-benzo[e]pyrrolo[1,2-a][1,4]diazepine-10(5H)-carboxylate 13h

**13g** (160 mg, 0.31 mmol) was dissolved in dichloromethane (5 mL), and sodium bicarbonate (154 mg, 1.83 mmol) and the Dess-Martin oxidant (390 mg, 0.92 mmol) were sequentially added under ice-bath conditions. After the addition, the ice-water bath was removed, and the mixture was warmed to room temperature and stirred for 1.5 h. The reaction mixture was quenched with saturated sodium bicarbonate solution (10 mL) and extracted with dichloromethane (8 mL × 3). The organic phases were combined, washed with saturated sodium chloride solution (30 mL), dried over anhydrous sodium sulfate, and filtered. Then the filtrate was concentrated under reduced pressure, and the resulting residue was purified using a CombiFlash preparative flash chromatograph with eluent system B to give the title product **13h** (127 mg, yield: 79.7%).

MS m/z (ESI): 521.0 [M+1].

### Step 8

### Allyl

### (S)-8-((1-(3-bromopropyl)cyclopropyl)methoxy)-7-methoxy-5-oxo-2-(((trifluoromethyl) sulfonyl)oxy)-11,11a-dihydro-1H-benzo[e]pyrrolo[1,2-a][1,4]diazepine-10(5H)-carbox ylate 13i

**13h** (127 mg, 0.24 mmol) was dissolved in dichloromethane (5 mL). The reaction system was purged with nitrogen gas three times and cooled in an acetonitrile-dry ice bath to -40 °C, and 2,6-lutidine (78 mg, 0.73 mmol) was added dropwise. The mixture was stirred for 5 min, and trifluoromethanesulfonic anhydride (138 mg, 0.49 mmol) was then added. After the addition, the mixture was stirred at -40 °C for another 40 min. The reaction mixture was quenched with saturated aqueous ammonium chloride (8 mL). Water (10 mL) was added, and extraction was performed with dichloromethane (6 mL × 3). The organic phases were combined, washed with saturated sodium chloride solution (15 mL), dried over anhydrous sodium sulfate, and filtered. Then the filtrate was concentrated under reduced pressure, and the resulting residue was purified using a CombiFlash preparative flash chromatograph with eluent system B to give the title product **13i** (66 mg, yield: 41.3%).

MS m/z (ESI): 653.0 [M+1].

### Step 9

### (S)-8-((1-(3-Bromopropyl)cyclopropyl)methoxy)-7-methoxy-2-(4-methoxyphenyl)-1,10 ,11,11a-tetrahydro-5H-benzo[e]pyrrolo[1,2-a][1,4]diazepin-5-one 13j

**13i** (66 mg, 0.10 mmol) and p-methoxyphenylboronic acid (46 mg, 0.30 mmol, Accela ChemBio) were dissolved in 3.5 mL of a mixed solvent of tetrahydrofuran and water (V/V = 6:1), and potassium carbonate (70 mg, 0.51 mmol) and [1,1'-bis(diphenylphosphino)ferrocene]palladium(II) dichloride (23 mg, 0.031 mmol, adamas) were sequentially added. After the addition, the reaction system was purged with nitrogen gas three times and stirred for 16 h. Water (15 mL) was added to the reaction mixture, and extraction was performed with ethyl acetate (8 mL × 3). The organic phases were combined, washed with saturated sodium chloride solution (15 mL), dried over anhydrous sodium sulfate, and filtered. Then the filtrate was concentrated under reduced pressure, and the resulting residue was purified using a CombiFlash preparative flash chromatograph with eluent system B to give the title product **13j** (31 mg, yield: 58.2%).

MS m/z (ESI): 527.1 [M+1].

### Step 10

### Allyl

### (S)-8-((1-(3-bromopropyl)cyclopropyl)methoxy)-7-methoxy-2-(4-methoxyphenyl)-5-ox o-11,11a-dihydro-1H-benzo[e]pyrrolo[1,2-a][1,4]diazepine-10(5H)-carboxylate 13k

**13j** (31 mg, 0.059 mmol) was dissolved in dichloromethane (3 mL), and pyridine (9.3 mg, 0.12 mmol) was added under ice-bath conditions. Allyl chloroformate (28 mg, 0.23 mmol) was added dropwise. After the addition, the mixture was stirred for 15 min. An ice-cold 10% citric acid solution (4 mL) was added to the reaction mixture, and extraction was performed with dichloromethane (6 mL × 3). The organic phases were combined, washed with saturated sodium chloride solution (20 mL), dried over anhydrous sodium sulfate, and filtered. Then the filtrate was concentrated under reduced pressure, and the resulting residue was purified using a CombiFlash preparative flash chromatograph with eluent system B to give the title product **13k** (33 mg, yield: 91.8%). MS m/z (ESI): 611.1 [M+1].

### Step 11

### Allyl

### (11aS)-8-(3-(1-((((S)-10-((allyloxy)carbonyl)-7-methoxy-2-(4-methoxyphenyl)-5-oxo-5, 10,11,11a-tetrahydro-1H-benzo[e]pyrrolo[1,2-a][1,4]diazepin-8-yl)oxy)methyl)cyclopr opyl)propoxy)-11-((tert-butyldimethylsilyl)oxy)-7-methoxy-5-oxo-11,11a-dihydro-1H,3 H-spiro[benzo[e]pyrrolo[1,2-a][1,4]diazepine-2,1'-cyclopropane]-10(5H)-carboxylate 131

**13k** (33 mg, 0.054 mmol) was dissolved in *N,N*-dimethylformamide (1.5 mL), and **1k** (29 mg, 0.059 mmol) and potassium carbonate (23 mg, 0.17 mmol) were added. After the addition, the reaction system was purged with nitrogen gas three times and stirred for 16 h. Water (10 mL) was added to the reaction mixture, and extraction was performed with ethyl acetate (8 mL × 3). The organic phases were combined, washed with saturated sodium chloride solution (15 mL), dried over anhydrous sodium sulfate, and filtered. Then the filtrate was concentrated under reduced pressure, and the resulting residue was purified by thin-layer chromatography with developing solvent system A to give the title product **13l** (44 mg, yield: 80.0%).

MS m/z (ESI): 1019.3 [M+1].

### Step 12

### Allyl

### (11aS)-8-(3-(1-((((S)-10-((allyloxy)carbonyl)-7-methoxy-2-(4-methoxyphenyl)-5-oxo-5, 10,11,1 1a-tetrahydro-1H-benzo[e]pyrrolo[1,2-a][1,4]diazepin-8-yl)oxy)methyl)cyclopr opyl)propoxy)-11-hydroxy-7-methoxy-5-oxo-11,11a-dihydro-1H,3H-spiro[benzo[e]pyrr olo[1,2-a][1,4]diazepine-2,1'-cyclopropane]-10(5H)-carboxylate 13m

**131** (44 mg, 0.043 mmol) was dissolved in tetrahydrofuran (3 mL), and glacial acetic acid (15 mg, 0.25 mmol) and a solution of tetrabutylammonium fluoride in tetrahydrofuran (1 M, 0.21 mL, 0.21 mmol) were sequentially added. The mixture was stirred for 1 h. The reaction mixture was diluted with ethyl acetate (5 mL) and washed with saturated aqueous sodium bicarbonate (8 mL) and saturated aqueous sodium chloride (10 mL) in sequence. The organic phase was dried over anhydrous sodium sulfate and filtered. Then the filtrate was concentrated under reduced pressure, and the resulting residue was purified using a CombiFlash preparative flash chromatograph with eluent system A to give the title product **13m** (36 mg, yield: 92.1%).

MS m/z (ESI): 905.8 [M+1].

### Step 13

### (S)-7-Methoxy-8-(3-(1-((((S)-7-methoxy-2-(4-methoxyphenyl)-5-oxo-5,10,11,11a-tetra hydro-1H-benzo[e]pyrrolo[1,2-a][1,4]diazepin-8-yl)oxy)methyl)cyclopropyl)propoxy)-1,11a-dihydro-3H,5H-spiro[benzo[e]pyrrolo[1,2-a][1,4]diazepine-2,1'-cyclopropan]-5-o ne 13

**13m** (18 mg, 0.02 mmol) was dissolved in dichloromethane (2 mL). The reaction system was purged with nitrogen gas three times, and pyrrolidine (8.9 mg, 0.13 mmol) and tetrakis(triphenylphosphine)palladium(0) (2.4 mg, 0.002 mmol) were sequentially added. The mixture was stirred for 30 min. The reaction mixture was concentrated under reduced pressure, and the resulting residue was purified by thin-layer chromatography with developing solvent system A to give the title product **13** (8 mg, yield: 56.0%).

MS m/z (ESI): 719.6 [M+1].

¹H NMR (500 MHz, DMSO-d6) δ 7.89 (d, 1H), 7.44 (s, 1H), 7.37 (d, 2H), 7.30 (t, 1H), 7.04 (s, 1H), 6.90 (d, 2H), 6.80 (s, 1H), 6.48 (d, 1H), 6.29-6.25 (m, 1H), 4.21-4.07 (m, 2H), 3.99-3.89 (m, 2H), 3.82-3.73 (m, 6H), 3.69-3.61 (m, 3H), 3.59-3.43 (m, 3H), 3.27-3.20 (m, 2H), 2.75 (dd, 1H), 2.44-2.27 (m, 2H), 2.07-1.95 (m, 2H), 1.91-1.81 (m, 2H), 1.65-1.56 (m, 2H), 1.34 (d, 1H), 0.87-0.81 (m, 1H), 0.69-0.62 (m, 2H), 0.59-0.46 (m, 4H).

### Example 2-14 14

### (S)-7-Methoxy-8-((1-(3-(((S)-7-methoxy-2-(4-methoxyphenyl)-5-oxo-5,10,11,11a-tetra hydro-1H-benzo[e]pyrrolo[1,2-a][1,4]diazepin-8-yl)oxy)propyl)cyclopropyl)methoxy)-1,11a-dihydro-3H,5H-spiro[benzo[e]pyrrolo[1,2-a][1,4]diazepine-2,1'-cyclopropan]-5-o ne 14

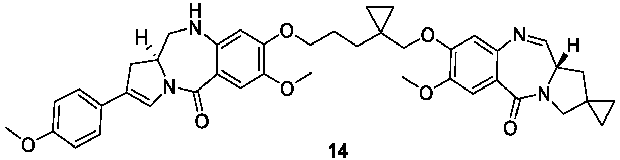

### Example 2-15 15

### (S)-8-((3-Amino-5-((((S)-7-methoxy-2-(4-methoxyphenyl)-5-oxo-5,10,11,11a-tetrahydr o-1H-benzo[e]pyrrolo[1,2-a][1,4]diazepin-8-yl)oxy)methyl)benzyl)oxy)-7-methoxy-1,1 1a-dihydro-3H,5H-spiro[benzo[e]pyrrolo[1,2-a][1,4]diazepine-2,1'-cyclopropan]-5-one 15

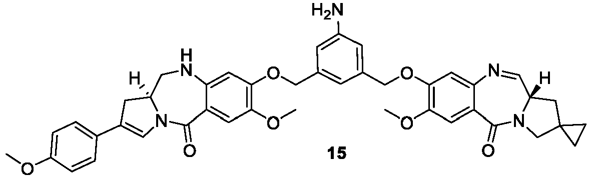

### Example 2-16 16

### (S)-7-Cyclopropoxy-8-((5-(((S)-7-methoxy-2-(4-methoxyphenyl)-5-oxo-5,10,11,11a-tet rahydro-1H-benzo[e]pyrrolo[1,2-a][1,4]diazepin-8-yl)oxy)pentyl)oxy)-1,11a-dihydro-3 H,5H-spiro[benzo[e]pyrrolo[1,2-a][1,4]diazepine-2,1 '-cyclopropan] -5-one 16

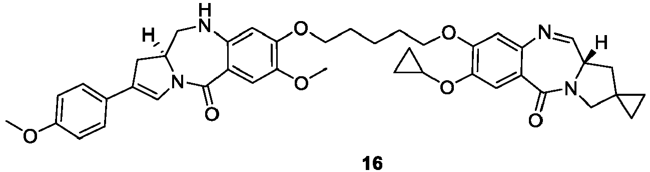

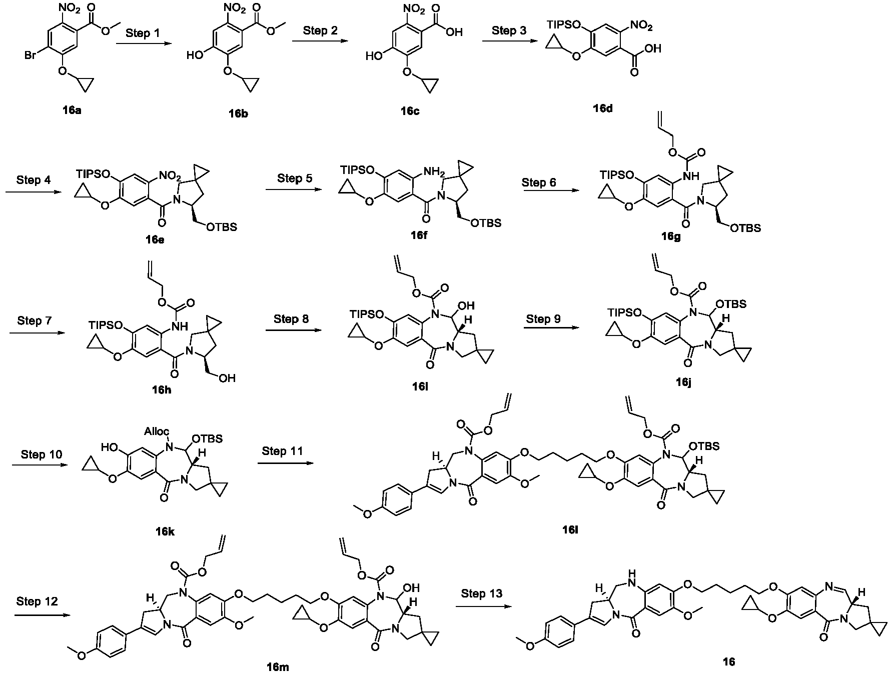

### Step 1

### Methyl 5-cyclopropoxy-4-hydroxy-2-nitrobenzoate 16b

Methyl 4-bromo-5-cyclopropoxy-2-nitrobenzoate **16a** (1.6 g, 5.07 mmol, prepared by the method disclosed in Example 18 on page 54 of the specification of the patent application "CN112110918 A") was placed in a reaction flask and dissolved in 1,4-dioxane (20 mL), and bis(pinacolato)diboron (3.22 g, 12.69 mmol), potassium acetate (1.49 g, 15.2 mmol), and bis(triphenylphosphine)palladium(II) dichloride (1.06 g, 1.51 mmol, adamas) were sequentially added. After the addition, the reaction system was purged with nitrogen gas 3 times, heated to 101 °C, and stirred for 16 h. The reaction mixture was filtered, and the filtrate was concentrated under reduced pressure. The resulting residue was dissolved in tetrahydrofuran (40 mL), and glacial acetic acid (5 mL) was added. The temperature was lowered to 0 °C, and hydrogen peroxide (10 mL, 35%) was added dropwise. After the addition, the mixture was naturally warmed to room temperature and stirred for 3 h. The reaction temperature was lowered to 0 °C, and saturated sodium thiosulfate solution (70 mL) was added dropwise. After the addition, extraction was performed with ethyl acetate (30 mL × 3). The organic phases were combined, washed with water (40 mL) and saturated sodium chloride solution (40 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16b** (902 mg, yield: 70.4%).

MS m/z (ESI): 252.2 [M-1].

### Step 2

### 5-Cyclopropoxy-4-hydroxy-2-nitrobenzoic acid 16c

**16b** (900 mg, 3.55 mmol) was placed in a reaction flask, and tetrahydrofuran (8 mL), methanol (2 mL), and water (2 mL) were added. Then lithium hydroxide monohydrate (373 mg, 8.88 mmol) was added, and the mixture was stirred at room temperature for 2 h. The reaction mixture was concentrated, and the resulting residue was dissolved in ethyl acetate (30 mL). The solution was washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system A to give the title product **16c** (770 mg, yield: 90.6%).

MS m/z (ESI): 238.2 [M-1].

### Step 3

### 5-Cyclopropoxy-2-nitro-4-((triisopropylsilyl)oxy)benzoic acid 16d

**16c** (1.25 g, 5.23 mmol) was dissolved in N,N-dimethylformamide (20 mL), and triisopropylchlorosilane (5.04 g, 26.14 mmol) was added. Then imidazole (2.8 g, 41.17 mmol) was added. After the addition, the mixture was stirred at room temperature for 20 h. Water (30 mL) was added to the reaction mixture, and extraction was performed with ethyl acetate (30 mL × 3). The organic phases were combined, washed with saturated sodium chloride solution (20 mL), dried over anhydrous sodium sulfate, and filtered. Then the filtrate was concentrated under reduced pressure, and methanol (10 mL) and water (2.5 mL) were added to the resulting residue. The mixture was stirred at room temperature for 3 h. The reaction mixture was concentrated under reduced pressure, and ethyl acetate (30 mL) was added to the residue. The mixture was washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system A to give the title product **16d** (1.4 g, yield: 67.9%).

MS m/z (ESI): 396.4 [M+1].

### Step 4

### (S)-(6-(((tert-Butyldimethylsilyl)oxy)methyl)-5-azaspiro[2.4]heptan-5-yl)(5-cyclopropo xy-2-nitro-4-((triisopropylsilyl)oxy)phenyl)methanone 16e

**16d** (1.4 g, 3.54 mmol) was dissolved in dichloromethane (25 mL). In an ice bath, the reaction system was purged with nitrogen gas 3 times, and *N,N,N',N*'-tetramethyl-*O*-(7-azabenzotriazol-1-yl)uronium hexafluorophosphate (1.62 g, 4.26 mmol, Accela ChemBio) and triethylamine (536 mg, 5.30 mmol) were added. After the addition, the mixture was stirred for 30 min, and (*S*)-6-(((tert-butyldimethylsilyl)oxy)methyl)-5-azaspiro[2.4]heptane (854 mg, 3.54 mmol, prepared by the method disclosed for intermediate 1-4 on page 98 of the specification of the patent "CN 111164208 A") was then added. After the addition, the mixture was stirred for 2 h. The reaction mixture was washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16e** (1.1 g, yield: 50.2%).

MS m/z (ESI): 619.3 [M+1].

### Step 5

### (S)-(2-Amino-5-cyclopropoxy-4-((triisopropylsilyl)oxy)phenyl)(6-(((tert-butyldimethyls ilyl)oxy)methyl)-5-azaspiro[2.4]heptan-5-yl)methanone 16f

**16e** (1.1 g, 1.78 mmol) was dissolved in a mixed solution of ethanol (15 mL) and tetrahydrofuran (5 mL), and palladium on carbon (200 mg, content 10%, dry) was added. The reaction system was purged with hydrogen gas three times and stirred at room temperature for 2 h. The reaction mixture was filtered through celite, and the filtrate was concentrated under reduced pressure. The resulting residue was purified using a CombiFlash preparative flash chromatograph with eluent system A to give the title product **16f** (620 mg, yield: 59.6%).

MS m/z (ESI): 589.4 [M+1].

### Step 6

### Allyl

### (S)-(2-(6-(((tert-butyldimethylsilyl)oxy)methyl)-5-azaspiro[2.4]heptane-5-carbonyl)-4-c yclopropoxy-5-((triisopropylsilyl)oxy)phenyl)carbamate 16g

**16f** (1 g, 1.70 mmol) was dissolved in dichloromethane (15 mL), and pyridine (403 mg, 5.10 mmol) was added. The reaction system was purged with nitrogen gas 3 times and cooled to -65 °C. Allyl chloroformate (205 mg, 1.70 mmol) was slowly added dropwise. After the addition, the mixture was stirred at -65 °C for another 20 min. The reaction mixture was quenched with ammonium chloride (20 mL) and separated. The organic phase was washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16g** (900 mg, yield: 78.9%).

MS m/z (ESI): 671.3 [M-1].

### Step 7

### Allyl

### (S)-(4-cyclopropoxy-2-(6-(hydroxymethyl)-5-azaspiro[2.4]heptane-5-carbonyl)-5-((triis opropylsilyl)oxy)phenyl)carbamate 16h

**16g** (900 mg, 1.34 mmol) was dissolved in a mixed solution of tetrahydrofuran (2 mL), methanol (2 mL), and water (2 mL), and glacial acetic acid (8 mL) was added. The reaction system was purged with nitrogen gas 3 times and stirred at room temperature for 5 h. The reaction mixture was concentrated under reduced pressure, and the residue was dissolved in ethyl acetate (20 mL). The solution was washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16h** (643 mg, yield: 86.1%).

MS m/z (ESI): 559.3 [M+1].

### Step 8

### Allyl

### (11aS)-7-cyclopropoxy-11-hydroxy-5-oxo-8-((triisopropylsilyl)oxy)-11,11a-dihydro-1H ,3H-spiro[benzo[e]pyrrolo[1,2-a][1,4]diazepine-2,1'-cyclopropane]-10(5H)-carboxylate 16i

**16h** (620 mg, 1.11 mmol) was dissolved in dichloromethane (8 mL), and iodobenzene diacetate (536.8 mg, 1.66 mmol) was added. The reaction system was purged with nitrogen gas 3 times, and 2,2,6,6-tetramethylpiperidine oxide (17.5 mg, 0.11 mmol) was added. The mixture was stirred at room temperature for 4 h. The reaction mixture was washed with water (10 mL) and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16i** (500 mg, yield: 81%).

MS m/z (ESI): 557.3 [M+1].

### Step 9

### Allyl

### (11aS)-11-((tert-butyldimethylsilyl)oxy)-7-cyclopropoxy-5-oxo-8-((triisopropylsilyl)ox y)-11,11a-dihydro-1H,3H-spiro[benzo[c]pyrrolo[1,2-a][1,4]diazepine-2,1'-cyclopropane ]-10(5H)-carboxylate 16j

**16i** (500 mg, 0.899 mmol) was dissolved in dichloromethane (8 mL). The reaction system was purged with nitrogen gas 3 times and cooled to -40 °C, and 4-dimethylaminopyridine (438.8 mg, 3.59 mmol) and tert-butyldimethylsilyl trifluoromethanesulfonate (712.2 mg, 2.69 mmol) were added. After the addition, the cooling bath was removed, and the mixture was stirred at room temperature for 16 h. After the temperature was lowered to -40 °C, 4-dimethylaminopyridine (438.8 mg, 3.59 mmol) and tert-butyldimethylsilyl trifluoromethanesulfonate (712.2 mg, 2.69 mmol) were added. Then the mixture was warmed to room temperature and allowed to react for 10 h. The reaction mixture was quenched with ammonium chloride (15 mL) and separated. The organic phase was collected, washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16j** (420 mg, yield: 69.7%).

MS m/z (ESI): 671.4 [M+1].

### Step 10

### Allyl

### (11aS)-11-((tert-butyldimethylsilyl)oxy)-7-cyclopropoxy-8-hydroxy-5-oxo-11,11a-dihy dro-1H,3H-spiro[benzo[e]pyrrolo[1,2-a][1,4]diazepine-2,1'-cyclopropane]-10(5H)-carb oxylate 16k

**16j** (420 mg, 0.626 mmol) was dissolved in a mixed solution of *N,N*-dimethylformamide (5 mL) and water (0.1 mL), and lithium acetate (49.6 mg, 0.751 mmol) was added. The mixture was stirred at room temperature for 3.5 h. Water (10 mL) was added, and extraction was performed with ethyl acetate (10 mL × 2). The organic phase was collected, washed with water (10 mL) and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **16k** (312 mg, yield: 96.8%).

MS m/z (ESI): 513.6 [M-1].

### Step 11

### Allyl

### (11aS)-8-((5-(((S)-10-((allyloxy)carbonyl)-7-methoxy-2-(4-methoxyphenyl)-5-oxo-5,10 ,11,11a-tetrahydro-1H-benzo[e]pyrrolo[1,2-a][1,4]diazepin-8-yl)oxy)pentyl)oxy)-1 1-((t ert-butyldimethylsilyl)oxy)-7-cyclopropoxy-5-oxo-11,11a-dihydro-1H,3H-spiro[benzo[ e]pyrrolo[1,2-a][1,4]diazepine-2,1'-cyclopropane] -10(5H)-carboxylate 16l

**16k** (26 mg, 0.051 mmol) was dissolved in *N,N-*dimethylformamide (2 mL), and allyl (*S*)-8-((5-bromopentyl)oxy)-7-methoxy-2-(4-methoxyphenyl)-5-oxy-11,11*a*-dihydro-1*H* -benzo[*e*]pyrrolo[1,2-*a*][1,4]diazepine-10(5*H*)-carboxylate (30 mg, 0.051 mmol, prepared by the method disclosed for intermediate 3-10 on page 103 of the specification of the patent "CN 111164208 A") was added. Potassium carbonate (21.3 mg, 0.154 mmol) was added, and the mixture was stirred at room temperature for 16 h. Water (6 mL) was added, and extraction was performed with ethyl acetate (10 mL × 2). The organic phase was collected, washed with water (10 mL) and saturated sodium chloride solution (10 mL) in sequence, dried over anhydrous sodium sulfate, and filtered. Then the filtrate was concentrated under reduced pressure, and the resulting residue was purified by thin-layer chromatography with developing solvent system A to give the title product **16l** (40 mg, yield: 76.4%).

MS m/z (ESI): 1020.4 [M+1].

### Step 12

### Allyl

### (11aS)-8-((5-(((S)-10-((allyloxy)carbonyl)-7-methoxy-2-(4-methoxyphenyl)-5-oxo-5,10 ,11,11a-tetrahydro-1H-benzo[e]pyrrolo[1,2-a][1,4]diazepin-8-yl)oxy)pentyl)oxy)-7-cycl opropoxy-11-hydroxy-5-oxo-11,11a-dihydro-1H,3H-spiro[benzo[e]pyrrolo[1,2-a][1,4]d iazepine-2,1'-cyclopropane]-10(5H)-carboxylate 16m

The title product **16m** (28 mg, yield: 78.9%) was obtained by replacing the starting material 1l of step 11 of Example 2-1 1 with **16l** (40 mg, 0.039 mmol) and using the same reaction conditions.

MS m/z (ESI): 903.2 [M-1].

### Step 13

### (S)-7-Cyclopropoxy-8-((5-(((S)-7-methoxy-2-(4-methoxyphenyl)-5-oxo-5,10,11,11a-tet rahydro-1H-benzo[e]pyrrolo[1,2-a][1,4]diazepin-8-yl)oxy)pentyl)oxy)-1,11a-dihydro-3 H,5H-spiro[benzo[e]pyrrolo[1,2-a][1,4]diazepine-2,1'-cyclopropan]-5-one 16

The title product **16** (20 mg, yield: 90.1%) was obtained by replacing the starting material 1m of step 12 of Example 2-1 1 with **16m** (28 mg, 0.031 mmol) and using the same reaction conditions.

MS m/z (ESI): 719.8 [M+1].

¹H NMR (500 MHz, DMSO-d6) δ 7.92 (d, 1H), 7.66 (s, 1H), 7.46-7.44 (m, 1H), 7.39-7.37 (m, 2H), 7.31-7.30 (m, 1H), 6.93-6.90 (m, 2H), 6.85-6.84 (m, 1H), 6.32-6.31 (m, 1H), 4.23-4.16 (m, 2H), 4.13-4.08 (m, 1H), 4.04-3.99 (m, 1H), 3.96-3.91 (m, 3H), 3.91-3.86 (m, 1H), 3.85-3.81 (m, 1H), 3.76 (s, 3H), 3.67 (s, 3H), 3.57-3.49 (m, 2H), 3.28-3.25 (m, 2H), 2.79-2.74 (m, 1H), 2.45-2.43 (m, 1H), 2.08-1.97 (m, 4H), 1.84-1.78 (m, 1H), 1.59-1.53 (m, 2H), 0.88-0.84 (m, 2H), 0.81-0.77 (m, 2H), 0.72-0.64 (m, 4H).

### Example 2-17 17

### (S)-4-((5-(((S)-7-Methoxy-2-(4-methoxyphenyl)-5-oxo-5,10,11,11a-tetrahydro-1H-benz o[e]pyrrolo[1,2-a][1,4]diazepin-8-yl)oxy)pentyl)oxy)-1,2,7a,8-tetrahydro-10H,12H-spir o[benzofuro[5,4-e]pyrrolo[1,2-a][1,4]diazepine-9,1'-cyclopropan]-12-one 17

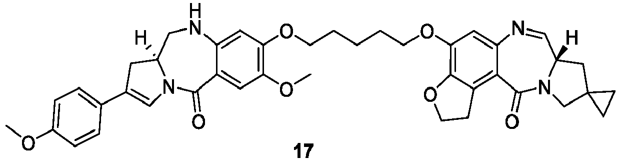

### Example 2-18 18

### (S)-8-((5-(((S)-7-Cyclopropoxy-2-(4-methoxyphenyl)-5-oxo-5,10,11,11a-tetrahydro-1H -benzo[e]pyrrolo[1,2-a][1,4]diazepin-8-yl)oxy)pentyl)oxy)-7-methoxy-1,11a-dihydro-3 H,5H-spiro[benzo[e]pyrrolo[1,2-a][1,4]diazepine-2,1'-cyclopropan]-5-one 18

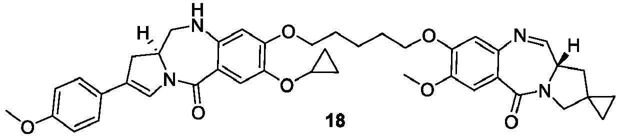

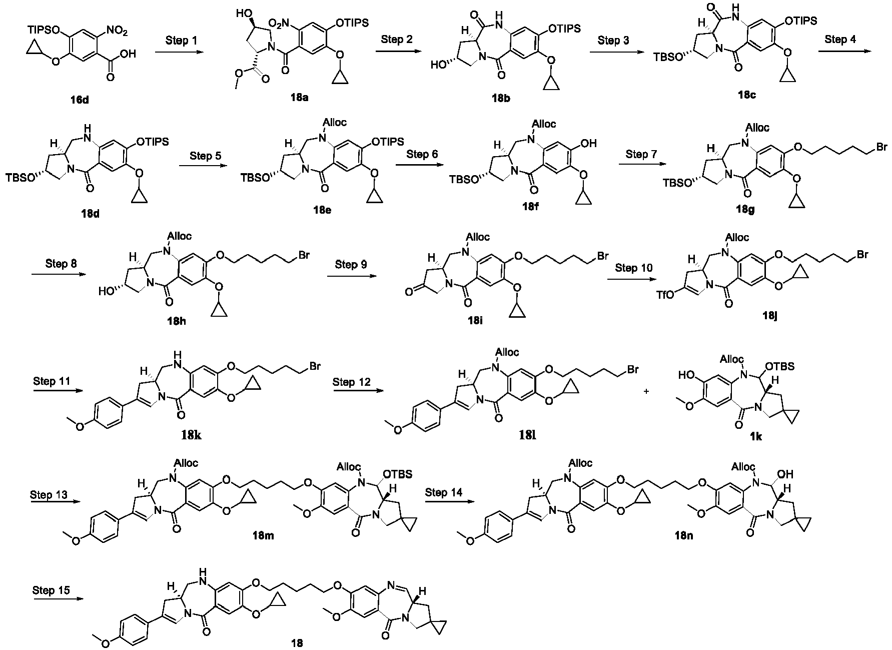

### Step 1

### Methyl

### (2S,4R)-1-(5-cyclopropoxy-2-nitro-4-((triisopropylsilyl)oxy)benzoyl)-4-hydroxypyrroli dine-2-carboxylate 18a

**16d** (6.5 g, 16.4 mmol) was dissolved in dichloromethane (60 mL). In an ice bath, the reaction system was purged with nitrogen gas 3 times, and oxalyl chloride (2.3 g, 18.1 mmol, Adamas) was added. Then *N,N-*dimethylformamide (0.3 mL) was added dropwise. After the dropwise addition, a large amount of gas was generated. The mixture was warmed to room temperature and stirred for 16 h. Methyl (4*R*)-4-hydroxypyrrolidine-2-carboxylate (2.4 g, 16.53 mmol, Accela ChemBio) was dissolved in dichloromethane (60 mL), and triethylamine (16 g, 158.1 mmol) was added under ice-bath conditions. The above solution was then added dropwise. After the dropwise addition, the mixture was warmed to room temperature and stirred for 20 h. The reaction mixture was washed with water (80 mL) and saturated sodium chloride solution (80 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18a** (8.6 g, yield: 100%).

### Step 2

### (2R,11aS)-7-Cyclopropoxy-2-hydroxy-8-((triisopropylsilyl)oxy)-1,2,3,11a-tetrahydro-5 H-benzo[e]pyrrolo[1,2-a][1,4]diazaheptane-5,11(10H)-dione 18b

**18a** (8.6 g, 16.50 mmol) was dissolved in ethanol (150 mL), and reduced iron powder (4.6 g, 82.40 mmol) was added. Then ammonium chloride (10.6 g, 198.20 mmol) was added. The reaction system was purged with nitrogen gas three times, heated to 70 °C, and stirred for 16 h. The reaction mixture was filtered through celite while hot. The filtrate was concentrated under reduced pressure, and the resulting residue was purified using a CombiFlash preparative flash chromatograph with eluent system B to give a crude product of the title product **18b** (5.9 g). The product was directly used in the next step.

MS m/z (ESI): 461.6 [M+1].

### Step 3

(2*R*,11*aS*)-2-((tert-Butyldimethylsilyl)oxy)-7-cyclopropoxy-8-((triisopropylsilyl)oxy)-1, 2,3,11*a*-tetrahydro-5*H*-benzo[*e*]pyrrolo[1,2-*a*][1,4]diazaheptane-5,11(10*H*)-dione **18c 18b** (5.9 g, 12.8 mmol) was dissolved in *N,N-*dimethylformamide (60 mL), and imidazole (5.2 g, 76.4 mmol), tert-butyldimethylchlorosilane (5.8 g, 38.5 mmol), and 4-dimethylaminopyridine (3.2 g, 25.9 mmol) were sequentially added. After the addition, the mixture was stirred at room temperature for 20 h. Water (120 mL) was added, and extraction was performed with ethyl acetate (100 mL × 2). The organic phase was collected, washed with water (100 mL) and saturated sodium chloride solution (100 mL) in sequence, dried over anhydrous sodium sulfate, and filtered. Then the filtrate was concentrated under reduced pressure, and the resulting residue was purified by thin-layer chromatography with developing solvent system A to give the title product **18c** (6.1 g, yield: 82.8%).

MS m/z (ESI): 575.2 [M+1].

### Step 4

### (2R,11aS)-2-((tert-Butyldimethylsilyl)oxy)-7-cyclopropoxy-8-((triisopropylsilyl)oxy)-1, 2,3,10,11,11a-hexahydro-5H-benzo[e]pyrrolo[1,2-a][1,4]diazaheptan-5-one 18d

**18c** (6 g, 10.4 mmol) was dissolved in tetrahydrofuran (60 mL), and a solution of lithium borohydride in tetrahydrofuran (1 M, 41.7 mL, 41.7 mmol) was added under ice-bath conditions. After the dropwise addition, the mixture was warmed to room temperature and stirred for 4 h. The reaction mixture was quenched by slowly adding methanol (20 mL) dropwise and concentrated. The resulting residue was dissolved in ethyl acetate (40 mL), and the solution was washed with water (25 mL) and saturated sodium chloride solution (25 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18d** (4.26 g, yield: 72.8%).

### Step 5

### Allyl

### (2R,11aS)-2-((tert-butyldimethylsilyl)oxy)-7-cyclopropoxy-5-oxo-8-((triisopropylsilyl)o xy)-2,3,11,11a-tetrahydro-1H-benzo[e]pyrrolo[1,2-a][1,4]diazaheptane-10(5H)-carboxy late 18e

**18d** (6.5 g, 11.6 mmol) was dissolved in dichloromethane (70 mL), and the solution was cooled to 0 °C in a nitrogen atmosphere. Pyridine (1.8 mg, 22.8 mmol) and allyl chloroformate (5.6 mg, 46.5 mmol) were sequentially added, and the mixture was stirred at 0 °C for 1 h. The reaction mixture was washed with water (50 mL) and saturated sodium chloride solution (50 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18e** (4.26 g, yield: 56.9%).

MS m/z (ESI): 645.3 [M+1].

### Step 6

### Allyl

### (2R,11aS)-2-((tert-butyldimethylsilyl)oxy)-7-cyclopropoxy-8-hydroxy-5-oxo-2,3,11,11a -tetrahydro-1H-benzo[e]pyrrolo[1,2-a][1,4]diazaheptane-10(5H)-carboxylate 18f

**18e** (4.26 g, 6.60 mmol) was dissolved in a mixed solution of *N,N*-dimethylformamide and water (V/V = 50/1, 25 mL), and lithium acetate (523 mg, 7.92 mmol) was added. After the addition, the mixture was left to react at room temperature for 4 h. Ethyl acetate (40 mL) was added, and the mixture was washed with water (35 mL × 2) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18f** (2.5 g, yield: 77.6%).

MS m/z (ESI): 489.2 [M+1].

### Step 7

### Allyl

### (2R,11a,S)-8-((5-bromopentyl)oxy)-2-((tert-butyldimethylsilyl)oxy)-7-cyclopropoxy-5-o xo-2,3,11,11a-tetrahydro-1H-benzo[e]pyrrolo[1,2-a][1,4]diazaheptane-10(5H)-carboxyl ate 18g

**18f** (2.5 mg, 5.12 mmol) was dissolved in *N*,*N*-dimethylformamide (20 mL), and 1,5-dibromopentane (5.88 g, 25.6 mmol, Adamas) and potassium carbonate (847 mg, 6.14 mmol) were added. The mixture was stirred at room temperature for 16 h. Ethyl acetate (50 mL) was added, and the mixture was washed with water (30 mL × 2) and saturated sodium chloride solution (30 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18g** (2.71 g, yield: 83.1%).

MS m/z (ESI): 637.2 [M+1].

### Step 8

### Allyl

### (2R,11aS)-8-((5-bromopentyl)oxy)-7-cyclopropoxy-2-hydroxy-5-oxo-2,3,11,11a-tetrahy dro-1H-benzo[e]pyrrolo[1,2-a][1,4]diazaheptane-10(5H)-carboxylate 18h

**18g** (2.71 g, 4.25 mmol) was dissolved in tetrahydrofuran (30 mL), and tetrabutylammonium fluoride (1 M, 6.35 mL, 6.35 mmol) was added. After the addition, the mixture was stirred at room temperature for 3 h. An ammonium chloride solution (30 mL) was added, and extraction was performed with ethyl acetate (50 mL × 2). The organic phases were combined, washed with water (30 mL × 2) and saturated sodium chloride solution (30 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18h** (2.24 g, crude product yield: >100%).

MS m/z (ESI): 523.2 [M+1].

### Step 9

### Allyl

### (S)-8-((5-bromopentyl)oxy)-7-cyclopropoxy-2,5-dioxo-2,3,11,11a-tetrahydro-1H-benzo [e]pyrrolo[1,2-a][1,4]diazepine-10(5H)-carboxylate 18i

**18h** (2.22 g, 4.25 mmol) was dissolved in dichloromethane (40 mL), and the solution was cooled to 0 °C. Sodium bicarbonate (2.2 g, 26.2 mmol) and Dess-Martin oxidant (5.44 g, 12.8 mmol, Adamas) were added. After the addition, the mixture was warmed to room temperature and stirred for 20 h. The reaction mixture was washed with water (30 mL × 2) and saturated sodium chloride solution (30 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **18i** (1.62 g, yield: 72.6%). MS m/z (ESI): 521.2 [M+1].

### Step 10

### Allyl

### (S)-8-((5-bromopentyl)oxy)-7-cyclopropoxy-5-oxo-2-(((trifluoromethyl)sulfonyl)oxy)-1

1,11*a*-dihydro-1*H*-benzo[*c*]pyrrolo[1,2-*a*][1,4]diazepine-10(5*H*)-carboxylate **18j** The title product **18j** (430 mg, yield: 66%) was obtained by replacing the starting material **1g** of step 7 of Example 2-1 1 with **18i** and using the same reaction conditions. MS m/z (ESI): 653.1 [M+1].

### Step 11

### (S)-8-((5-Bromopentyl)oxy)-7-cyclopropoxy-2-(4-methoxyphenyl)-1,10,11,11a-tetrahy dro-5H-benzo[e]pyrrolo[1,2-a][1,4]diazepin-5-one 18k

The title product **18k** (82 mg, yield: 70%) was obtained by replacing the starting material **1h** of step 8 of Example 2-1 1 with **18j** and the 4-(4-methyl-1-piperazinyl)phenylboronic acid with 4-methoxyphenylboronic acid (Accela ChemBio) and using the same reaction conditions.

MS m/z (ESI): 527.1 [M+1].

### Step 12

### Allyl

### (S)-8-((5-bromopentyl)oxy)-7-cyclopropoxy-2-(4-methoxyphenyl)-5-oxo-11,11a-dihydr o-1H-benzo[e]pyrrolo[1,2-a][1,4]diazepine-10(5H)-carboxylate 18l

The title product **18l** (88 mg, yield: 93%) was obtained by replacing the starting material **1i** of step 9 of Example 2-1 1 with **18k** and using the same reaction conditions. MS m/z (ESI): 611.2 [M+1].

### Step 13

### Allyl

### (11aS)-8-((5-(((S)-10-((allyloxy)carbonyl)-7-cyclopropoxy-2-(4-methoxyphenyl)-5-oxo -5,10,11,11a-tetrahydro-1H-benzo[e]pyrrolo[1,2-a][1,4]diazepin-8-yl)oxy)pentyl)oxy)-1 1-((tert-butyldimethylsilyl)oxy)-7-methoxy-5-oxo-11,11a-dihydro-1H,3H-spiro[benzo[e ]pyrrolo[1,2-a][1,4]diazepine-2,1'-cyclopropane]-10(5H)-carboxylate 18m

The title product **18m** (45 mg, yield: 79.4%) was obtained by replacing the starting material **1j** of step 10 of Example 2-1 1 with **18l** (34 mg, 0.06 mmol) and using the same reaction conditions.

MS m/z (ESI): 1019.9 [M+1].

### Step 14

### Allyl

### (11aS)-8-((5-(((S)-10-((allyloxy)carbonyl)-7-cyclopropoxy-2-(4-methoxyphenyl)-5-oxo -5,10,11,11a-tetrahydro-1H-benzo[e]pyrrolo[1,2-a][1,4]diazepin-8-yl)oxy)pentyl)oxy)-1 1-hydroxy-7-methoxy-5-oxo-11,11a-dihydro-1H,3H-spiro[benzo[e]pyrrolo[1,2-a][1,4]d iazepine-2,1'-cyclopropane]-10(5H)-carboxylate 18n

The title product **18n** (40 mg, yield: 100%) was obtained by replacing the starting material **1l** of step 11 of Example 2-1 1 with **18m** (45 mg, 0.04 mmol) and using the same reaction conditions.

MS m/z (ESI): 905.7 [M+1].

### Step 15

### (S)-8-((5-(((S)-7-Cyclopropoxy-2-(4-methoxyphenyl)-5-oxo-5,10,11,11a-tetrahydro-1H -benzo[e]pyrrolo[1,2-a][1,4]diazepin-8-yl)oxy)pentyl)oxy)-7-methoxy-1,11a-dihydro-3 H,5H-spiro[benzo[e]pyrrolo[1,2-a][1,4]diazepine-2,1'-cyclopropan]-5-one 18

The title product **18** (20 mg, yield: 62.9%) was obtained by replacing the starting material **1m** of step 12 of Example 2-1 1 with **18n** (40 mg, 0.04 mmol) and using the same reaction conditions.

MS m/z (ESI): 719.6 [M+1].

¹H NMR (500 MHz, DMSO-d6) δ 7.91 (d, 1H), 7.56 (s, 1H), 7.45 (s, 1H), 7.38 (d, 2H), 7.33 (s, 1H), 6.91 (d, 2H), 6.85 (s, 1H), 6.55 (d, 1H), 6.31 (s, 1H), 4.24-4.18 (m, 1H), 4.14-4.08 (m, 1H), 4.05-3.99 (m, 1H), 3.96-3.91 (m, 2H), 3.82 (s, 3H), 3.76 (s, 3H), 3.73-3.70 (m, 1H), 3.62-3.58 (m, 1H), 3.57-3.52 (m, 1H), 3.50 (d, 1H), 3.27-3.22 (m, 1H), 2.79-2.74 (m, 1H), 2.45-2.40 (m, 1H), 2.06 (d, 1H), 2.03-1.97 (m, 1H), 1.84-1.78 (m, 4H), 1.60-1.53 (m, 2H), 1.37-1.33 (m, 1H), 0.86 (t, 1H), 0.81-0.76 (m, 1H), 0.69-0.60 (m, 6H).

### Example 2-19 19

### (S)-7-Cyclopropoxy-8-((5-(((S)-7-cyclopropoxy-2-(4-methoxyphenyl)-5-oxo-5,10,11,11 a-tetrahydro-1H-benzo[e]pyrrolo[1,2-a][1,4]diazepin-8-yl)oxy)pentyl)oxy)-1,11a-dihyd ro-3H,5H-spiro[benzo[e]pyrrolo[1,2-a][1,4]diazepine-2,1'-cyclopropan]-5-one 19

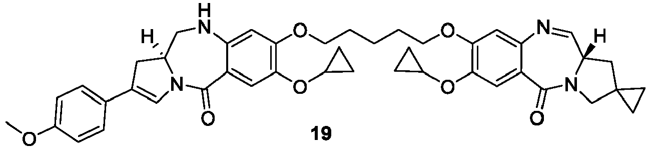

### Examples 2-20 20 and 21

### (1aS,9aR,10aS)-5-Methoxy-6-((5-(((S)-7-methoxy-2-(4-methoxyphenyl)-5-oxo-5, 10,11, 1 1a-tetrahydro-1H-benzo[e]pyrrolo[1,2-a][1,4]diazepin-8-yl)oxy)pentyl)oxy)-1a,9a,10, 10a-tetrahydrobenzo[e]cyclopropa[4,5]pyrrolo[1,2-a][1,4]diazepin-3(1H)-one and

### (1aS,9aS,10a,S)-5-Methoxy-6-((5-(((S)-7-methoxy-2-(4-methoxyphenyl)-5-oxo-5,10,11, 1 1a-tetrahydro-1H-benzo[e]pyrrolo[1,2-a][1,4]diazepin-8-yl)oxy)pentyl)oxy)-1a,9a,10, 10a-tetrahydrobenzo[e]cyclopropa[4,5]pyrrolo[1,2-a][1,4]diazepin-3(1H)-one

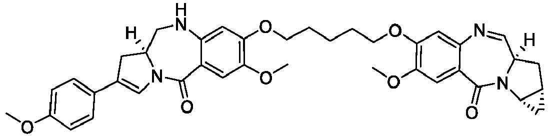

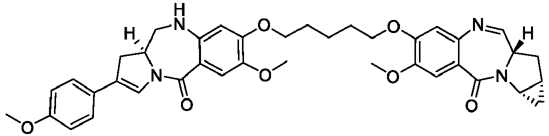

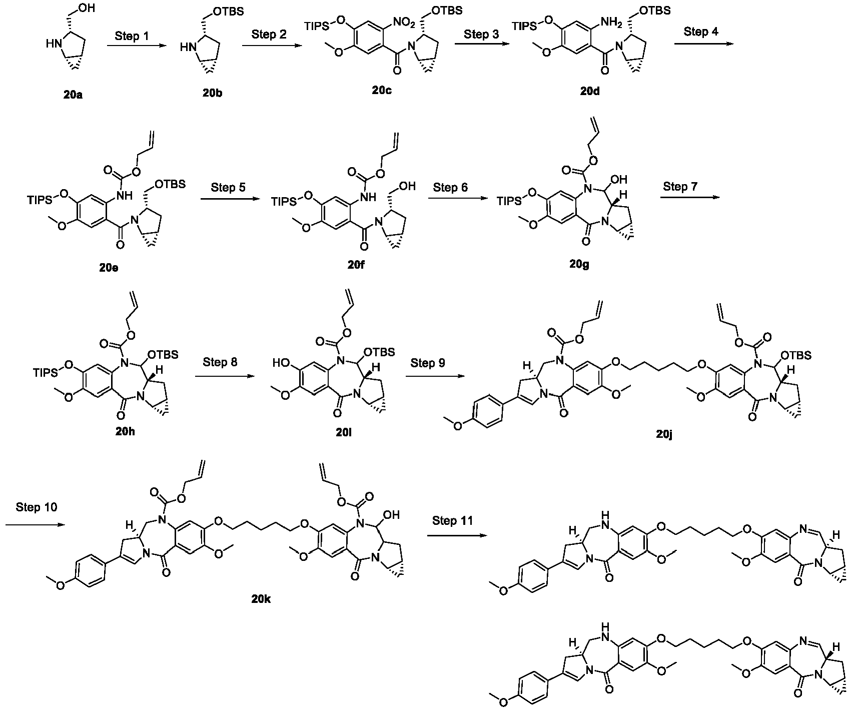

### Step 1

(1*S*,3*S*,5*S*)-3-(((tert-Butyldimethylsilyl)oxy)methyl)-2-azabicyclo[3.1.0]hexane **20b** ((1*S*,3*S*,5*S*)-2-Azabicyclo[3.1.0]hexan-3-yl)methanol **20a** (246 mg, 2.17 mmol, prepared by the method disclosed in Example 5 on page 5 of the specification of the patent application "CN104098481 A") was placed in a reaction flask and dissolved in dichloromethane (6 mL), and triethylamine (217 mg, 2.14 mmol) and 2,6-lutidine (455 mg, 4.25 mmol) were added. The temperature was lowered to 0 °C, and the reaction system was purged with nitrogen gas 3 times. tert-Butyldimethylsilyl trifluoromethanesulfonate (862 mg, 3.26 mmol) was added dropwise, and the mixture was warmed to room temperature and stirred for 2 h. The reaction mixture was quenched with ammonium chloride (8 mL) and separated. The organic phase was collected, washed with water (6 mL) and saturated sodium chloride solution (6 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **20b** (470 mg, yield: 94.5%).

MS m/z (ESI): 228.2 [M+1].

### Step 2

### ((1S,3S,5S)-3-(((tert-Butyldimethylsilyl)oxy)methyl)-2-azabicyclo[3.1.0]hexan-2-yl)(5-methoxy-2-nitro-4-((triisopropylsilyl)oxy)phenyl)methanone 20c 5-Methoxy-2-nitro-4-((triisopropylsilyl)oxy)benzoic acid (657 mg, 1.78 mmol, prepared by the method disclosed for compound 2 on page 60 of the specification of the patent application "WO2013053872 A1") was placed in a reaction flask, and dichloromethane (6 mL) and triethylamine (297 mg, 2.94 mmol) were added. N,N,N',N'-Tetramethyl-O-(7-azabenzotriazol-1-yl)uronium hexafluorophosphate (812 mg, 2.13 mmol, Accela ChemBio) was added under ice-bath conditions, and the mixture was warmed to room temperature and stirred for 1 h. Then the temperature was lowered to 0 °C, and a solution of 20b (450 mg, 1.98 mmol) and triethylamine (297 mg, 2.94 mmol) in dichloromethane (6 mL) was added dropwise to the reaction mixture. After the addition, the mixture was warmed to room temperature and stirred for 1 h, washed with water (10 mL) and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give the title product 20c (930 mg, yield: 90.3%).

MS m/z (ESI): 579.3 [M+1].

### Step 3

### (2-Amino-5-methoxy-4-((triisopropylsilyl)oxy)phenyl)((1S,3S,5S)-3-(((tert-butyldimeth ylsilyl)oxy)methyl)-2-azabicyclo[3.1.0]hexan-2-yl)methanone 20d

**20c** (903 mg, 1.56 mmol) was dissolved in ethanol (20 mL), and palladium on carbon (180 mg, content 10%, dry) was added. The reaction system was purged with hydrogen gas three times and stirred at room temperature for 2 h. The reaction mixture was filtered through celite, and the filtrate was concentrated under reduced pressure and dried *in vacuo* to give a crude product of the title product **20d** (866 mg).

MS m/z (ESI): 549.3 [M+1].

### Step 4

### Allyl

### (2-((1S,3S,5S)-3-(((tert-butyldimethylsilyl)oxy)methyl)-2-azabicyclo[3.1.0]hexane-2-car bonyl)-4-methoxy-5-((triisopropylsilyl)oxy)phenyl)carbamate 20e

**20d** (347 mg, 0.633 mmol) was dissolved in dichloromethane (8 mL), and pyridine (147 mg, 1.86 mmol) was added. The reaction system was purged with nitrogen gas 3 times and cooled to -65 °C, and allyl chloroformate (76 mg, 0.631 mmol) was slowly added dropwise. After the addition, the mixture was warmed to room temperature and stirred for 15 min. The reaction mixture was quenched with a 10% citric acid solution (6 mL) and separated. The organic phase was collected, washed with water (10 mL), a sodium bicarbonate solution (10 mL), and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **20e** (360 mg, yield: 90%).

MS m/z (ESI): 633.4 [M+1].

### Step 5

### Allyl

### (2-((1S,3S,5S)-3-(hydroxymethyl)-2-azabicyclo[3.1.0]hexane-2-carbonyl)-4-methoxy-5-((triisopropylsilyl)oxy)phenyl)carbamate 20f

**20e** (360 mg, 0.569 mmol) was dissolved in a mixed solution of tetrahydrofuran (2 mL), methanol (2 mL), and water (2 mL), and glacial acetic acid (8 mL) was added. The reaction system was purged with nitrogen gas 3 times and stirred at room temperature for 3 h. The reaction mixture was concentrated under reduced pressure to remove the organic phase, and the aqueous phase was extracted with ethyl acetate (10 mL × 3). The organic phases were combined, washed with water (10 mL), a sodium bicarbonate solution (10 mL), and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **20f** (266 mg, yield: 90.1%).

MS m/z (ESI): 519.2 [M+1].

### Step 6

### Allyl

### (1aS,9aS,10aS)-9-hydroxy-5-methoxy-3-oxo-6-((triisopropylsilyl)oxy)-1,1a,9,9a,10,10 a-hexahydrobenzo[e]cyclopropa[4,5]pyrrolo[1,2-a][1,4]diazepine-8(3H)-carboxylate 20g

**20f** (266 mg, 0.513 mmol) was dissolved in dichloromethane (8 mL). The reaction system was purged with nitrogen gas 3 times and cooled to 0 °C, and sodium bicarbonate (103.5 mg, 1.23 mmol) was added. Then (1,1,1-triacetoxy)-1,1-dihydro-1,2-benziodoxol-3(1*H*)-one (261 mg, 0.615 mmol, adamas) was added. After the addition, the mixture was warmed to room temperature and stirred for 1.5 h. The reaction mixture was diluted with dichloromethane (10 mL), washed with saturated sodium bicarbonate solution (10 mL), water (10 mL), and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **20g** (260 mg, yield: 98.1%).

MS m/z (ESI): 517.2 [M+1].

### Step 7

### Allyl

### (1aS,9aS,10aS)-9-((tert-butyldimethylsilyl)oxy)-5-methoxy-3-oxo-6-((triisopropylsilyl) oxy)-1,1a,9,9a,10,10a-hexahydrobenzo[e]cyclopropa[4,5]pyrrolo[1,2-a][1,4]diazepine-8(3H)-carboxylate 20h

**20g** (260 mg, 0.503 mmol) was dissolved in dichloromethane (8 mL). The reaction system was purged with nitrogen gas 3 times and cooled to -40 °C, and 4-dimethylaminopyridine (438.8 mg, 3.59 mmol) was added. tert-Butyldimethylsilyl trifluoromethanesulfonate (799 mg, 3.02 mmol) was added. After the addition, the cooling bath was removed, and the mixture was stirred at room temperature for 16 h. After the temperature was lowered to -40 °C, 4-dimethylaminopyridine (246 mg, 2.01 mmol) and tert-butyldimethylsilyl trifluoromethanesulfonate (399 mg, 1.51 mmol) were added. Then the mixture was warmed to room temperature and allowed to react for 6 h. The reaction mixture was quenched with ammonium chloride (15 mL) and separated. The organic phase was collected, washed with water (15 mL) and saturated sodium chloride solution (15 mL) in sequence, dried over anhydrous sodium sulfate, and filtered. Then the filtrate was concentrated under reduced pressure, and the resulting residue was purified using a CombiFlash preparative flash chromatograph with eluent system B to give the title product **20h** (209 mg, yield: 65.8%).

MS m/z (ESI): 631.3 [M+1].

### Step 8

### Allyl

### (1aS,9aS,10aS)-9-((tert-butyldimethylsilyl)oxy)-6-hydroxy-5-methoxy-3-oxo-1,1a,9,9a, 10,10a-hexahydrobenzo[e]cyclopropa[4,5]pyrrolo[1,2-a][1,4]diazepine-8(3H)-carboxyl ate 20i

**20h** (209 mg, 0.331 mmol) was dissolved in a mixed solution of *N*,*N*-dimethylformamide (3 mL) and water (0.06 mL), and lithium acetate (26.3 mg, 0.398 mmol) was added. The reaction system was purged with nitrogen gas 3 times and stirred at room temperature for 3 h. Water (10 mL) was added to the reaction mixture, and extraction was performed with ethyl acetate (10 mL × 3). The organic phases were combined, washed with water (10 mL) and saturated sodium chloride solution (10 mL) in sequence, dried over anhydrous sodium sulfate, and filtered. Then the filtrate was concentrated under reduced pressure, and the resulting residue was purified using a CombiFlash preparative flash chromatograph with eluent system B to give a crude product of the title product **20i** (160 mg).

MS m/z (ESI): 473.1 [M-1].

### Step 9

### Allyl

### (1aS,9aS,10aS)-6-((5-(((5)-10-((allyloxy)carbonyl)-7-methoxy-2-(4-methoxyphenyl)-5-oxo-5,10,11,11a-tetrahydro-1H-benzo[e]pyrrolo[1,2-a][1,4]diazepin-8-yl)oxy)pentyl)ox y)-9-((tert-butyldimethylsilyl)oxy)-5-methoxy-3-oxo-1,1a,9,9a,10,10a-hexahydrobenzo [e]cyclopropa[4,5]pyrrolo[1,2-a][1,4]diazepine-8(3H)-carboxylate 20j

The title product **20j** (41 mg, yield: 31.7%) was obtained by replacing the starting material 1k of step 10 of Example 2-1 1 with **20i** (71.8 mg, 0.151 mmol) and allyl (*S*)-8-((5-bromopentyl)oxy)-7-methoxy-2-(4-methoxyphenyl)-5-oxo-11,11*a*-dihydro-1*H* -benzo[*e*]pyrrolo[1,2-*a*][1,4]diazepine-10(5*H*)-carboxylate (77 mg, 0.131 mmol, prepared by the method disclosed for intermediate 3-10 on page 103 of the specification of the patent application "CN 111164208 A") and using the same reaction conditions. MS m/z (ESI): 979.3 [M+1].

### Step 10

### Allyl

### (1aS,10aS)-6-((5-(((5)-10-(allyloxy)carbonyl)-7-methoxy-2-(4-methoxyphenyl)-5-oxo-5 ,10,11,11a-tetrahydro-1H-benzo[e]pyrrolo[1,2-a][1,4]diazepin-8-yl)oxy)pentyl)oxy)-9-hydroxy-5-methoxy-3-oxo-1,1a,9,9a,10,10a-hexahydrobenzo[e]cyclopropa[4,5]pyrrolo [1,2-a][1,4]diazepine-8(3H)-carboxylate 20k

The title product **20k** (26.5 mg, yield: 100%) was obtained by replacing the starting material 1l of step 11 of Example 2-1 1 with **20j** (30 mg, 0.03 mmol) and using the same reaction conditions.

MS m/z (ESI): 865.3 [M+1].

### Step 11

### (1aS,9aR,10aS)-5-Methoxy-6-((5-(((S)-7-methoxy-2-(4-methoxyphenyl)-5-oxo-5,10,11, 11a-tetrahydro-1H-benzo[e]pyrrolo[1,2-a][1,4]diazepin-8-yl)oxy)pentyl)oxy)-1a,9a,10, 10a-tetrahydrobenzo[e]cyclopropa[4,5]pyrrolo[1,2-a][1,4]diazepin-3(1H)-one 20

### (1aS,9aS,10aS)-5-Methoxy-6-((5-(((S)-7-methoxy-2-(4-methoxyphenyl)-5-oxo-5,10,11, 11a-tetrahydro-1H-benzo[e]pyrrolo[1,2-a][1,4]diazepin-8-yl)oxy)pentyl)oxy)-1a,9a,10, 10a-tetrahydrobenzo[e]cyclopropa[4,5]pyrrolo[1,2-a][1,4]diazepin-3(1H)-one 21

A crude product of the title product was obtained by replacing the starting material 1m of step 12 of Example 2-1 1 with **20k** (26.5 mg, 0.03 mmol) and using the same reaction conditions. The crude product was purified by thin-layer chromatography with developing solvent system A to give the title product (8 mg, 12 mg).

### Single-configuration compound (shorter retention time):

MS m/z (ESI): 679.3 [M+1].

HPLC analysis: retention time 8.929 min, (column: Waters Sunfire C18 4.6 × 75 mm, 3.5 µm; mobile phases: A (%) 0.1% FA-H₂O/B (%) 0.08% FA-ACN = 70/5 ==> 30/95). ¹H NMR (500 MHz, DMSO-d6) δ 7.83 (d, 1H), 7.45 (s, 1H), 7.41-7.35 (m, 3H), 7.30 (s, 1H), 6.93-6.88 (m, 2H), 6.84-6.81 (m, 1H), 6.33-6.29 (m, 1H), 4.23-4.16 (m, 1H), 4.13-4.08 (m, 1H), 4.04-3.99 (m, 1H), 3.96-3.91 (m, 2H), 3.83 (s, 3H), 3.76 (s, 3H), 3.66 (s, 3H), 3.65-3.60 (m, 2H), 3.58-3.51 (m, 2H), 3.28-3.21 (m, 2H), 2.79-2.74 (m, 1H), 2.46-2.42 (m, 1H), 2.03-1.96 (m, 2H), 1.84-1.80 (m, 2H), 1.61-1.53 (m, 2H), 0.93-0.81 (m, 4H).

### Single-configuration compound (longer retention time):

MS m/z (ESI): 679.3 [M+1].

HPLC analysis: retention time 9.612 min. (Column: Waters Sunfire C18 4.6 × 75 mm, 3.5 µm; mobile phases: A (%) 0.1% FA-H₂O/B (%) 0.08% FA-ACN = 70/5 ==> 30/95). ¹H NMR (500 MHz, DMSO-d6) δ 7.72 (d, 1H), 7.45 (s, 1H), 7.40-7.36 (m, 2H), 7.31-7.27 (m, 2H), 6.93-6.89 (m, 2H), 6.79 (s, 1H), 6.31 (s, 1H), 4.22-4.17 (m, 1H), 4.13-4.04 (m, 2H), 4.03-3.97 (m, 1H), 3.96-3.91 (m, 2H), 3.82 (s, 3H), 3.76 (s, 3H), 3.74-3.71 (m, 1H), 3.67 (s, 3H), 3.58-3.52 (m, 2H), 3.29-3.21 (m, 2H), 2.79-2.73 (m, 1H), 2.42-2.36 (m, 1H), 2.03-1.96 (m, 2H), 1.84-1.80 (m, 2H), 1.61-1.53 (m, 2H), 1.05-0.99 (m, 2H), 0.88-0.83 (m, 2H).

### Example 2-22 22

### (S)-7-Methoxy-8-((5-(((S)-7-methoxy-5-oxo-5,11a-dihydro-1H,3H-spiro[benzo[e]pyrrol o[1,2-a][1,4]diazepine-2,1'-cyclopropan]-8-yl)oxy)pentyl)oxy)-2-(4-methoxyphenyl)-1, 11a-dihydro-5H-benzo[e]pyrrolo[1,2-a][1,4]diazepine-5,11(10H)-dione 22

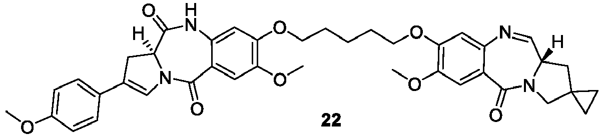

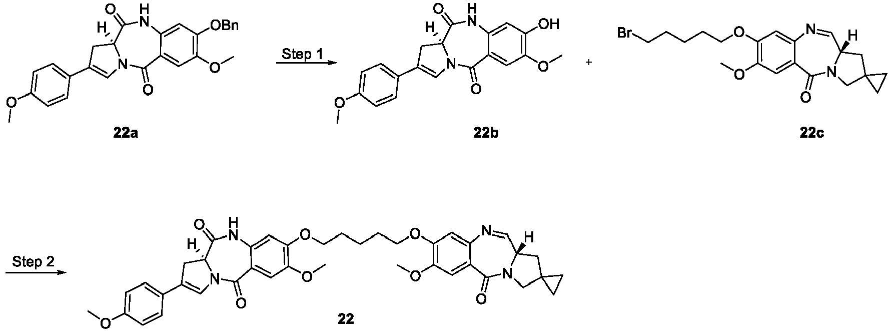

### Step 1

### (S)-8-Hydroxy-7-methoxy-2-(4-methoxyphenyl)-1,11a-dihydro-5H-benzo[e]pyrrolo[1,2 -a][1,4]diazepine-5,1 1(10H)-dione 22b

To a 100 mL single-necked flask was added the compound (*S*)-8-(benzyloxy)-7-methoxy-2-(4-methoxyphenyl)-1,11*a*-dihydro-5*H*-benzo[*e*]pyrrolo[ 1,2-*a*][1,4]diazepine-5,11(10*H*)-dione **22a** (300 mg, 657.18 µmol, prepared by the method disclosed for intermediate 005 on page 8 of the specification of the patent application "CN109180681A") followed by dichloromethane (10 mL). The mixture was stirred and cooled in an ice bath to 0-5 °C, and methanesulfonic acid (3 mL) was added dropwise. The mixture was stirred at 0-5 °C for 2 h. Water was added to the reaction mixture, and a white solid precipitated. The mixture was filtered under reduced pressure. The filter cake was rinsed with a small amount of water, collected, and dried *in vacuo* to give a crude product of the title product (180 mg). The obtained crude product was stirred with dichloromethane (2 mL × 2). The reaction mixture was filtered under reduced pressure. The filter cake was rinsed with a small amount of dichloromethane, collected, and dried *in vacuo* to give the title product **22b** (100 mg, yield: 42.0%).

MS m/z (ESI): 367.50 [M+1].

### Step 2

### (S)-7-Methoxy-8-((5-(((S)-7-methoxy-5-oxo-5,11a-dihydro-1H,3H-spiro[benzo[e]pyrrol o[1,2-a][1,4]diazepine-2,1'-cyclopropan]-8-yl)oxy)pentyl)oxy)-2-(4-methoxyphenyl)-1, 11a-dihydro-5H-benzo[e]pyrrolo[1,2-a][1,4]diazepine-5,11(10H)-dione 22

To a 10 mL single-necked flask were added compound **22b** (60 mg, 163.77 µmol) and the compound (*S*)-8-((5-bromopentyl)oxy)-7-methoxy-1,11*a*-dihydro-3*H*,5*H*-spiro[benzo[*e*]pyrrolo[1, 2-*a*][1,4]diazepine-2,1'-cyclopropan]-5-one **22c** (69 mg, 163.77 µmol, prepared by the method disclosed for intermediate 6-7 on page 105 of the specification of the patent application "CN113166764A"). Potassium carbonate (45 mg, 327.54 µmol) was added, and *N*,*N*-dimethylformamide (1 mL) was then added. The mixture was heated to 50 °C and stirred for 3 h. The reaction mixture was cooled to room temperature and purified by high performance liquid chromatography (GILSON, column: PNtulips C18, 30 × 150 mm, 5 µm; mobile phases: aqueous phase (20 mmol/L ammonium bicarbonate) and methanol, gradient ratio: methanol 70%-80%, flow rate: 30 mL/min). The corresponding fraction was collected to give the title product **22** (29 mg, yield: 25.2%).

MS m/z (ESI): 707.30 [M+1].

¹H-NMR (400 MHz, DMSO-d6) δ 7.33-7.44 (m, 5H), 7.16 (s, 1H), 6.91-6.93 (m, 3H), 6.68 (s, 1H), 6.51 (s, 1H), 4.67-4.70 (m, 1H), 4.05-4.10 (m, 4H), 3.78-3.80 (s, 6H), 3.80-3.82 (s, 3H), 3.15-3.27 (m, 3H), 2.39-2.47 (m, 1H), 2.21-2.31 (m, 1H),1.87-1.94 (m, 6H), 1.67-1.71 (m, 2H), 0.63-0.71 (m, 4H).

### Example 2-26 26

### (S)-7-Methoxy-8-(2-(2-(((S)-7-methoxy-2-(4-methoxyphenyl)-5-oxo-5,10,11,11a-tetrah ydro-1H-benzo[e]pyrrolo[1,2-a][1,4]diazepin-8-yl)oxy)ethoxy)ethoxy)-1,11a-dihydro-3 H,5H-spiro[benzo[e]pyrrolo[1,2-a][1,4]diazepine-2,1'-cyclopropan]-5-one 26

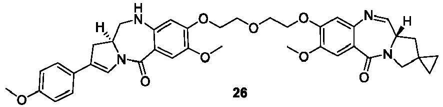

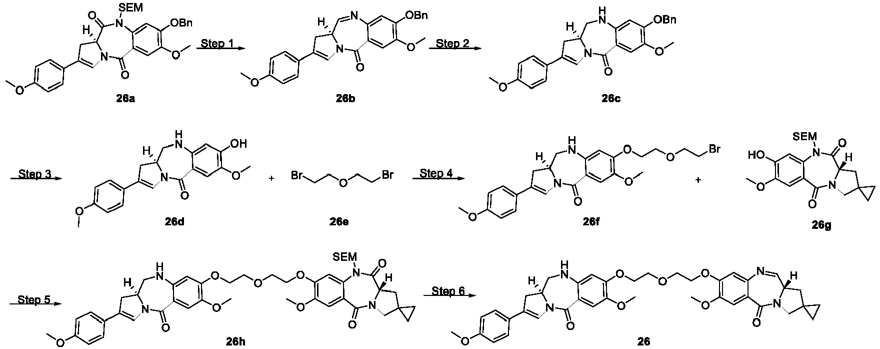

### Step 1

### (S)-8-(Benzyloxy)-7-methoxy-2-(4-methoxyphenyl)-1,11a-dihydro-5H-benzo[e]pyrrolo [1,2-a][1,4]diazepin-5-one 26b

Tetrahydrofuran (2 mL) was added to the starting material (*S*)-8-(benzyloxy)-7-methoxy-2-(4-methoxyphenyl)-10-((2-(trimethylsilyl)ethoxy)meth yl)-1,11*a*-dihydro-5*H*-benzo[*e*]pyrrolo[1,2-*a*][1,4]diazepine-5,11(10*H*)-dione **26a** (250 mg, 426.07 µmol, prepared by the method disclosed for intermediate 006 on page 8 of the specification of the patent application CN109180681 A). The mixture was stirred at room temperature, and the reaction system was purged with nitrogen gas 3 times and cooled in a dry ice-ethanol bath to -78 °C. Lithium tetraborate (2.13 mL, 4.26 mmol, 2 M in tetrahydrofuran) was added dropwise, and the mixture was stirred at -5 °C for 24 h. The reaction mixture was quenched with ice water (20 mL), and the aqueous phase was extracted with dichloromethane (20 mL × 3). The organic phases were combined, washed with saturated sodium chloride solution (20 mL), dried over anhydrous sodium sulfate, and filtered. Then the filtrate was concentrated under reduced pressure, and the resulting residue was purified by thin-layer chromatography with developing solvent system A to give the title product **26b** (118 mg).

MS m/z (ESI): 441.17 [M+1].

### Step 2

### (S)-8-(Benzyloxy)-7-methoxy-2-(4-methoxyphenyl)-1,10,11,11a-tetrahydro-5H-benzo[e ]pyrrolo[1,2-a][1,4]diazepin-5-one 26c

The starting material **26b** (118 mg, 267.88 µmol) was dissolved in dichloromethane (1 mL), and the solution was cooled in an ice bath to 0-5 °C. Sodium triacetoxyborohydride (114 mg, 535.76 µmol) was added portionwise, and the mixture was stirred at 0-5 °C for 1 h. The reaction mixture was directly purified by thin-layer chromatography with developing solvent system A to give the title product **26c** (90 mg). MS m/z (ESI): 443.19 [M+1].

### Step 3

### (S)-8-Hydroxy-7-methoxy-2-(4-methoxyphenyl)-1,10,11,11a-tetrahydro-5H-benzo[e]py rrolo[1,2-a][1,4]diazepin-5-one 26d

Compound **26c** (90 mg, 203.38 µmol) was dissolved in dichloromethane (2.8 mL). The reaction system was purged with nitrogen gas 3 times and cooled in an ice bath to 0-5 °C. Methanesulfonic acid (1 mL) was added dropwise, and the mixture was stirred at 0-5 °C for 2 h. The reaction mixture was quenched with ice water (20 mL), and the aqueous phase was extracted with dichloromethane (20 mL × 3). The organic phases were combined, washed with saturated sodium chloride solution (20 mL), dried over anhydrous sodium sulfate, and filtered. Then the filtrate was concentrated under reduced pressure, and the resulting residue was purified by thin-layer chromatography with developing solvent system A to give the title product **26d** (20 mg).

MS m/z (ESI): 353.15 [M+1].

### Step 4

### (S)-8-(2-(2-Bromoethoxy)ethoxy)-7-methoxy-2-(4-methoxyphenyl)-1,10,11,11a-tetrahy dro-5H-benzo[e]pyrrolo[1,2-a][1,4]diazepin-5-one 26f

To a 10 mL single-necked flask were added the starting materials **26d** (35 mg, 99.32 µmol), 1-bromo-2-(2-bromoethoxy)ethane **26e** (115 mg, 496.61 µmol), and potassium carbonate (82 mg, 595.93 µmol) followed by *N*,*N*-dimethylformamide (0.5 mL). The mixture was heated to 50 °C and stirred for 3 h. Water (5 mL) was added to the reaction mixture, and the aqueous phase was extracted with dichloromethane (5 mL × 3). The organic phases were combined, washed with saturated sodium chloride solution (5 mL), dried over anhydrous sodium sulfate, and filtered. Then the filtrate was concentrated under reduced pressure, and the resulting residue was purified by thin-layer chromatography with developing solvent system A to give the title product **26f** (30 mg). MS m/z (ESI): 503.25 [M+1].

### Step 5

### (S)-7-Methoxy-8-(2-(2-(((S)-7-methoxy-2-(4-methoxyphenyl)-5-oxo-5,10,11,11a-tetrah ydro-1H-benzo[e]pyrrolo[1,2-a][1,4]diazepin-8-yl)oxy)ethoxy)ethoxy)-10-((2-(trimethy lsilyl)ethoxy)methyl)-1,11a-dihydro-3H,5H-spiro[benzo[e]pyrrolo[1,2-a][1,4]diazepine -2,1'-cyclopropane]-5,11(10H)-dione 26h

To a 10 mL single-necked flask were added the starting materials **26f** (30 mg, 59.60 µmol),
(*S*)-8-hydroxy-7-methoxy-10-((2-(trimethylsilyl)ethoxy)methyl)-1,11*a*-dihydro-3*H*,5*H-*spiro[benzo[*e*]pyrrolo[1,2-*a*][1,4]diazepine-2,1'-cyclopropane]-5,11(10*H*)-dione **26g** (25 mg, 59.60 µmol, prepared by the method disclosed for intermediate 6-5 on page 105 of the specification of the patent application "CN113166764A"), and potassium carbonate (16 mg, 119.19 µmol) followed by *N*,*N*-dimethylformamide (0.5 mL). The mixture was heated to 50 °C and stirred for 3 h. Water (5 mL) was added to the reaction mixture, and the aqueous phase was extracted with dichloromethane (5 mL × 3). The organic phases were combined, washed with saturated sodium chloride solution (5 mL), dried over anhydrous sodium sulfate, and filtered. Then the filtrate was concentrated under reduced pressure, and the resulting residue was purified by thin-layer chromatography with developing solvent system A to give the title product **26h** (40 mg).

MS m/z (ESI): 841.55 [M+1].

### Step 6

### (S)-7-Methoxy-8-(2-(2-(((S)-7-methoxy-2-(4-methoxyphenyl)-5-oxo-5,10,11,11a-tetrah ydro-1H-benzo[e]pyrrolo[1,2-a][1,4]diazepin-8-yl)oxy)ethoxy)ethoxy)-1,11a-dihydro-3 H,5H-spiro[benzo[e]pyrrolo[1,2-a][1,4]diazepine-2,1 '-cyclopropan] -5-one 26

To a 25 mL reaction tube was added the starting material **26h** (40 mg, 47.56 µmol) followed by tetrahydrofuran (2 mL). The mixture was stirred, and the reaction system was purged with nitrogen gas 3 times and cooled in a dry ice-ethanol bath to -78 °C. Lithium tetraborate (0.71 mL, 1.43 mmol, 2 M in tetrahydrofuran) was added dropwise, and the mixture was stirred at -5 °C for 7 h. The reaction mixture was quenched with ice water (5 mL), and the aqueous phase was extracted with dichloromethane (5 mL × 3).

The organic phases were combined, washed with saturated sodium chloride solution (5 mL), dried over anhydrous sodium sulfate, and filtered. Then the filtrate was concentrated under reduced pressure, and the resulting residue was purified by high performance liquid chromatography (GILSON, column: PNtulips C18, 30 × 150 mm, 5 µm; mobile phases: aqueous phase (20 mmol/L ammonium bicarbonate) and methanol, gradient ratio: methanol 75%-90%, flow rate: 30 mL/min) to give the title product **26** (8.7 mg, yield: 26.3%).

MS m/z (ESI): 695.30 [M+1].

¹H-NMR (400 MHz, DMSO-d6) δ 7.45-7.47 (m, 5H), 7.18-7.19 (m, 1H), 6.87-6.99 (m, 3H), 6.31 (s, 1H), 4.20-4.25 (m, 1H), 3.92-3.97 (m, 4H), 3.38-3.90 (m, 12H), 3.63-3.64 (m, 1H), 3.35-3.44(m, 4H), 3.21-3.30(m, 2H), 2.74-2.84 (m, 1H), 2.40-2.48 (m, 1H), 2.01-2.09(m, 1H), 0.63-0.79(m, 4H).

### Example 2-27 27

### (S)-7-Methoxy-8-((5-(((S)-7-methoxy-2-(4-methoxyphenyl)-5-oxo-5,10,11,11a-tetrahyd ro-1H-benzo[e]pyrrolo[1,2-a][1,4]diazepin-8-yl)oxy)pentyl)oxy)-1,10,11,11a-tetrahydr o-3H,5H-spiro[benzo[e]pyrrolo[1,2-a][1,4]diazepine-2,1'-cyclopropan]-5-one 27

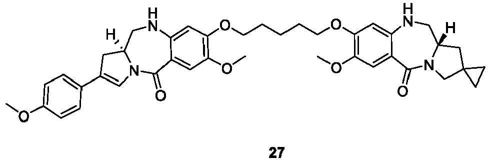

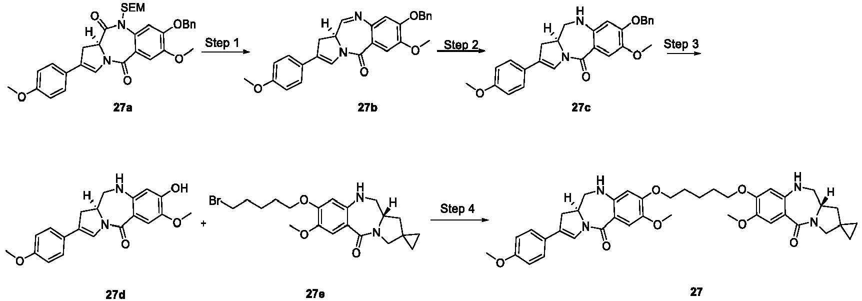

### Step 1

### (S)-8-(Benzyloxy)-7-methoxy-2-(4-methoxyphenyl)-1,11a-dihydro-5H-benzo[e]pyrrolo [1,2-a][1,4]diazepin-5-one 27b

To a 25 mL reaction tube was added (*S*)-8-(benzyloxy)-7-methoxy-2-(4-methoxyphenyl)-10-((2-(trimethylsilyl)ethoxy)meth yl)-1,11*a*-dihydro-5*H*-benzo[*e*]pyrrolo[1,2-*a*][1,4]diazepine-5,11(10*H*)-dione **27a** (250 mg, 426.07 µmol, prepared by the method disclosed for compound 006 on page 8 of the specification of the patent application "CN109180681A") followed by tetrahydrofuran (2 mL). The mixture was stirred, and the reaction system was purged with nitrogen gas 3 times and cooled in a dry ice-ethanol bath to -78 °C. Lithium tetraborate (2.13 mL, 4.26 mmol, 2.0 M in tetrahydrofuran) was added dropwise, and the mixture was stirred at -5 °C for 24 h. The reaction mixture was quenched with ice water (20 mL), and the aqueous phase was extracted with dichloromethane (20 mL × 3). The organic phases were combined, washed with saturated sodium chloride solution (20 mL), dried over anhydrous sodium sulfate, and filtered. Then the filtrate was concentrated under reduced pressure, and the resulting residue was purified by silica gel column chromatography with eluent system A to give the title product **27b** (118 mg).

MS m/z (ESI): 441.17 [M+1].

### Step 2

### (S)-8-(Benzyloxy)-7-methoxy-2-(4-methoxyphenyl)-1,10,11,11a-tetrahydro-5H-benzo[e ]pyrrolo[1,2-a][1,4]diazepin-5-one 27c

The starting material **27b** (118 mg, 267.88 µmol) was added to a 25 mL single-necked flask and dissolved in dichloromethane (1 mL) with stirring, and the solution was cooled in an ice bath to 0-5 °C. Sodium triacetoxyborohydride (114 mg, 535.76 µmol) was added portionwise, and the mixture was stirred at 0-5 °C for 1 h. The reaction mixture was purified by thin-layer chromatography with developing solvent system A to give the title product **27c** (90 mg).

MS m/z (ESI): 443.19 [M+1].

### Step 3

### (S)-8-Hydroxy-7-methoxy-2-(4-methoxyphenyl)-1,10,11,11a-tetrahydro-5H-benzo[e]py rrolo[1,2-a][1,4]diazepin-5-one 27d

The starting material **27c** (90 mg, 203.38 µmol) was added to a 100 mL single-necked flask and dissolved in dichloromethane (2.8 mL) with stirring. The reaction system was purged with nitrogen gas 3 times and cooled in an ice bath to 0-5 °C. Methanesulfonic acid (1 mL) was added dropwise, and the mixture was stirred at 0-5 °C for 2 h. The reaction mixture was quenched with ice water (20 mL), and the aqueous phase was extracted with dichloromethane (20 mL × 3). The organic phases were combined, washed with saturated sodium chloride solution (20 mL), dried over anhydrous sodium sulfate, and filtered. Then the filtrate was concentrated under reduced pressure, and the resulting residue was purified by thin-layer chromatography with developing solvent system A to give the title product **27d** (20 mg).

MS m/z (ESI): 353.15 [M+1].

### Step 4

### (S)-7-Methoxy-8-((5-(((S)-7-methoxy-2-(4-methoxyphenyl)-5-oxo-5,10,11,11a-tetrahyd ro-1H-benzo[e]pyrrolo[1,2-a][1,4]diazepin-8-yl)oxy)pentyl)oxy)-1,10,11,11a-tetrahydr o-3H,5H-spiro[benzo[e]pyrrolo[1,2-a][1,4]diazepine-2,1 '-cyclopropan]-5-one 27

**27d** (15 mg, 0.426 mmol) was placed in a reaction flask, and the compound (*S*)-8-((5-bromopentyl)oxy)-7-methoxy-1,10,11,11*a*-tetrahydro-3*H*,5*H*-spiro[benzo[*e*]py rrolo[1,2-*a*][1,4]diazepine-2,1'-cyclopropan]-5-one **27e** (34 mg, 0.639 mmol, prepared by the method disclosed for intermediate 6-7 on page 124 of the specification of the patent application "CN113166764, A") was added. Potassium carbonate (10 mg, 0.724 mmol) was added, and *N*,*N*-dimethylformamide (0.5 mL) was then added. The reaction system was purged with nitrogen gas three times, heated to 50 °C, and stirred for 3 h. The reaction mixture was cooled to room temperature and directly purified by high performance liquid chromatography (GILSON, column: PNtulips C18, 30 × 150 mm, 5 µm; mobile phases: aqueous phase (20 mmol/L ammonium bicarbonate) and acetonitrile, gradient ratio: acetonitrile 40%-70%, flow rate: 30 mL/min) to give the title product **27** (16 mg, yield: 54.2%).

MS m/z (ESI): 695.4 [M+1].

¹H-NMR (400 MHz, DMSO) δ 7.42-7.47 (m, 1H), 7.34-7.40 (m, 2H), 7.27-7.343 (m, 2H), 6.87-6.93 (m, 2H), 6.50-6.57 (d, 1H), 6.28-6.32 (d, 2H), 6.23-6.28 (d, 1H), 4.13-4.29 (m, 1H), 3.88-3.99 (m, 4H), 3.79-3.87 (m, 1H), 3.72-3.78 (s, 3H), 3.60-3.69 (s, 6H), 3.43-3.57 (m, 3H), 3.08-3.31 (m, 4H), 2.69-2.81 (m, 1H), 1.91-2.01(m, 1H), 1.76-1.86 (m, 5H), 1.50-1.61 (m, 2H), 0.46-0.69 (m, 4H).

### Example 2-29 29

### (S)-7-Methoxy-8-((5-((((S)-7-methoxy-2-(4-(methoxyphenyl)-5-oxo-5,10,11,11a-tetrah ydro-1H-benzo[e]pyrrolo[1,2-a][1,4]diazepin-8-yl)oxy)methyl)pyridin-3-yl)methoxy)-1 ,11a-dihydro-3H,5H-spiro[benzo[e]pyrrolo[1,2-a][1,4]diazepine-2,1'-cyclopropan]-5-on e 29

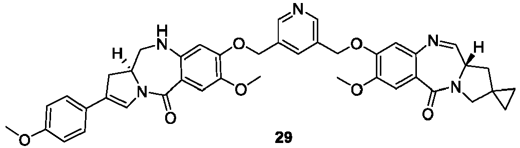

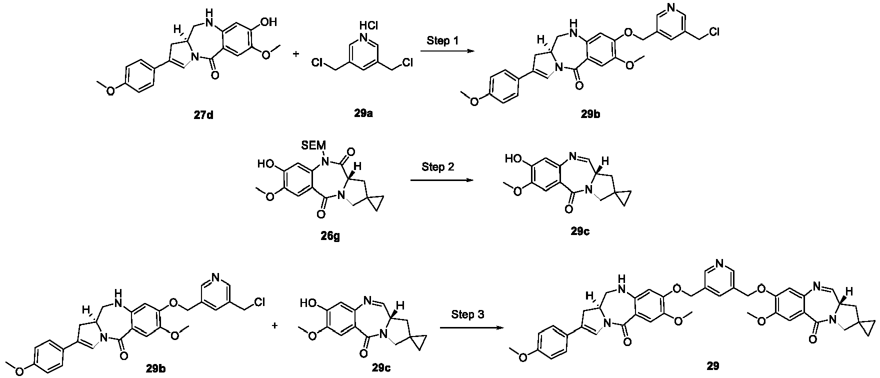

### Step 1

### (S)-8-((5-(Chloromethyl)pyridin-3-yl)methoxy)-7-methoxy-2-(4-methoxyphenyl)-1,10,1 1,11a-tetrahydro-5H-benzo[e]pyrrolo[1,2-a][1,4]diazepin-5-one 29b

To a 10 mL single-necked flask were added the starting materials **27d** (50 mg, 141.89 µmol), 3,5-bis(chloromethyl)pyridine hydrochloride **29a** (75 mg, 354.72 µmol), and potassium carbonate (78 mg, 567.55 µmol) followed by *N*,*N*-dimethylformamide (0.5 mL). The mixture was heated to 50 °C and stirred for 3 h. The reaction mixture was directly purified by high performance liquid chromatography (column: Batch ODS-C18, 80 g Flash; mobile phases: aqueous phase and acetonitrile, gradient ratio: acetonitrile 0%-60%, flow rate: 50 mL/min) to give the title product **29b** (27 mg).

MS m/z (ESI): 492.20 [M+1].

### Step 2

### (S)-8-Hydroxy-7-methoxy-1,11a-dihydro-3H,5H-spiro[benzo[e]pyrrolo[1,2-a][1,4]diaz epine-2,1'-cyclopropan]-5-one 29c

The starting material **26g** (1 g, 2.39 mmol) was added to a 250 mL three-necked flask and dissolved in tetrahydrofuran (20 mL) with stirring. The reaction system was purged with nitrogen gas 3 times and cooled in a dry ice-ethanol bath to -78 °C. Lithium triacetoxyborohydride (24 mL, 23.9 mmol, 1.0 M in tetrahydrofuran) was added dropwise, and the mixture was stirred at -78 °C for 1 h. After the reaction was complete, the reaction mixture was slowly poured into ice water (200 mL). The aqueous phase was extracted with dichloromethane (200 mL × 3), and the organic phases were combined, washed with saturated sodium chloride solution (100 mL), dried over anhydrous sodium sulfate, and filtered. Then the filtrate was concentrated under reduced pressure to give the title product **29c** (870 mg).

Ms (ESI): m/z 273.12 [M+1].

### Step 3

### (S)-7-Methoxy-8-((5-((((S)-7-methoxy-2-(4-(methoxyphenyl)-5-oxo-5,10,11,11a-tetrah ydro-1H-benzo[e]pyrrolo[1,2-a][1,4]diazepin-8-yl)oxy)methyl)pyridin-3-yl)methoxy)-1 ,11a-dihydro-3H,5H-spiro[benzo[e]pyrrolo[1,2-a][1,4]diazepine-2,1'-cyclopropan]-5-on e 29

To a 10 mL single-necked flask were added the starting materials **29b** (27 mg, 54.88 µmol), **29c** (25 mg, 54.88 µmol), and potassium carbonate (19 mg, 137.20 µmol) followed by *N*,*N*-dimethylformamide (0.5 mL). The mixture was heated to 50 °C and stirred for 3 h. The reaction mixture was directly purified by high performance liquid chromatography (GILSON, column: Xtimate C18, 30 × 150 mm, 5 µm; mobile phases: aqueous phase (20 mmol/L ammonium bicarbonate) and methanol, gradient ratio: methanol 65%-80%, flow rate: 30 mL/min) to give the title product **29** (2.1 mg).

MS m/z (ESI): 728.30 [M+1].

¹H-NMR (400 MHz, CD3OD) δ 8.60 (s, 2H), 8.02 (s, 2H),7.32-7.46 (m, 4H), 7.20 (s, 1H), 6.88-6.90 (m, 2H), 6.66 (s, 1H), 6.34 (s, 1H), 5.17-5.21 (m, 5H), 4.18-4.28 (m, 1H), 3.75-3.82 (m,9H), 3.70-3.75 (m, 1H), 3.55-3.68 (m, 2H), 3.38-3.42(m, 1H), 3.23-3.26(m, 1H),2.73-2.85 (m, 1H), 2.37-2.45 (m, 1H),1.23-1.38 (m, 2H), 0.60-0.69 (m, 4H).

### Example 2-30 30

### (S)-8-((5-(((S)-2-(4-(4-(2-Hydroxyethyl)piperazin-1-yl)phenyl)-7-methoxy-5-oxo-5,10,1 1,11a-tetrahydro-1H-benzo[e]pyrrolo[1,2-a][1,4]diazepin-8-yl)oxy)pentyl)oxy)-7-meth oxy-1,11a-dihydro-3H,5H-spiro[benzo[e]pyrrolo[1,2-a][1,4]diazepine-2,1'-cyclopropan ]-5-one 30

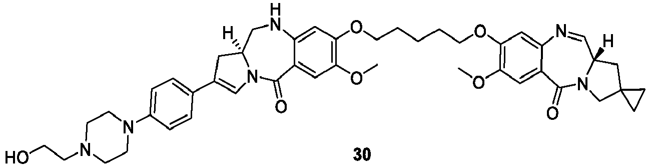

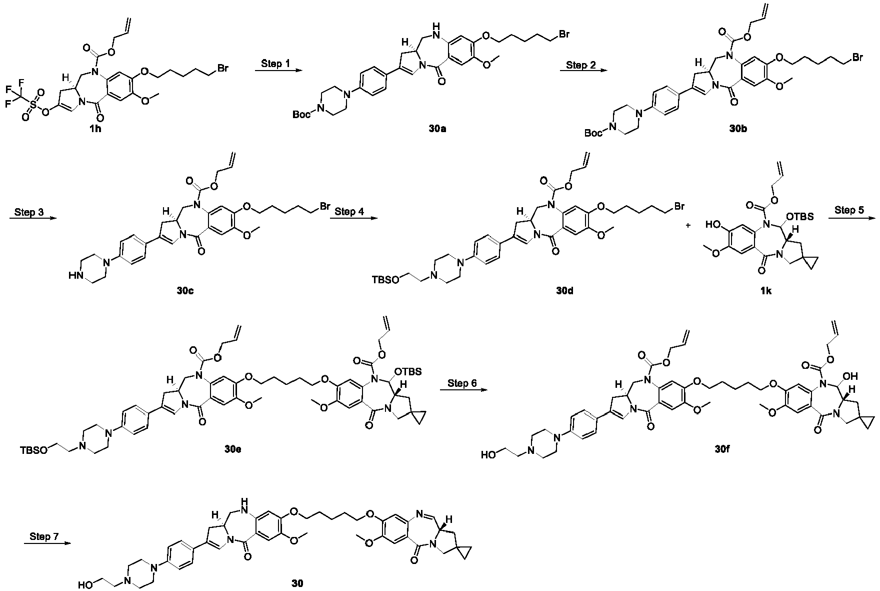

### Step 1

### tert-Butyl

### (S)-4-(4-(8-((5-bromopentyl)oxy)-7-methoxy-5-oxo-5,10,11,11a-tetrahydro-1H-benzo[e ]pyrrolo[1,2-a][1,4]diazepin-2-yl)phenyl)piperazine-1-carboxylate 30a

The title product **30a** (228 mg, yield: 62.3%) was obtained by replacing the starting material 4-(4-methyl-1-piperazinyl)phenylboronic acid of step 8 of Example 2-1 1 with 4-(4-tert-butoxycarbonylpiperazinyl)phenylboronic acid pinacol ester and using the same conditions.

MS m/z (ESI): 655.5 [M+1].

### Step 2

### Allyl

### (S)-8-((5-bromopentyl)oxy)-2-(4-(4-(tert-butoxycarbonyl)piperazin-1-yl)phenyl)-7-meth oxy-5-oxo-11,11a-dihydro-1H-benzo[e]pyrrolo[1,2-a][1,4]diazepine-10(5H)-carboxylat e 30b

The title product **30b** (204 mg, yield: 79.3%) was obtained by replacing the starting material **1i** of step 9 of Example 2-1 1 with **30a** (228 mg, 0.04 mmol) and using the same reaction conditions.

MS m/z (ESI): 739.5 [M+1].

### Step 3

### Allyl

### (S)-8-((5-bromopentyl)oxy)-7-methoxy-5-oxo-2-(4-(piperazin-1-yl)phenyl)-11,11a-dihy dro-1H-benzo[e]pyrrolo[1,2-a][1,4]diazepine-10(5H)-carboxylate 30c

**30b** (100 mg, 0.14 mmol) was dissolved in anhydrous dichloromethane (1 mL), and trifluoroacetic acid (0.3 mL) was added. The mixture was stirred at room temperature for 1 h. The reaction mixture was concentrated under reduced pressure to remove the organic solvent and dried using an oil pump. The residue was dissolved in ethyl acetate (20 mL), and the solution was washed with saturated sodium bicarbonate solution (10 mL × 2) and saturated sodium chloride solution (10 mL) in sequence. The organic phase was dried over anhydrous sodium sulfate and filtered. Then the filtrate was concentrated under reduced pressure, and the residue was dried *in vacuo* to give a crude product of the title product **30c** (70 mg, yield: 80.7%). The product was directly used in the next step without purification.

MS m/z (ESI): 639.5 [M+1].

### Step 4

### Allyl

### (S)-8-((5-bromopentyl)oxy)-2-(4-(4-(2-((tert-butyldimethylsilyl)oxy)ethyl)piperazin-1-y l)phenyl)-7-methoxy-5-oxo-11,11a-dihydro-1H-benzo[e]pyrrolo[1,2-a][1,4]diazepine-1 0(5H)-carboxylate 30d

**30c** (70 mg, 0.11 mmol) was dissolved in anhydrous tetrahydrofuran (3 mL), and (tert-butyldimethylsilyloxy)acetaldehyde (96 mg, 0.55 mmol, Acros) was added. The mixture was stirred at room temperature for 30 min. Sodium triacetoxyborohydride (140 mg, 0.66 mmol) was slowly added to the reaction mixture. After the addition, the mixture was stirred at room temperature for another 2 h. Saturated aqueous sodium bicarbonate solution (10 mL) was added to the reaction mixture, and extraction was performed with ethyl acetate (20 mL × 3). The organic phases were combined, washed with saturated sodium chloride solution (10 mL), dried over anhydrous sodium sulfate, and filtered. Then the filtrate was concentrated under reduced pressure, and the resulting residue was purified by thin-layer chromatography with developing solvent system A to give the title product **30d** (60 mg, yield: 68.7%).

MS m/z (ESI): 797.5 [M+1].

### Step 5

### Allyl

### (11aS)-8-((5-(((S)-10-((allyloxy)carbonyl)-2-(4-(4-(2-((tert-butyldimethylsilyl)oxy)ethyl )piperazin-1-yl)phenyl)-7-methoxy-5-oxo-5,10,11,11a-tetrahydro-1H-benzo[e]pyrrolo[1 ,2-a][1,4]diazepin-8-yl)oxy)pentyl)oxy)-11-((tert-butyldimethylsilyl)oxy)-7-methoxy-5-oxo-11,11a-dihydro-1H,3H-spiro[benzo[e]pyrrolo[1,2-a][1,4]diazepine-2,1'-cyclopropa ne]-10(5H)-carboxylate 30e

The title product **30e** (62 mg, yield: 68.4%) was obtained by replacing the starting material **1j** of step 10 of Example 2-1 1 with **30d** (45 mg, 0.07 mmol) and using the same reaction conditions.

MS m/z (ESI): 1205.7 [M+1].

### Step 6

### Allyl

### (11aS)-8-((5-(((S)-10-((allyloxy)carbonyl)-2-(4-(4-(2-hydroxyethyl)piperazin-1-yl)phen yl)-7-methoxy-5-oxo-5,10,11,11a-tetrahydro-1H-benzo[e]pyrrolo[1,2-a][1,4]diazepin-8 -yl)oxy)pentyl)oxy)-11-hydroxy-7-methoxy-5-oxo-11,11a-dihydro-1H,3H-spiro[benzo[ e]pyrrolo[1,2-a][1,4]diazepine-2,1'-cyclopropane]-10(5H)-carboxylate 30f

The title product **30f** (20 mg, yield: 39.8%) was obtained by replacing the starting material **11** of step 11 of Example 2-1 1 with **30e** (62 mg, 0.05 mmol) and using the same reaction conditions.

MS m/z (ESI): 978.0 [M+1].

### Step 7

### (S)-8-((5-(((S)-2-(4-(4-(2-Hydroxyethyl)piperazin-1-yl)phenyl)-7-methoxy-5-oxo-5,10,1 1,11a-tetrahydro-1H-benzo[e]pyrrolo[1,2-a][1,4]diazepin-8-yl)oxy)pentyl)oxy)-7-meth oxy-1,11a-dihydro-1H,3H-spiro[benzo[e]pyrrolo[1,2-a][1,4]diazepine-2,1'-cyclopropan ]-5-one 30

The title product **30** (10 mg, yield: 61.8%) was obtained by replacing the starting material **1m** of step 12 of Example 2-1 1 with **30f** (20 mg, 0.02 mmol) and using the same reaction conditions.

MS m/z (ESI): 791.6 [M+1].

¹H NMR (500 MHz, DMSO-d6) δ 7.92 (d, 1H), 7.39 (s, 1H), 7.33 (s, 1H), 7.29 (d, 2H), 7.21-7.15 (m, 1H), 6.90 (d, 2H), 6.85 (s, 1H), 6.68-6.62 (m, 1H), 6.54-6.50 (m, 1H), 6.32 (s, 1H), 5.33 (t, 1H), 4.44-4.40 (m, 1H), 4.22-4.09 (m, 4H), 4.05-4.01 (m, 1H), 3.97-3.90 (m, 3H), 3.82 (s, 2H), 3.67 (s, 3H), 3.57-3.49 (m, 4H), 3.27-3.20 (m, 2H), 3.17-3.09 (m, 4H), 2.03-1.95 (m, 3H), 1.86-1.78 (m, 3H), 1.62-1.55 (m, 2H), 1.49-1.44 (m, 2H), 1.35 (d, 1H), 0.86 (t, 3H), 0.80-0.76 (m, 1H), 0.69-0.64 (m, 2H).

### Example 2-3131

### (S)-8-((5-(((S)-2-(4-(4-((R)-2,3-Dihydroxypropyl)piperazin-1-yl)phenyl)-7-methoxy-5-o xo-5,10,11,11a-tetrahydro-1H-benzo[e]pyrrolo[1,2-a][1,4]diazepin-8-yl)oxy)pentyl)oxy )-7-methoxy-1,11a-dihydro-3H,5H-spiro[benzo[e]pyrrolo[1,2-a][1,4]diazepine-2,1'-cycl opropan]-5-one 31

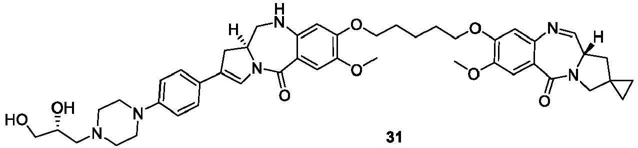

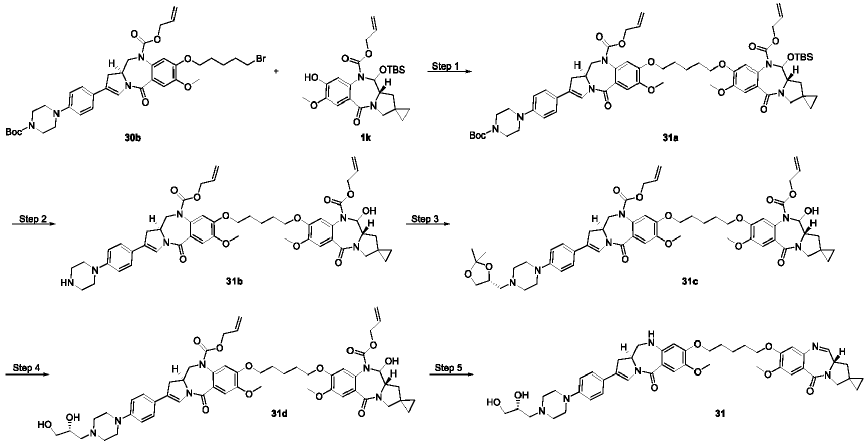

### Step 1

### Allyl

### (11aS)-8-((5-(((S)-10-((allyloxy)carbonyl)-2-(4-(4-(tert-butoxycarbonyl)piperazin-1-yl) phenyl)-7-methoxy-5-oxo-5,10,11,11a-tetrahydro-1H-benzo[e]pyrrolo[1,2-a][1,4]diaze pin-8-yl)oxy)pentyl)oxy)-11-((tert-butyldimethylsilyl)oxy)-7-methoxy-5-oxo-11,11a-di hydro-1H,3H-spiro[benzo[e]pyrrolo[1,2-a][1,4]diazepine-2,1'-cyclopropane]-10(5H)-ca rboxylate 31a

The title product **31a** (360 mg, yield: 89.3%) was obtained by replacing the starting material **1j** of step 10 of Example 2-1 1 with **30b** (260 mg, 0.35 mmol) and using the same reaction conditions.

MS m/z (ESI): 1147.1 [M+1].

### Step 2

### Allyl

### (11aS)-8-((5-(((S)-10-((allyloxy)carbonyl)-7-methoxy-5-oxo-2-(4-(piperazin-1-yl)pheny l)-5,10,11,11a-tetrahydro-1H-benzo[e]pyrrolo[1,2-a][1,4]diazepin-8-yl)oxy)pentyl)oxy) -11-hydroxy-7-methoxy-5-oxo-11,11a-dihydro-1H,3H-spiro[benzo[e]pyrrolo[1,2-a][1,4 ]diazepine-2,1'-cyclopropane]-10(5H)-carboxylate 31b

A crude product of the title product **31b** (300 mg) was obtained by replacing the starting material **30b** of step 3 of Example 2-30 30 with **31a** (360 mg, 0.31 mmol) and using the same reaction conditions.

MS m/z (ESI): 933.5 [M+1].

### Step 3

### Allyl

### (11aS)-8-((5-(((S)-10-((allyloxy)carbonyl)-2-(4-(4-(((R)-2,2-dimethyl-1,3-dioxolan-4-yl )methyl)piperazin-1-yl)phenyl)-7-methoxy-5-oxo-5,10,11,11a-tetrahydro-1H-benzo[e]p yrrolo[1,2-a][1,4]diazepin-8-yl)oxy)pentyl)oxy)-11-hydroxy-7-methoxy-5-oxo-11,11a-d ihydro-1H,3H-spiro[benzo[e]pyrrolo[1,2-a][1,4]diazepine-2,1'-cyclopropane]-10(5H)-c arboxylate 31c

**31b** (65 mg, 0.07 mmol) was dissolved in anhydrous tetrahydrofuran (4 mL), and (*S*)-2,2-dimethyl-1,3-dioxolane-4-carboxaldehyde (200 mg, 0.77 mmol, Bide) was added. The mixture was stirred at room temperature for 30 min. Sodium triacetoxyborohydride (115 mg, 0.54 mmol) was slowly added to the reaction mixture. After the addition, the mixture was stirred at room temperature for another 1 h. Saturated aqueous sodium bicarbonate solution (10 mL) was added to the reaction mixture, and extraction was performed with ethyl acetate (20 mL × 3). The organic phases were combined, washed with saturated sodium chloride solution (10 mL), dried over anhydrous sodium sulfate, and filtered. Then the filtrate was concentrated under reduced pressure, and the resulting residue was purified by thin-layer chromatography with developing solvent system A to give the title product **31c** (60 mg, yield: 82.2%).

MS m/z (ESI): 1045.7 [M-1].

### Step 4

### Allyl

### (11aS)-8-((5-(((S)-10-((allyloxy)carbonyl)-2-(4-(4-((R)-2,3-dihydroxypropyl)piperazin-1-yl)phenyl)-7-methoxy-5-oxo-5,10,11,11a-tetrahydro-1H-benzo[e]pyrrolo[1,2-a][1,4]d iazepin-8-yl)oxy)pentyl)oxy)-11-hydroxy-7-methoxy-5-oxo-11,11a-dihydro-1H,3H-spir o[benzo[e]pyrrolo[1,2-a][1,4]diazepine-2,1'-cyclopropane]-10(5H)-carboxylate 31d

**31c** (60 mg, 0.06 mmol) was dissolved in anhydrous dichloromethane (2 mL), and trifluoroacetic acid (0.4 mL) was added. The mixture was stirred at room temperature for 10 min. The reaction mixture was concentrated under reduced pressure and dried using an oil pump. The residue was dissolved in ethyl acetate (20 mL), and the solution was washed with saturated sodium bicarbonate solution (10 mL × 2) and saturated sodium chloride solution (10 mL) in sequence. The organic phase was dried over anhydrous sodium sulfate and filtered. Then the filtrate was concentrated under reduced pressure, and the resulting residue was purified by thin-layer chromatography with developing solvent system A to give the title product **31d** (14 mg, yield: 24.3%).

MS m/z (ESI): 1007.9 [M+1].

### Step 5

### (S)-8-((5-(((S)-2-(4-(4-((R)-2,3-Dihydroxypropyl)piperazin-1-yl)phenyl)-7-methoxy-5-o xo-5,10,11,11a-tetrahydro-1H-benzo[e]pyrrolo[1,2-a][1,4]diazepin-8-yl)oxy)pentyl)oxy )-7-methoxy-1, 11a-dihydro-3H,5H-spiro[benzo[e]pyrrolo[1,2-a][1,4]diazepine-2,1'-cycl opropan]-5-one 31

The title product **31** (10 mg, yield: 87.6%) was obtained by replacing the starting material **1m** of step 12 of Example 2-1 1 with **31d** (14 mg, 0.01 mmol) and using the same reaction conditions.

MS m/z (ESI): 821.6 [M+1].

¹H NMR (500 MHz, DMSO-d6) δ 7.92 (d, 1H), 7.40-7.38 (m, 1H), 7.33 (s, 1H), 7.31-7.28 (m, 2H), 6.91 (d, 1H), 6.85 (s, 1H), 6.67-6.64 (m, 1H), 6.53 (d, 1H), 6.32 (s, 1H), 5.33 (t, 1H), 4.54-4.50 (m, 1H), 4.44-4.41 (m, 1H), 4.18-4.11 (m, 2H), 4.04-4.01 (m, 1H), 3.95-3.92 (m, 2H), 3.82 (s, 2H), 3.66 (s, 4H), 3.56-3.50 (m, 3H), 3.17-3.03 (m, 6H), 2.74-2.71 (m, 1H), 2.03-1.95 (m, 6H), 1.85-1.77 (m, 5H), 1.61-1.55 (m, 2H), 1.48-1.44 (m, 2H), 1.35 (d, 1H), 0.86 (t, 4H), 0.69-0.64 (m, 2H).

### Example 2-32 32

### (S)-8-((5-(((S)-2-(4-(4-(2-Hydroxyacetyl)piperazin-1-yl)phenyl)-7-methoxy-5-oxo-5,10, 11,11a-tetrahydro-1H-benzo[e]pyrrolo[1,2-a][1,4]diazepin-8-yl)oxy)pentyl)oxy)-7-met hoxy-1,11a-dihydro-3H,5H-spiro[benzo[e]pyrrolo[1,2-a][1,4]diazepine-2,1'-cyclopropa n]-5-one 32

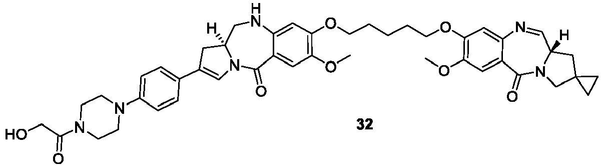

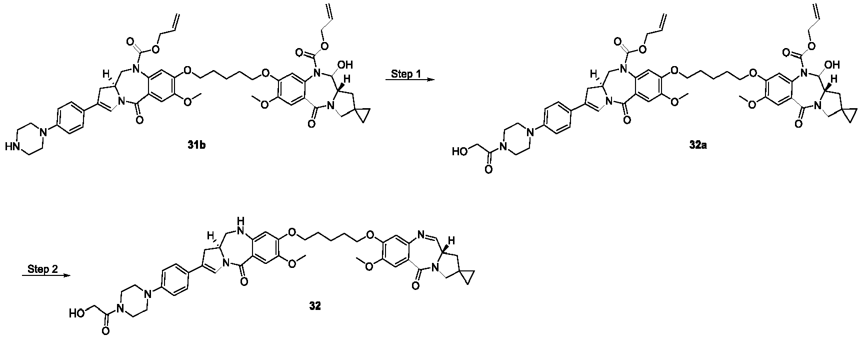

### Step 1

### Allyl

### (11aS)-8-((5-(((S)-10-((allyloxy)carbonyl)-2-(4-(4-(2-hydroxyacetyl)piperazin-1-yl)phe nyl)-7-methoxy-5-oxo-5,10,11,11a-tetrahydro-1H-benzo[e]pyrrolo[1,2-a][1,4]diazepin-8-yl)oxy)pentyl)oxy)-11-hydroxy-7-methoxy-5-oxo-11,11a-dihydro-1H,3H-spiro[benzo [e]pyrrolo[1,2-a][1,4]diazepine-2,1'-cyclopropane]-10(5H)-carboxylate 32a

**31b** (50 mg, 0.05 mmol) was dissolved in anhydrous dichloromethane (5 mL) and methanol (0.5 mL), and 2-hydroxyacetic acid (4.1 mg, 0.05 mmol, Innochem) and 4-(4,6-dimethoxy-1,3,5-triazin-2-yl)-4-methylmorpholinium chloride (30 mg, 0.11 mmol, Accela ChemBio) were sequentially added. The mixture was stirred at room temperature for 1 h. The reaction mixture was concentrated under reduced pressure, and the resulting residue was purified by thin-layer chromatography with developing solvent system A to give the title product **32a** (34 mg, yield: 64.0%).

MS m/z (ESI): 991.8 [M+1].

### Step 2

### (S)-8-((5-(((S)-2-(4-(4-(2-Hydroxyacetyl)piperazin-1-yl)phenyl)-7-methoxy-5-oxo-5,10, 11,11a-tetrahydro-1H-benzo[e]pyrrolo[1,2-a][1,4]diazepin-8-yl)oxy)pentyl)oxy)-7-met hoxy-1,11a-dihydro-3H,5H-spiro[benzo[e]pyrrolo[1,2-a][1,4]diazepine-2,1'-cyclopropa n]-5-one 32

The title product **32** (25 mg, yield: 90.5%) was obtained by replacing the starting material **1m** of step 12 of Example 2-1 1 with **32a** (34 mg, 0.03 mmol) and using the same reaction conditions.

MS m/z (ESI): 805.9 [M+1].

¹H NMR (500 MHz, DMSO-d6) δ 7.90 (d, 1H), 7.39 (s, 1H), 7.33-7.24 (m, 2H), 7.04 (s, 1H), 6.91 (d, 1H), 6.83 (s, 1H), 6.56 (s, 1H), 6.52-6.47 (m, 1H), 6.29 (s, 1H), 4.60 (t, 1H), 4.19-4.08 (m, 3H), 3.98-3.89 (m, 3H), 3.82-3.77 (m, 1H), 3.69-3.62 (m, 3H), 3.61-3.56 (m, 2H), 3.55-3.40 (m, 6H), 3.25-3.19 (m, 2H), 3.17-3.09 (m, 3H), 2.72 (dd, 1H), 2.36-2.25 (m, 2H), 2.19-2.15 (m, 1H), 2.05-1.93 (m, 2H), 1.86-1.73 (m, 4H), 1.60-1.51 (m, 2H), 1.46-1.41 (m, 1H), 0.86-0.77 (m, 2H), 0.69-0.51 (m, 3H).

### Example 2-33 33

### (S)-8-((5-(((S)-2-(4-(4-((S)-2,3-Dihydroxypropyl)piperazin-1-yl)phenyl)-7-methoxy-5-o xo-5,10,11,11a-tetrahydro-1H-benzo[e]pyrrolo[1,2-a][1,4]diazepin-8-yl)oxy)pentyl)oxy )-7-methoxy-1,11a-dihydro-3H,5H-spiro[benzo[e]pyrrolo[1,2-a][1,4]diazepine-2,1'-cycl opropan]-5-one 33

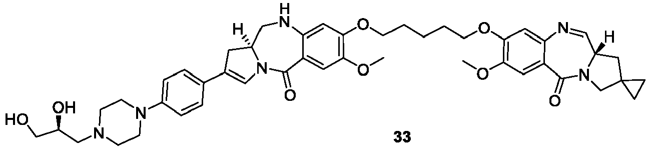

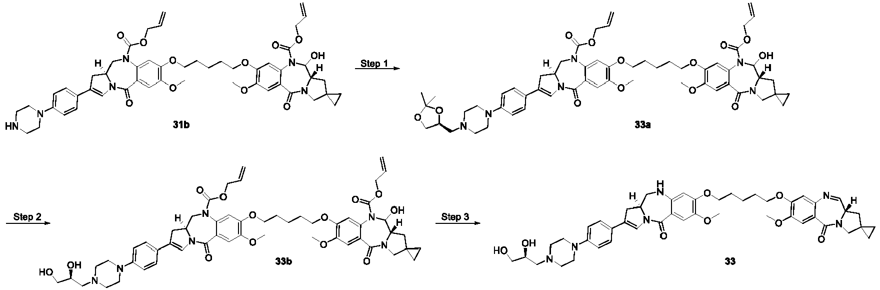

### Step 1

### Allyl

### (11aS)-8-((5-(((S)-10-((allyloxy)carbonyl)-2-(4-(4-(((S)-2,2-dimethyl-1,3-dioxolan-4-yl) methyl)piperazin-1-yl)phenyl)-7-methoxy-5-oxo-5,10,11,11a-tetrahydro-1H-benzo[e]py rrolo[1,2-a][1,4]diazepin-8-yl)oxy)pentyl)oxy)-11-hydroxy-7-methoxy-5-oxo-11,11a-di hydro-1H,3H-spiro[benzo[e]pyrrolo[1,2-a][1,4]diazepine-2,1'-cyclopropane]-10(5H)-ca rboxylate 33a

The title product **33a** (23 mg, yield: 82.0%) was obtained by replacing the starting material (*S*)-2,2-dimethyl-1,3-dioxolane-4-carboxaldehyde of step 3 of Example 2-31 31 with (*R*)-2,2-dimethyl-1,3-dioxolane-4-carboxaldehyde and using the same reaction conditions.

MS m/z (ESI): 1047.9 [M+1].

### Step 2

### Allyl

### (11aS)-8-((5-(((S)-10-((allyloxy)carbonyl)-2-(4-(4-((S)-2,3-dihydroxypropyl)piperazin-1 -yl)phenyl)-7-methoxy-5-oxo-5,10,11,11a-tetrahydro-1H-benzo[e]pyrrolo[1,2-a][1,4]di azepin-8-yl)oxy)pentyl)oxy)-11-hydroxy-7-methoxy-5-oxo-11,11a-dihydro-1H,3H-spir o[benzo[e]pyrrolo[1,2-a][1,4]diazepine-2,1'-cyclopropane]-10(5H)-carboxylate 33b

**33a** (23 mg, 0.02 mmol) was dissolved in acetonitrile (2 mL), and ytterbium(III) trifluoromethanesulfonate (28 mg, 0.05 mmol, Accela ChemBio) was added. The mixture was heated to 60 °C and stirred for another 16 h. The reaction mixture was cooled to room temperature and concentrated under reduced pressure, and the resulting residue was purified by thin-layer chromatography with developing solvent system A to give the title product **33b** (10 mg, yield: 45.2%).

MS m/z (ESI): 1005.8 [M-1].

### Step 3

### (S)-8-((5-(((S)-2-(4-(4-((S)-2,3-Dihydroxypropyl)piperazin-1-yl)phenyl)-7-methoxy-5-o xo-5,10,11,11a-tetrahydro-1H-benzo[c]pyrrolo[1,2-a][1,4]diazepin-8-yl)oxy)pentyl)oxy )-7-methoxy-1,11a-dihydro-3H,5H-spiro[benzo[e]pyrrolo[1,2-a][1,4]diazepine-2,1'-cycl opropan]-5-one 33

The title product **33** (5 mg, yield: 61.3%) was obtained by replacing the starting material **1m** of step 12 of Example 2-1 1 with **33b** (10 mg, 0.01 mmol) and using the same reaction conditions.

MS m/z (ESI): 821.6 [M+1].

¹H NMR (500 MHz, DMSO-d6) δ 7.89 (d, 1H), 7.36 (s, 1H), 7.31 (s, 1H), 7.30-7.22 (m, 2H), 6.88 (d, 1H), 6.83 (s, 1H), 6.63 (br, 1H), 6.52-6.49 (m, 1H), 6.29 (s, 1H), 5.30 (t, 1H), 4.50 (br, 1H), 4.40 (d, 1H), 4.17-4.06 (m, 2H), 4.03-3.98 (m, 1H), 3.95-3.87 (m, 2H), 3.82-3.76 (m, 2H), 3.70-3.58 (m, 4H), 3.55-3.44 (m, 3H), 3.20-3.05 (m, 6H), 2.72 (dd, 1H), 2.35-2.14 (m, 6H), 2.02-1.93 (m, 3H), 1.84-1.74 (m, 2H), 1.58-1.51 (m, 2H), 1.47-1.39 (m, 2H), 1.34-1.30 (m, 1H), 0.90-0.76 (m, 4H), 0.67-0.59 (m, 2H).

### Example 2-34 34

### (S)-8-((5-(((S)-2-(4-((Cyclopropylmethyl)thio)phenyl)-7-methoxy-5-oxo-5,10,11,11a-tet rahydro-1H-benzo[e]pyrrolo[1,2-a][1,4]diazepin-8-yl)oxy)pentyl)oxy)-7-methoxy-1,11 a-dihydro-3H,5H-spiro[benzo[e]pyrrolo[1,2-a][1,4]diazepine-2,1'-cyclopropan]-5-one 34

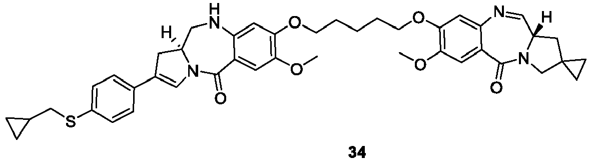

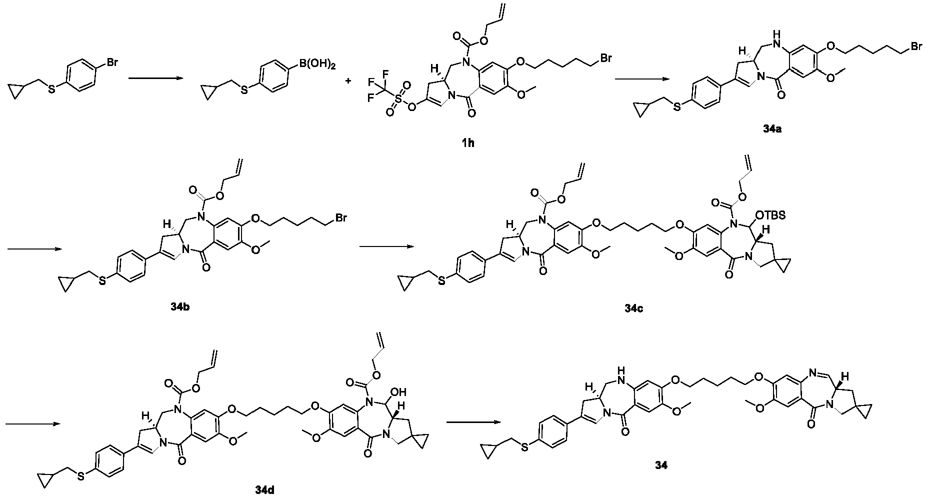

### Step 1

### (4-((Cyclopropylmethyl)thio)phenyl)boronic acid

Tetrahydrofuran (1 mL) was placed in a reaction flask. The reaction system was purged with nitrogen gas three times and cooled in a dry ice-acetone bath to -78 °C, and a 2.5 M n-butyllithium solution (1.89 mL, 4.73 mmol) was added dropwise. After the dropwise addition, the mixture was stirred for 10 min. A solution of (4-bromophenyl)(cyclopropylmethyl)sulfane (700 mg, 2.88 mmol, prepared by the method disclosed in Example 7 on page 43 of the specification of the patent application "WO2006/134341, A1") in 5 mL of tetrahydrofuran was added dropwise. After the dropwise addition, the mixture was stirred at -78 °C for another 1 h, and a solution of triisopropyl borate (740 mg, 3.93 mmol) in 5 mL of tetrahydrofuran was added dropwise. After the dropwise addition, the reaction mixture was warmed to room temperature and stirred for another 3 h. 20 mL of water was added to the reaction mixture, and the mixture was stirred for 30 min. Then 1 M hydrochloric acid was added to adjust the pH of the reaction mixture to 1. After the addition, the mixture was stirred for 30 min. The reaction mixture was concentrated under reduced pressure, extracted with ethyl acetate (15 mL × 3), and washed with saturated sodium chloride solution (30 mL). The organic phase was dried over anhydrous sodium sulfate and filtered. Then the filtrate was concentrated under reduced pressure, and the resulting residue was purified on a CombiFlash preparative flash chromatograph with eluent system B to give the title product (4-((cyclopropylmethyl)thio)phenyl)boronic acid (352 mg, yield: 58.7%).

MS m/z (ESI): 207.1 [M-1].

The title product **34** (12 mg, overall yield: 11.9%) was prepared by using the synthesis scheme of Example **2-1 1** and replacing the reagent 4-(4-methyl-1-piperazinyl)phenylboronic acid of step 8 with (4-((cyclopropylmethyl)thio)phenyl)boronic acid.

MS m/z (ESI): 749.5 [M+1].

¹H NMR (500 MHz, DMSO-d6) δ 7.89 (d, 1H), 7.55 (s, 1H),7.39-7.33 (m, 2H), 7.32-7.22 (m, 3H), 7.03 (s, 1H), 6.84-6.81 (m, 1H), 6.58-6.53 (m, 1H), 6.32-6.28 (m, 1H), 4.23-4.16 (m, 1H), 4.13-4.07 (m, 1H), 4.03-3.98 (m, 1H), 3.96-3.90 (m, 2H), 3.82-3.76 (m, 2H), 3.70-3.62 (m, 4H), 3.60-3.46 (m, 3H), 3.27-3.19 (m, 2H), 2.90 (d, 2H), 2.75 (dd, 1H), 2.43-2.38 (m, 1H), 2.34-2.25 (m, 1H), 2.19-2.14 (m, 1H), 2.06-1.94 (m, 2H), 1.83-1.72 (m, 3H), 1.62-1.51 (m, 2H), 1.35-1.31 (m, 1H), 1.00-0.94 (m, 1H), 0.86-0.80 (m, 1H), 0.64-0.57 (m, 1H), 0.52-0.45 (m, 2H), 0.26-0.20 (m, 2H).

### Example 2-35 35

### (S)-8-((5-(((S)-2-(4-(Cyclobutylthio)phenyl)-7-methoxy-5-oxo-5,10,11,11a-tetrahydro-1 H-benzo[e]pyrrolo[1,2-a][1,4]diazepin-8-yl)oxy)pentyl)oxy)-7-methoxy-1,11a-dihydro-3H,5H-spiro[benzo[e]pyrrolo[1,2-a][1,4]diazepine-2,1'-cyclopropan]-5-one 35

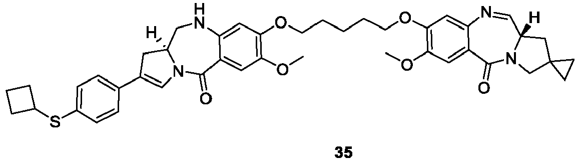

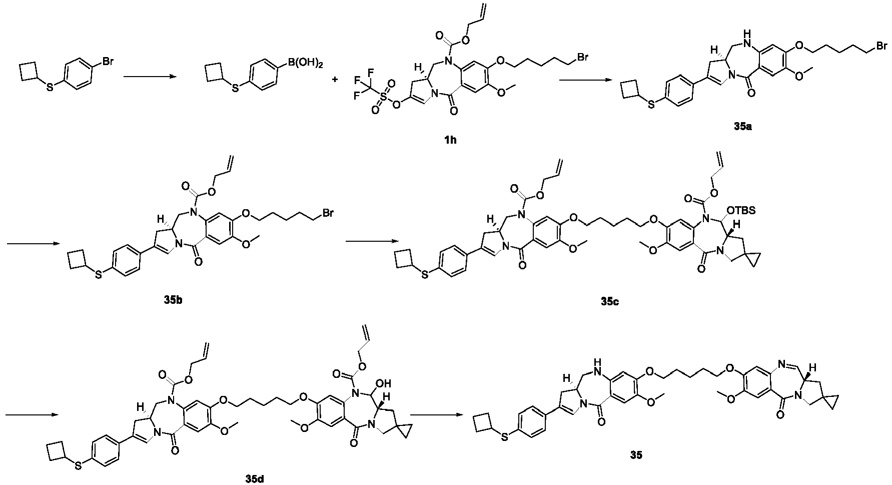

### Step 1

### (4-(Cyclobutylthio)phenyl)boronic acid

Tetrahydrofuran (3 mL) was placed in a reaction flask. The reaction system was purged with nitrogen gas three times and cooled in a dry ice-acetone bath to -78 °C, and a 2.5 M n-butyllithium solution (3.23 mL, 8.07 mmol) was added dropwise. After the dropwise addition, the mixture was stirred for 10 min. A solution of (4-bromophenyl)(cyclobutyl)sulfane (1.2 g, 4.94 mmol, prepared by the method disclosed in Example 14 on page 17 of the specification of the patent application "EP1921074A1") in 10 mL of tetrahydrofuran was added dropwise. After the dropwise addition, the mixture was stirred at -78 °C for another 1 h, and a solution of triisopropyl borate (1.27 g, 6.75 mmol) in 8 mL of tetrahydrofuran was added dropwise. After the dropwise addition, the reaction mixture was warmed to room temperature and stirred for another 3 h. 30 mL of water was added to the reaction mixture, and the mixture was stirred for 30 min. Then 1 M hydrochloric acid was added to adjust the pH of the reaction mixture to 1, and the mixture was stirred for 30 min. The reaction mixture was concentrated under reduced pressure, extracted with ethyl acetate (20 mL × 3), and washed with saturated sodium chloride solution (50 mL). The organic phase was dried over anhydrous sodium sulfate and filtered. Then the filtrate was concentrated under reduced pressure, and the resulting residue was purified on a CombiFlash preparative flash chromatograph with eluent system B to give the title product (4-(cyclobutylthio)phenyl)boronic acid (821 mg, yield: 79.9%).

MS m/z (ESI): 207.1 [M-1].

The title product **35** (10 mg, overall yield: 12.8%) was prepared by using the synthesis scheme of Example **2-1 1** and replacing the reagent 4-(4-methyl-1-piperazinyl)phenylboronic acid of step 8 with (4-(cyclobutylthio)phenyl)boronic acid.

MS m/z (ESI): 749.5 [M+1].

¹H NMR (500 MHz, DMSO-d6) δ 7.90 (d, 1H), 7.57 (s, 1H), 7.37 (d, 2H), 7.29 (t, 2H), 7.18 (d, 2H), 7.06-7.03 (m, 1H), 6.85-6.82 (m, 1H), 6.59-6.54 (m, 1H), 6.33-6.29 (m, 1H), 4.24-4.16 (m, 2H), 4.14-4.07 (m, 1H), 4.04-3.92 (m, 4H), 3.83-3.78 (m, 1H), 3.71-3.64 (m, 4H), 3.62-3.47 (m, 3H), 3.26-3.19 (m, 2H), 2.76 (dd, 1H), 2.43-2.32 (m, 2H), 2.21-2.15 (m, 1H), 2.05-1.96 (m, 2H), 1.84-1.76 (m, 4H), 1.61-1.53 (m, 2H), 1.36-1.32 (m, 1H), 0.87-0.81 (m, 2H), 0.69-0.53 (m, 4H).

### Example 2-36 36

### (S)-8-((5-(((S)-2-(4-(1-Cyclopropylpiperidin-4-yl)phenyl)-7-methoxy-5-oxo-5,10,11,11 a-tetrahydro-1H-benzo[e]pyrrolo[1,2-a][1,4]diazepin-8-yl)oxy)pentyl)oxy)-7-methoxy-1,11a-dihydro-3H,5H-spiro[benzo[e]pyrrolo[1,2-a][1,4]diazepine-2,1'-cyclopropan]-5-o ne 36

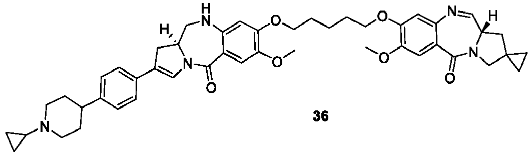

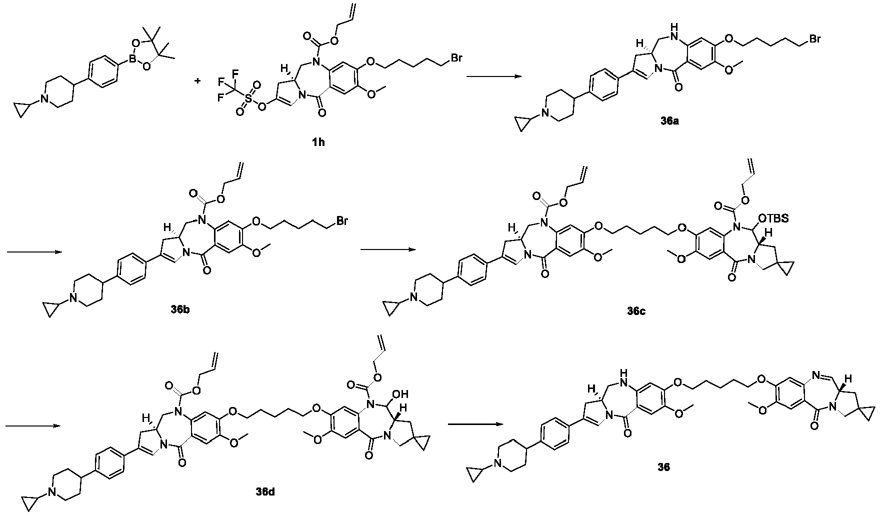

The title product **36** (25 mg, overall yield: 20.9%) was prepared by using the synthesis scheme of Example **2-1 1** and replacing the reagent 4-(4-methyl-1-piperazinyl)phenylboronic acid of step 8 with 1-cyclopropyl-4-(4-(4,4, 5, 5-tetramethyl-1,3,2-dioxaboran-2-yl)phenyl)piperidine (prepared by the method disclosed in Example 10 on page 59 of the specification of the patent application "KR102019/0109007A").

MS m/z (ESI): 786.5 [M+1].

¹H NMR (500 MHz, DMSO-d6) δ 7.90 (d, 1H), 7.52 (s, 1H), 7.38-7.31 (m, 2H), 7.29 (s, 1H), 7.19 (d, 2H), 6.84 (s, 1H), 6.54 (d, 1H), 6.32 (s, 1H), 4.24-4.16 (m, 1H), 4.15-4.08 (m, 1H), 4.05-3.99 (m, 1H), 3.98-3.91 (m, 2H), 3.81 (s, 3H), 3.65 (s, 3H), 3.57-3.46 (m, 2H), 3.2 7-3.18 (m, 2H), 3.02 (br, 2H), 2.76 (dd, 1H), 2.46-2.37 (m, 2H), 2.29-2.17 (m, 2H), 2.08-1.95 (m, 2H), 1.88-1.76 (m, 4H), 1.74-1.66 (m, 2H), 1.63-1.51 (m, 4H), 1.37-1.32 (m, 1H), 0.89-0.74 (m, 3H), 0.71-0.60 (m, 3H), 0.48-0.38 (m, 2H), 0.34-0.25 (m, 2H).

### Example 2-37 37

### (S)-7-Methoxy-8-((5-(((S)-7-methoxy-5-oxo-2-(4-(1-(2,2,2-trifluoroethyl)piperidin-4-yl )phenyl)-5,10,11,11a-tetrahydro-1H-benzo[e]pyrrolo[1,2-a][1,4]diazepin-8-yl)oxy)pent yl)oxy)-1,11a-dihydro-3H,5H-spiro[benzo[e]pyrrolo[1,2-a][1,4]diazepine-2,1'-cyclopro pan]-5-one 37

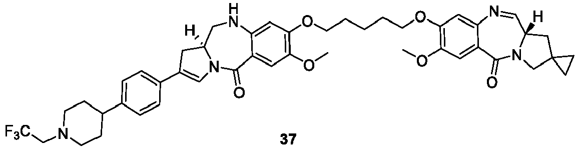

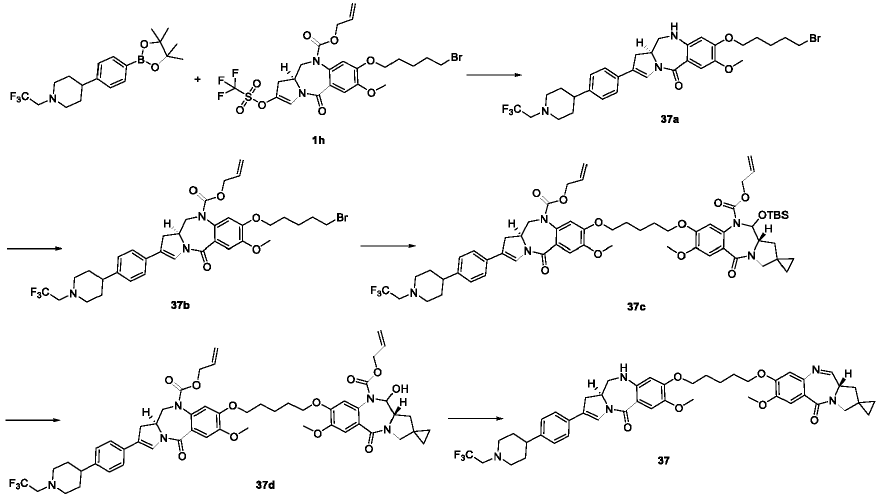

The title product **37** (16 mg, overall yield: 20.7%) was prepared by using the synthesis scheme of Example **2-1 1** and replacing the reagent 4-(4-methyl-1-piperazinyl)phenylboronic acid of step 8 with 4-(4-(4,4,5,5-tetramethyl-15-tetramethyl-1,3,2-dioxaboran-2-yl)phenyl)-1-(2,2,2-trifluoroethyl)piperidi ne (prepared by the method disclosed in Example 60 on page 40 of the specification of the patent application "US2013/210798, A1").

MS m/z (ESI): 828.6 [M+1].

¹H NMR (500 MHz, DMSO-d6) δ 7.90 (d, 1H), 7.50 (s, 1H), 7.37-7.30 (m, 2H), 7.28 (s, 1H), 7.19 (d, 2H), 6.83 (s, 1H), 6.57-6.50 (m, 1H), 6.29 (s, 1H), 4.22-4.14 (m, 1H), 4.12-4.06 (m, 1H), 4.03-3.97 (m, 1H), 3.96-3.89 (m, 2H), 3.82-3.76 (m, 2H), 3.70-3.61 (m, 4H), 3.60-3.44 (m, 3H), 3.25-3.09 (m, 4H), 2.99 (d, 2H), 2.75 (dd, 1H), 2.43-2.36 (m, 2H), 2.34-2.25 (m, 1H), 2.05-1.93 (m, 2H), 1.83-1.74 (m, 4H), 1.71-1.52 (m, 6H), 1.33 (d, 1H), 0.83 (t, 1H), 0.79-0.73 (m, 1H), 0.69-0.51 (m, 3H).

### Example 2-38 38

### (S)-8-((5-(((S)-2-(4-(1-(2,2-Difluoroethyl)piperidin-4-yl)phenyl)-7-methoxy-5-oxo-5,10 ,11,11a-tetrahydro-1H-benzo[e]pyrrolo[1,2-a][1,4]diazepin-8-yl)oxy)pentyl)oxy)-7-met hoxy-1,11a-dihydro-3H,5H-spiro[benzo[e]pyrrolo[1,2-a][1,4]diazepine-2,1'-cyclopropa n]-5-one 38

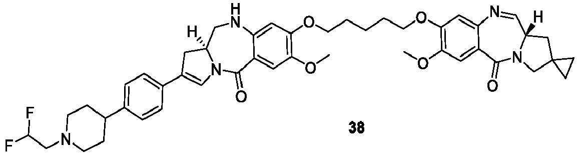

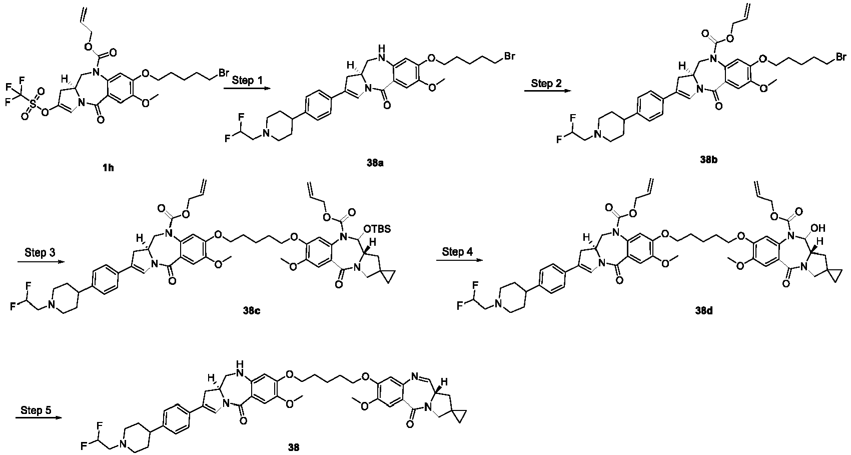

The title product **38** (13 mg, overall yield: 15.7%) was prepared by using the synthesis scheme of Example **2-1 1** and replacing the reagent 4-(4-methyl-1-piperazinyl)phenylboronic acid of step 8 with 1-(2,2-difluoroethyl)-4-(4-(4,4,5,5-tetramethyl-1,3,2-dioxaboran-2-yl)phenyl)piperidine (prepared by the method disclosed in step 2 of Example 59 on page 122 of the specification of the patent application "WO2020002487, A1").

MS m/z (ESI): 810.7 [M+1].

¹H NMR (500 MHz, DMSO-d6) δ 7.90 (d, 1H), 7.52 (s, 1H), 7.38-7.32 (m, 2H), 7.29 (s, 1H), 7.21 (d, 2H), 6.84 (s, 1H), 6.57-6.52 (m, 1H), 6.32 (s, 1H), 5.32 (t, 1H), 4.23-4.17 (m, 1H), 4.14-4.09 (m, 1H), 4.05-3.99 (m, 1H), 3.97-3.91 (m, 2H), 3.83-3.80 (m, 2H), 3.68-3.64 (m, 3H), 3.58-3.47 (m, 3H), 3.27-3.19 (m, 2H), 3.00 (d, 2H), 2.80-2.70 (m, 3H), 2.47-2.40 (m, 2H), 2.30-2.22 (m, 2H), 2.08-1.96 (m, 3H), 1.85-1.78 (m, 3H), 1.74-1.54 (m, 6H), 1.37-1.31 (m, 1H), 0.86 (t, 1H), 0.81-0.75 (m, 1H), 0.72-0.61 (m, 3H).

### Example 2-39 39

### (S)-7-Cyclopropoxy-8-((5-(((S)-7-methoxy-5-oxo-2-(4-(tetrahydro-2H-pyran-4-yl)phen yl)-5,10,11,11a-tetrahydro-1H-benzo[e]pyrrolo[1,2-a][1,4]diazepin-8-yl)oxy)pentyl)oxy )-1,11a-dihydro-3H,5H-spiro[benzo[e]pyrrolo[1,2-a][1,4]diazepine-2,1'-cyclopropan]-5-one 39

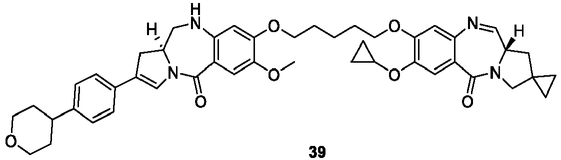

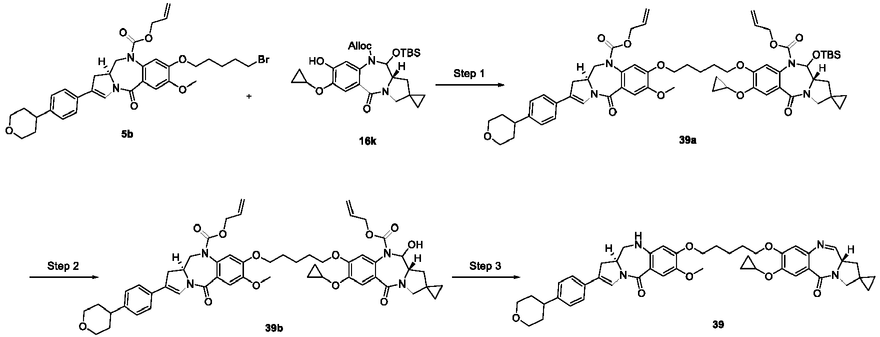

The title product **39** (20 mg, overall yield: 16.3%) was prepared by using the synthesis scheme of Example **2-1 1** and replacing the reagent **1j** of step 10 with **5b** and **1k** with **16k.**

MS m/z (ESI): 773.6 [M+1].

¹H NMR (500 MHz, DMSO-d6) δ 7.99 (s, 1H), 7.73-7.65 (m, 2H), 7.64-7.56 (m, 2H), 7.47-7.39 (m, 1H), 7.35 (s, 1H), 7.28 (d, 1H), 6.91 (s, 1H), 6.64-6.58 (m, 1H), 6.40-6.37 (m, 1H), 4.30-4.14 (m, 2H), 4.10-3.86 (m, 5H), 3.73 (s, 2H), 3.70-3.45 (m, 5H), 3.32-3.27 (m, 1H), 3.20 (d, 1H), 2.89-2.76 (m, 2H), 2.70 (s, 1H), 2.48-2.35 (m, 2H), 2.14-1.99 (m, 2H), 1.86 (br, 4H), 1.78-1.55 (m, 5H), 1.43-1.39 (m, 1H), 0.94-0.82 (m, 3H), 0.80-0.61 (m, 5H).

### III. Linker-Drug Examples

### Example 3-1 LD-1

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-5-[4-(4-methylpiperazin-1-yl) phenyl]-2-oxo-3,9-diazatricyclo[8.4.0.0^{3,7}]tetradeca-1(14),4,10,12-tetraen-12-yl]oxy}pe ntyl)oxy]-2'-oxo-3',9'-diazaspiro[cyclopropane-1,5'-tricyclo[8.4.0.0^{3,7}]tetradecane]-1'(14 '),10',12'-triene-9'-carboxylate LD-1

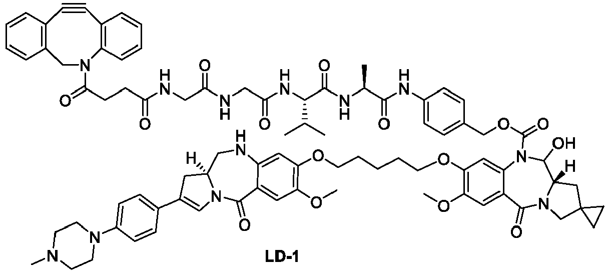

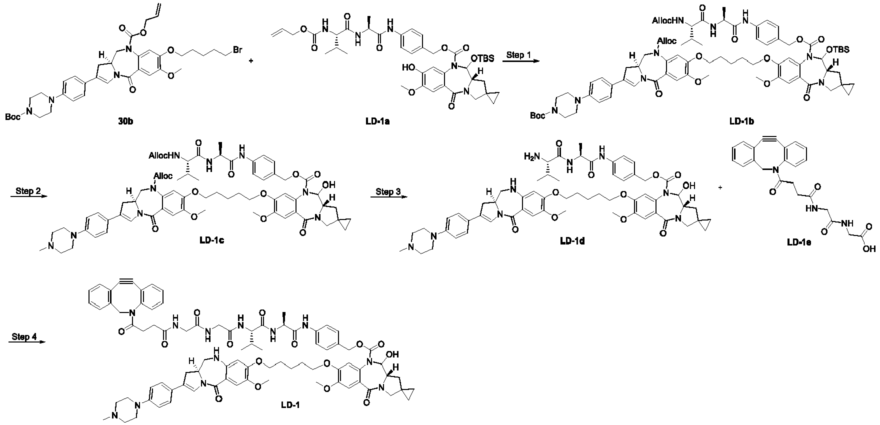

### Step 1

### 4-((S)-2-((S)-2-(((Allyloxy)carbonyl)amino)-3-methylbutanamido)propanamido)benzyl (11aS)-8-((5-(((S)-10-((allyloxy)carbonyl)-2-(4-(4-(tert-butoxycarbonyl)piperazin-1-yl) phenyl)-7-methoxy-5-oxo-5,10,11,11a-tetrahydro-1H-benzo[e]pyrrolo[1,2-a][1,4]diaze pin-8-yl)oxy)pentyl)oxy)-11-((tert-butyldimethylsilyl)oxy)-7-methoxy-5-oxo-11,11a-di hydro-1H,3H-spiro[benzo[e]pyrrolo[1,2-a][1,4]diazepine-2,1'-cyclopropane]-10(5H)-ca rboxylate LD-1b

**30b** (66 mg, 0.09 mmol) was dissolved in *N*,*N*-dimethylformamide (3 mL), and 4-((*S*)-2-((*S*)-2-(((allyloxy)carbonyl)amino)-3-methylbutanamido)propanamido)benzyl (11*aS*)-11-((tert-butyldimethylsilyl)oxy)-8-hydroxy-7-methoxy-5-oxo-11,11*a*-dihydro-1 *H*,3*H*-spiro[benzo[*e*]pyrrolo[1,2-*a*][1,4]diazepine-2,1'-cyclopropane]-10(5*H*)-carboxylat e **LD-1a** (60 mg, 0.07 mmol, prepared by the method disclosed in Example 1 on page 123 of the specification of the patent application "CN 111164208 A") was added. Then anhydrous potassium carbonate (31 mg, 0.22 mmol) was added, and the mixture was stirred at room temperature for 16 h. Water (10 mL) was added to the reaction mixture, and extraction was performed with ethyl acetate (15 mL × 3). The organic phases were combined, washed with water (15 mL × 3) and saturated sodium chloride solution (20 mL) in sequence, dried over anhydrous sodium sulfate, and filtered. Then the filtrate was concentrated under reduced pressure, and the resulting residue was purified by thin-layer chromatography with developing solvent system A to give the title product **LD-1b** (89 mg, yield: 81.7%).

MS m/z (ESI): 1466.4 [M+1].

### Step 2

### 4-((S)-2-((S)-2-(((Allyloxy)carbonyl)amino)-3-methylbutanamido)propanamido)benzyl (11aS)-8-((5-(((S)-10-((allyloxy)carbonyl)-7-methoxy-2-(4-(4-methylpiperazin-1-yl)phe nyl)-5-oxo-5,10,11,11a-tetrahydro-1H-benzo[e]pyrrolo[1,2-a][1,4]diazepin-8-yl)oxy)pe ntyl)oxy)-11-hydroxy-7-methoxy-5-oxo-11,11a-dihydro-1H,3H-spiro[benzo[e]pyrrolo[ 1,2-a][1,4]diazepine-2,1'-cyclopropane]-10(5H)-carboxylate LD-1c

**LD-1b** (89 mg, 0.06 mmol) was dissolved in anhydrous dichloromethane (0.5 mL), and a 4 N solution of hydrochloric acid in 1,4-dioxane (1 mL) was added. The mixture was stirred at room temperature for 1 h. The reaction mixture was concentrated under reduced pressure and dried using an oil pump. The resulting residue was dissolved in ethyl acetate (20 mL), and the solution was washed with saturated sodium bicarbonate solution (5 mL × 2) and saturated brine (5 mL) in sequence, dried over anhydrous sodium sulfate, and filtered. Then the filtrate was concentrated under reduced pressure. The resulting residue was dissolved in anhydrous tetrahydrofuran (4 mL), and an aqueous formaldehyde solution (50 mg, content 37%, 0.62 mmol) was added. The mixture was stirred at room temperature for 30 min. Sodium triacetoxyborohydride (39 mg, 0.18 mmol) was slowly added to the reaction mixture, and the mixture was stirred at room temperature for another 1 h. The reaction mixture was cooled in an ice bath, quenched with saturated sodium bicarbonate solution (10 mL), and extracted with ethyl acetate (15 mL × 3). The organic phases were combined, washed with saturated sodium chloride solution (20 mL), dried over anhydrous sodium sulfate, and filtered. Then the filtrate was concentrated under reduced pressure, and the resulting residue was purified by thin-layer chromatography with developing solvent system A to give the title product **LD-1c** (34 mg, yield: 44.2%).

MS m/z (ESI): 1266.9 [M+1].

### Step 3

### 4-((S)-2-((S)-2-Amino-3-methylbutanamido)propanamido)benzyl

### (11aS)-11-hydroxy-7-methoxy-8-((5-(((S)-7-methoxy-2-(4-(4-methylpiperazin-1-yl)phe nyl)-5-oxo-5,10,11,11a-tetrahydro-1H-benzo[e]pyrrolo[1,2-a][1,4]diazepin-8-yl)oxy)pe ntyl)oxy)-5-oxo-11,11a-dihydro-1H,3H-spiro[benzo[e]pyrrolo[1,2-a][1,4]diazepine-2,1' -cyclopropane]-10(5H)-carboxylate LD-1d

The title product **LD-1d** (40 mg, crude product yield: >100%) was obtained by replacing the starting material **1m** of step 12 of Example **2-1 1** with **LD-1c** (34 mg, 0.03 mmol) and using the same reaction conditions.

MS m/z (ESI): 1098.7 [M+1].

### Step 4

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-5-[4-(4-methylpiperazin-1-yl) phenyl]-2-oxo-3,9-diazatricyclo[8.4.0.0^{3,7}]tetradeca-1(14),4,10,12-tetraen-12-yl]oxy}pe ntyl)oxy]-2'-oxo-3',9'-diazaspiro[cyclopropane-1,5'-tricyclo[8.4.0.0^{3,7}]tetradecane]-1'(14 '),10',12'-triene-9'-carboxylate LD-1

**LD-1d** (10 mg, 0.01 mmol) was dissolved in 1.1 mL of a mixed solvent of anhydrous dichloromethane and methanol (V/V = 10:1), and 2-[2-(4-{2-azatricyclo[10.4.0.0^{4,9}]hexadeca-1(12),4,6,8,13,15-hexaen-10-yn-2-yl}-4-ox obutanamido)acetamido]acetic acid **LD-1e** (3.9 mg, 0.01 mmol, prepared by the method disclosed in Example 2 on page 127 of the specification of the patent application "CN 111164208 A") and 4-(4,6-dimethoxytriazin-2-yl)-4-methylmorpholine hydrochloride (5 mg, 0.02 mmol, Accela ChemBio) were sequentially added. The reaction system was purged with nitrogen gas three times and stirred at room temperature for 30 min. The reaction mixture was concentrated under reduced pressure to remove the organic solvent, and the resulting residue was purified by thin-layer chromatography with developing solvent system A to give the title product **LD-1** (10 mg, yield: 73.2%).

MS m/z (ESI): 1499.8 [M/2+1].

¹H NMR (500 MHz, DMSO-d6) δ 9.90 (s, 1H), 8.25-8.20 (m, 1H), 8.19-8.10 (m, 1H), 8.08-8.00 (m, 1H), 7.79-7.64 (m, 2H), 7.60 (t, 1H), 7.57-7.53 (m, 1H), 7.51-7.47 (m, 1H), 7.46-7.42 (m, 2H), 7.37-7.32 (m, 4H), 7.29 (s, 2H), 7.20 (d, 2H), 7.05 (s, 1H), 6.97 (d, 1H), 6.72 (s, 1H), 6.68-6.63 (m, 1H), 6.56-6.52 (m, 1H), 6.32 (s, 1H), 5.76 (t, 1H), 5.32 (t, 2H), 5.19 (d, 1H), 5.02 (d, 1H), 4.84-4.76 (m, 1H), 4.40-4.33 (m, 1H), 4.23-4.15 (m, 2H), 3.97-3.91 (m, 3H), 3.80-3.73 (m, 5H), 3.66 (s, 3H), 3.62-3.58 (m, 2H), 3.56-3.51 (m, 2H), 3.41 (t, 1H), 2.78-2.71 (m, 1H), 2.64 (s, 2H), 2.31-2.24 (m, 2H), 2.04-1.96 (m, 9H), 1.83-1.74 (m, 5H), 1.59-1.44 (m, 8H), 1.37-1.33 (m, 5H), 0.89-0.86 (m, 5H), 0.82-0.78 (m, 3H), 0.65-0.55 (m, 4H).

### Example 3-2 LD-2

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-5-[4-(morpholin-4-yl)phenyl]-2-oxo-3,9-diazatricyclo[8.4.0.0^{3,7}]tetradeca-1(14),4,10,12-tetraen-12-yl]oxylpentyl)oxy ]-2'-oxo-3',9'-diazaspiro[cyclopropane-1,5'-tricyclo[8.4.0.0^{3,7}]tetradecane]-1'(14'),10',12' -triene-9'-carboxylate LD-2

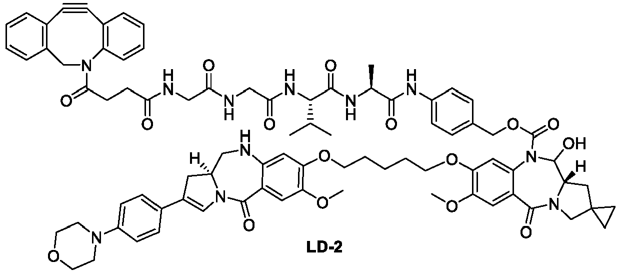

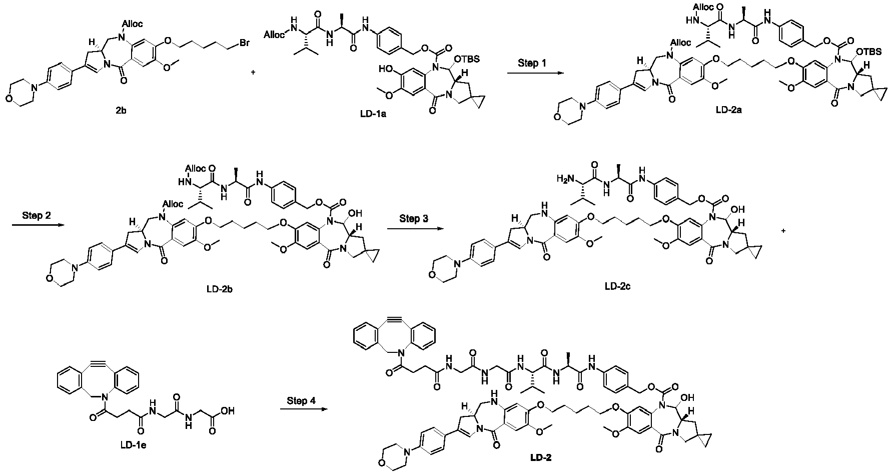

### Step 1

### 4-((S)-2-((S)-2-(((Allyloxy)carbonyl)amino)-3-methylbutanamido)propanamido)benzyl (11aS)-8-((5-(((S)-10-((allyloxy)carbonyl)-7-methoxy-2-(4-morpholinophenyl)-5-oxo-5, 10,11,11a-tetrahydro-1H-benzo[e]pyrrolo[1,2-a][1,4]diaza-8-yl)oxy)pentyl)oxy)-11-((te rt-butyldimethylsilyl)oxy)-7-methoxy-5-oxy-11,11a-dihydro-1H,3H-spiro[benzo[e]pyrr olo[1,2-a][1,4]diaza-2,1'-cyclopropane]-10(5H)-carboxylate LD-2a

**2b** (51.4 mg, 0.08 mmol) was dissolved in *N*,*N*-dimethylformamide (2 mL), and **LD-1a** (50 mg, 0.06 mmol) and anhydrous potassium carbonate (25.6 mg, 0.19 mmol) were added. The mixture was stirred at room temperature for 16 h. Water (6 mL) was added to the reaction mixture, and extraction was performed with ethyl acetate (10 mL × 3). The organic phases were combined, washed with water (10 mL × 3) and saturated sodium chloride solution (10 mL) in sequence, dried over anhydrous sodium sulfate, and filtered. Then the filtrate was concentrated under reduced pressure, and the resulting residue was purified by silica gel column chromatography with eluent system B to give the title product **LD-2a** (65 mg, yield: 76.9%).

MS m/z (ESI): 1367.9 [M+1].

### Step 2

### 4-((S)-2-((S)-2-(((Allyloxy)carbonyl)amino)-3-methylbutanamido)propanamido)benzyl (11aS)-8-((5-(((S)-10-((allyloxy)carbonyl)-7-methoxy-2-(4-morpholinophenyl)-5-oxo-5, 10,11,11a-tetrahydro-1H-benzo[e]pyrrolo[1,2-a][1,4]diaza-8-yl)oxy)pentyl)oxy)-11-hyd roxy-7-methoxy-5-oxo-11,11a-dihydro-1H,3H-spiro[benzo[e]pyrrolo[1,2-a][1,4]diaza-2 ,1'-cyclopropane]-10(5H)-carboxylate LD-2b

**LD-2a** (65 mg, 0.05 mmol) was dissolved in anhydrous tetrahydrofuran (3 mL), and the solution was cooled in an ice-water bath. Glacial acetic acid (17.1 mg, 0.29 mmol) was added, and then a solution of tetrabutylammonium fluoride in tetrahydrofuran (1 M, 0.24 mL, 0.24 mmol) was added. After the addition, the ice-water bath was removed, and the mixture was stirred at room temperature for 22 h. The reaction mixture was diluted with ethyl acetate (6 mL) and washed with saturated aqueous ammonium chloride solution (10 mL × 2) and saturated aqueous sodium chloride solution (10 mL × 2) in sequence. The organic phase was dried over anhydrous sodium sulfate and filtered. Then the filtrate was concentrated under reduced pressure, and the resulting residue was purified by silica gel column chromatography with eluent system A to give the title product **LD-2b** (59.5 mg, yield: 100%).

MS m/z (ESI): 1253.8 [M+1].

### Step 3

### 4-((S)-2-((S)-2-Amino-3-methylbutanamido)propanamido)benzyl

### (11aS)-11-hydroxy-7-methoxy-8-((5-(((S)-7-methoxy-2-(4-morpholinophenyl)-5-oxo-5, 10,11,11a-tetrahydro-1H-benzo[e]pyrrolo[1,2-a][1,4]diaza-8-yl)oxy)pentyl)oxy)-5-oxo-11,11a-dihydro-1H,3H-spiro[benzo[e]pyrrolo[1,2-a][1,4]diaza-2,1'-cyclopropane]-10(5 H)-carboxylate LD-2c

**LD-2b** (59.5 mg, 0.047 mmol) was dissolved in anhydrous dichloromethane (2 mL), and pyrrolidine (33.8 mg, 0.48 mmol) and tetrakis(triphenylphosphine)palladium(0) (5.5 mg, 0.0047 mmol) were sequentially added. The mixture was stirred for 30 min in a nitrogen atmosphere. The organic solvent was removed by concentration under reduced pressure, and the resulting residue was purified by thin-layer chromatography with developing solvent system A to give the title product **LD-2c** (45 mg, yield: 87.3%).

MS m/z (ESI): 1085.6 [M+1].

### Step 4

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-5-[4-(morpholin-4-yl)phenyl]-2-oxo-3,9-diazatricyclo[8.4.0.0^{3,7}]tetradeca-1(14),4,10,12-tetraen-12-yl]oxy}pentyl)oxy ]-2'-oxo-3',9'-diazaspiro[cyclopropane-1,5'-tricyclo[8.4.0.0^{3,7}]tetradecane]-1'(14'),10',12' -triene-9'-carboxylate LD-2

**LD-2c** (10 mg, 0.01 mmol) was dissolved in a mixture of dichloromethane and methanol (V/V = 10/1, 2 mL), and **LD-1e** (3.86 mg, 0.01 mmol) and 4-(4,6-dimethoxytriazin-2-yl)-4-methylmorpholine hydrochloride (6.4 mg, 0.02 mmol) were sequentially added. The mixture was stirred for 30 min in a nitrogen atmosphere. The organic solvent was removed by concentration under reduced pressure, and the resulting residue was purified by thin-layer chromatography with developing solvent system A to give the title product **LD-2** (2.1 mg, yield: 15.4%).

MS m/z (ESI): 1486.9 [M+1].

¹H NMR (500 MHz, DMSO-d6) δ12.42 (s, 1H), 9.89 (s, 1H),8.24-8.18 (m, 1H), 8.18-8.14 (m, 1H), 8.14-8.10 (m, 1H), 8.07-7.99 (m, 1H), 7.78-7.73 (m, 1H), 7.72-7.63 (m, 2H), 7.62-7.57 (m, 1H), 7.57-7.51 (m, 1H), 7.51-7.39 (m, 3H), 7.38-7.27 (m, 3H), 7.23-7.16 (m, 2H), 7.07-7.02 (m, 1H), 6.93-6.88 (m, 1H), 6.75-6.70 (m, 1H), 6.69-6.62 (m, 1H), 6.57-6.48 (m, 2H), 6.31 (s, 1H), 5.35-5.30 (m, 1H), 5.23-5.14 (m, 1H), 5.04-4.98 (m, 1H), 4.84-4.77 (m, 1H), 4.40-4.31 (m, 2H), 4.23-4.12 (m, 2H), 4.03-3.83 (m, 6H), 3.81-3.71 (m, 5H), 3.66 (s, 2H), 3.63-3.48 (m, 5H), 3.43-3.38 (m, 2H), 3.28-3.20 (m, 2H), 3.16-3.08 (m, 3H), 2.80-2.60 (m, 7H), 2.39-2.22 (m, 3H), 2.10-1.92 (m, 4H), 1.84-1.71 (m, 3H), 1.61-1.49 (m, 3H), 1.49-1.41 (m, 2H), 1.37-1.32 (m, 1H), 0.90-0.77 (m, 5H), 0.74-0.67 (m, 1H), 0.64-0.54 (m, 2H).

### Example 3-3 LD-3

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-12'-[(5-{[(7S)-5-[4-(cyclopropylthio)phenyl]-13-methoxy-2-oxo-3,9-diazatricyclo[ 8.4.0.0^{3,7}]tetradeca-1(14),4,10,12-tetraen-12-yl]oxy}pentyl)oxy]-8'-hydroxy-13'-methox y-2'-oxo-3',9'-diazaspiro[cyclopropane-1,5'-tricyclo[8.4.0.0^{3,7}]tetradecane]-1'(14'),10',12 '-triene-9'-carboxylate LD-3

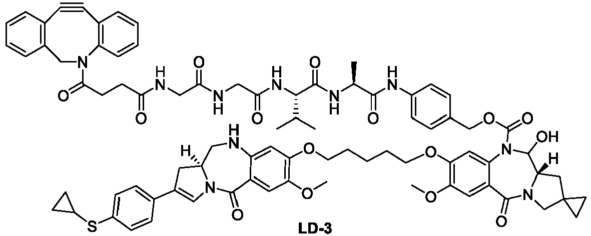

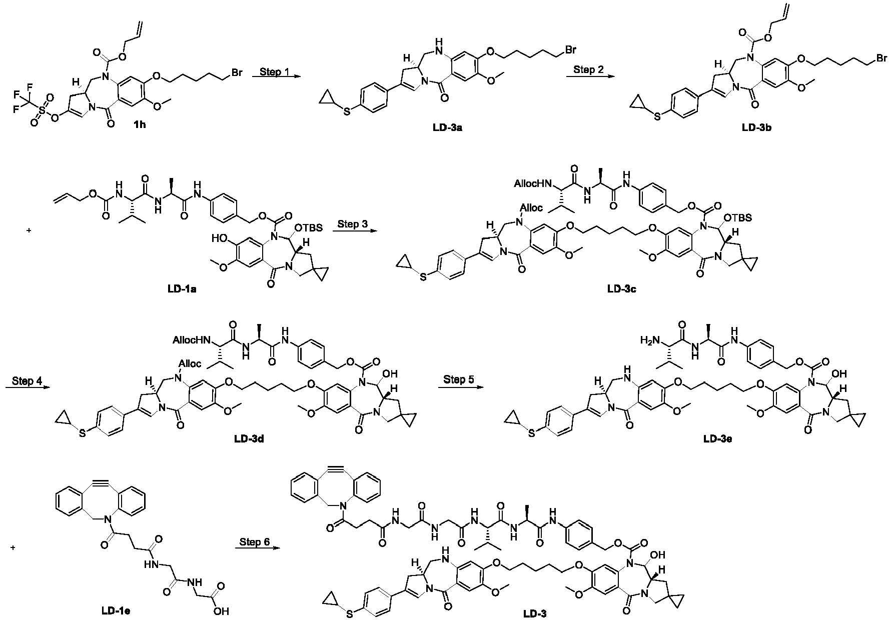

### Step 1

### (S)-8-((5-Bromopentyl)oxy)-2-(4-(cyclopropylthio)phenyl)-7-methoxy-1,10,11,11a-tetr ahydro-5H-benzo[e]pyrrolo[1,2-a][1,4]diazepin-5-one LD-3a

The title product **LD-3a** (110 mg, yield: 69.9%) was obtained by replacing the starting material 4-(4-methyl-1-piperazinyl)phenylboronic acid of step 8 of Example **2-1 1** with ((4-cyclopropylthio)phenyl)boronic acid and using the same conditions.

MS m/z (ESI): 543.1 [M+1].

### Step 2

### Allyl

### (S)-8-((5-bromopentyl)oxy)-2-(4-(cyclopropylthio)phenyl)-7-methoxy-5-oxo-11,11a-di hydro-1H-benzo[e]pyrrolo[1,2-a][1,4]diazepine-10(5H)-carboxylate LD-3b

The title product **LD-3b** (108 mg, yield: 85.0%) was obtained by replacing the starting material **1i** of step 9 of Example **2-1** 1 with **LD-3a** (110 mg, 0.20 mmol) and using the same reaction conditions.

MS m/z (ESI): 627.1 [M+1].

### Step 3

### 4-((S)-2-((S)-2-(((Allyloxy)carbonyl)amino)-3-methylbutanamido)propanamido)benzyl (11aS)-8-((5-(((S)-10-((allyloxy)carbonyl)-2-(4-(cyclopropylthio)phenyl)-7-methoxy-5-oxo-5,10,11,11a-tetrahydro-1H-benzo[e]pyrrolo[1,2-a][1,4]diazepin-8-yl)oxy)pentyl)ox y)-11-((tert-butyldimethylsilyl)oxy)-7-methoxy-5-oxo-11,11a-dihydro-1H,3H-spiro[ben zo[e]pyrrolo[1,2-a][1,4]diazepine-2,1'-cyclopropane]-10(5H)-carboxylate LD-3c

The title product **LD-3c** (90 mg, yield: 82.6%) was obtained by replacing the starting material **30b** of step 1 of Example **3-1 LD-1** with **LD-3b** (56 mg, 0.09 mmol) and using the same reaction conditions.

MS m/z (ESI): 1354.8 [M+1].

### Step 4

### 4-((S)-2-((S)-2-(((Allyloxy)carbonyl)amino)-3-methylbutanamido)propanamido)benzyl (11aS)-8-((5-(((S)-10-(allyloxy)carbonyl)-2-(4-(cyclopropylthio)phenyl)-7-methoxy-5-o xo-5,10,11,11a-tetrahydro-1H-benzo[e]pyrrolo[1,2-a][1,4]diazepin-8-yl)oxy)pentyl)oxy )-11-hydroxy-7-methoxy-5-oxo-11,11a-dihydro-1H,3H-spiro[benzo[e]pyrrolo[1,2-a][1, 4]diazepine-2,1'-cyclopropane]-10(5H)-carboxylate LD-3d

The title product **LD-3d** (75 mg, yield: 91.0%) was obtained by replacing the starting material **1l** of step 11 of Example **2-1 1** with **LD-3c** (90 mg, 0.07 mmol) and using the same reaction conditions.

MS m/z (ESI): 1240.8 [M+1].

### Step 5

### 4-((S)-2-((S)-2-Amino-3-methylbutanamido)propanamido)benzyl

### (11aS)-8-((5-(((S)-2-(4-(cyclopropylthio)phenyl)-7-methoxy-5-oxo-5,10,11,11a-tetrahy dro-1H-benzo[e]pyrrolo[1,2-a][1,4]diazepin-8-yl)oxy)pentyl)oxy)-11-hydroxy-7-metho xy-5-oxo-11,11a-dihydro-1H,3H-spiro[benzo[e]pyrrolo[1,2-a][1,4]diazepine-2,1'-cyclo propane]-10(5H)-carboxylate LD-3e

The title product **LD-3e** (51 mg, yield: 78.7%) was obtained by replacing the starting material **1m** of step 12 of Example **2-1 1** with **LD-3d** (75 mg, 0.06 mmol) and using the same reaction conditions.

MS m/z (ESI): 1072.8 [M+1].

### Step 6

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-12'-[(5-{[(7S)-5-[4-(cyclopropylthio)phenyl]-13-methoxy-2-oxo-3,9-diazatricyclo[ 8.4.0.0^{3,7}]tetradeca-1(14),4,10,12-tetraen-12-yl]oxy}pentyl)oxy]-8'-hydroxy-13'-methox y-2'-oxo-3',9'-diazaspiro[cyclopropane-1,5'-tricyclo[8.4.0.0^{3,7}]tetradecane]-1'(14'),10',12 '-triene-9'-carboxylate LD-3

The title product **LD-3** (5 mg, yield: 21.4%) was obtained by replacing the starting material **LD-1d** of step 4 of Example **3-1 LD-1** with **LD-3e** (17 mg, 0.02 mmol) and using the same reaction conditions.

MS m/z (ESI): 1473.5 [M+1].

¹H NMR (500 MHz, DMSO-d6) δ 9.90 (s, 1H), 8.25-8.11 (m, 2H), 8.09-8.00 (m, 1H), 7.76 (d, 1H), 7.74-7.64 (m, 2H), 7.61 (t, 1H), 7.57 (s, 1H), 7.55-7.53 (m, 1H), 7.50-7.48 (m, 1H), 7.45-7.43 (m, 1H), 7.40 (d, 2H), 7.38-7.34 (m, 2H), 7.33 (s, 1H), 7.31 (s, 2H), 7.22-7.17 (m, 2H), 7.05 (s, 1H), 6.73 (s, 1H), 6.59-6.51 (m, 2H), 6.32 (s, 1H), 5.77 (t, 1H), 5.33 (t, 1H), 5.19 (d, 1H), 5.06-4.98 (m, 1H), 4.40-4.32 (m, 1H), 4.25-4.17 (m, 2H), 3.94 (t, 3H), 3.79 (s, 3H), 3.76-3.72 (s, 2H), 3.67 (s, 3H), 3.62-3.58 (m, 3H), 3.56-3.52 (m, 3H), 3.42 (t, 2H), 3.15 (d, 1H), 2.81-2.75 (m, 2H), 2.30-2.24 (m, 2H), 2.11-2.03 (m, 2H), 2.02-1.95 (m, 5H), 1.83-1.76 (m, 4H), 1.61-1.51 (m, 4H), 1.50-1.43 (m, 2H), 1.10-1.07 (m, 2H), 0.88-0.84 (m, 5H), 0.81 (t, 3H), 0.61-0.57 (m, 4H).

### Example 3-4 LD-4

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-2-oxo-5-[4-(1H-pyrazol-1-yl) phenyl]-3,9-diazatricyclo[8.4.0.0^{3,7}]tetradeca-1(14),4,10,12-tetraen-12-yl]oxylpentyl)ox y]-2'-oxo-3',9'-diazaspiro[cyclopropane-1,5'-tricyclo[8.4.0.0^{3,7}]tetradecane]-1'(14'),10',1 2'-triene-9'-carboxylate LD-4

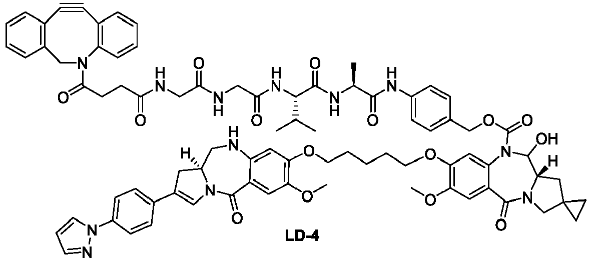

The title product **LD-4** (15 mg, overall yield: 43%) was prepared by using the synthesis scheme of Example **3-2 LD-2** and replacing the reagent **2b** of step 1 with **4b.**

MS m/z (ESI): 1467.6 [M+1].

¹H NMR (500 MHz, DMSO) δ 9.90 (s, 1H), 8.52-8.50 (m, 1H), 8.29-7.98 (m, 4H), 7.81 (d, 2H), 7.78-7.64 (m, 3H), 7.64-7.52 (m, 3H), 7.52-7.42 (m, 3H), 7.40-7.29 (m, 3H), 7.25-7.15 (m, 3H), 7.10-7.02 (m,1H), 6.77-6.63 (m, 2H), 6.60-6.52 (m, 2H), 6.33 (s, 1H), 5.22-5.18 (m, 1H), 5.05-5.00 (m, 1H), 4.86-4.80 (m, 1H), 4.40-4.32 (m, 1H), 4.28-4.19 (m, 2H), 4.02-3.38 (m, 24H), 3.20-3.15 (m, 1H), 2.72-2.61 (m, 2H), 2.33-2.23 (m, 2H), 2.07-1.92 (m, 2H), 1.89-1.68 (m, 4H), 1.63-1.53 (m, 3H), 1.49-1.45 (m, 3H), 1.00-0.78 (m, 6H), 0.73-0.53 (m, 4H).

### Example 3-5 LD-5

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-5-[4-(oxan-4-yl)phenyl]-2-ox o-3,9-diazatricyclo[8.4.0.0^{3,7}]tetradeca-1(14),4,10,12-tetraen-12-yl]oxy}pentyl)oxy]-2'-oxo-3',9'-diazaspiro[cyclopropane-1,5'-tricyclo[8.4.0.0^{3,7}]tetradecane]-1'(14'),10',12'-trie ne-9'-carboxylate LD-5

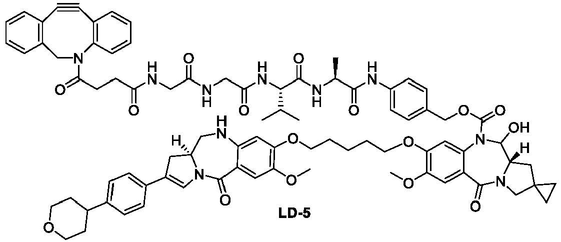

The title product **LD-5** (18 mg, overall yield: 42%) was prepared by using the synthesis scheme of Example **3-2 LD-2** and replacing the reagent **2b** of step 1 with **5b.**

MS m/z (ESI): 1485.6 [M+1].

¹H NMR (500 MHz, DMSO) δ 9.89 (s, 1H), 8.31-7.94 (m, 3H), 7.79-7.64 (m, 2H), 7.63-7.44 (m, 7H), 7.40-7.28 (m, 6H), 7.25-7.15 (m, 1H), 7.07-7.02 (m, 1H), 6.80-6.64 (m, 2H), 6.60-6.50 (m, 2H), 6.32 (s, 1H), 5.82-5.70 (m, 1H), 5.26-5.15 (m, 1H), 5.07-4.97 (m, 1H), 4.87-4.76 (m, 1H), 4.41-4.34 (m, 1H), 4.25-4.16 (m, 2H), 4.00-3.51 (m, 27H), 3.18-3.12 (m, 1H), 2.80-2.70 (m, 1H), 2.68-2.60 (m, 2H), 2.33-2.23 (m, 2H), 2.10-1.94 (m, 6H), 1.85-1.74 (m, 4H), 1.62-1.53 (m, 3H), 1.51-1.43 (m, 3H), 0.87-0.80 (m, 6H), 0.76-0.53 (m, 4H).

### Example 3-6 LD-6

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-12'-[(5-{[(7S)-5-{4-[1-(2-hydroxyacetyl)piperidin-4-yl]phenyl}-13-me thoxy-2-oxo-3,9-diazatricyclo[8.4.0.0^{3,7}]tetradeca-1(14),4,10,12-tetraen-12-yl]oxy}pent yl)oxy]-13'-methoxy-2'-oxo-3',9'-diazaspiro[cyclopropane-1,5'-tricyclo[8.4.0.0^{3,7}]tetrade cane]-1'(14'),10',12'-triene-9'-carboxylate LD-6

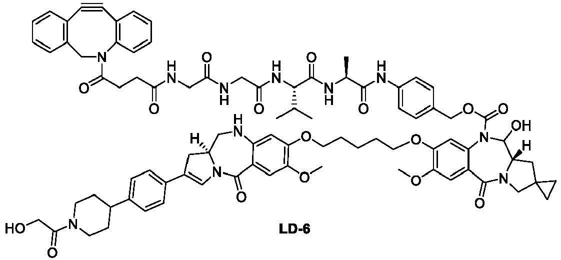

### Example 3-7 LD-7

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl }-4-oxobutanamido)acetamido] acetamido } -3 -methylbutanamido]propa nami do] phenyl} methyl

### (7'S)-8'-hydroxy-13'-methoxy-12'-[(5-{[(7S)-13-methoxy-2-oxo-5-[4-(2H-1,2,3-triazol-2-yl)phenyl]-3,9-diazatricyclo[8.4.0.0^{3,7}]tetradeca-1(14),4,10,12-tetraen-12-yl]oxy}pent yl)oxy]-2'-oxo-3',9'-diazaspiro[cyclopropane-1,5'-tricyclo[8.4.0.0^{3,7}]tetradecane]-1'(14'), 10',12'-triene-9'-carboxylate LD-7

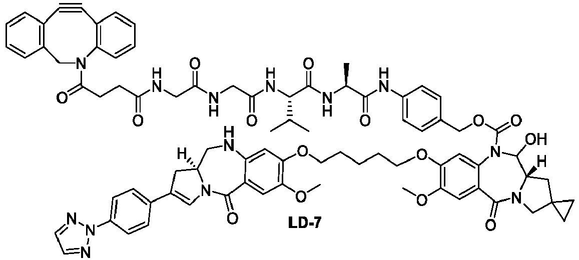

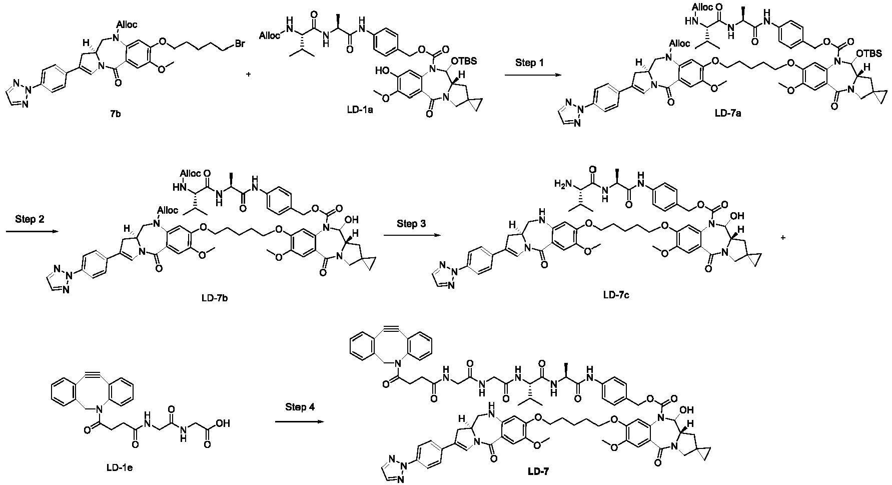

The title product **LD-7** (13 mg, overall yield: 7.8%) was prepared by using the synthesis scheme of Example **3-2 LD-2** and replacing the reagent **2b** of step 1 with **7b** (70 mg, 0.11 mmol).

MS m/z (ESI): 735.0 [M/2+1].

¹H NMR (500 MHz, DMSO-d6) δ 9.89 (s, 1H),8.23-8.15 (m, 2H), 8.12 (s, 2H), 8.08-8.00 (m, 1H), 7.98 (d, 1H), 7.76 (d, 1H), 7.73-7.55 (m, 4H), 7.57-7.52 (m, 1H), 7.51-7.40 (m, 2H), 7.39-7.27 (m, 3H), 7.23-7.16 (m, 2H), 7.07-7.02 (m, 1H), 6.75-6.70 (m, 1H), 6.68-6.50 (m, 3H), 6.32 (s, 1H), 5.35-5.30 (m, 1H), 5.22-5.15 (m, 1H), 5.04-4.98 (m, 1H), 4.84-4.77 (m, 1H), 4.40-4.32 (m, 2H), 4.29-4.16 (m, 2H), 3.99-3.89 (m, 3H), 3.87-3.71 (m, 5H), 3.67 (s, 2H), 3.64-3.50 (m, 4H), 3.43-3.35 (m, 2H), 3.29-3.22 (m, 2H), 3.18-3.09 (m, 2H), 2.88-2.79 (m, 2H), 2.69-2.59 (m, 3H), 2.40-2.32 (m, 2H), 2.31-2.21 (m, 2H), 2.10-1.92 (m, 4H), 1.84-1.71 (m, 4H), 1.61-1.50 (m, 3H), 1.49-1.41 (m, 2H), 1.39-1.32 (m, 1H), 0.90-0.77 (m, 5H), 0.75-0.66 (m, 1H), 0.64-0.54 (m, 2H).

### Example 3-8 LD-8

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-[(5-{[(7S)-13-methoxy-5-[4-(1-methylpiperidin-4-yl) phenyl]-2-oxo-3,9-diazatricyclo[8.4.0.0^{3,7}]tetradeca-1(14),4,10,12-tetraen-12-yl]oxy}pe ntyl)oxy]-2'-oxo-3',9'-diazaspiro[cyclopropane-1,5'-tricyclo[8.4.0.0^{3,7}]tetradecane]-1'(14 '),10',12'-triene-9'-carboxylate LD-8

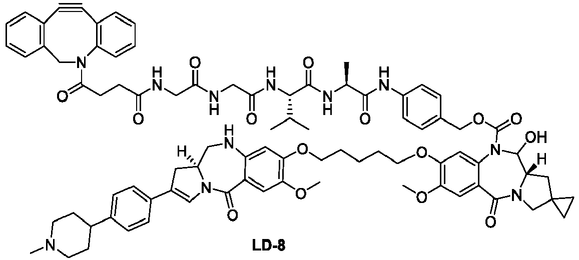

The title product **LD-8** (13 mg, overall yield: 29%) was prepared by using the synthesis scheme of Example **3-2 LD-2** and replacing the reagent **2b** of step 1 with **6b.**

MS m/z (ESI): 1498.7 [M+1].

¹H NMR (500 MHz, DMSO) δ 9.89 (s, 1H), 8.27-7.96 (m, 3H), 7.83-7.63 (m, 2H), 7.62-7.26 (m, 12H), 7.24-7.14 (m, 3H), 7.08-7.02 (m, 1H), 6.75-6.68 (m, 1H), 6.61-6.50 (m, 2H), 6.32 (s, 1H), 5.80-5.72 (m, 1H), 5.24-5.15 (m, 1H), 5.05-4.98 (m, 1H), 4.87-4.77 (m, 1H), 4.41-4.31 (m, 1H), 4.25-4.16 (m, 2H), 3.97-3.90 (m, 5H), 3.88-3.72 (m, 9H), 3.70-3.38 (m, 14H), 3.27-3.10 (m, 2H), 2.81-2.60 (m, 4H), 2.31-2.22 (m, 2H), 2.09-1.95 (m, 6H), 1.86-1.69 (m, 4H), 1.60-1.51 (m, 3H), 1.49-1.42 (m, 3H), 0.92-0.76 (m, 6H), 0.73-0.55 (m, 4H).

### Example 3-9 LD-9

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-{3-[1-({[(7S)-13-methoxy-5-(4-methoxyphenyl)-2-ox o-3,9-diazatricyclo[8.4.0.0^{3,7}]tetradeca-1(14),4,10,12-tetraen-12-yl]oxy}methyl)cyclopr opyl]propoxy}-2'-oxo-3',9'-diazaspiro[cyclopropane-1,5'-tricyclo[8.4.0.0^{3,7}]tetradecane] -1'(14'),10',12'-triene-9'-carboxylate LD-9

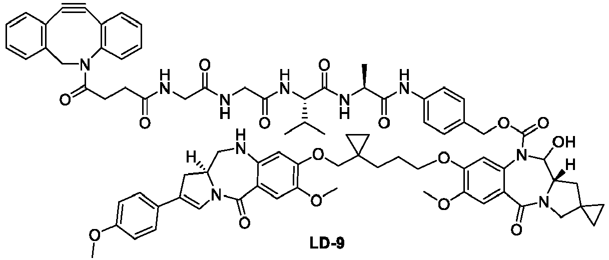

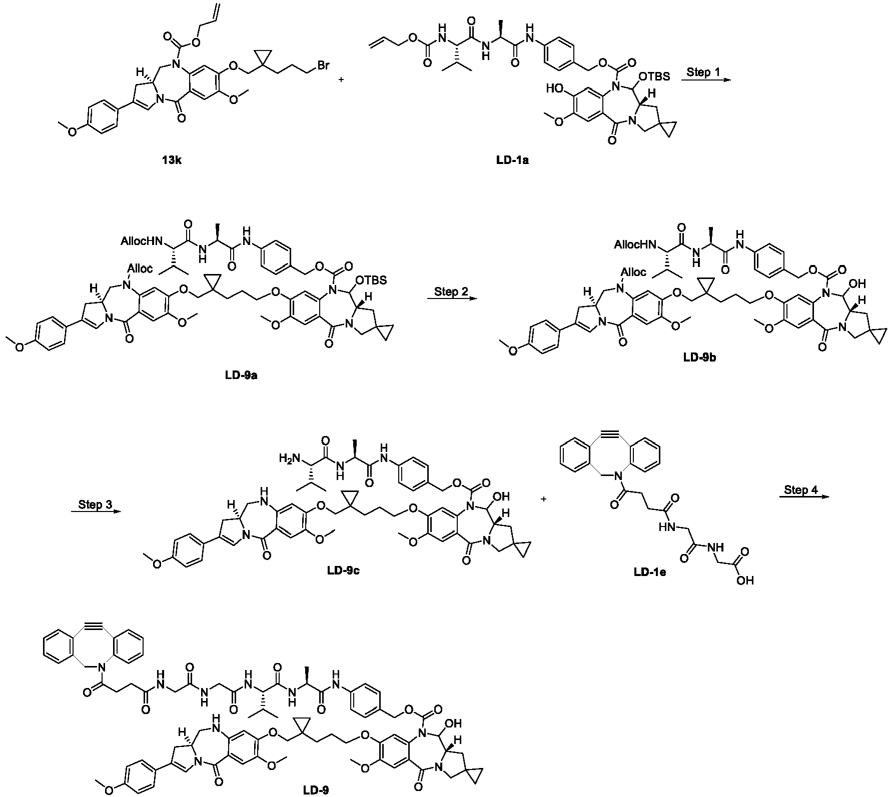

### Step 1

### 4-((S)-2-((S)-2-(((Allyloxy)carbonyl)amino)-3-methylbutanamido)propanamido)benzyl (11aS)-8-(3-(1-((((S)-10-((allyloxy)carbonyl)-7-methoxy-2-(4-methoxyphenyl)-5-oxo-5, 10,11,11a-tetrahydro-1H-benzo[e]pyrrolo[1,2-a][1,4]diazepin-8-yl)oxy)methyl)cyclopr opyl)propoxy)-11-((tert-butyldimethylsilyl)oxy)-7-methoxy-5-oxo-11,11a-dihydro-1H,3 H-spiro[benzo[e]pyrrolo[1,2-a][1,4]diazepine-2,1'-cyclopropane]-10(5H)-carboxylate LD-9a

The title product **LD-9a** (98 mg, yield: 84.5%) was obtained by replacing the starting material **30b** of step 1 of Example **3-1 LD-1** with **13k** (58 mg, 0.01 mmol) and using the same reaction conditions.

MS m/z (ESI): 1338.6 [M+1].

### Step 2

### 4-((S)-2-((S)-2-(((Allyloxy)carbonyl)amino)-3-methylbutanamido)propanamido)benzyl (11aS)-8-(3-(1-((((S)-10-((allyloxy)carbonyl)-7-methoxy-2-(4-methoxyphenyl)-5-oxo-5, 10,11,11a-tetrahydro-1H-benzo[e]pyrrolo[1,2-a][1,4]diazepin-8-yl)oxy)methyl)cyclopr opyl)propoxy)-11-hydroxy-7-methoxy-5-oxo-11,11a-dihydro-1H,3H-spiro[benzo[e]pyrr olo[1,2-a][1,4]diazepine-2,1'-cyclopropane]-10(5H)-carboxylate LD-9b

The title product **LD-9b** (73 mg, yield: 81.4%) was obtained by replacing the starting material **1l** of step 11 of Example **2-1** 1 with **LD-9a** (98 mg, 0.07 mmol) and using the same reaction conditions.

MS m/z (ESI): 1224.7 [M+1].

### Step 3

### 4-((S)-2-((S)-2-Amino-3-methylbutanamido)propanamido)benzyl

### (11aS)-11-hydroxy-7-methoxy-8-(3-(1-((((S)-7-methoxy-2-(4-methoxyphenyl)-5-oxo-5, 10,11,11a-tetrahydro-1H-benzo[e]pyrrolo[1,2-a][1,4]diazepin-8-yl)oxy)methyl)cyclopr opyl)propoxy)-5-oxo-11,11a-dihydro-1H,3H-spiro[benzo[e]pyrrolo[1,2-a][1,4]diazepin e-2,1'-cyclopropane]-10(5H)-carboxylate LD-9c

The title product **LD-9c** (58 mg, yield: 88.4%) was obtained by replacing the starting material **1m** of step 12 of Example **2-1 1** with **LD-9b** (73 mg, 0.06 mmol) and using the same reaction conditions.

MS m/z (ESI): 1056.8 [M+1].

### Step 4

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-{3-[1-({[(7S)-13-methoxy-5-(4-methoxyphenyl)-2-ox o-3,9-diazatricyclo[8.4.0.0^{3,7}]tetradeca-1(14),4,10,12-tetraen-12-yl]oxy}methyl)cyclopr opyl]propoxy}-2'-oxo-3',9'-diazaspiro[cyclopropane-1,5'-tricyclo[8.4.0.0^{3,7}]tetradecane] -1'(14'),10',12'-triene-9'-carboxylate LD-9

The title product **LD-9** (17 mg, yield: 82.1%) was obtained by replacing the starting material **LD-1d** of step 4 of Example **3-1 LD-1** with **LD-9c** (15 mg, 0.01 mmol) and using the same reaction conditions.

MS m/z (ESI): 729.8 [M/2+1].

¹H NMR (500 MHz, DMSO-d6) δ 12.44 (s, 1H), 9.87 (s, 1H), 8.26-8.09 (m, 2H), 8.08-7.99 (m, 1H), 7.81-7.64 (m, 2H), 7.60 (s, 1H), 7.55-7.42 (m, 5H), 7.40-7.27 (m, 6H), 7.22-7.11 (m, 2H), 7.04 (s, 1H), 6.91 (d, 2H), 6.78-6.69 (m, 1H), 6.27 (s, 1H), 5.75 (s, 1H), 5.13 (d, 1H), 5.02 (d, 1H), 4.82 (d, 1H), 4.36 (s, 1H), 4.26-4.13 (m, 2H), 3.99-3.93 (m, 1H), 3.86 (s, 6H), 3.79-3.72 (m, 9H), 3.67 (s, 3H), 3.61-3.57 (m, 3H), 3.53 (d, 2H), 3.43-3.37 (m, 2H), 3.14 (d, 1H), 2.76 (d, 1H), 2.65-2.62 (m, 1H), 2.38-2.29 (m, 2H), 2.00-1.95 (m, 2H), 1.88-1.75 (m, 4H), 1.62-1.54 (m, 3H), 0.88-0.79 (m, 8H), 0.64-0.56 (m, 3H), 0.54-0.42 (m, 4H).

### Example 3-10 LD-10

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-8'-hydroxy-13'-methoxy-12'-{[1-(3-{[(7S)-13-methoxy-5-(4-methoxyphenyl)-2-ox o-3,9-diazatricyclo[8.4.0.0^{3,7}]tetradeca-1(14),4,10,12-tetraen-12-yl]oxy}propyl)cyclopr opyl]methoxy}-2'-oxo-3',9'-diazaspiro[cyclopropane-1,5'-tricyclo[8.4.0.0^{3,7}]tetradecane] -1'(14'),10',12'-triene-9'-carboxylate LD-10

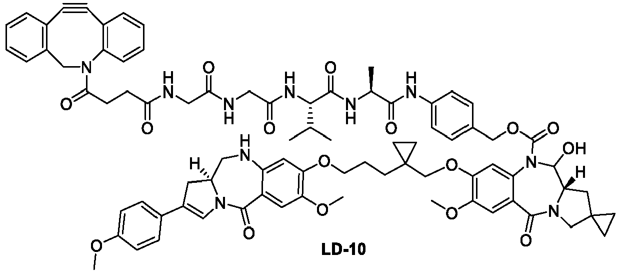

### Example 3-11 LD-11

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa namido]phenyl}methyl

### (7'S)-13'-cyclopropoxy-8'-hydroxy-12'-[(5-{[(7S)-13-methoxy-5-(4-methoxyphenyl)-2-o xo-3,9-diazatricyclo[8.4.0.0^{3,7}]tetradeca-1(14),4,10,12-tetraen-12-yl]oxy}pentyl)oxy]-2' -oxo-3',9'-diazaspiro[cyclopropane-1,5'-tricyclo[8.4.0.0^{3,7}]tetradecane]-1'(14'),10',12'-tri ene-9'-carboxylate LD-11

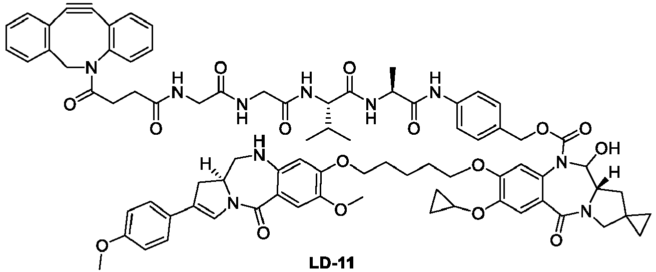

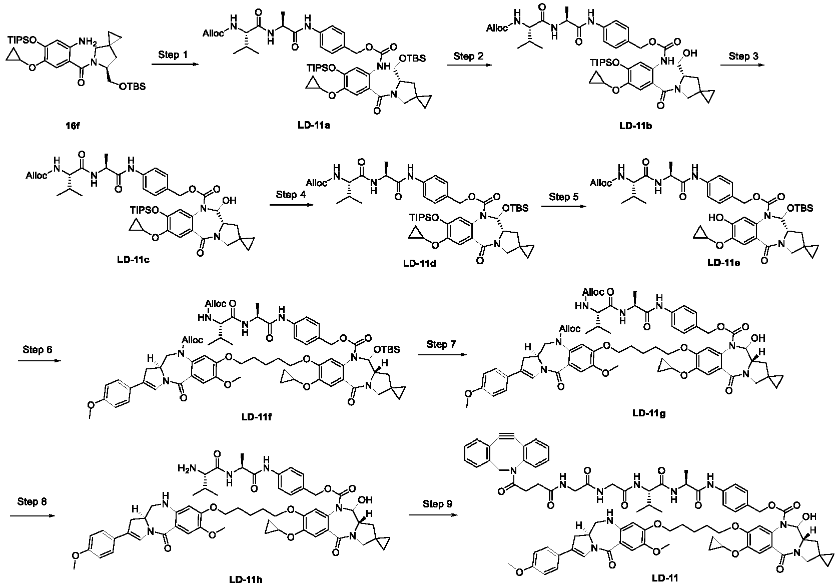

### Step 1

4-((*S*)-2-((*S*)-2-(((Allyloxy)carbonyl)amino)-3-methylbutanamido)propanamido)benzyl (2-((*S*)-6-(((tert-butyldimethylsilyl)oxy)methyl)-5-azaspiro[2.4]heptane-5-carbonyl)-4-c yclopropoxy-5-((triisopropylsilyl)oxy)phenyl)carbamate **LD-11a**
**16f** (900 mg, 1.53 mmol) was dissolved in tetrahydrofuran (10 mL), and triethylamine (231 mg, 2.29 mmol) was added. In a nitrogen atmosphere, the temperature was lowered to 0 °C, and triphosgene (204 mg, 0.69 mmol) was added. After 10 min of stirring, a mixed solution of N [(prop-2-en-1-yloxy)carbonyl]-L-valinyl-N [4-(hydroxymethyl)phenyl]-L-alaninamid e (693 mg, 1.84 mmol, synthesized by following the method shown in Example 113 on page 161 of the specification of the patent "WO2020/006722 A1") and triethylamine (231 mg, 2.29 mmol) in tetrahydrofuran (4 mL) and N,N-dimethylformamide (0.4 mL) was added dropwise. After the addition, the mixture was heated to 40 °C and stirred for 19 h. Water (15 mL) was added to the reaction mixture, and extraction was performed with ethyl acetate (20 mL × 3). The organic phases were combined, washed with water (15 mL × 2) and saturated sodium chloride solution (15 mL) in sequence, dried over anhydrous sodium sulfate, and filtered. Then the filtrate was concentrated under reduced pressure, and the resulting residue was purified by silica gel column chromatography with eluent system B to give the title product **LD-11a** (937 mg, yield: 73.3%).

### Step 2

### 4-((S)-2-((S)-2-(((Allyloxy)carbonyl)amino)-3-methylbutanamido)propanamido)benzyl (4-cyclopropoxy-2-((S)-6-(hydroxymethyl)-5-azaspiro[2.4]heptane-5-carbonyl)-5-((triis opropylsilyl)oxy)phenyl)carbamate LD-11b

**LD-11a** (930 mg, 0.05 mmol) was dissolved in a mixed solution of tetrahydrofuran, methanol, and water (V/V/V = 1/1/1, 8 mL), and glacial acetic acid (8 mL) was added. The mixture was stirred at room temperature for 5 h. The reaction mixture was concentrated, and the resulting residue was dissolved in ethyl acetate (10 mL). The solution was washed with water (10 mL × 2) and saturated sodium chloride solution (10 mL) in sequence, dried over anhydrous sodium sulfate, and filtered. Then the filtrate was concentrated under reduced pressure, and the resulting residue was purified by silica gel column chromatography with eluent system B to give the title product **LD-11b** (630 mg, yield: 76.5%).

MS m/z (ESI): 878.3 [M+1].

### Step 3

### 4-((S)-2-((S)-2-(((Allyloxycarbonyl)amino)-3-methylbutanamido)propanamido)benzyl (11S,11aS)-7-cyclopropoxy-11-hydroxy-5-oxo-8-((triisopropylsilyl)oxy)-11,11a-dihydr o-1H,3H-spiro[benzo[e]pyrrolo[1,2-a][1,4]diaza-2,1'-cyclopropane]-10(5H)-carboxylat e LD-11c

**LD-11b** (580 mg, 0.66 mmol) was dissolved in anhydrous dichloromethane (6 mL), and iodobenzene diacetate (319.5 mg, 0.99 mmol) and 2,2,6,6-tetramethylpiperidine oxide (10.4 mg, 0.07 mmol) were sequentially added. The mixture was stirred for 16 h in a nitrogen atmosphere. The reaction mixture was quenched with an ammonium chloride solution (10 mL) and separated. The organic phase was washed with water (10 mL × 2) and saturated sodium chloride solution (10 mL) in sequence, dried over anhydrous sodium sulfate, and filtered. Then the filtrate was concentrated under reduced pressure, and the resulting residue was purified by silica gel column chromatography with eluent system B to give the title product **LD-11c** (489 mg, yield: 84.6%).

MS m/z (ESI): 876.3 [M+1].

### Step 4

### 4-((S)-2-((S)-2-(((Allyloxy)carbonyl)amino)-3-methylbutanamido)propanamido)benzyl (11S,11aS)-11-((tert-butyldimethylsilyl)oxy)-7-cyclopropoxy-5-oxo-8-((triisopropylsilyl )oxy)-11,11a-dihydro-1H,3H-spiro[benzo[e]pyrrolo[1,2-a][1,4]diaza-2,1'-cyclopropane] -10(5H)-carboxylate LD-11d

**LD-11c** (489 mg, 0.56 mmol) was dissolved in *N*,*N*-dimethylformamide (2 mL). In a nitrogen atmosphere, the temperature was lowered to -40 °C, and 4-dimethylaminopyridine (545 mg, 4.46 mmol) and tert-butyldimethylsilyl trifluoromethanesulfonate (1 g, 3.79 mmol) were sequentially added. The mixture was warmed to room temperature and stirred for 16 h. The reaction mixture was quenched with an ammonium chloride solution (10 mL) and separated. The organic phase was washed with water (10 mL × 2) and saturated sodium chloride solution (10 mL) in sequence, dried over anhydrous sodium sulfate, and filtered. Then the filtrate was concentrated under reduced pressure, and the resulting residue was purified by silica gel column chromatography with eluent system B to give the title product **LD-11d** (499 mg, yield: 90.3%).

### Step 5

### 4-((S)-2-((S)-2-(((Allyloxy)carbonyl)amino)-3-methylbutanamido)propanamido)benzyl (11S,11aS)-11-((tert-butyldimethylsilyl)oxy)-7-cyclopropoxy-8-hydroxy-5-oxo-11,11a-dihydro-1H,3H-spiro[benzo[e]pyrrolo[1,2-a][1,4]diaza-2,1'-cyclopropane]-10(5H)-carb oxylate LD-lle

**LD-11d** (499 mg, 0.50 mmol) was dissolved in a mixed solution of *N*,*N*-dimethylformamide and water (V/V = 50/1, 5 mL), and lithium acetate (40 mg, 0.61 mmol) was then added. The mixture was stirred at room temperature for 3 h in a nitrogen atmosphere. Water (10 mL) was added to the reaction mixture, and extraction was performed with ethyl acetate (10 mL × 2). The organic phase was washed with water (10 mL × 2) and saturated sodium chloride solution (10 mL), dried over anhydrous sodium sulfate, and filtered. Then the filtrate was concentrated under reduced pressure, and the resulting residue was purified by silica gel column chromatography with eluent system B to give the title product **LD-11e** (419 mg, yield: 99.7%).

### Step 6

### 4-((S)-2-((S)-2-(((Allyloxy)carbonyl)amino)-3-methylbutanamido)propanamido)benzyl (11aS)-8-((5-(((S)-10-((allyloxy)carbonyl)-7-methoxy-2-(4-methoxyphenyl)-5-oxo-5,10 ,11,11a-tetrahydro-1H-benzo[e]pyrrolo[1,2-a][1,4]diaza-8-yl)oxy)pentyl)oxy)-11-((tert-butyldimethylsilyl)oxy)-7-cyclopropoxy-5-oxo-11,11a-dihydro-1H,3H-spiro[benzo[e]p yrrolo[1,2-a][1,4]diaza-2,1'-cyclopropane]-10(5H)-carboxylate LD-11f

**LD-11e** (70 mg, 0.08 mmol) was dissolved in N,N-dimethylformamide (2 mL), and allyl (*S*)-8-((5-bromopentyl)oxy)-7-methoxy-2-(4-methoxyphenyl)-5-oxy-11,11*a*-dihydro-1*H* -benzo[*e*]pyrrolo[1,2-*a*][1,4]diazepine-10(5*H*)-carboxylate (58.9 mg, 0.10 mmol, prepared by the method disclosed for intermediate 3-10 on page 103 of the specification of the patent "CN 111164208 A") was added. Potassium carbonate (34.8 mg, 0.25 mmol) was added, and the mixture was stirred at room temperature for 16 h. Water (6 mL) was added, and extraction was performed with ethyl acetate (10 mL × 2). The organic phase was collected, washed with water (10 mL) and saturated sodium chloride solution (10 mL) in sequence, dried over anhydrous sodium sulfate, and filtered. Then the filtrate was concentrated under reduced pressure, and the resulting residue was purified by thin-layer chromatography with developing solvent system A to give the title product **LD-11f** (111 mg, yield: 98.7%).

MS m/z (ESI): 1338.6 [M+1].

### Step 7

### 4-((S)-2-((S)-2-(((Allyloxy)carbonyl)amino)-3-methylbutanamido)propanamido)benzyl (11aS)-8-((5-(((S)-10-((allyloxy)carbonyl)-7-methoxy-2-(4-methoxyphenyl)-5-oxo-5,10 ,11,11a-tetrahydro-1H-benzo[e]pyrrolo[1,2-a][1,4]diaza-8-yl)oxy)pentyl)oxy)-7-cyclopr opoxy-11-hydroxy-5-oxo-11,11a-dihydro-1H,3H-spiro[benzo[e]pyrrolo[1,2-a][1,4]diaz a-2,1'-cyclopropane]-10(5H)-carboxylate LD-11g

**LD-**11**f** (111 mg, 0.08 mmol) was dissolved in anhydrous tetrahydrofuran (6 mL), and the solution was cooled in an ice-water bath. Glacial acetic acid (29.9 mg, 0.49 mmol) was added, and then a solution of tetrabutylammonium fluoride in tetrahydrofuran (1 M, 0.4 mL, 0.42 mmol) was added. After the addition, the ice-water bath was removed, and the mixture was stirred at room temperature for 22 h. The reaction mixture was diluted with ethyl acetate (10 mL) and washed with saturated aqueous ammonium chloride solution (10 mL × 2) and saturated aqueous sodium chloride solution (10 mL × 2) in sequence. The organic phase was dried over anhydrous sodium sulfate and filtered. Then the filtrate was concentrated under reduced pressure, and the resulting residue was purified by silica gel column chromatography with eluent system A to give the title product **LD-11g** (92 mg, yield: 90.6%).

MS m/z (ESI): 1224.4 [M+1].

### Step 8

### 4-((S)-2-((S)-2-Amino-3-methylbutanamido)propanamido)benzyl

### (11aS)-7-cyclopropoxy-11-hydroxy-8-((5-(((S)-7-methoxy-2-(4-methoxyphenyl)-5-oxo-5,10,11,11a-tetrahydro-1H-benzo[e]pyrrolo[1,2-a][1,4]diaza-8-yl)oxy)pentyl)oxy)-5-ox o-11,11a-dihydro-1H,3H-spiro[benzo[e]pyrrolo[1,2-a][1,4]diaza-2,1'-cyclopropane]-10( 5H)-carboxylate LD-11h

**LD-11g** (92 mg, 0.08 mmol) was dissolved in anhydrous dichloromethane (3 mL), and pyrrolidine (53.5 mg, 0.75 mmol) and tetrakis(triphenylphosphine)palladium(0) (8.7 mg, 0.0075 mmol) were sequentially added. The mixture was stirred for 30 min in a nitrogen atmosphere. The organic solvent was removed by concentration under reduced pressure, and the resulting residue was purified by thin-layer chromatography with developing solvent system A to give the title product **LD-11h** (49 mg, yield: 61.7%).

MS m/z (ESI): 1056.9 [M+1].

### Step 9

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (7'S)-13'-cyclopropoxy-8'-hydroxy-12'-[(5-{[(7S)-13-methoxy-5-(4-methoxyphenyl)-2-o xo-3,9-diazatricyclo[8.4.0.0^{3,7}]tetradeca-1(14),4,10,12-tetraen-12-yl]oxy}pentyl)oxy]-2' -oxo-3',9'-diazaspiro[cyclopropane-1,5'-tricyclo[8.4.0.0^{3,7}]tetradecane]-1'(14'),10',12'-tri ene-9'-carboxylate LD-11

**LD-11h** (25 mg, 0.02 mmol) was dissolved in a mixture of dichloromethane and methanol (V/V = 10/1, 2 mL), and **LD-1e** (9.9 mg, 0.02 mmol) and 4-(4,6-dimethoxytriazin-2-yl)-4-methylmorpholine hydrochloride (16.4 mg, 0.06 mmol) were sequentially added. The mixture was stirred for 30 min in a nitrogen atmosphere. The organic solvent was removed by concentration under reduced pressure, and the resulting residue was purified by thin-layer chromatography with developing solvent system A to give the title product **LD-11** (14 mg, yield: 40.5%).

MS m/z (ESI): 729.8 [M/2+1].

¹H NMR (500 MHz, DMSO-d6) δ 9.89 (s, 1H), 8.25-7.99 (m, 4H), 7.76 (d, 1H), 7.73-7.57 (m, 3H), 7.57-7.51 (m, 1H), 7.51-7.41 (m, 3H), 7.40-7.27 (m, 4H), 7.24-7.16 (m, 2H), 6.93-6.88 (m, 1H), 6.76-6.70 (m, 1H), 6.68-6.61 (m, 1H), 6.57-6.49 (m, 2H), 6.31 (s, 1H), 5.35-5.30 (m, 1H), 5.23-5.16 (m, 1H), 5.04-4.97 (m, 1H), 4.85-4.76 (m, 1H), 4.42-4.30 (m, 2H), 4.23-4.14 (m, 2H), 3.99-3.82 (m, 3H), 3.76 (s, 3H), 3.67 (s, 2H), 3.63-3.50 (m, 5H), 3.44-3.39 (m, 2H), 3.27-3.21 (m, 1H), 3.18-3.09 (m, 2H), 2.78-2.62 (m, 7H), 2.40-2.33 (m, 2H), 2.31-2.23 (m, 2H), 2.20-2.16 (m, 1H), 2.10-1.93 (m, 4H), 1.84-1.68 (m, 4H), 1.61-1.41 (m, 5H), 1.36 (s, 1H), 0.89-0.73 (m, 6H), 0.72-0.51 (m, 5H).

### Example 3-12 LD-12

### {4-[(2S)-2-[(2S)-2-{2-[2-(4-{2-Azatricyclo[10.4.0.0^{4,9}]hexadeca-1(12),4(9),5,7,13,15-he xaen-10-yn-2-yl}-4-oxobutanamido)acetamido]acetamido}-3-methylbutanamido]propa nami do] phenyl} methyl

### (4S,6S,8S)-9-hydroxy-14-methoxy-13-[(5-{[(7S)-13-methoxy-5-(4-methoxyphenyl)-2-o xo-3,9-diazatricyclo[8.4.0.0^{3,7}]tetradeca-1(14),4,10,12-tetraen-12-yl]oxy}pentyl)oxy]-2-oxo-3,10-diazatetracyclo[9.4.0.0^{3,8}.0^{4,6}]pentadeca-1(15),11,13-triene-10-carboxylate LD-12

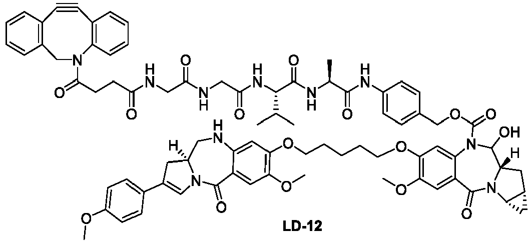

### Example 3-13 LD-13

### (2S,3S,4S,5R)-6-(4-{[(7S)-12-({5-[(7'S)-9'-[({4-[(2S)-2-[(2S)-2-{2-[2-(4-{2-Azatricyclo [10.4.0.0^{4,9}]hexadeca-1(12),4(9),5,7,13,15-hexaen-10-yn-2-yl}-4-oxobutanamido)aceta mido]acetamido}-3-methylbutanamido]propanamido]phenyl}methoxy)carbonyl]-8'-hyd roxy-13'-methoxy-2'-oxo-3',9'-diazaspiro[cyclopropane-1,5'-tricyclo[8.4.0.0^{3,7}]tetradeca ne]-1'(14'),10',12'-trien-12'-yloxy]pentyl}oxy)-13-methoxy-5-(morpholin-4-yl)-2-oxo-3, 9-diazatricyclo[8.4.0.0^{3,7}]tetradeca-1(14),4,10,12-tetraene-9-carbonyloxy]methyl}-2-[(2 -methoxyethyl)aminocarbonyl]phenoxy)-3,4,5-trihydroxyoxane-2-carboxylic acid LD-13

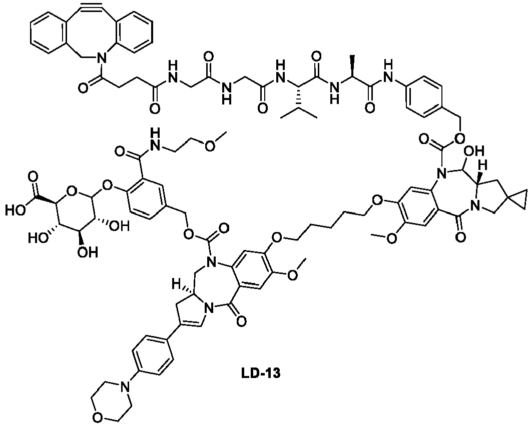

### Example 3-14 LD-14

### (2S,3S,4S,5R)-6-(4-{[(7S)-12-({5-[(7'S)-9'-[({4-[(2S)-2-[(2S)-2-{2-[2-(4-{2-Azatricyclo [10.4.0.0^{4,9}]hexadeca-1(12),4(9),5,7,13,15-hexaen-10-yn-2-yl}-4-oxobutanamido)aceta mido]acetamido}-3-methylbutanamido]propanamido]phenyl}methoxy)carbonyl]-8'-hyd roxy-13'-methoxy-2'-oxo-3',9'-diazaspiro[cyclopropane-1,5'-tricyclo[8.4.0.0^{3,7}]tetradeca ne]-1'(14'),10',12'-trien-12'-yloxy]pentyl}oxy)-5-[4-(cyclopropylthio)phenyl]-13-metho xy-2-oxo-3,9-diazatricyclo[8.4.0.0^{3,7}]tetradeca-1(14),4,10,12-tetraene-9-carbonyloxy]m ethyl}-2-[(2-methoxyethyl)aminocarbonyl]phenoxy)-3,4,5-trihydroxyoxane-2-carboxyli c acid LD-14

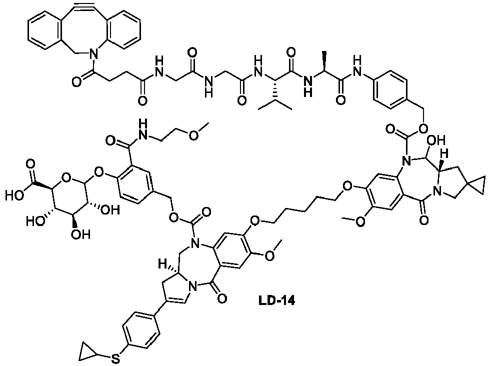

### Example 3-15 LD-15

### (2S,3S,4S,5R)-6-(4-{[(7S)-12-[(1-{3-[(7'S)-9'-[({4-[(2S)-2-[(2S)-2-{2-[2-(4-{2-Azatricy clo[10.4.0.0^{4,9}]hexadeca-1(12),4(9),5,7,13,15-hexaen-10-yn-2-yl}-4-oxobutanamido)ace tamido]acetamido}-3-methylbutanamido]propanamido]phenyl}methoxy)carbonyl]-8'-h ydroxy-13'-methoxy-2'-oxo-3',9'-diazaspiro[cyclopropane-1,5'-tricyclo[8.4.0.0^{3,7}]tetrade cane]-1'(14'),10',12'-trien-12'-yloxy]propyl}cyclopropyl)methoxy]-13-methoxy-5-(4-me thoxyphenyl)-2-oxo-3,9-diazatricyclo[8.4.0.0^{3,7}]tetradeca-1(14),4,10,12-tetraene-9-carb onyloxy]methyl }-2-[(2-methoxyethyl)aminocarbonyl]phenoxy)-3,4, 5-trihydroxyoxane-2-carboxylic acid LD-15

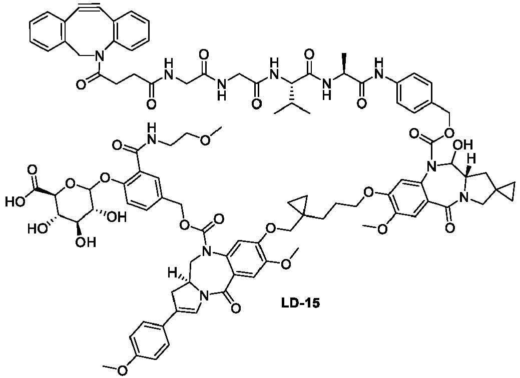

### Example 3-16 LD-16

### {4-[(2S)-2-[(2S)-2-{2-[2-(4-{2-Azatricyclo[10.4.0.0^{4,9}]hexadeca-1(12),4(9),5,7,13,15-h exaen-10-yn-2-yl}-4-oxobutanamido)acetamido]acetamido}-3-methylbutanamido]prop anamido]phenyl}methyl

### (7'S)-12'-[(S-{[(7S)-13-cyclopropoxy-5-(4-methoxyphenyl)-2-oxo-3,9-diazatricyclo[8.4. 0.0^{3,7}]tetradeca-1(14),4,10,12-tetraen-12-yl]oxy}pentyl)oxy]-8'-hydroxy-13'-methoxy-2' -oxo-3',9'-diazaspiro[cyclopropane-1,5'-tricyclo[8.4.0.0^{3,7}]tetradecane]-1'(14'),10',12'-tri ene-9'-carboxylate LD-16

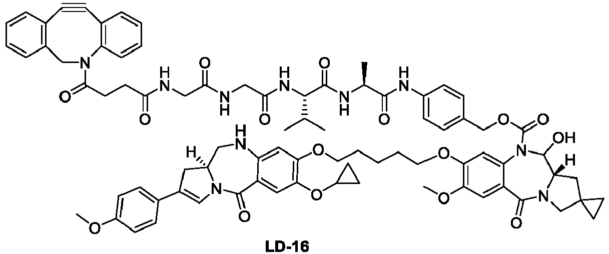

The title product **LD-16** (16 mg, overall yield: 50%) was prepared by using the synthesis scheme of Example **3-2 LD-2** and replacing the reagent **2b** of step 1 with **181.** MS m/z (ESI): 1457.6 [M+1].

1H NMR (500 MHz, DMSO) δ 9.89 (s, 1H), 8.28-7.96 (m, 3H), 7.79-7.64 (m, 2H), 7.63-7.28 (m, 11H), 7.24-7.17 (m, 2H), 7.08-7.02 (m, 1H), 6.91 (d, 2H), 6.75-6.70 (m, 1H), 6.60-6.51 (m, 2H), 6.31 (s, 1H), 5.80-5.74 (m, 1H), 5.22-5.15 (m, 1H), 5.05-5.00 (m, 1H), 4.85-4.78 (m, 1H), 4.40-4.33 (m, 1H), 4.25-4.18 (m, 2H), 4.00-3.70 (m, 20H), 3.65-3.50 (m, 4H), 3.18-3.10 (m, 1H), 2.82-2.73 (m, 2H), 2.31-2.25 (m, 2H), 2.09-1.94 (m, 2H), 1.84-1.72 (m, 4H), 1.58-1.52 (m, 3H), 1.50-1.43 (m, 3H), 0.88-0.78 (m, 6H), 0.68-0.55 (m, 8H).

### Example 3-17 LD-17

### {4-[(2S)-2-[(2S)-2-{2-[2-(4-{2-Azatricyclo[10.4.0.0^{4,9}]hexadeca-1(12),4(9),5,7,13,15-h exaen-10-yn-2-yl}-4-oxobutanamido)acetamido]acetamido}-3-methylbutanamido]prop anamidophenyl}methyl

### (7'S)-12'-[(5-{[(7S)-5-[4-(1-cyclopropylpiperidin-4-yl)phenyl]-13-methoxy-2-oxo-3,9-d iazatricyclo[8.4.0.0^{3,7}]tetradeca-1(14),4,10,12-tetraen-12-yl]oxy}pentyl)oxy]-8'-hydroxy -13'-methoxy-2'-oxo-3',9'-diazaspiro[cyclopropane-1,5'-tricyclo[8.4.0.0^{3,7}]tetradecane]-1 '(14'),10',12'-triene-9'-carboxylate LD-17

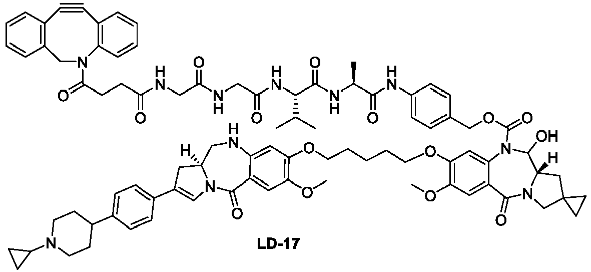

The title product **LD-17** (17 mg, overall yield: 46%) was prepared by using the synthesis scheme of Example **3-2 LD-2** and replacing the reagent **2b** of step 1 with propylene

(*S*)-8-((5-bromopentyl)oxy)-2-(4-(1-cyclopropylpiperidin-4-yl)phenyl)-7-methoxy-5-ox o-11,11*a*-dihydro-1*H*-benzo[*e*]pyrrolo[1,2-*a*][1,4]diazepine-10(5*H*)-carboxylate **36b.**

MS m/z (ESI): 1524.7 [M+1].

¹H NMR (500 MHz, DMSO) δ 9.89 (s, 1H), 8.32-8.00 (m, 3H), 7.79-7.42 (m, 9H), 7.40-7.25 (m, 5H), 7.25-7.15 (m, 2H),7.09-7.01 (m, 1H), 6.75-6.62 (m, 2H), 6.60-6.50 (m, 2H), 6.32 (s, 1H), 5.80-5.72 (m, 1H), 5.24-5.14 (m, 1H), 5.05-4.98 (m, 1H), 4.85-4.77 (m, 1H), 4.40-4.31 (m, 1H), 4.26-4.17 (m, 2H), 4.00-3.87 (m, 6H), 3.85-3.51 (m, 20H), 3.27-3.14 (m, 3H), 3.07-2.97 (m, 1H), 2.80-2.72 (m, 2H), 2.32-2.20 (m, 2H), 2.06-1.93 (m, 6H), 1.86-1.70 (m, 4H), 1.66-1.51 (m, 3H), 1.50-1.42 (m, 3H), 0.90-0.80 (m, 6H), 0.66-0.29 (m, 8H).

### Example 3-18 LD-18

### {4-[(2S)-2-[(2S)-2-{2-[2-(4-{2-Azatricyclo[10.4.0.0^{4,9}]hexadeca-1(12),4(9),5,7,13,15-h exaen-10-yn-2-yl}-4-oxobutanamido)acetamido]acetamido}-3-methylbutanamido]prop anamido]phenyl}methyl

### (7'S)-8'-hydroxy-13'-methoxy-12'-[(5-{[(7S)-13-methoxy-2-oxo-5-{4-[1-(2,2,2-trifluoro ethyl)piperidin-4-yl]phenyl}-3,9-diazatricyclo[8.4.0.0^{3,7}]tetradeca-1(14),4,10,12-tetraen -12-yl]oxy}pentyl)oxy]-2'-oxo-3',9'-diazaspiro[cyclopropane-1,5'-tricyclo[8.4.0.0^{3,7}]tetr adecane]-1'(14'),10',12'-triene-9'-carboxylate LD-18

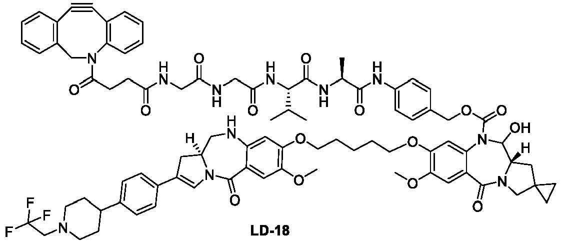

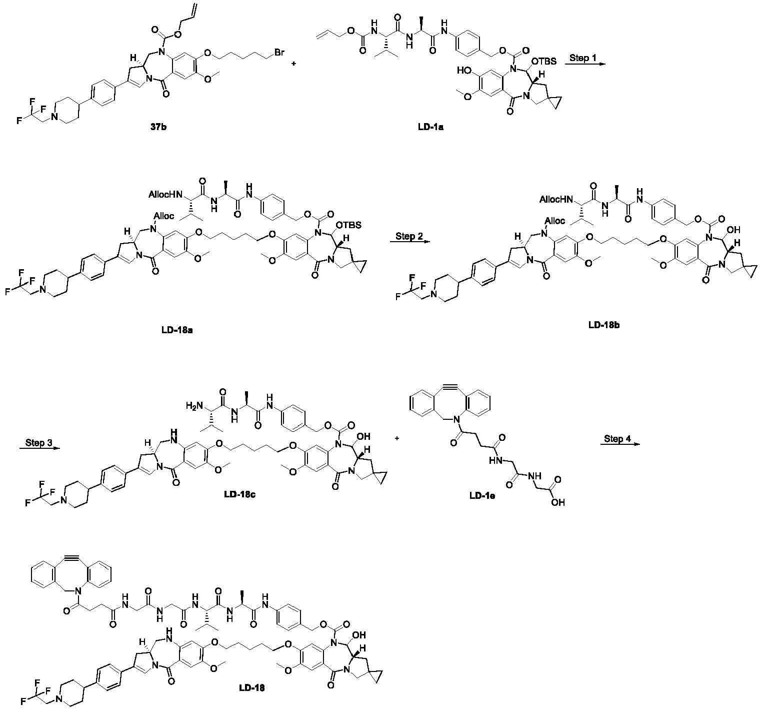

The title product **LD-18** (20 mg, overall yield: 20.6%) was prepared by using the synthesis scheme of Example **3-9 LD-9** and replacing the reagent **13k** of step 1 with (*S*)-8-((5-bromopentyl)oxy)-7-methoxy-2-(4-(1-(2,2,2-trifluoroethyl)piperidin-4-yl)phen yl)-1,10,11,11*a*-tetrahydro-5*H*-benzo[*e*]pyrrolo[1,2-*a*][1,4]diazepin-5-one **37b**.

MS m/z (ESI): 1566.4 [M+1].

¹H NMR (500 MHz, DMSO) δ 9.91 (s, 1H), 8.29-8.10 (m, 2H), 8.09-7.97 (m, 1H), 7.78-7.63 (m, 2H), 7.62-7.57 (m, 1H), 7.57-7.51 (m, 2H), 7.50-7.46 (m, 1H), 7.46-7.41 (m, 1H), 2.40-7.26 (m, 7H), 7.25-7.15 (m, 4H), 7.10-7.01 (m, 1H), 6.72 (s, 1H), 6.53 (s, 2H), 6.35-6.27 (m, 1H), 5.76 (s, 1H), 5.38-5.28 (m, 1H), 5.24-5.14 (m, 1H), 5.06-4.97 (m, 1H), 4.86-4.75 (m, 1H), 4.35 (s, 1H), 4.20 (s, 2H), 3.93 (s, 3H), 3.84 (s, 4H), 3.81-3.71 (m, 6H), 3.68-3.64 (m, 3H), 3.63-3.57 (m, 5H), 3.56-3.51 (m, 2H), 3.05-2.97 (m, 2H), 2.81-2.72 (m, 1H), 2.36 (s, 1H), 2.30-2.24 (m, 1H), 2.06-1.92 (m, 3H), 1.86-1.64 (m, 10H), 1.55 (s, 3H), 1.49-1.43 (m, 1H), 1.37-1.32 (m, 2H), 0.90-0.77 (m, 8H), 0.75-0.66 (m, 1H), 0.65-0.54 (m, 3H).

### IV Glycan Chain Synthesis Examples

### Example 4-1. Synthesis of Glycan Chain OLS-1 (Including OLS-1-a and OLS-1-b)

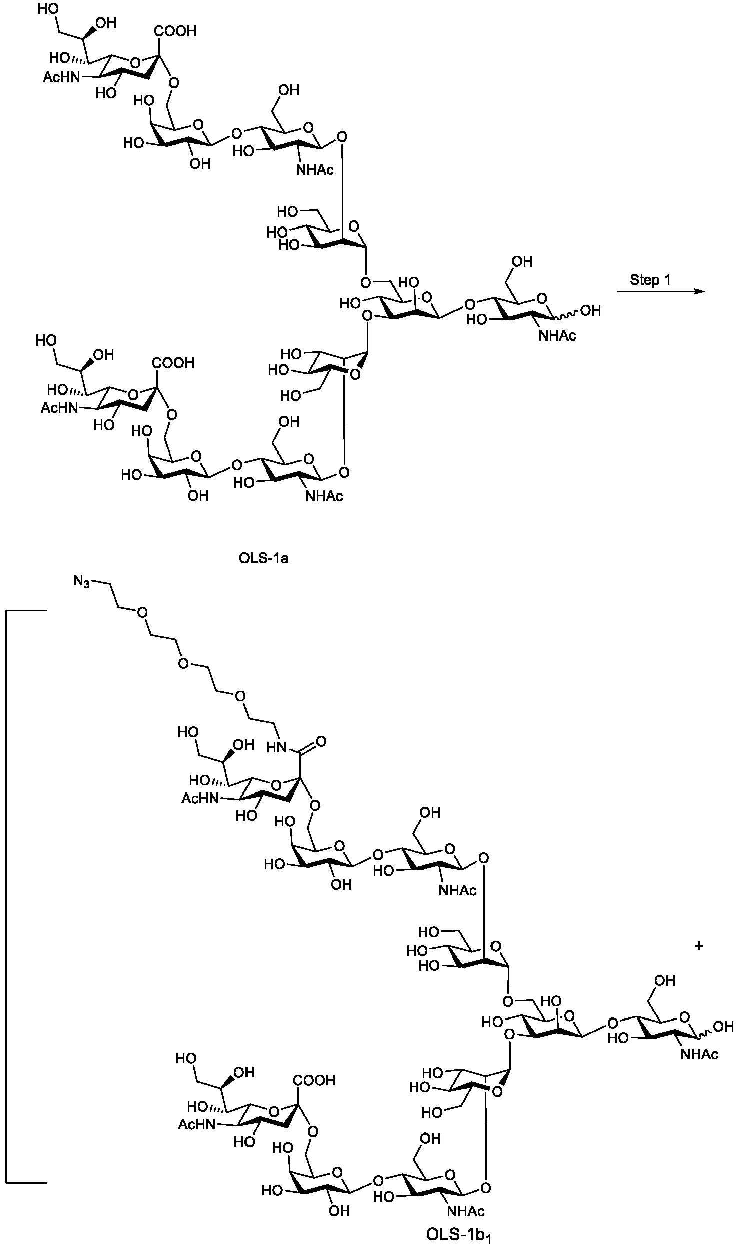

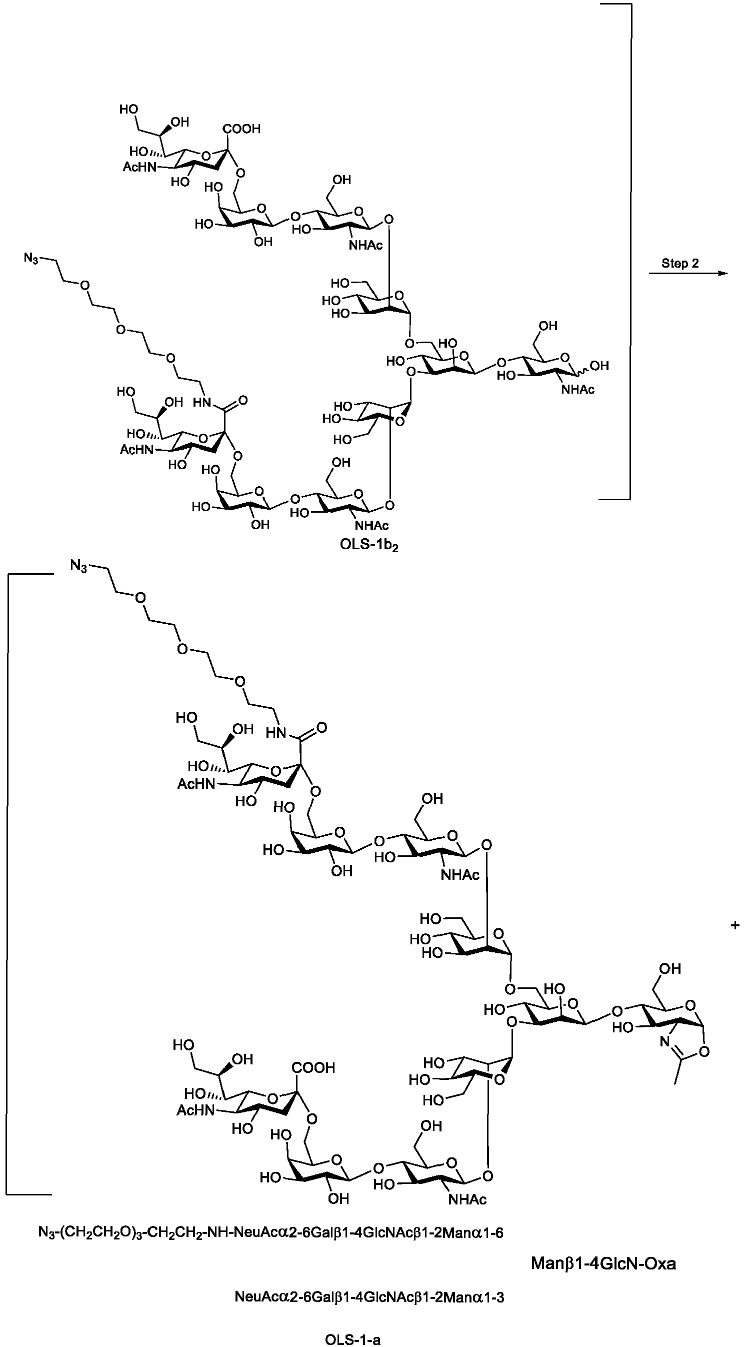

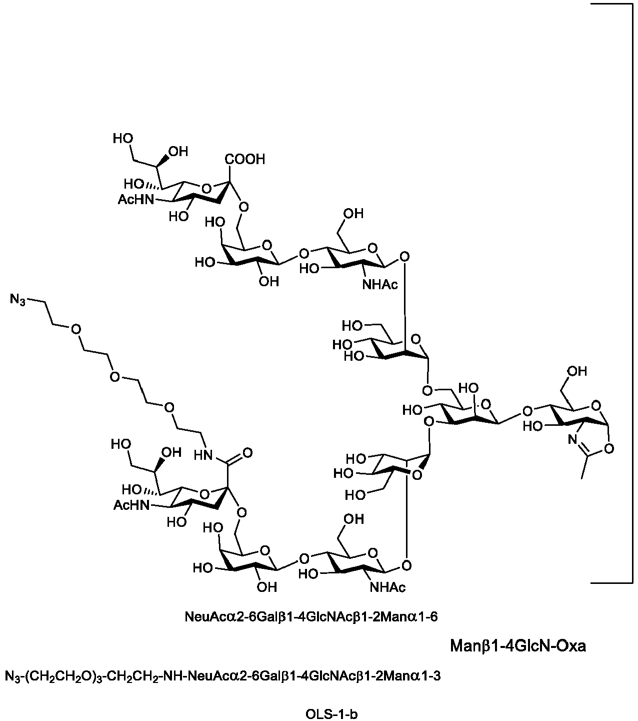

### Step 1

### Synthesis of OLS-1b (including OLS-1b₁ and OLS-1b₂)

OLS-1a (20 mg, 0.01 mmol, prepared by the well-known method "Journal of Carbohydrate Chemistry, 2017, 36, 336-346") was dissolved in 1.2 mL of a mixed solvent of dimethyl sulfoxide and *N,N*-dimethylformamide (V/V = 5:7), and a solution of *N*,*N*-diisopropylethylamine (2.55 mg, 0.02 mmol) in *N,N*-dimethylformamide (0.1 mL), a solution of 11-azido-3,6,9-trioxaundecan-1-amine (1.51 mg, 0.007 mmol, purchased from Energy) in *N,N*-dimethylformamide (0.1 mL), and a solution of *N*,*N*,*N*',*N*'-tetramethyl-O-(7-azabenzotriazol-1-yl)uronium hexafluorophosphate (HATU, 3.16 mg, 0.008 mmol) in *N*,*N*-dimethylformamide (0.1 mL) were sequentially added. The mixture was stirred at 28 °C for 6 h. The reaction mixture was purified by high performance liquid chromatography (separation conditions: column: XBridge Prep Amide 5 µm 19 × 150 mm; mobile phases: A-water (0.01% ammonia water):B-acetonitrile, gradient elution, flow rate: 30 mL/min), and the corresponding fraction was collected and lyophilized to give the title product OLS-1b (including OLS-1b₁ and OLS-1b₂) (4.7 mg).

MS m/z (ESI): 1110.5 [M/2+1].

### Step 2

### Synthesis of OLS-1 (including OLS-1-a and OLS-1-b)

OLS-1b (including OLS-1b₁ and OLS-1b₂) (1 mg, 0.45 µmol) was dissolved in water (0.2 mL), and an aqueous solution (0.1 mL) of triethylamine (2 mg, 0.02 mmol) and 2-chloro-1,3-dimethylimidazolium chloride (1.14 mg, 6.75 µmol) was added under ice-bath conditions. The mixture was stirred under ice-bath conditions, and every 2 h an aqueous solution (0.1 mL) of triethylamine (2 mg, 0.02 mmol) and 2-chloro-1,3-dimethylimidazolium chloride (DMC, 1.14 mg, 6.75 µmol) was added. The mixture was stirred for a total of 8 h. The reaction mixture was purified by high performance liquid chromatography (separation conditions: column: Welch HILIC 5 µm 10 × 250 mm; mobile phases: A-water (0.01% ammonia water):B-acetonitrile, gradient elution, flow rate: 15 mL/min), and the corresponding fraction was collected and lyophilized to give the title product OLS-1 (including OLS-1-a and OLS-1-b) (0.2 mg). MS m/z (ESI): 1101.7 [M/2+1].

### Example 4-2. Synthesis of Glycan Chain OLS-2 (Including OLS-2-a and OLS-2-b)

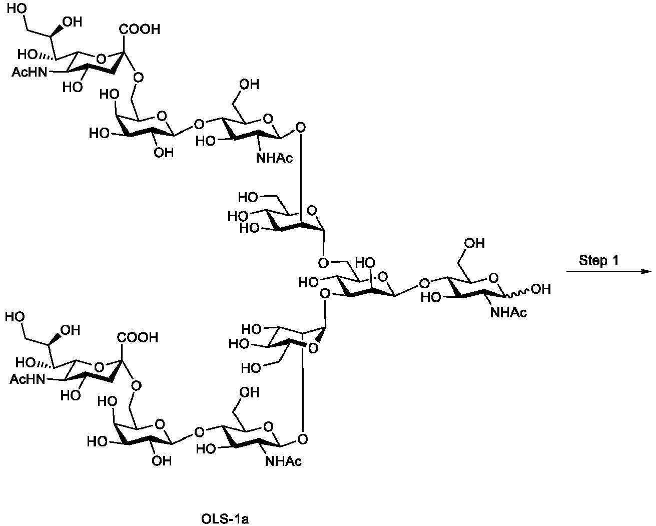

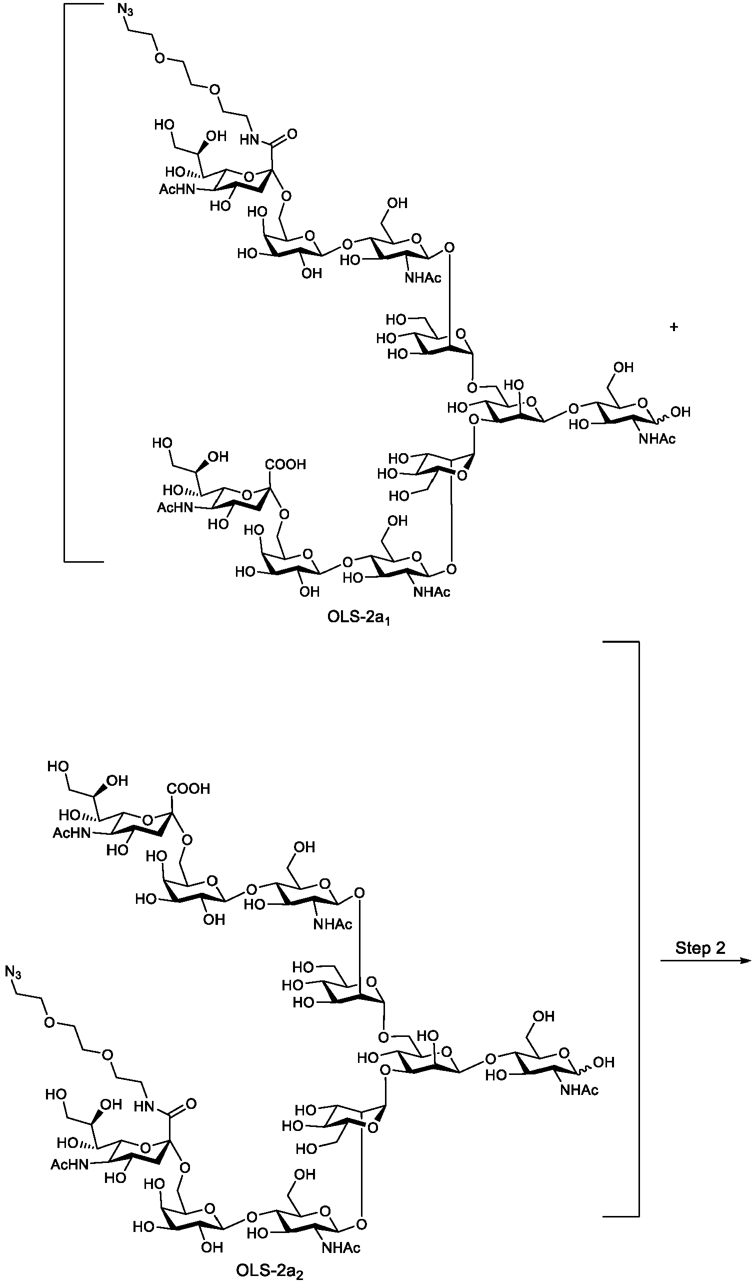

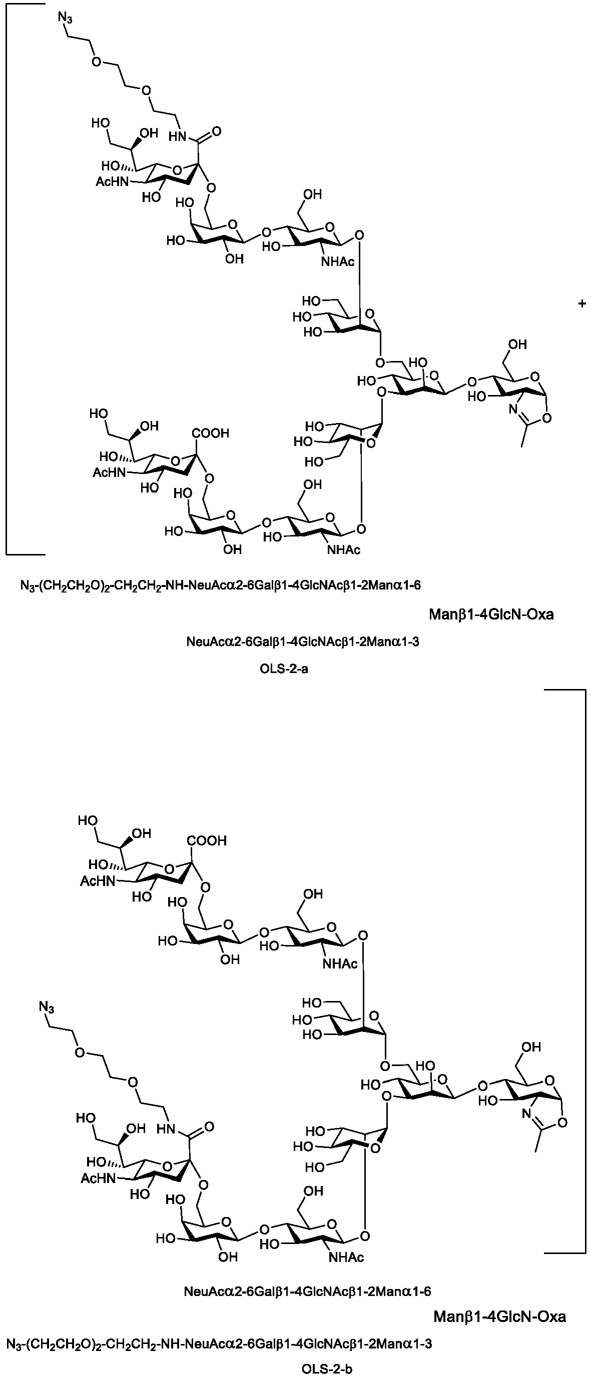

### Step 1

### Synthesis of OLS-2a (including OLS-2a₁ and OLS-2a₂)

OLS-1a (100 mg, 49.5 µmol) was dissolved in 1 mL of a 0.2 M, pH 5.0 phosphate buffer to give solution A; 2-(2-(2-azidoethoxy)ethoxy)ethan-1-amine (N₃-PEG₂-NH₂, 100 mg, 574 µmol, Macklin Reagent) was dissolved in 1 mL of a 0.2 M, pH 7.0 phosphate buffer to give solution B; and 4-(4,6-dimethoxy-1,3,5-triazin-2-yl)-4-methylmorpholinium chloride (DMTMM, 100 mg, 361 µmol, Bide Pharmatech) was dissolved in 1 mL of water to give solution C.

700 µL of solution A, 186 µL of solution B, and 466 µL of solution C were mixed, and the pH was adjusted to 5.0. The mixture was incubated at 50 °C for 3 h. Another 466 µL of solution C was added, and the mixture was incubated at 50 °C for 1.5 h. The reaction mixture was purified by high performance liquid chromatography (separation conditions: column: XBridge^{®} Prep C18 5 µm OBD^{™} (19 × 250 mm); mobile phases: A-water:B-acetonitrile, gradient elution, flow rate: 8 mL/min), and the corresponding fraction was collected and lyophilized to give the title product OLS-2a (including OLS-2a₁ and OLS-2a₂) (61 mg).

MS m/z (ESI): 1088.9 [M/2+1].

### Step 2

### Synthesis of OLS-2 (including OLS-2-a and OLS-2-b)

OLS-2a (61 mg, 28 mmol), 2-chloro-1,3-dimethyl-1*H*-benzimidazol-3-ium chloride (CDMBI, 30.4 mg, 140 mmol), and potassium phosphate (89.2 mg, 420 mmol) were mixed in 2.8 mL of water, and the mixture was cooled in an ice bath and stirred for 3 h. The reaction was complete when a precipitate was generated in the reaction mixture. The reaction mixture was purified by gel column chromatography (packing material: GE Healthcare Sephadex^{™} G-25 Fine, mobile phase: 2 %o aqueous system of triethylamine), and the corresponding fraction was collected and lyophilized to give the title product OLS-2 (including OLS-2-a and OLS-2-b).

MS m/z (ESI): 1079.9 [M/2+1].

### Example 4-3. Synthesis of Glycan Chain OLS-3

### (2S,3S,4S,5R,6R)-4-(2-(2-(2-Azidoethoxy)ethoxy)ethoxy)-2-(((3aR,5R,6S,7R,7aR)-7-hy droxy-5-(hydroxymethyl)-2-methyl-3a,6,7,7a-tetrahydro-5H-pyrano[3,2-d]oxazol-6-yl) oxy)-6-(hydroxymethyl)tetrahydro-2H-pyran-3,5-diol OLS-3

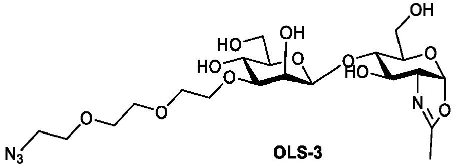

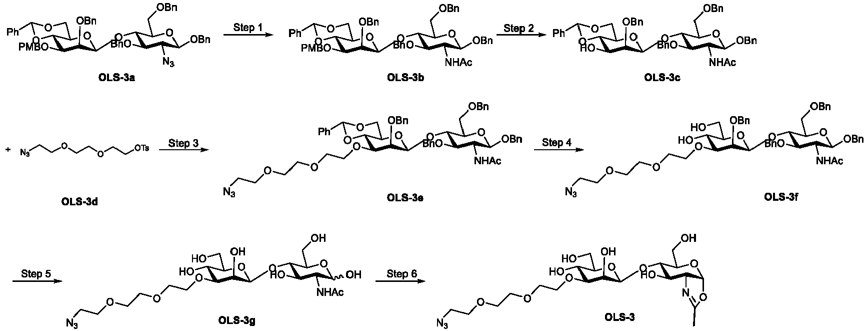

### Step 1

### N-((2R,3R,4R,5S,6R)-2,4-Bis(benzyloxy)-5-(((2R,4aR,6S,7S,8S,8aR)-7-(benzyloxy)-8-( (4-methoxybenzyl)oxy)-2-phenylhexahydropyrano[3,2-d][1,3]dioxin-6-yl)oxy)-6-((benz yloxy)methyl)tetrahydro-2H-pyran-3-yl)acetamide OLS-3b

To a reaction flask containing (2*R*,4*aR*,6*S*,7*S*,8*S*,8*aR*)-6-(((2*R*,3*S*,4*R*,5*R*,6*R*)-5-azido-4,6-bis(benzyloxy)-2-((benzylox y)methyl)tetrahydro-2H-pyran-3-yl)oxy)-7-(benzyloxy)-8-((4-methoxybenzyl)oxy)-2-p henylhexahydropyrano[3,2-d][1,3]dioxine **OLS-3a** (214 mg, 0.23 mmol, prepared by the well-known method *"*Journal of the American Chemistry Society, 2008, 130, 13790-13803") were added isopropanol (10 mL) and ethyl acetate (10 mL) followed by nickel (125 mg). The reaction system was purged with hydrogen gas three times and left to react at 15-25 °C for about 65 h. The reaction mixture was filtered through celite, and the filtrate was concentrated under reduced pressure. Pyridine (5 mL) and acetic anhydride (69 mg, 0.68 mmol) were sequentially added to the resulting residue, and the mixture was left to react at 15-25 °C for about 2.5 h. The reaction mixture was diluted with water (20 mL) and ethyl acetate (30 mL) and separated. The organic phase was collected, and the aqueous phase was further extracted with ethyl acetate (10 mL). The organic phases were combined, washed with saturated sodium bicarbonate solution (10 mL × 2), water (10 mL), and saturated sodium chloride solution (10 mL) in sequence, dried over anhydrous sodium sulfate, and filtered. The filtrate was concentrated under reduced pressure to give a crude product of the title product **OLS-3b** (185 mg). The product was directly used in the next step without purification.

### Step 2

### N-((2R,3R,4R,5S,6R)-2,4-Bis(benzyloxy)-5-(((2R,4aR,6S,7S,8S,8aS)-7-(benzyloxy)-8-h ydroxy-2-phenylhexahydropyrano[3,2-d][1,3]dioxin-6-yl)oxy)-6-((benzyloxy)methyl)te trahydro-2H-pyran-3-yl)acetamide OLS-3c

To a reaction flask containing **OLS-3b** (185 mg, 0.19 mmol) were sequentially added acetonitrile (5 mL), water (0.5 mL), and ceric ammonium nitrate (220 mg, 0.41 mmol), and the mixture was left to react at 20-30 °C for about 1.5 h. The reaction mixture was poured into a mixed solution of saturated sodium bicarbonate solution (20 mL) and ethyl acetate (20 mL), and the mixture was separated. The organic phase was collected, and the aqueous phase was further extracted with ethyl acetate (20 mL). The organic phases were combined, washed with saturated sodium sulfite solution (20 mL) and saturated sodium chloride solution (20 mL) in sequence, dried over anhydrous sodium sulfate, and filtered. The filtrate was concentrated under reduced pressure, and the resulting residue was purified by thin-layer chromatography with developing solvent system C to give the title product **OLS-3c** (65 mg, yield: 40.1%).

MS m/z (ESI): 832.3 [M+1].

¹H NMR (400 MHz, CDCl3): δ 7.41-7.17 (m, 25H), 5.57 (d, 1H), 5.39 (s, 1H), 4.95-4.81 (m, 4H), 4.62 (d, 1H), 4.58-4.47 (m, 4H), 4.41 (d, 1H), 4.09-4.02 (m, 2H), 3.90 (t, 1H), 3.72-3.62 (m, 4H), 3.54-3.46 (m, 3H), 3.40-3.34 (m, 1H), 3.09-3.03 (m, 1H), 2.25 (s, 1H), 1.71 (s, 3H).

### Step 3

### N-((2R,3R,4R,5S,6R)-5-(((2R,4aR,6S,7S,8S,8aR)-8-(2-(2-(2-Azidoethoxy)ethoxy)ethox y)-7-(benzyloxy)-2-phenylhexahydropyrano[3,2-d][1,3]dioxin-6-yl)oxy)-2,4-bis(benzyl

oxy)-6-((benzyloxy)methyl)tetrahydro-2*H*-pyran-3-yl)acetamide **OLS-3e** *N,N*-Dimethylformamide (2 mL) and 2-(2-(2-azidoethoxy)ethoxy)ethyl 4-methylbenzenesulfonate **OLS-3d** (24 mg, 0.073 mmol, Amatek) were sequentially added to a reaction flask containing **OLS-3c** (28 mg, 0.034 mmol), and the mixture was cooled to 0 °C. Sodium hydride (11 mg, 0.28 mmol, content 60%) was added. After the addition, the mixture was left to react at 0 °C for about 1 h and then at 15-25 °C for about 15.5 h. The reaction mixture was poured into a mixed solution of saturated ammonium chloride solution (5 mL) and water (20 mL), and extraction was performed with ethyl acetate (20 mL × 2). The organic phases were combined, washed with water (10 mL) and saturated sodium chloride solution (10 mL) in sequence, dried over anhydrous sodium sulfate, and filtered. The filtrate was concentrated under reduced pressure, and the resulting residue was purified by thin-layer chromatography with developing solvent system C to give the title product **OLS-3e** (33 mg, yield: 100.0%).

MS m/z (ESI): 989.4 [M+1].

¹H NMR (400 MHz, CDCl3) δ 7.40-7.16 (m, 25H), 5.70 (d, 1H), 5.43 (s, 1H), 4.87 (d, 1H), 4.84-4.76 (m, 3H), 4.70 (d, 1H), 4.58-4.50 (m, 4H), 4.39 (d, 1H), 4.08-3.95 (m, 2H), 3.93-3.85 (m, 2H), 3.76-3.68 (m, 3H), 3.62-3.51 (m, 9H), 3.50-3.47 (m, 4H), 3.34-3.31 (m, 1H), 3.24-3.21 (m, 2H), 3.11-3.06 (m, 1H), 1.65 (s, 3H).

### Step 4

### N-((2R,3R,4R,5S,6R)-5-(((2S,3S,4S,5R,6R)-4-(2-(2-(2-Azidoethoxy)ethoxy)ethoxy)-3-(b enzyloxy)-5-hydroxy-6-(hydroxymethyl)tetrahydro-2H-pyran-2-yl)oxy)-2,4-bis(benzylo xy)-6-((benzyloxy)methyl)tetrahydro-2H-pyran-3-yl)acetamide OLS-3f

Compound **OLS-3e** (84 mg, 0.085 mmol) was dissolved in dichloromethane (8 mL). In a nitrogen atmosphere, the temperature was lowered to -20 °C, and trifluoroacetic acid (1.5 mL) was added dropwise. After the dropwise addition, the mixture was warmed to 0 °C and stirred for about 3 h. The reaction mixture was quenched with methanol (4 mL), diluted with dichloromethane (20 mL), and washed with saturated sodium bicarbonate solution (10 mL). The aqueous phase was extracted with dichloromethane (20 mL). The organic phases were combined, washed with saturated sodium chloride solution (10 mL), dried over anhydrous sodium sulfate, and filtered. The filtrate was concentrated under reduced pressure, and the resulting residue was purified by thin-layer chromatography with developing solvent system D to give the title product **OLS-3f** (46 mg, yield: 60.1%).

MS m/z (ESI): 901.4 [M+1].

¹H NMR (400 MHz, CDCl3) δ 7.31-7.15 (m, 20H), 5.70 (d, 1H), 4.88-4.78 (m, 3H), 4.69 (s, 2H), 4.57-4.50 (m, 3H), 4.43-4.39 (m, 2H), 4.08-4.02 (m, 2H), 3.82 (t, 2H), 3.74-3.49 (m, 16H), 3.46-3.37 (m, 2H), 3.29 (t, 2H), 3.11-3.06 (m, 1H), 3.01-2.98 (m, 1H), 1.66 (s, 3H).

### Step 5

### N-((3R,4R,5S,6R)-5-(((2S,3S,4S,5R,6R)-4-(2-(2-(2-Azidoethoxy)ethoxy)ethoxy)-3,5-dih ydroxy-6-(hydroxymethyl)tetrahydro-2H-pyran-2-yl)oxy)-2,4-dihydroxy-6-(hydroxyme thyl)tetrahydro-2H-pyran-3-yl)acetamide OLS-3g (a mixture of diastereomers)

Compound **OLS-3f** (50 mg, 0.055 mmol) was dissolved in tetrahydrofuran (5 mL) and water (1 mL), and 5% palladium hydroxide (14 mg), 10% palladium on carbon (15 mg), and hydrochloric acid (1 drop) were added. The reaction system was purged with hydrogen gas three times. The mixture was stirred at room temperature for about 3 h. The reaction mixture was filtered, and the filtrate was concentrated under reduced pressure to give a crude intermediate. To another reaction flask were sequentially added imidazole-1-sulfonyl azide hydrochloride (29.1 mg, 0.057 mmol, prepared by the well-known method *"*Organic Letters, 2007, 9, 3797-3800"), potassium carbonate (39.2 mg, 0.28 mmol), copper(II) sulfate pentahydrate (5.4 mg, 0.022 mmol), and methanol (2 mL). Then the crude intermediate was dissolved in methanol (2 mL), and the solution was added to the reaction flask. The mixture was left to react at room temperature for about 16 h. The reaction mixture was filtered, and the filtrate was concentrated under reduced pressure. Ethanol (6 mL) was added. After filtration, the resulting filtrate was concentrated under reduced pressure to give a crude product 1 of the title product **OLS-3g.**

Compound **OLS-3f** (144 mg, 0.16 mmol) was dissolved in tetrahydrofuran (4 mL) and water (1 mL), and 5% palladium hydroxide (7 mg), 10% palladium on carbon (5 mg), and hydrochloric acid (22 mg, 0.22 mmol) were added. The reaction system was purged with hydrogen gas three times and stirred at room temperature for about 3.5 h. 5% palladium hydroxide (32 mg) and 10% palladium on carbon (31 mg) were added to the reaction mixture. The reaction system was purged with hydrogen gas three times and stirred at room temperature for about 1.5 h. The reaction mixture was filtered, and the filtrate was concentrated under reduced pressure to give a crude intermediate (112 mg). To another reaction flask were sequentially added imidazole-1-sulfonyl azide hydrochloride (74.3 mg, 0.35 mmol), potassium carbonate (102.7 mg, 0.74 mmol), copper(II) sulfate pentahydrate (11.8 mg, 0.047 mmol), and methanol (6 mL). Then the crude intermediate (89.3 mg) was added to the reaction flask, and the mixture was left to react at room temperature for about 4.5 h. The reaction mixture was filtered, and the filtrate was concentrated under reduced pressure. Methanol (12 mL) was added. After filtration, the resulting filtrate was concentrated under reduced pressure to give a crude product 2 of the title product **OLS-3g.**

The two crude products of the title product **OLS-3g** described above were combined and purified by high performance liquid chromatography (column: Phenomenex C18, 150 × 25 mm, 10 µm; mobile phases: aqueous phase (10 mM ammonium bicarbonate) and acetonitrile, gradient ratio: acetonitrile 0%-20%, flow rate: 25 mL/min) to give the title product **OLS-3g** (80 mg, yield: 68.7%).

MS m/z (ESI): 541.2 [M+1].

¹H NMR (400 MHz, D2O) δ 5.20 (d, 1H), 4.75 (s, 1H), 4.30-4.24 (m, 1H), 3.94-3.84 (m, 5H), 3.81-3.77 (m, 1H), 3.76-3.70 (m, 11H), 3.67-3.62 (m, 2H), 3.60-3.52 (m, 1H), 3.51-3.44 (m, 4H), 2.03 (s, 3H).

### Step 6

### (2S,3S,4S,5R,6R)-4-(2-(2-(2-Azidoethoxy)ethoxy)ethoxy)-2-(((3aR,5R,6S,7R,7aR)-7-hy droxy-5-(hydroxymethyl)-2-methyl-3a,6,7,7a-tetrahydro-5H-pyrano[3,2-d]oxazol-6-yl) oxy)-6-(hydroxymethyl)tetrahydro-2H-pyran-3,5-diol OLS-3

**OLS-3g** (50 mg, 0.093 mmol) was dissolved in purified water (12 mL) under ice-bath conditions, and cesium carbonate (1.80 g, 5.52 mmol) and 2-chloro-1,3-dimethyl-1*H*-benzimidazol-3-ium chloride (420 mg, 1.93 mmol, prepared by the well-known method *"*Helvetica Chimica Acta, 2012, vol. 95, 1928-1936") were added. The mixture was stirred at 0 °C for about 13.5 h. The reaction mixture was purified by high performance liquid chromatography (column: Phenomenex C18, 150 × 25 mm, 10 µm; mobile phase: water and acetonitrile, gradient ratio: acetonitrile 0%-20%, flow rate: 25 mL/min) to give the title product **OLS-3** (15 mg, yield: 31.0%). MS m/z (ESI): 523.1 [M+1].

¹H NMR (400 MHz, CDCl3) δ 6.00 (d, 1H), 5.38 (s, 1H), 4.99 (s, 1H), 4.65 (s, 1H), 4.44-4.31 (m, 3H), 4.12-4.02 (m, 3H), 3.99-3.95 (m, 2H), 3.89-3.87 (m, 1H), 3.84-3.76 (m, 3H), 3.75-3.65 (m, 9H), 3.40 (t, 2H), 3.37-3.35 (m, 1H), 3.31-3.28 (m, 1H), 2.67 (s, 2H), 2.02 (s, 3H).

### Example 4-4. Synthesis of Glycan Chain OLS-4

### (2S,3S,4S,5R,6R)-4-(2-(3-(2-(2-Azidoethoxy)ethoxy)propoxy)ethoxy)-2-(((3aR,5R,6S,7 R,7aR)-7-hydroxy-5-(hydroxymethyl)-2-methyl-5,6,7,7a-tetrahydro-3aH-pyrano[3,2-d] oxazol-6-yl)oxy)-6-(hydroxymethyl)tetrahydro-2H-pyran-3,5-diol OLS-4

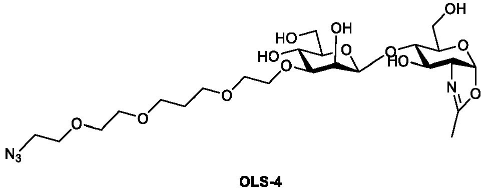

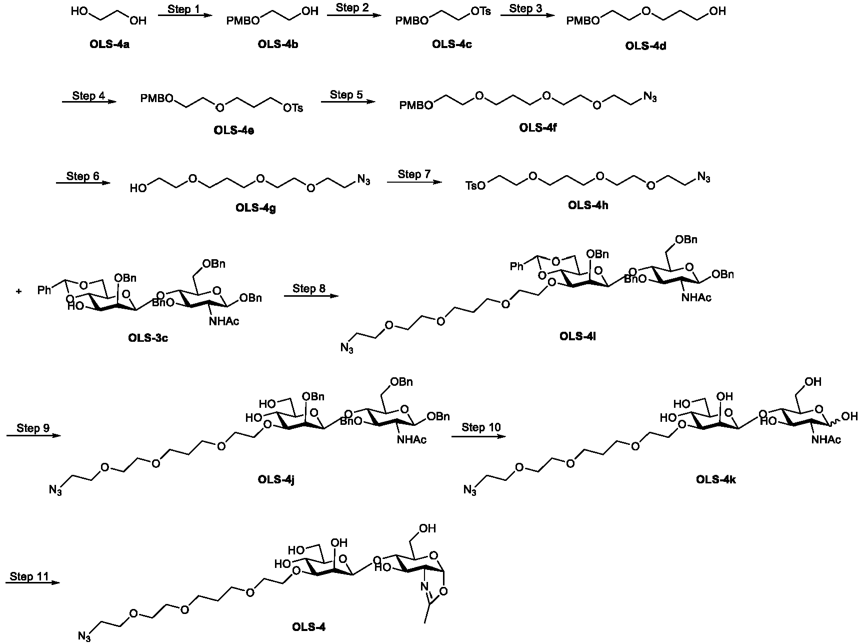

### Step 1

### 2-((4-Methoxybenzyl)oxy)ethan-1-ol OLS-4b

Ethylene glycol **OLS-4a** (50.00 g, 805.57 mmol) was dissolved in anhydrous tetrahydrofuran (300 mL), and sodium hydride (4.93 g, 128.89 mmol, content 60%) and tetrabutylammonium iodide (5.37 g, 16.11 mmol) were sequentially added. In a nitrogen atmosphere, the mixture was stirred at 0 °C for 30 min, and then p-methoxybenzyl chloride (20.19 g, 128.89 mmol) was slowly added dropwise over about 30 min. The mixture was heated to 80 °C and stirred for 16 h. The reaction mixture was poured into ice water and stirred to be quenched. Ethyl acetate (500 mL) was added, and the mixture was stirred and separated. The resulting aqueous phase was extracted with ethyl acetate (150 mL × 3). The organic phases were combined, washed with water (250 mL × 3) and saturated sodium chloride solution (250 mL × 2), dried over anhydrous sodium sulfate, and filtered. The filtrate was concentrated under reduced pressure, and the resulting residue was purified by silica gel column chromatography with eluent system C to give the title product **OLS-4b** (22.00 g, yield: 14.9%).

MS m/z (ESI): 205.2 [M+23].

### Step 2

### 2-((4-Methoxybenzyl)oxy)ethyl 4-methylbenzenesulfonate OLS-4c

**OLS-4b** (22.00 g, 120.74 mmol) was dissolved in pyridine (98 mL), and p-toluenesulfonyl chloride (27.62 g, 144.88 mmol) was added at 0 °C. In a nitrogen atmosphere, the mixture was stirred at 0 °C, slowly warmed to room temperature, and left to react for 16 h. The reaction mixture was diluted with ethyl acetate (300 mL) and washed with hydrochloric acid (10%, 150 mL × 3). The organic phase was washed with water (150 mL × 3) and saturated sodium chloride solution (250 mL × 2) in sequence, dried over anhydrous sodium sulfate for 30 min, and filtered. The filtrate was concentrated under reduced pressure, and the resulting residue was purified by silica gel column chromatography with eluent system C to give the title product **OLS-4c** (36.40 g, yield: 89.6%).

¹H NMR (400 MHz, CDCl3) δ 7.81 (d, 2H), 7.33 (d, 2H), 7.21 (d, 2H), 6.88 (d, 2H), 4.43 (s, 2H), 4.23-4.17 (m, 2H), 3.82 (s, 3H), 3.68-3.61 (m, 2H), 2.45 (s, 3H).

### Step 3

### 3-(2-((4-Methoxybenzyl)oxy)ethoxy)propan-1-ol OLS-4d

**OLS-4c** (36.40 g, 108.20 mmol) and 1,3-propanediol (82.34 g, 1.08 mol) were dissolved in toluene (100 mL), and powdered potassium hydroxide (15.18 g, 270.51 mmol) was added at room temperature. The mixture was heated to 50 °C in a nitrogen atmosphere and stirred for 72 h. The reaction mixture was poured into ice water (200 mL), and concentrated hydrochloric acid was added dropwise until the pH of the reaction mixture was 3-4. Ethyl acetate (300 mL) was added for extraction. The mixture was separated. The aqueous phase was extracted with ethyl acetate (100 mL × 3), and the organic phases were combined, dried over anhydrous sodium sulfate for 30 min, and filtered. The filtrate was concentrated under reduced pressure, and the resulting residue was purified by silica gel column chromatography with eluent system C to give the title product **OLS-4d** (15.60 g, yield: 60.0%).

¹H NMR (400 MHz, CDCl3) δ 7.28 (d, 2H), 6.89 (d, 2H), 4.51 (s, 2H), 3.81 (s, 3H), 3.79 (t, 2H), 3.68 (t, 2H), 3.65-3.59 (m, 4H), 2.51 (br.s, 1H), 1.88-1.82 (m, 2H).

### Step 4

### 3-(2-((4-Methoxybenzyl)oxy)ethoxy)propyl 4-methylbenzenesulfonate OLS-4e

**OLS-4d** (15.60 g, 64.92 mmol) was dissolved in a mixed solution of pyridine (52 mL) and dichloromethane (52 mL), and p-toluenesulfonyl chloride (18.56 g, 97.38 mmol) was added at 0 °C. In a nitrogen atmosphere, the mixture was stirred at 0 °C, slowly warmed to room temperature, and left to react for 16 h. The reaction mixture was diluted with ethyl acetate (300 mL), washed with hydrochloric acid (10%, 150 mL × 3), water (150 mL × 3), and saturated sodium chloride solution (250 mL × 2) in sequence, dried over anhydrous sodium sulfate for 30 min, and filtered. The filtrate was concentrated under reduced pressure, and the resulting residue was purified by silica gel column chromatography with eluent system C to give the title product **OLS-4e** (18.00 g, yield: 70.3%).

MS m/z (ESI): 417.3 [M+23].

¹H NMR (400 MHz, CDCl3) δ 7.80 (d, 2H), 7.34 (d, 2H), 7.27 (d, 2H), 6.90 (d, 2H), 4.48 (s, 2H), 4.16 (t, 2H), 3.83 (s, 3H), 3.53-3.49 (m, 6H), 2.45 (s, 3H), 1.96-1.91 (m, 2H).

### Step 5

### 14-Azido-1-(4-methoxyphenyl)-2,5,9,12-tetraoxatetradecane OLS-4f

**OLS-4e** (8.00 g, 20.28 mmol) and 2-(2-azidoethoxy)ethanol (3.72 g, 28.39 mol, Amatek) were dissolved in toluene (33 mL), and powdered potassium hydroxide (2.84 g, 50.70 mmol) was added at room temperature. The mixture was heated to 60 °C in a nitrogen atmosphere and stirred for 16 h. The reaction mixture was poured into ice water (100 mL), and concentrated hydrochloric acid was added dropwise until the pH of the reaction mixture was 3-4. Ethyl acetate (100 mL) was added for extraction. After separation, the aqueous phase was extracted with ethyl acetate (50 mL × 3). The organic phases were combined, dried over anhydrous sodium sulfate for 30 min, and filtered. The filtrate was concentrated under reduced pressure, and the resulting residue was purified by silica gel column chromatography with eluent system C to give the title product **OLS-4f** (6.3 g, yield: 87.9%).

MS m/z (ESI): 376.3 [M+23].

¹H NMR (400 MHz, CDCl3) δ 7.29 (d, 2H), 6.89 (d, 2H), 4.52 (s, 2H), 3.82 (s, 3H), 3.70-3.56 (m, 14H), 3.40 (t, 2H), 1.93-1.87 (m, 2H).

### Step 6

### 2-(3-(2-(2-Azidoethoxy)ethoxy)propoxy)ethan-1-ol OLS-4g

**OLS-4f** (6.30 g, 17.83 mmol) was dissolved in dichloromethane (31 mL) and water (3.1 mL), and dichlorodicyanobenzoquinone (4.45 g, 19.61 mmol, Aladdin) was added at room temperature. The mixture was stirred at 25 °C for 1.5 h in a nitrogen atmosphere. The reaction mixture was filtered through celite. The filtrate was diluted with dichloromethane (150 mL), washed with saturated sodium bicarbonate solution (100 mL × 2), saturated sodium bisulfite solution (100 mL × 2), water (100 mL), and saturated sodium chloride solution (100 mL × 2) in sequence, dried over anhydrous sodium sulfate for 30 min, and filtered. The filtrate was concentrated under reduced pressure, and the resulting residue was purified by silica gel column chromatography with eluent system C to give the title product **OLS-4g** (3.2 g, yield: 76.9%).

### Step 7

### 2-(3-(2-(2-Azidoethoxy)ethoxy)propoxy)ethyl 4-methylbenzenesulfonate OLS-4h

**OLS-4g** (3.20 g, 13.72 mmol) was dissolved in pyridine (11 mL) and dichloromethane (11 mL). The solution was cooled to 0 °C, and p-toluenesulfonyl chloride (3.14 g, 16.46 mmol) was added. In a nitrogen atmosphere, the mixture was stirred at 0 °C, slowly warmed to room temperature, and left to react for 16 h. The reaction mixture was diluted with ethyl acetate (200 mL), washed with hydrochloric acid (10%, 150 mL × 3), water (150 mL × 3), and saturated sodium chloride solution (250 mL × 2) in sequence, dried over anhydrous sodium sulfate for 30 min, and filtered. The filtrate was concentrated under reduced pressure, and the resulting residue was purified by silica gel column chromatography with eluent system C to give the title product **OLS-4h** (4.80 g, yield: 90.3%).

¹H NMR (400 MHz, CDCl3) δ 7.79 (d, 2H), 7.34 (d, 2H), 4.14 (t, 2H), 3.67-3.57 (m, 8H), 3.51-3.46 (m, 4H), 3.38 (t, 2H), 2.44 (s, 3H), 1.81-1.75 (m, 2H).

### Step 8

### N-((2R,3R,4R,5S,6R)-5-(((2R,4aR,6S,7S,8S,8aR)-8-(2-(3-(2-(2-Azidoethoxy)ethoxy)pro poxy)ethoxy)-7-(benzyloxy)-2-phenylhexahydropyrano[3,2-d][1,3]dioxin-6-yl)oxy)-2,4 -bis(benzyloxy)-6-((benzyloxy)methyl)tetrahydro-2H-pyran-3-yl)acetamide OLS-4i

**OLS-3c** (340 mg, 0.41 mmol), *N,N*-dimethylformamide (4.8 mL), and 2 mL of a solution of **OLS-4h** (398 mg, 1.03 mmol) in *N*,*N*-dimethylformamide were sequentially added to a reaction flask and cooled to 0 °C. Sodium hydride (86 mg, 2.15 mmol, content 60%) was added, and the mixture was left to react at 0 °C for about 0.5 h and then at 20-30 °C for about 0.5 h. The reaction mixture was poured into a mixed solution of saturated ammonium chloride solution (10 mL) and water (10 mL), and extraction was performed with ethyl acetate (10 mL × 3). The organic phases were combined, washed with water (10 mL) and saturated sodium chloride solution (10 mL) in sequence, dried over anhydrous sodium sulfate, and filtered. The filtrate was concentrated under reduced pressure, and the resulting residue was purified by thin-layer chromatography with developing solvent system C to give the title product **OLS-4i** (362 mg, yield: 84.6%).

MS m/z (ESI): 1047.4 [M+1].

¹H NMR (400 MHz, CDCl3) δ 7.40-7.16 (m, 25H), 5.70 (d, 1H), 5.44 (s, 1H), 4.87 (d, 1H), 4.84-4.78 (m, 3H), 4.71-4.68 (m, 1H), 4.57-4.50 (m, 4H), 4.41-4.38 (m, 1H), 4.04-4.01 (m, 1H), 3.97 (t, 1H), 3.93-3.84 (m, 2H), 3.76-3.68 (m, 3H), 3.61-3.40 (m, 16H), 3.34-3.27 (m, 3H), 3.11-3.05 (m, 2H), 1.78-1.72 (m, 2H), 1.51 (s, 3H).

### Step 9

### N-((2R,3R,4R,5S,6R)-5-(((2S,3S,4S,5R,6R)-4-(2-(3-(2-(2-Azidoethoxy)ethoxy)propoxy) ethoxy)-3-(benzyloxy)-5-hydroxy-6-(hydroxymethyl)tetrahydro-2H-pyran-2-yl)oxy)-2, 4-bis(benzyloxy)-6-((benzyloxy)methyl)tetrahydro-2H-pyran-3-yl)acetamide OLS-4j

Compound **OLS-4i** (360 mg, 0.34 mmol) and dichloromethane (26 mL) were sequentially added to a reaction flask. In a nitrogen atmosphere, the temperature was lowered to -30 to -20 °C, and trifluoroacetic acid (3.6 mL) was added dropwise. After the dropwise addition, the mixture was warmed to -5 to 5 °C and stirred for about 1.5 h. The reaction mixture was quenched with methanol (5.1 mL), diluted with dichloromethane (12.3 mL), washed with saturated sodium bicarbonate solution (26 mL × 2) and saturated sodium chloride solution (13 mL) in sequence, dried over anhydrous sodium sulfate, and filtered. The filtrate was concentrated under reduced pressure, and the resulting residue was purified by thin-layer chromatography with developing solvent system C to give the title product **OLS-4j** (234 mg, yield: 71.0%).

MS m/z (ESI): 959.7 [M+1].

¹H NMR (400 MHz, CDCl3) δ 7.31-7.17 (m, 20H), 5.64 (d, 1H), 4.87 (d, 1H), 4.84-4.79 (m, 2H), 4.70 (s, 2H), 4.58-4.50 (m, 3H), 4.43-4.39 (m, 2H), 4.05 (t, 1H), 3.82 (t, 1H), 3.74-3.36 (m, 23H), 3.30 (t, 2H), 3.10-3.07 (m, 1H), 3.00-2.97 (m, 1H), 2.21 (br.s, 1H), 1.81-1.75 (m, 2H), 1.66 (s, 3H).

### Step 10

### N-((3R,4R,5S,6R)-5-(((2S,3S,4S,5R,6R)-4-(2-(3-(2-(2-Azidoethoxy)ethoxy)propoxy)eth oxy)-3,5-dihydroxy-6-(hydroxymethyl)tetrahydro-2H-pyran-2-yl)oxy)-2,4-dihydroxy-6-(hydroxymethyl)tetrahydro-2H-pyran-3-yl)acetamide OLS-4k (a mixture of diastereomers)

**OLS-4j** (150 mg, 158.72 µmol) was dissolved in tetrahydrofuran (8 mL) and water (2 mL), and palladium hydroxide (50.0 mg, content 15%), palladium on carbon (61 mg, content 10%), and hydrochloric acid (1 drop) were added. The reaction system was purged with hydrogen gas three times and stirred at room temperature for 12 h. The catalyst was removed by filtration. The filtrate was concentrated under reduced pressure, and the resulting residue was dissolved in methanol (7 mL). Imidazole-1-sulfonyl azide hydrochloride (6 mg, 220.41 µmol), potassium carbonate (68 mg, 492.00 µmol), and copper sulfate (5.7 mg, 22.83 µmol) were added, and the mixture was stirred at room temperature for 12 h. The inorganic salts were removed by filtration, and the solids were rinsed with methanol (3 mL). The organic phases were combined, and the filtrate was concentrated under reduced pressure to give a crude product of the title product **OLS-4k** (161 mg). The product was directly used in the next step without purification.

MS m/z (ESI): 599.3 [M+1].

### Step 11

### (2S,3S,4S,5R,6R)-4-(2-(3-(2-(2-Azidoethoxy)ethoxy)propoxy)ethoxy)-2-(((3aR,5R,6S,7 R,7aR)-7-hydroxy-5-(hydroxymethyl)-2-methyl-5,6,7,7a-tetrahydro-3aH-pyrano[3,2-d] oxazol-6-yl)oxy)-6-(hydroxymethyl)tetrahydro-2H-pyran-3,5-diol OLS-4

The crude product of compound **OLS-4k** (161 mg, 148.01 µmol) was dissolved in purified water (8 mL) under ice-bath conditions, and 2-chloro-1,3-dimethyl-1*H*-benzimidazol-3-ium chloride (557 mg, 3.07 mmol) and cesium carbonate (3.00 g, 9.20 mmol) were added. The mixture was stirred at 0 °C for 12 h. The reaction mixture was purified by high performance liquid chromatography (column: Phenomenex C18, 150 × 25 mm, 10 µm; mobile phases: water (0.01% ammonia water) and acetonitrile, gradient ratio: acetonitrile 0%-30%, flow rate: 25 mL/min), and lyophilized in a 5 mol% sodium hydroxide solution to give the title product **OLS-4** (35 mg, yield over steps 10 and 11: 38.2%).

MS m/z (ESI): 581.3 [M+1].

¹H NMR (400 MHz, CDCl3) δ 6.02 (d, 1H), 4.65 (s, 2H), 4.30 (s, 2H), 4.09-3.93(m, 5H), 3.86-3.77 (m, 4H), 3.73-3.51 (m, 16H), 3.40-3.28 (m, 5H), 2.01 (s, 3H), 1.88-1.81 (m, 2H).

### Example 4-5. Synthesis of Glycan Chain OLS-5

### (2S,3S,4S,5R,6R)-4-(2-(3-(2-Azidoethoxy)propoxy)ethoxy)-2-(((3aR,5R,6S,7R,7aR)-7-h ydroxy-5-(hydroxymethyl)-2-methyl-3a,6,7,7a-tetrahydro-5H-pyrano[3,2-d]oxazol-6-yl )oxy)-6-(hydroxymethyl)tetrahydro-2H-pyran-3,5-diol OLS-5

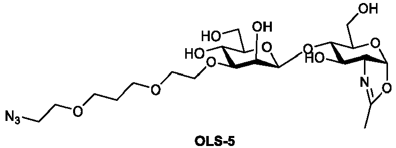

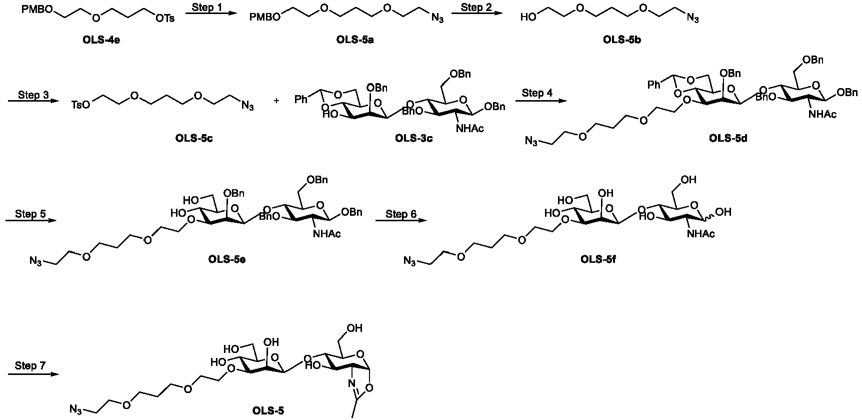

### Step 1

### 1-((2-(3-(2-Azidoethoxy)propoxy)ethoxy)methyl)-4-methoxybenzene OLS-5a

**OLS-4e** (2.0 g, 5.07 mmol) and 2-azidoethanol (593 mg, 6.08 mmol) were dissolved in toluene (7 mL), and powdered potassium hydroxide (711 mg, 12.67 mmol) was added at room temperature. The mixture was heated to 60 °C in a nitrogen atmosphere and stirred for 16 h. The reaction mixture was poured into ice water (100 mL), and concentrated hydrochloric acid was added dropwise until the pH of the reaction mixture was 3-4. Extraction was performed with ethyl acetate (50 mL × 3). The organic phases were combined, dried over anhydrous sodium sulfate for 30 min, and filtered. The filtrate was concentrated under reduced pressure, and the resulting residue was purified by silica gel column chromatography with eluent system C to give the title product **OLS-5a** (1.09 g, yield: 69.4%).

¹H NMR (400 MHz, CDCl3) δ 7.27 (d, 2H), 6.88 (d, 2H), 4.50 (s, 2H), 3.80 (s, 3H), 3.62-3.55 (m, 10H), 3.35 (t, 2H), 1.91-1.85 (m, 2H).

### Step 2

### 2-(3-(2-Azidoethoxy)propoxy)ethan-1-ol OLS-5b

**OLS-5a** (1090 mg, 3.56 mmol) was dissolved in dichloromethane (5.0 mL) and water (0.5 mL), and dichlorodicyanobenzoquinone (888 mg, 3.91 mmol) was added at room temperature. The mixture was stirred at 25 °C for 1 h in a nitrogen atmosphere. The reaction mixture was diluted with dichloromethane (150 mL) and washed with saturated sodium bicarbonate solution (100 mL × 2), saturated sodium bisulfite solution (100 mL × 2), water (100 mL), and saturated sodium chloride solution (100 mL × 2) in sequence. The organic phase was dried over anhydrous sodium sulfate for 30 min and filtered. The filtrate was concentrated under reduced pressure, and the resulting residue was purified by silica gel column chromatography with eluent system C to give the title product **OLS-5b** (385 mg, yield: 57.2%).

### Step 3

### 2-(3-(2-Azidoethoxy)propoxy)ethyl 4-methylbenzenesulfonate OLS-5c

**OLS-5b** (385 mg, 2.03 mmol) was dissolved in pyridine (3 mL). The solution was cooled to 0 °C, and p-toluenesulfonyl chloride (466 mg, 2.44 mmol) was added. In a nitrogen atmosphere, the mixture was stirred at 0 °C, slowly warmed to room temperature, and left to react for 4 h. The reaction mixture was diluted with ethyl acetate (200 mL), washed with hydrochloric acid (10%, 150 mL × 3 mL), water (150 mL × 3), and saturated sodium chloride solution (250 mL × 2) in sequence, dried over anhydrous sodium sulfate for 30 min, and filtered. The filtrate was concentrated under reduced pressure, and the resulting residue was purified by silica gel column chromatography with eluent system C to give the title product **OLS-5c** (509 mg, yield: 72.9%).

¹H NMR (400 MHz, CDCl3) δ 7.80 (d, 2H), 7.34 (d, 2H), 4.15 (t, 2H), 3.63-3.59 (m, 4H), 3.53-3.49 (m, 4H), 3.35 (t, 2H), 2.45 (s, 3H), 1.82-1.76 (m, 2H).

### Step 4

### N-((2R,3R,4R,5S,6R)-5-(((2R,4aR,6S,7S,8S,8aR)-8-(2-(3-(2-Azidoethoxy)propoxy)etho xy)-7-(benzyloxy)-2-phenylhexahydropyrano[3,2-d][1,3]dioxin-6-yl)oxy)-2,4-bis(benzy loxy)-6-((benzyloxy)methyl)tetrahydro-2H-pyran-3-yl)acetamide OLS-5d

**OLS-3c** (437 mg, 0.53 mmol), **OLS-5c** (451 mg, 1.31 mmol), and *N*,*N*-dimethylformamide (9.0 mL) were sequentially added to a reaction flask. The mixture was cooled in an ice-water bath, and sodium hydride (106 mg, 2.65 mmol, content 60%) was added. The mixture was left to react at 0-10 °C for about 0.5 h and then at 20-30 °C for about 2.5 h. The reaction mixture was poured into a mixed solution of saturated ammonium chloride solution (10 mL) and water (10 mL), and extraction was performed with ethyl acetate (15 mL × 3). The organic phases were combined, washed with water (15 mL) and saturated sodium chloride solution (15 mL) in sequence, dried over anhydrous sodium sulfate, and filtered. The filtrate was concentrated under reduced pressure, and the resulting residue was purified by thin-layer chromatography with developing solvent system C to give the title product **OLS-5d** (358 mg, yield: 67.9%).

MS m/z (ESI): 1003.7 [M+1].

¹H NMR (400 MHz, CDCl3) δ 7.40-7.16 (m, 25H), 5.71 (d, 1H), 5.44 (s, 1H), 4.87 (d, 1H), 4.84-4.78 (m, 3H), 4.70 (d, 1H), 4.57-4.50 (m, 4H), 4.39 (d, 1H), 4.06-4.01 (m, 1H), 3.98-3.84 (m, 3H), 3.76-3.68 (m, 3H), 3.61-3.40 (m, 13H), 3.34-3.31 (m, 1H), 3.22 (t, 2H), 3.11-3.05 (m, 1H), 1.77-1.71 (m, 2H), 1.64 (s, 3H).

### Step 5

### N-((2R,3R,4R,5S,6R)-5-(((2S,3S,4S,5R,6R)-4-(2-(3-(2-Azidoethoxy)propoxy)ethoxy)-3-(benzyloxy)-5-hydroxy-6-(hydroxymethyl)tetrahydro-2H-pyran-2-yl)oxy)-2,4-bis(benz yloxy)-6-((benzyloxy)methyl)tetrahydro-2H-pyran-3-yl)acetamide OLS-5e

**OLS-5d** (342 mg, 0.34 mmol) was dissolved in dichloromethane (20 mL). In a nitrogen atmosphere, the temperature was lowered to -20 °C, and trifluoroacetic acid (3.4 mL) was added dropwise with the temperature controlled at -30 to -20 °C. After the dropwise addition, the mixture was warmed to 0 °C and stirred for about 1.5 h. The reaction mixture was quenched with methanol (4 mL) and poured into saturated sodium bicarbonate solution (20 mL), and the pH was adjusted to 7-8 with solid sodium bicarbonate. The mixture was separated. The aqueous phase was extracted with a mixed solvent of dichloromethane/methanol (v/v = 10:1, 10 mL × 2), and the organic phases were combined. The organic phases were washed with saturated sodium chloride solution (10 mL), dried over anhydrous sodium sulfate, and filtered. The filtrate was concentrated under reduced pressure, and the resulting residue was purified by thin-layer chromatography with developing solvent system D to give the title product **OLS-5e** (193 mg, yield: 61.9%).

MS m/z (ESI): 915.7 [M+1].

¹H NMR (400 MHz, CDCl3) δ 7.31-7.17 (m, 20H), 5.65 (d, 1H), 4.87-4.79 (m, 3H), 4.70 (s, 2H), 4.58-4.50 (m, 3H), 4.44-4.39 (m, 2H), 4.04 (t, 1H), 3.82 (t, 1H), 3.74-3.63 (m, 10H), 3.53-3.45 (m, 10H), 3.26 (t, 2H), 3.11-3.05 (m, 1H), 3.00-2.98 (m, 1H), 1.81-1.75 (m, 2H), 1.66 (s, 3H).

### Step 6

### N-((3R,4R,5S,6R)-5-(((2S,3S,4S,5R,6R)-4-(2-(3-(2-Azidoethoxy)propoxy)ethoxy)-3,5-di hydroxy-6-(hydroxymethyl)tetrahydro-2H-pyran-2-yl)oxy)-2,4-dihydroxy-6-(hydroxym ethyl)tetrahydro-2H-pyran-3-yl)acetamide OLS-5f (a mixture of diastereomers)

**OLS-5e** (190 mg, 207.64 µmol) was dissolved in tetrahydrofuran (8 mL) and water (2 mL), and palladium hydroxide (66 mg, content 15%), palladium on carbon (77 mg, content 10%), and hydrochloric acid (1 drop) were added. The reaction system was purged with hydrogen gas three times and stirred at room temperature for 12 h. The catalyst was removed by filtration. The filtrate was concentrated under reduced pressure, and the resulting residue was dissolved in methanol (8 mL). Imidazole-1-sulfonyl azide hydrochloride (56.7 mg, 270.50 µmol), potassium carbonate (71 mg, 510.11 µmol), and copper sulfate (9.3 mg, 37.25 µmol) were added, and the mixture was stirred at room temperature for 12 h. The inorganic salts were removed by filtration, and the solids were rinsed with methanol (3 mL). The filtrate was concentrated under reduced pressure to give a crude product of the title product **OLS-5f** (194 mg). The product was directly used in the next step without purification.

MS m/z (ESI): 555.4 [M+1].

### Step 7

### (2S,3S,4S,5R,6R)-4-(2-(3-(2-Azidoethoxy)propoxy)ethoxy)-2-(((3aR,5R,6S,7R,7aR)-7-h ydroxy-5-(hydroxymethyl)-2-methyl-3a,6,7,7a-tetrahydro-5H-pyrano[3,2-d]oxazol-6-yl )oxy)-6-(hydroxymethyl)tetrahydro-2H-pyran-3,5-diol OLS-5

The crude product of **OLS-5f** (100 mg, 90.16 µmol) was dissolved in purified water (6 mL) under ice-bath conditions, and 2-chloro-1,3-dimethyl-1*H*-benzimidazol-3-ium chloride (344 mg, 1.89 mmol) and cesium carbonate (1.76 g, 5.40 mmol) were added. The mixture was stirred at 0 °C for 12 h to give reaction mixture 1.

The crude product of **OLS-5f** (100 mg, 90.16 µmol) was dissolved in purified water (6 mL) under ice-bath conditions, and 2-chloro-1,3-dimethyl-1*H*-benzimidazol-3-ium chloride (339 mg, 1.87 mmol) and cesium carbonate (1.75 g, 5.39 mmol) were added. The mixture was stirred at 0 °C for 12 h to give reaction mixture 2.

Reaction mixture 1 and reaction mixture 2 were combined, purified by high performance liquid chromatography (column: Phenomenex C18, 150 × 25 mm, 10 µm; mobile phases: water (0.01% ammonia water) and acetonitrile, gradient ratio: acetonitrile 0%-30%, flow rate: 25 mL/min), and lyophilized in a 5 mol% sodium hydroxide solution to give the title product **OLS-5** (22 mg, yield over steps 6 and 7: 19.8%).

MS m/z (ESI): 537.1 [M+1].

¹H NMR (400 MHz, CDCl3) δ 6.02 (d, 1H), 4.66 (s, 2H), 4.30 (s, 2H), 4.10-3.95 (m, 5H), 3.88-3.78 (m, 4H), 3.68-3.53 (m, 12H), 3.37-3.29 (m, 5H).

### Example 4-6. Synthesis of Glycan Chain OLS-6

### (2R,3S,4S,5S,6S)-2-(14-Azido-2,5,9,12-tetraoxatetradecyl)-6-(((3aR,5R,6S,7R,7aR)-7-h ydroxy-5-(hydroxymethyl)-2-methyl-3a,6,7,7a-tetrahydro-5H-pyrano[3,2-d]oxazol-6-yl )oxy)tetrahydro-2H-pyran-3,4,5-triol OLS-6

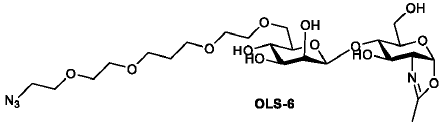

### Example 4-7. Synthesis of Glycan Chain OLS-7

### (2R,3S,4S,5S,6S)-2-((2-(3-(2-Azidoethoxy)propoxy)ethoxy)methyl)-6-(((3aR,5R,6S,7R, 7aR)-7-hydroxy-5-(hydroxymethyl)-2-methyl-3a,6,7,7a-tetrahydro-5H-pyrano[3,2-d]ox azol-6-yl)oxy)tetrahydro-2H-pyran-3,4,5-triol OLS-7

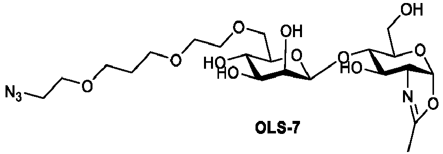

### Example 4-8. Synthesis of Glycan Chain OLS-8

### (2R,3R,4S,5S,6S)-2-(14-Azido-2,5,9,12-tetraoxatetradecyl)-4-(2-(2-(2-azidoethoxy)etho xy)ethoxy)-6-(((3aR,5R,6S,7R,7aR)-7-hydroxy-5-(hydroxymethyl)-2-methyl-3a,6,7,7a-t etrahydro-5H-pyrano[3,2-d]oxazol-6-yl)oxy)tetrahydro-2H-pyran-3,5-diol OLS-8

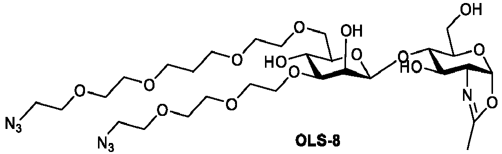

### Example 4-9. Synthesis of Glycan Chain OLS-9

### (2R,3R,4S,5S,6S)-4-(2-(2-(2-Azidoethoxy)ethoxy)ethoxy)-2-((2-(3-(2-azidoethoxy)prop oxy)ethoxy)methyl)-6-(((3aR,5R,6S,7R,7aR)-7-hydroxy-5-(hydroxymethyl)-2-methyl-3 a,6,7,7a-tetrahydro-5H-pyrano[3,2-d]oxazol-6-yl)oxy)tetrahydro-2H-pyran-3,5-diol OLS-9

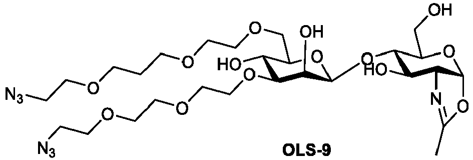

### Example 4-10. Synthesis of Glycan Chain OLS-10

### (2R,3R,4S,5S,6S)-2-((2-(2-(2-Azidoethoxy)ethoxy)ethoxy)methyl)-4-(2-(3-(2-(2-azidoet hoxy)ethoxy)propoxy)ethoxy)-6-(((3aR,5R,6S,7R,7aR)-7-hydroxy-5-(hydroxymethyl)-2 -methyl-3a,6,7,7a-tetrahydro-5H-pyrano[3,2-d]oxazol-6-yl)oxy)tetrahydro-2H-pyran-3, 5-diol OLS-10

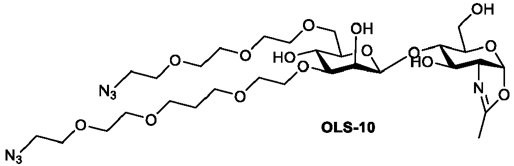

### Example 4-11. Synthesis of Glycan Chain OLS-11

### (2R,3R,4S,5S,6S)-2-((2-(2-(2-Azidoethoxy)ethoxy)ethoxy)methyl)-4-(2-(3-(2-(2-azidoet hoxy)propoxy)ethoxy)-6-(((3aR,5R,6S,7R,7aR)-7-hydroxy-5-(hydroxymethyl)-2-methyl -3a,6,7,7a-tetrahydro-5H-pyrano[3,2-d]oxazol-6-yl)oxy)tetrahydro-2H-pyran-3,5-diol OLS-11

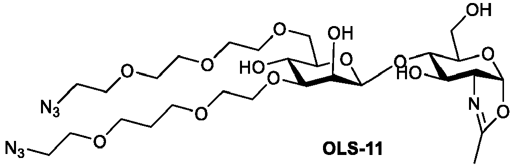

### Example 4-12. Synthesis of Glycan Chain OLS-12

### (2R,3R,4S,5S,6S)-2-(14-Azido-2,5,9,12-tetraoxatetradecyl)-4-(2-(3-(2-(2-azidoethoxy)et hoxy)propoxy)ethoxy)-6-(((3aR,5R,6S,7R,7aR)-7-hydroxy-5-(hydroxymethyl)-2-methyl -3a,6,7,7a-tetrahydro-5H-pyrano[3,2-d]oxazol-6-yl)oxy)tetrahydro-2H-pyran-3,5-diol OLS-12

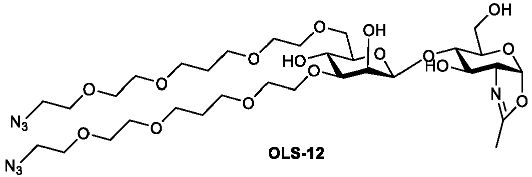

### V ADC Synthesis Examples

### Example 5-1. ADC-1

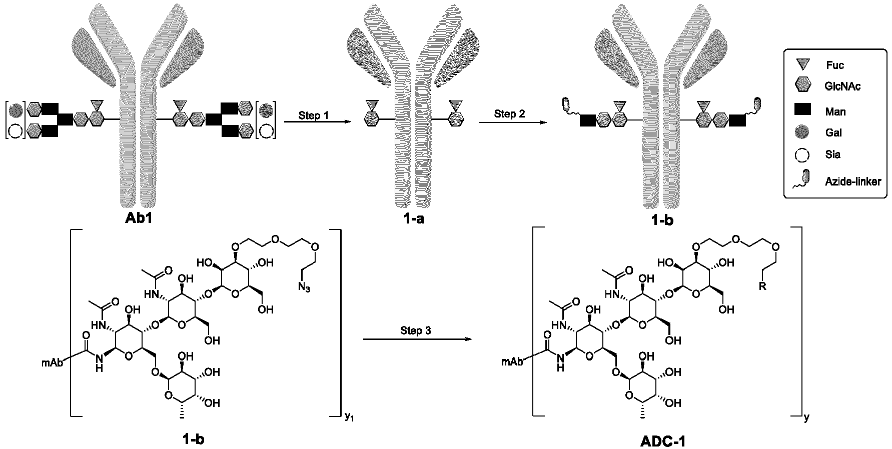

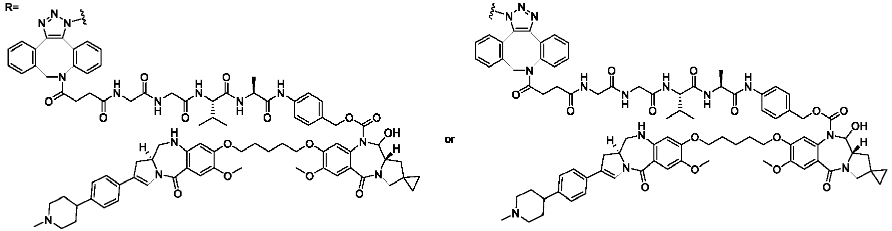

(The triazole ring formed in step 3 has a geometric structure, and the obtained compound comprises the two structures shown above as R.)

### Step 1

Endo S enzyme (19.16 mg/mL, 0.052 mL) was added to antibody **Ab1** in an aqueous PBS buffer solution (a pH 6.5, 0.05 M aqueous PBS buffer solution; 10 mg/mL, 20 mL) at 37 °C. The mixture was shaken on a water bath shaker at 37 °C for 12 h, and the reaction was stopped. The reaction mixture was purified using a Hitrap Protein A HP protein purification column (elution phase: a pH 3.0 aqueous acetic acid buffer solution) to give an affinity eluate of **1-a,** and the eluate was refrigerated at 4 °C.

### Step 2

Endo S enzyme (1 mg/mL, 0.02 mL) and **OLS-3** (0.28 mg) were added to **1-a** in an aqueous PBS buffer solution (a pH 6.5, 0.05 M aqueous PBS buffer solution; 10.0 mg/mL, 0.2 mL) at 37 °C. The mixture was shaken on a water bath shaker at 37 °C for 1 h, and the reaction was stopped. The reaction mixture was purified using a Hitrap Protein A HP protein purification column (elution phase: a pH 3.0 aqueous acetic acid buffer solution) to give an affinity eluate of **1-b,** and the eluate was refrigerated at 4 °C. y¹ represents the average number of glycan chains remodeled to the N297 sites of the heavy chains of the antibody; two glycan chains or one glycan chain may be remodeled at the two N297 sites of the heavy chains of the antibody.

### Step 3

1,2-Propanediol (0.112 mL) was added to **1-b** in an aqueous PBS buffer solution (a pH 7.4 aqueous PBS buffer solution; 8.02 mg/mL, 0.125 mL) at 25 °C. Compound **LD-8** (0.20 mg) was dissolved in 13 µL of dimethyl sulfoxide, and the solution was added to the above reaction mixture. The mixture was shaken on a water bath shaker at 25 °C for 5 h, and the reaction was stopped. The reaction mixture was desalted and purified using a Sephadex G25 gel column (elution phase: a pH 7.4 aqueous PBS buffer solution) to give the title product **ADC-1** in PBS buffer, and the product was refrigerated at 4 °C.

The average number of bound drugs per antibody molecule was calculated by MS: y = 1.88.

An antibody with a similar structure and a similar molecular weight, such as Ab2, Ab3, or Ab4, can be deglycosylated by a step similar to the preparation of **1-a,** and a glycan chain can be remodeled by a second step similar to the preparation of **1-b.** Then conjugation to a drug linker (LD) can be performed by a third step similar to the preparation of **ADC-1** to give an ADC.

### Example 5-2. ADC-2

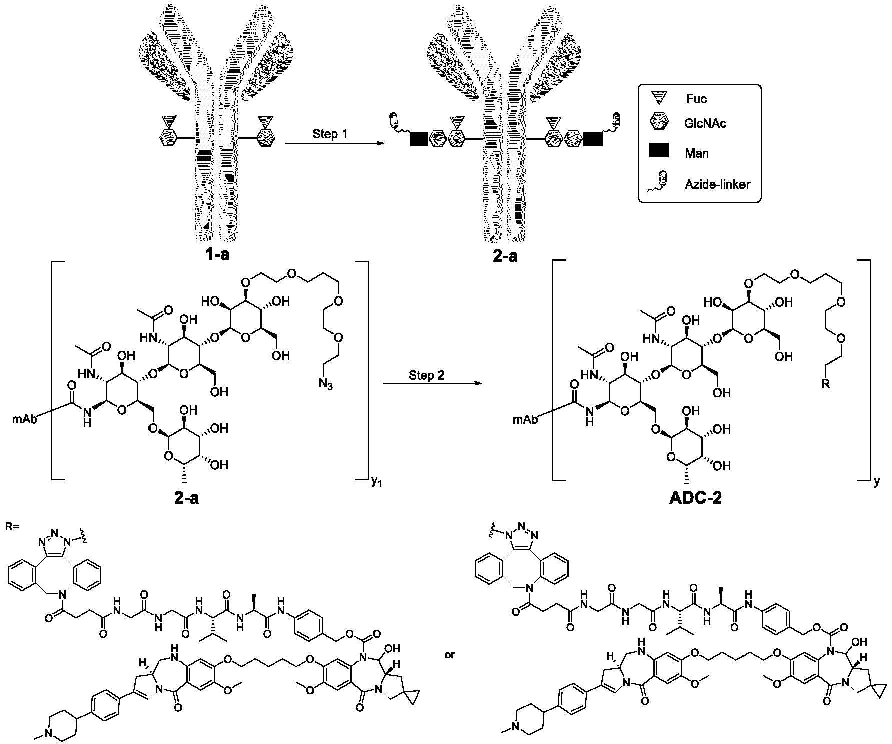

(The triazole ring formed in step 2 has a geometric structure, and the obtained compound comprises the two structures shown above as R.)

### Step 1

Endo S enzyme (1 mg/mL, 0.02 mL) and **OLS-4** (0.31 mg) were added to 1-a in an aqueous PBS buffer solution (a pH 6.5, 0.05 M aqueous PBS buffer solution; 10.0 mg/mL, 0.2 mL) at 37 °C. The mixture was shaken on a water bath shaker at 37 °C for 1 h, and the reaction was stopped. The reaction mixture was purified using a Hitrap Protein A HP protein purification column (elution phase: a pH 3.0 aqueous acetic acid buffer solution) to give an affinity eluate of **2-a,** and the eluate was refrigerated at 4 °C. y¹ represents the average number of glycan chains remodeled to the N297 sites of the heavy chains of the antibody; two glycan chains or one glycan chain may be remodeled at the two N297 sites of the heavy chains of the antibody.

### Step 2

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-8** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-2** in PBS buffer, and the product was refrigerated at 4 °C.

The average number of bound drugs per antibody molecule was calculated by MS: y = 1.98.

### Example 5-3. ADC-3

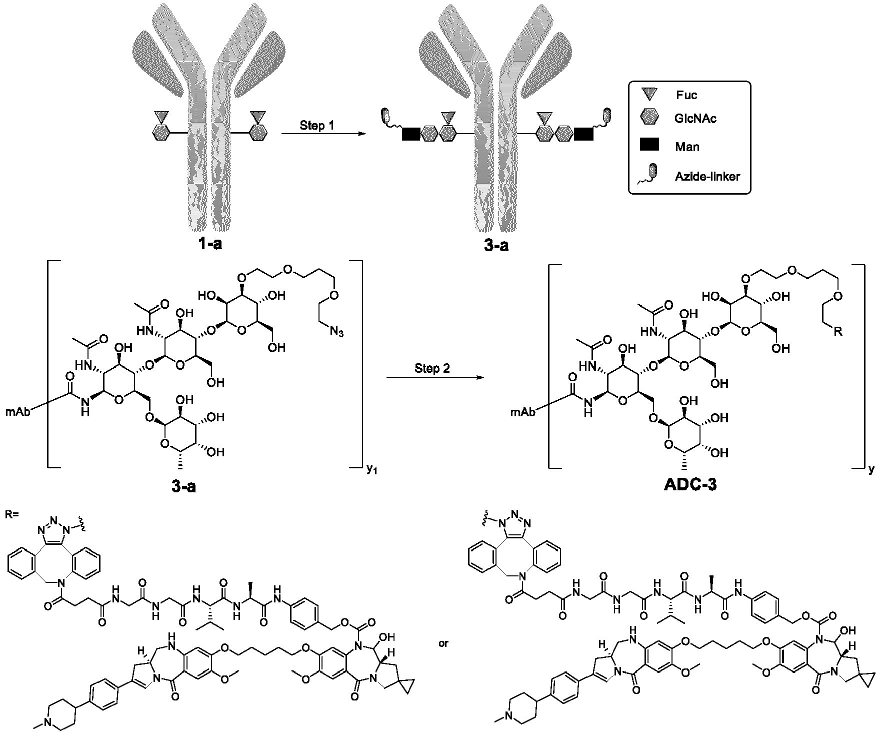

(The triazole ring formed in step 2 has a geometric structure, and the obtained compound comprises the two structures shown above as R.)

### Step 1

Endo S enzyme (1 mg/mL, 0.02 mL) and **OLS-5** (0.29 mg) were added to **1-a** in an aqueous PBS buffer solution (a pH 6.5, 0.05 M aqueous PBS buffer solution; 10.0 mg/mL, 0.2 mL) at 37 °C. The mixture was shaken on a water bath shaker at 37 °C for 1 h, and the reaction was stopped. The reaction mixture was purified using a Hitrap Protein A HP protein purification column (elution phase: a pH 3.0 aqueous acetic acid buffer solution) to give an affinity eluate of **3-a,** and the eluate was refrigerated at 4 °C. y¹ represents the average number of glycan chains remodeled to the N297 sites of the heavy chains of the antibody; two glycan chains or one glycan chain may be remodeled at the two N297 sites of the heavy chains of the antibody.

### Step 2

1,2-Propanediol (0.13 mL) was added to **3-a** in an aqueous PBS buffer solution (a pH 7.4 aqueous PBS buffer solution; 6.88 mg/mL, 0.145 mL) at 25 °C. Compound **LD-8** (0.20 mg) was dissolved in 15 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-3** in PBS buffer, and the product was refrigerated at 4 °C.

The average number of bound drugs per antibody molecule was calculated by MS: y = 2.

### Example 5. Remodeling of Antibody and Glycan Chain OLS-1 and ADC Conjugation

The following steps are suitable for preparing ADC-4, ADC-5, ADC-6, ADC-7, ADC-8, and ADC-9:

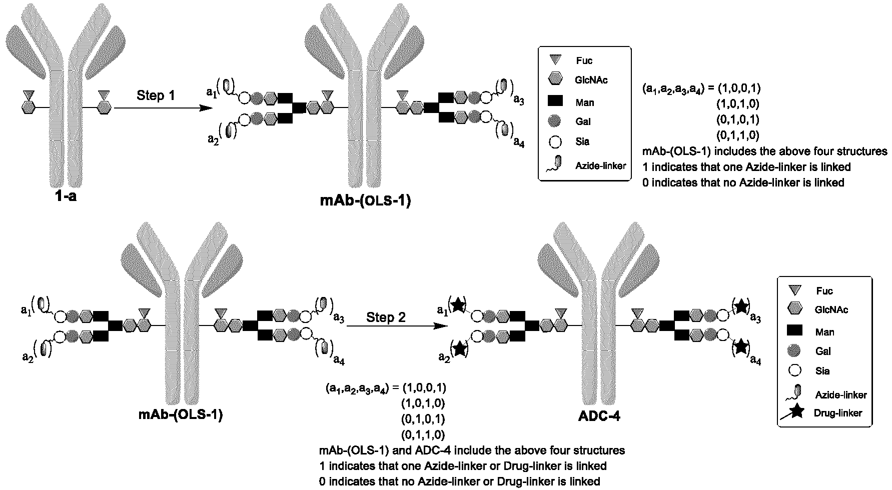

### Step 1. Remodeling of antibody glycan chain mAb-(OLS-1)

Endo F3 (D126A) enzyme (prepared by the method disclosed on page 21 of the patent application US10851174B2) (20 mg/mL, 0.05 mL), an aqueous PBS buffer solution (pH = 7.4, 0.05 mL), and **OLS-1** (1.19 mg) were added to **1-a** in an aqueous PBS buffer solution (a pH 7.4 aqueous PBS buffer solution; 20.0 mg/mL, 0.1 mL) at 37 °C. The mixture was shaken on a water bath shaker at 37 °C for 5 min, and the reaction was stopped. The reaction mixture was purified using a Hitrap Protein A HP protein purification column (elution phase: a pH 3.0 aqueous acetic acid buffer solution) to give an affinity eluate of mAb-(OLS-1), and the eluate was refrigerated at 4 °C.

mAb-(OLS-1) includes the following four structures: mAb-(OLS-1)**a₁**, mAb-(OLS-1)**a₂**, mAb-(OLS-1)**a₃**, and mAb-(OLS-1)**a₄**:

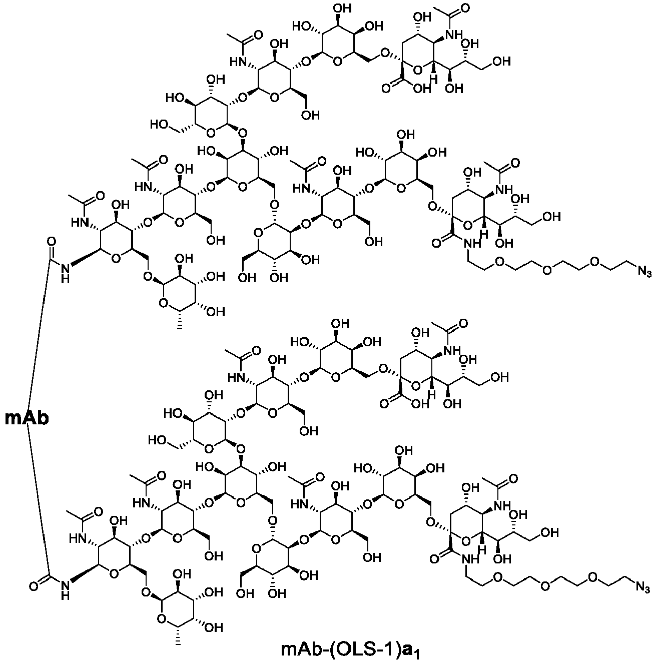

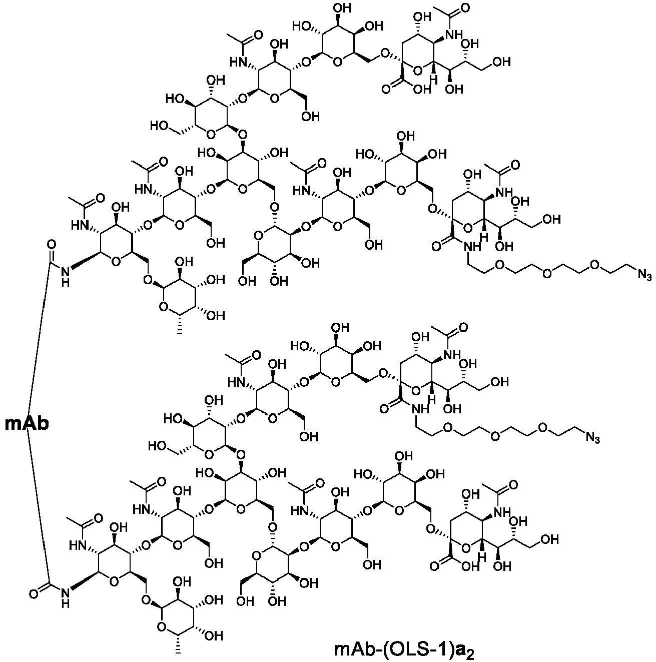

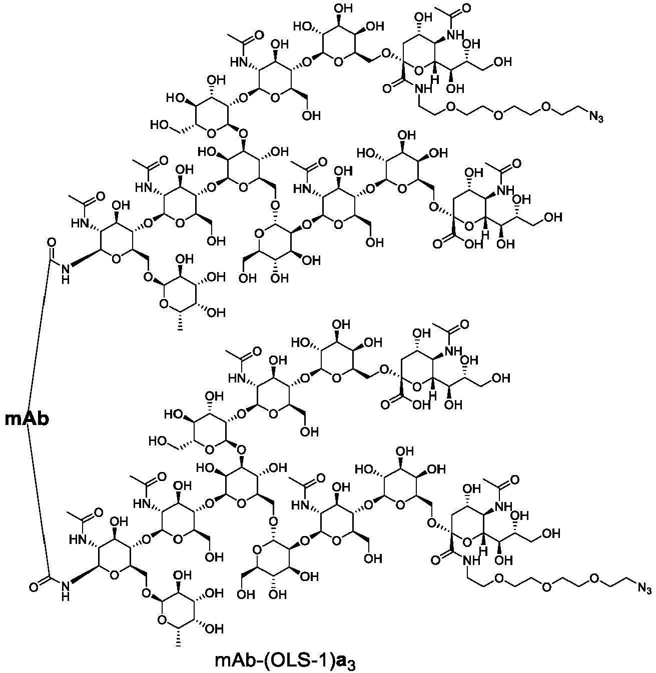

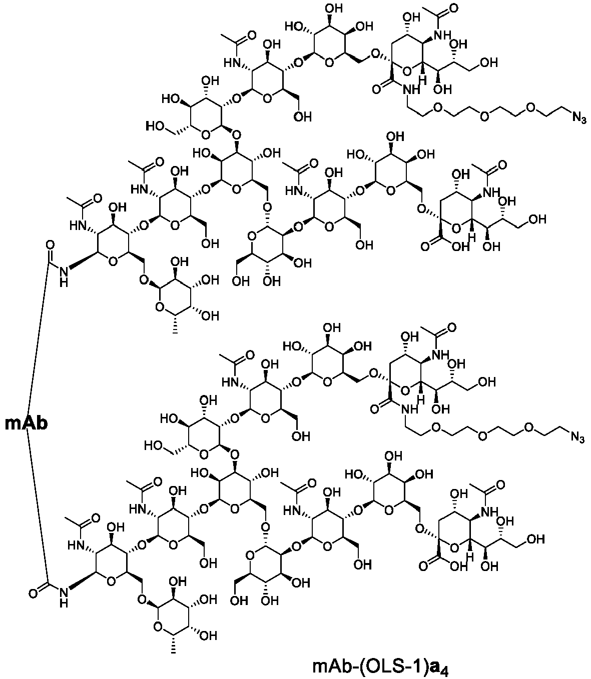

### Step 2. ADC Synthesis

### Preparation of examples ADC-4, ADC-5, ADC-6, ADC-7, ADC-8, and ADC-9:

mAb-OLS-1 was conjugated to different drug linkers to give mAb-(OLS-1)-R.
mAb-(OLS-1)-R includes the following four structures: mAb-(OLS-1)-R**a**,
mAb-(OLS-1)-R**b**, mAb-(OLS-1)-R**c**, and mAb-(OLS-1)-R**d**:

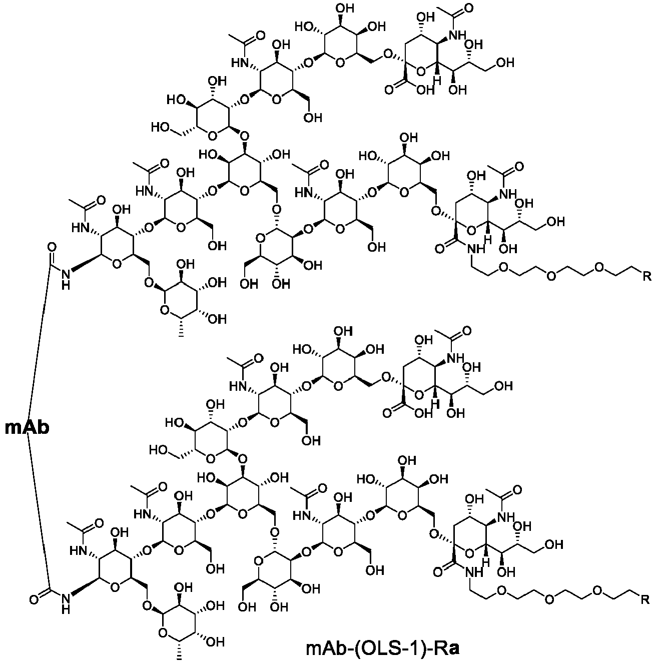

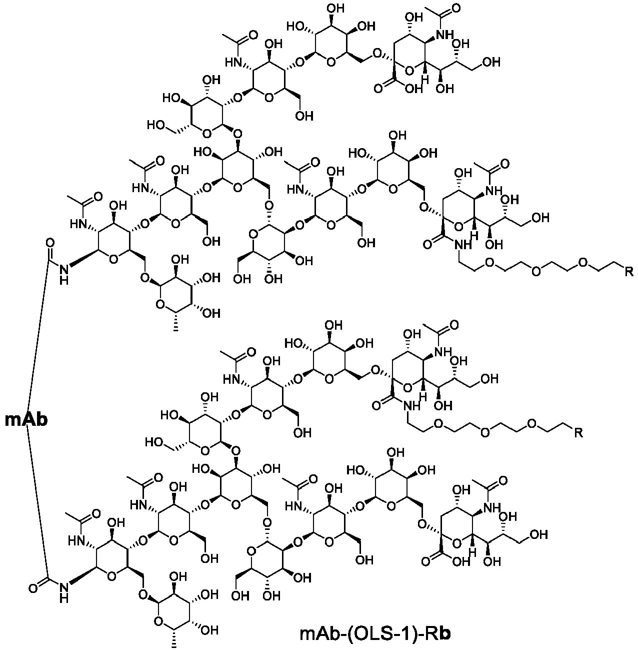

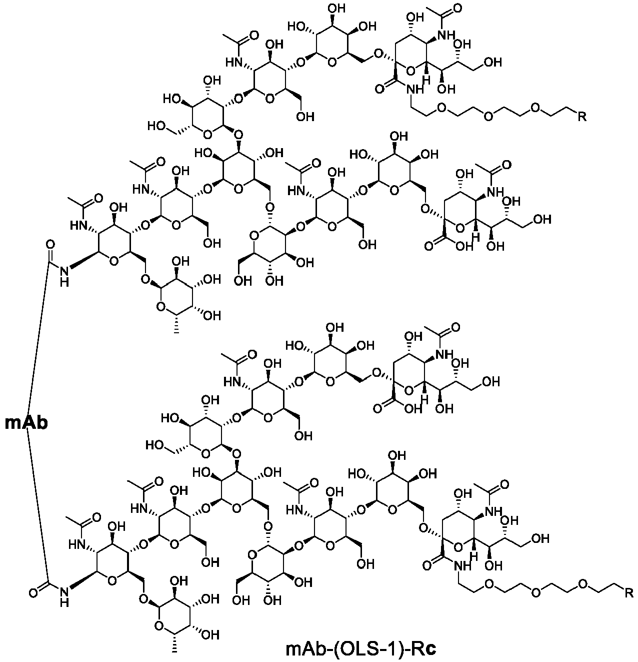

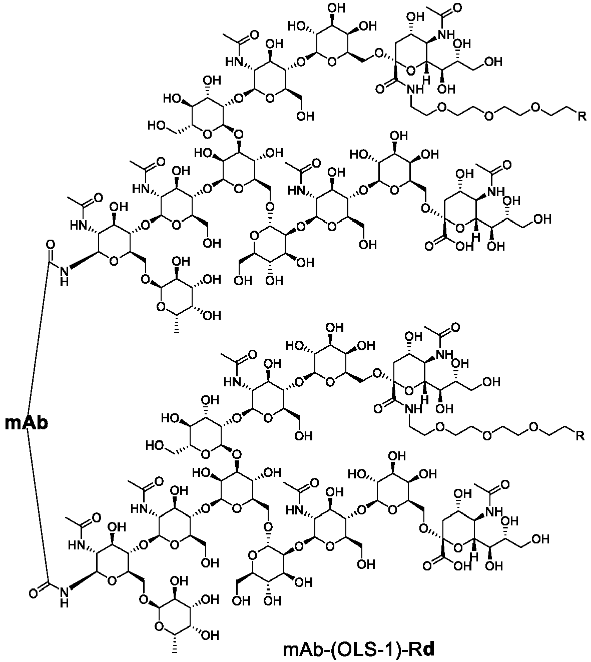

The structural formula for ADC-4, ADC-5, ADC-6, ADC-7, ADC-8, and ADC-9 is represented by mAb-(OLS-1)-R, where the R group varies depending on the drug linkers used in the examples, and meanwhile the triazole ring of the R group has two structures. An antibody with a similar structure and a similar molecular weight, such as Ab2, Ab3, or Ab4, can be deglycosylated by a step similar to the preparation of **1-a,** and a glycan chain can be remodeled by a step similar to the preparation of mAb-(OLS-1). Then conjugation to a drug linker (LD) can be performed by a step similar to the preparation of mAb-(OLS-1)-R.

### Example 5-4. ADC-4

R is selected from the group consisting of:

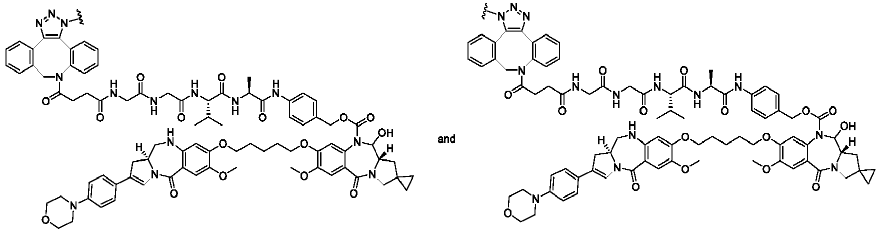

1,2-Propanediol (0.125 mL) was added to mAb-(OLS-1) in an aqueous PBS buffer solution (a pH 7.4 aqueous PBS buffer solution; 7.2 mg/mL, 0.139 mL) at 25 °C. Compound **LD-2** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-4** in PBS buffer, and the product was refrigerated at 4 °C.

The average number of bound drugs per antibody molecule (DAR) was calculated by MS: y = 1.76.

### Example 5-5. ADC-5

R is selected from the group consisting of:

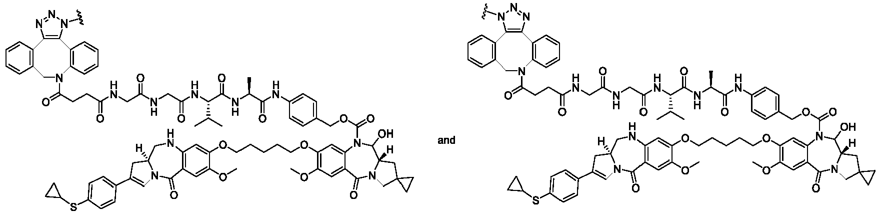

(The triazole ring formed has a geometric structure, and the obtained compound comprises the two structures shown above as R.)

1,2-Propanediol (0.125 mL) was added to mAb-(OLS-1) in an aqueous PBS buffer solution (a pH 7.4 aqueous PBS buffer solution; 7.2 mg/mL, 0.139 mL) at 25 °C. Compound **LD-3** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-5** in PBS buffer, and the product was refrigerated at 4 °C.

The average number of bound drugs per antibody molecule (DAR) was calculated by MS: y = 1.37.

### Example 5-6. ADC-6

R is selected from the group consisting of:

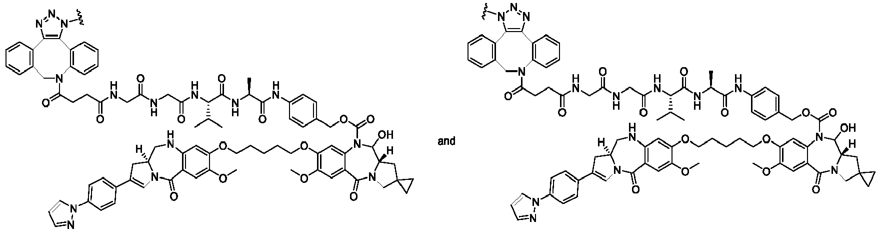

(The triazole ring formed has a geometric structure, and the obtained compound comprises the two structures shown above as R.)

1,2-Propanediol (0.125 mL) was added to mAb-(OLS-1) in an aqueous PBS buffer solution (a pH 7.4 aqueous PBS buffer solution; 7.2 mg/mL, 0.139 mL) at 25 °C. Compound **LD-4** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-6** in PBS buffer, and the product was refrigerated at 4 °C.

The average number of bound drugs per antibody molecule (DAR) was calculated by MS: y = 1.89.

### Example 5-7. ADC-7

R is selected from the group consisting of:

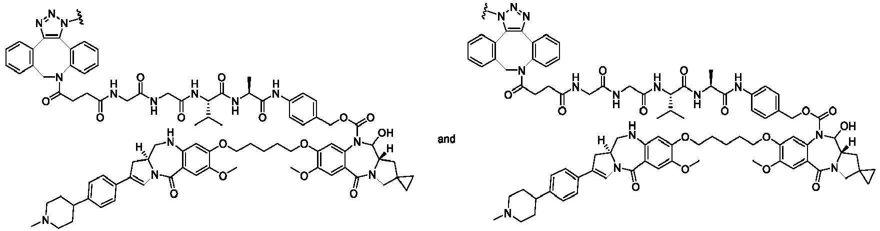

(The triazole ring formed has a geometric structure, and the obtained compound comprises the two structures shown above as R.)

1,2-Propanediol (0.125 mL) was added to mAb-(OLS-1) in an aqueous PBS buffer solution (a pH 7.4 aqueous PBS buffer solution; 7.2 mg/mL, 0.139 mL) at 25 °C. Compound **LD-8** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-7** in PBS buffer, and the product was refrigerated at 4 °C.

The average number of bound drugs per antibody molecule (DAR) was calculated by MS: y = 1.80.

### Example 5-8. ADC-8

R is selected from the group consisting of:

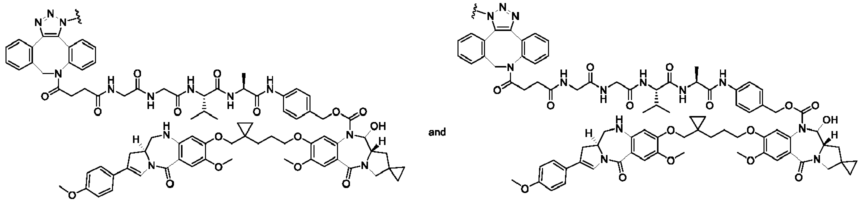

(The triazole ring formed has a geometric structure, and the obtained compound comprises the two structures shown above as R.)

1,2-Propanediol (0.125 mL) was added to mAb-(OLS-1) in an aqueous PBS buffer solution (a pH 7.4 aqueous PBS buffer solution; 7.2 mg/mL, 0.139 mL) at 25 °C. Compound **LD-9** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-8** in PBS buffer, and the product was refrigerated at 4 °C.

The average number of bound drugs per antibody molecule (DAR) was calculated by MS: y = 1.80.

### Example 5-9. ADC-9

R is selected from the group consisting of:

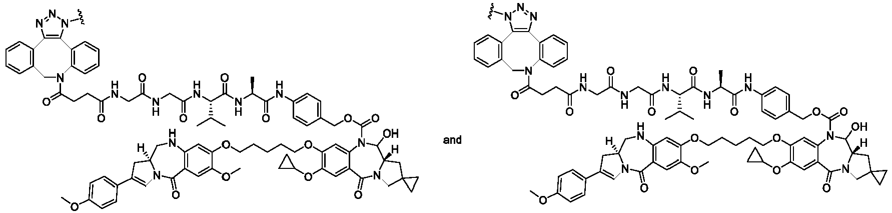

(The triazole ring formed has a geometric structure, and the obtained compound comprises the two structures shown above as R.)

1,2-Propanediol (0.125 mL) was added to mAb-(OLS-1) in an aqueous PBS buffer solution (a pH 7.4 aqueous PBS buffer solution; 7.2 mg/mL, 0.139 mL) at 25 °C. Compound **LD-11** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-9** in PBS buffer, and the product was refrigerated at 4 °C.

The average number of bound drugs per antibody molecule (DAR) was calculated by MS: y = 1.31.

### Example 5-10. ADC-10

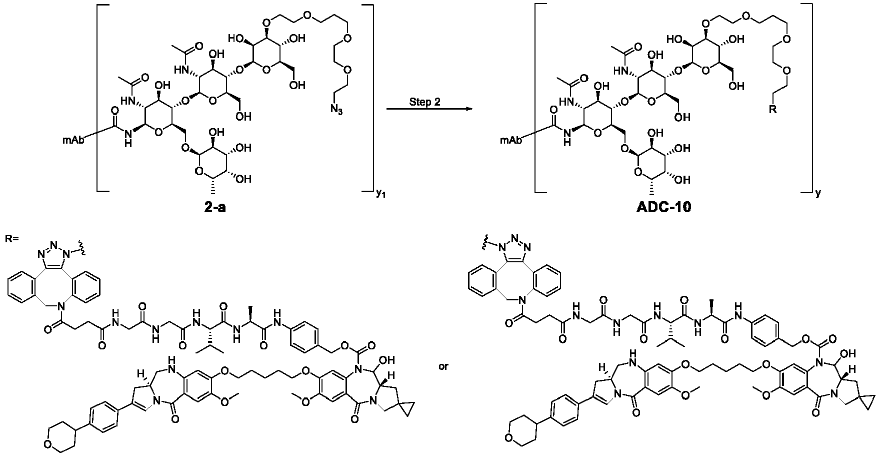

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-5** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-10** in PBS buffer, and the product was refrigerated at 4 °C.

The average number of bound drugs per antibody molecule was calculated by MS: y = 1.99.

### Example 5-11. ADC-11

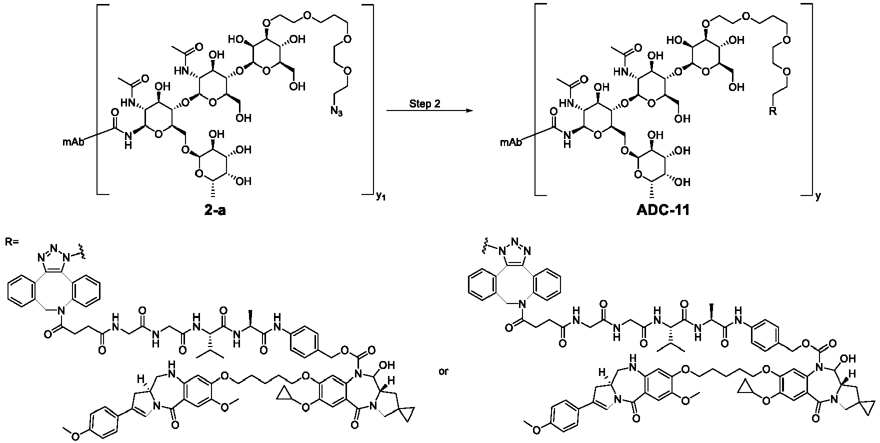

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-11** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-11** in PBS buffer, and the product was refrigerated at 4 °C.

The average number of bound drugs per antibody molecule was calculated by MS: y = 1.93.

### Example 5-12. ADC-12

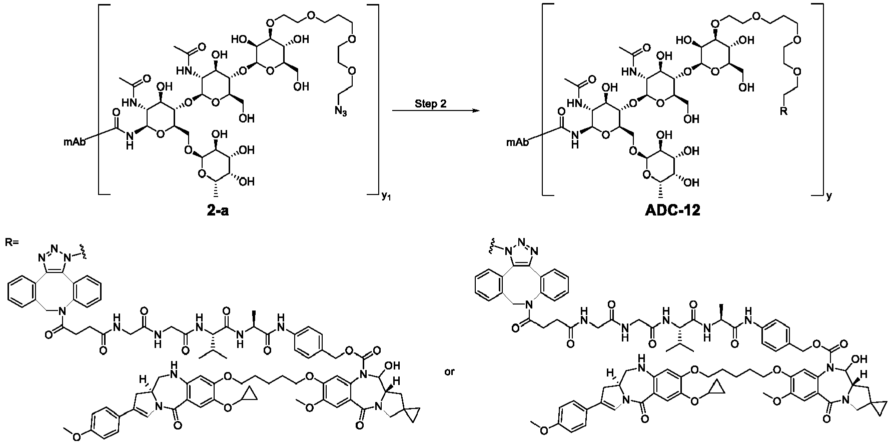

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-16** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-12** in PBS buffer, and the product was refrigerated at 4 °C.

The average number of bound drugs per antibody molecule was calculated by MS: y = 1.99.

### Example 5-13. ADC-13

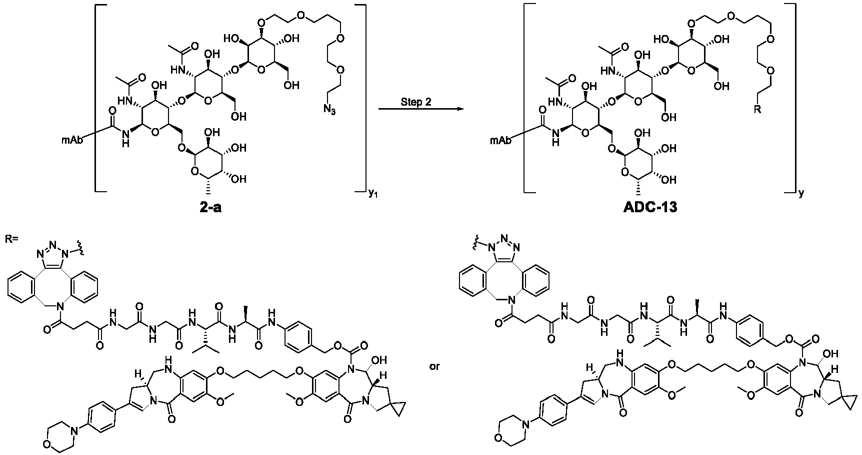

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-2** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-13** in PBS buffer, and the product was refrigerated at 4 °C.

The average number of bound drugs per antibody molecule was calculated by MS: y = 1.99.

### Example 5-14. ADC-14

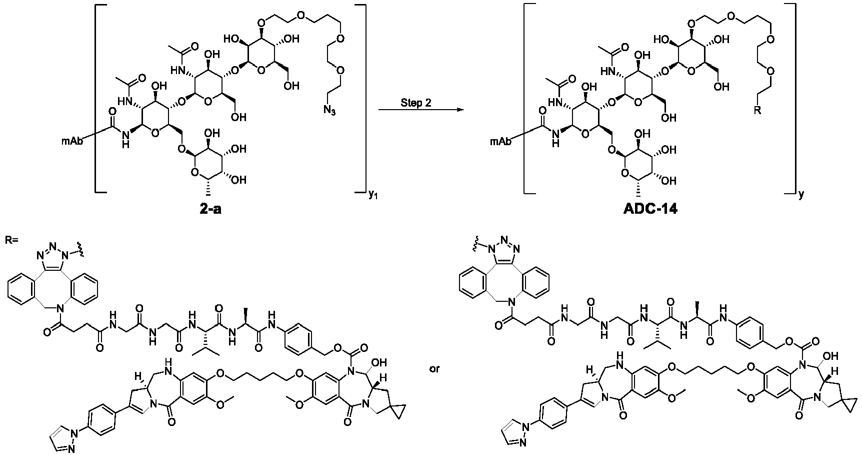

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-4** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-14** in PBS buffer, and the product was refrigerated at 4 °C.

The average number of bound drugs per antibody molecule was calculated by MS: y = 2.0.

### Example 5-15. ADC-15

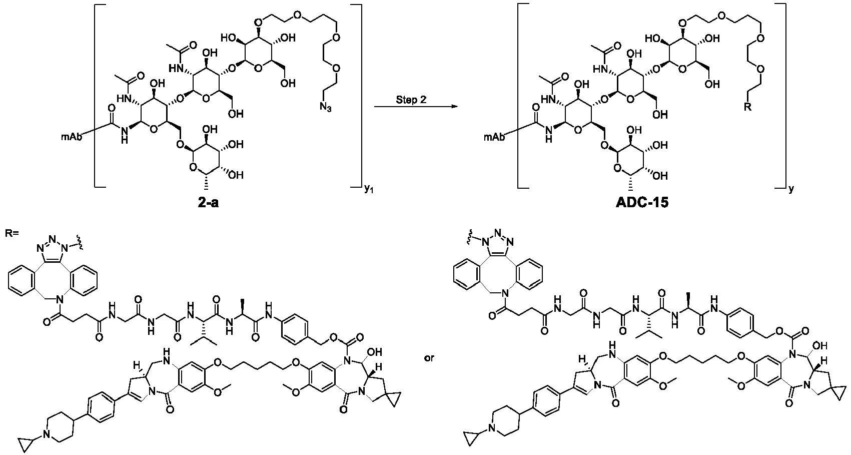

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-17** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-15** in PBS buffer, and the product was refrigerated at 4 °C.

The average number of bound drugs per antibody molecule was calculated by MS: y = 1.48.

### Example 5-16. ADC-16

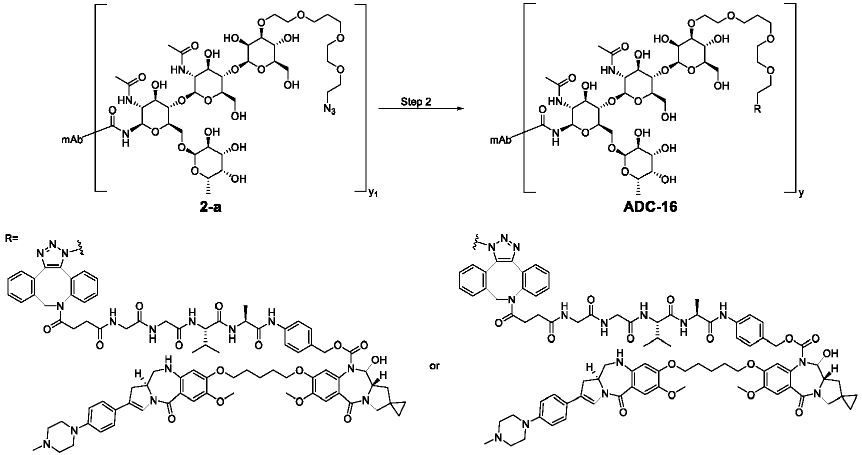

(The triazole ring formed has a geometric structure, and the obtained compound comprises the two structures shown above as R.)

1,2-Propanediol (0.12 mL) was added to **2-a** in an aqueous PBS buffer solution (a pH 7.4 aqueous PBS buffer solution; 7.44 mg/mL, 0.134 mL) at 25 °C. Compound **LD-1** (0.20 mg) was dissolved in 14 µL of dimethyl sulfoxide, and the solution was added to the above reaction mixture. The mixture was shaken on a water bath shaker at 25 °C for 12 h, and the reaction was stopped. The reaction mixture was desalted and purified using a Sephadex G25 gel column (elution phase: a pH 7.4 aqueous PBS buffer solution) to give the title product **ADC-16** in PBS buffer, and the product was refrigerated at 4 °C.

The average number of bound drugs per antibody molecule was calculated by MS: y = 1.97.

### Example 5-18. ADC-18

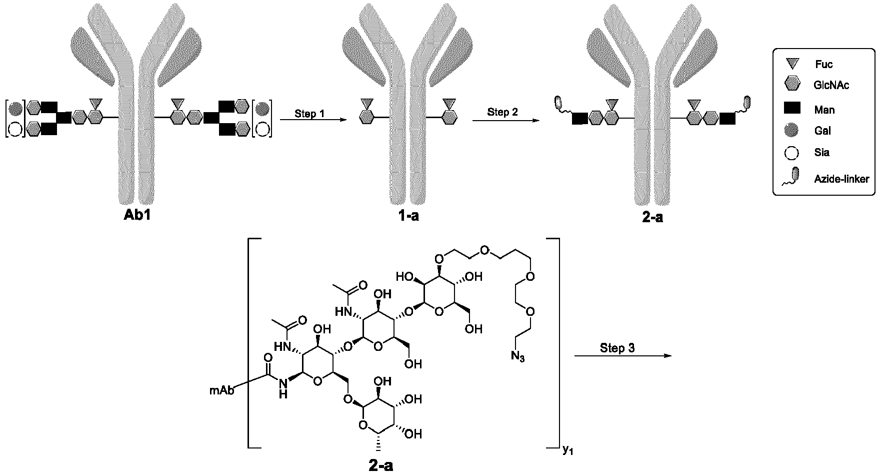

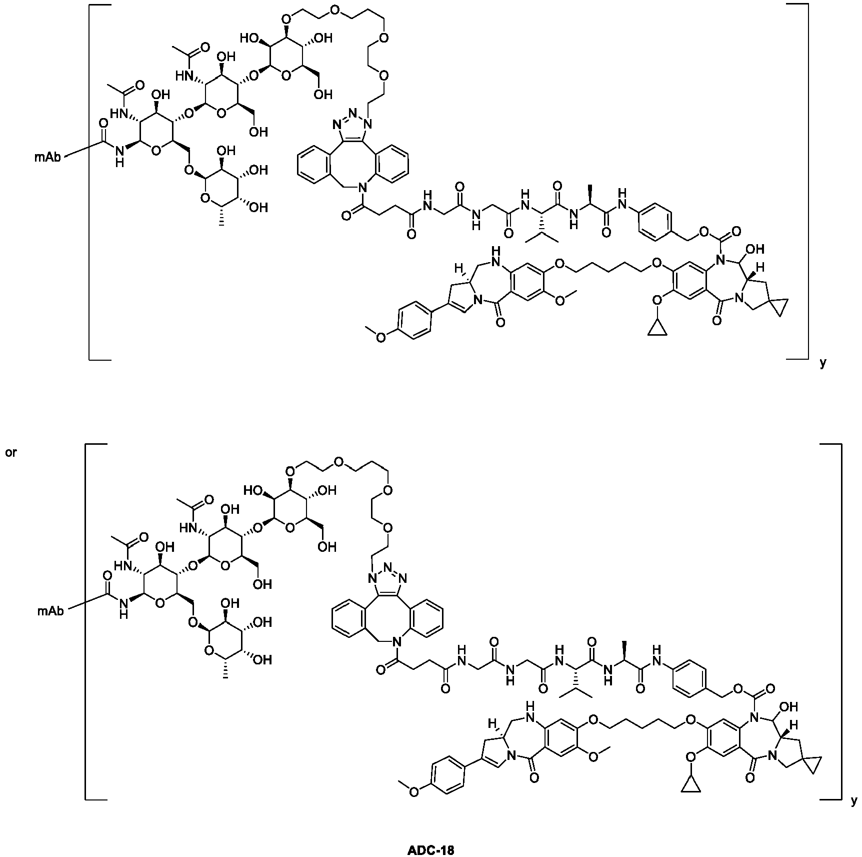

### Step 1

Endo S(WT) (2.74 mg/mL, 13 µL) was added to antibody **Ab1** in an aqueous PBS buffer solution (10.0 mg/mL, 0.35 mL, 23.6 nmol, a pH 6.5, 0.05 M aqueous PBS buffer solution) at 37 °C. After the addition, the mixture was placed on a 37 °C shaker. After 3 h, the reaction was stopped, and a crude product **1-a** was obtained.

### Step 2

**OLS-4** (0.41 mg, 709 nmol) was added to the reaction mixture of the crude product **1-a.** After the addition, the mixture was placed on a 37 °C shaker. After 1 h, the reaction was stopped. The reaction mixture was subjected to centrifugal buffer exchange several times using PBS 7.4 buffer solution to remove excess azide until the final volume was 410 µL, and a crude product **2-a** was obtained.

### Step 3

269 µL of 1,2-propanediol was added to the treated reaction mixture of the crude product **2-a.** After the mixture was well mixed, a solution **of LD-11** (0.21 mg, 142 nmol, dissolved in 41 µL of dimethyl sulfoxide and 100 µL of 1,2-propanediol) was added. The mixture was left to react on a shaker at room temperature for 12 h. The reaction mixture was centrifuged, and the supernatant was diluted with 1 mL of PBS 7.4 buffer solution and then purified using a Hitrap Protein A HP protein purification column (elution phase: a pH 3.0 aqueous acetic acid buffer solution). The pH of the resulting solution was adjusted to about 5.0 with a Tris hydrochloride solution (1 M, pH = 8.0) to give an affinity eluate of the title product **ADC-18,** and the eluate was refrigerated at 4 °C.

The average value was calculated by MS: y = 1.95.

### Example 5-20. ADC-20

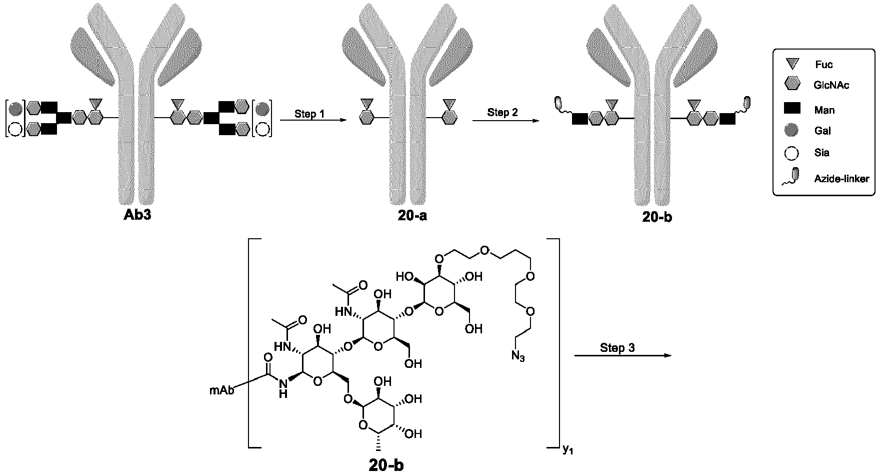

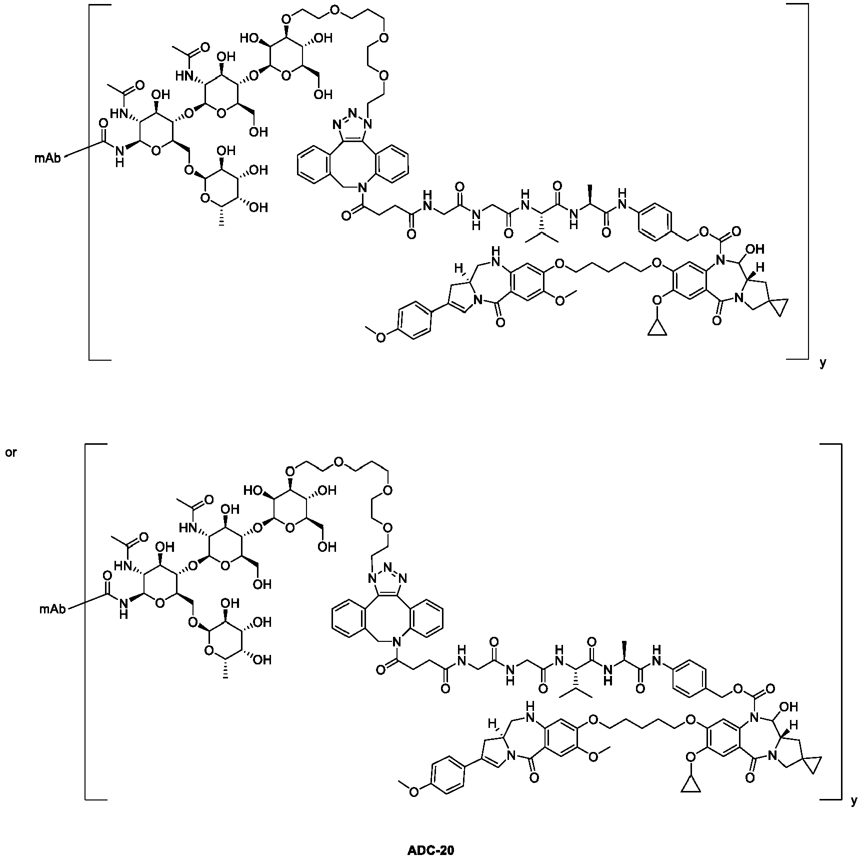

### Step 1

Endo S(WT) (Endo S(WT) 2.74 mg/mL, 13 µL) was added to antibody **Ab3** in an aqueous PBS buffer solution (10.0 mg/mL, 0.35 mL, 24.1 nmol, a pH 6.5, 0.05 M aqueous PBS buffer solution) at 37 °C. After the addition, the mixture was placed on a 37 °C shaker. After 3 h, the reaction was stopped, and a crude product **20-a** was obtained.

### Step 2

**OLS-4** (0.42 mg, 724 nmol) was added to the reaction mixture of the crude product **20-a.** After the addition, the mixture was placed on a 37 °C shaker. After 1 h, the reaction was stopped. The reaction mixture was subjected to centrifugal buffer exchange several times using PBS 7.4 buffer solution to remove excess azide until the final volume was 410 µL, and a crude product **20-b** was obtained.

### Step 3

269 µL of 1,2-propanediol was added to the treated reaction mixture of the crude product **20-b**. After the mixture was well mixed, a solution of **LD-11** (0.21 mg, 145 nmol, dissolved in 41 µL of dimethyl sulfoxide and 100 µL of 1,2-propanediol) was added. The mixture was left to react on a shaker at room temperature for 12 h. The reaction mixture was centrifuged, and the supernatant was diluted with 1 mL of PBS 7.4 buffer solution and then purified using a Hitrap Protein A HP protein purification column (elution phase: a pH 3.0 aqueous acetic acid buffer solution). The pH value of the resulting solution was adjusted to about 5.0 with a Tris hydrochloride solution (1 M, pH = 8.0) to give an affinity eluate of the title product **ADC-20,** and the eluate was refrigerated at 4 °C.

The average value was calculated by MS: y = 1.98.

### Example 5-22. ADC-22

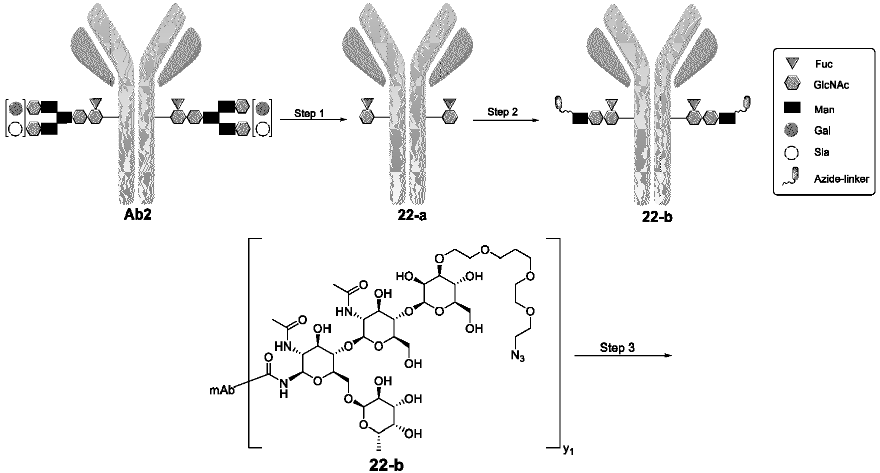

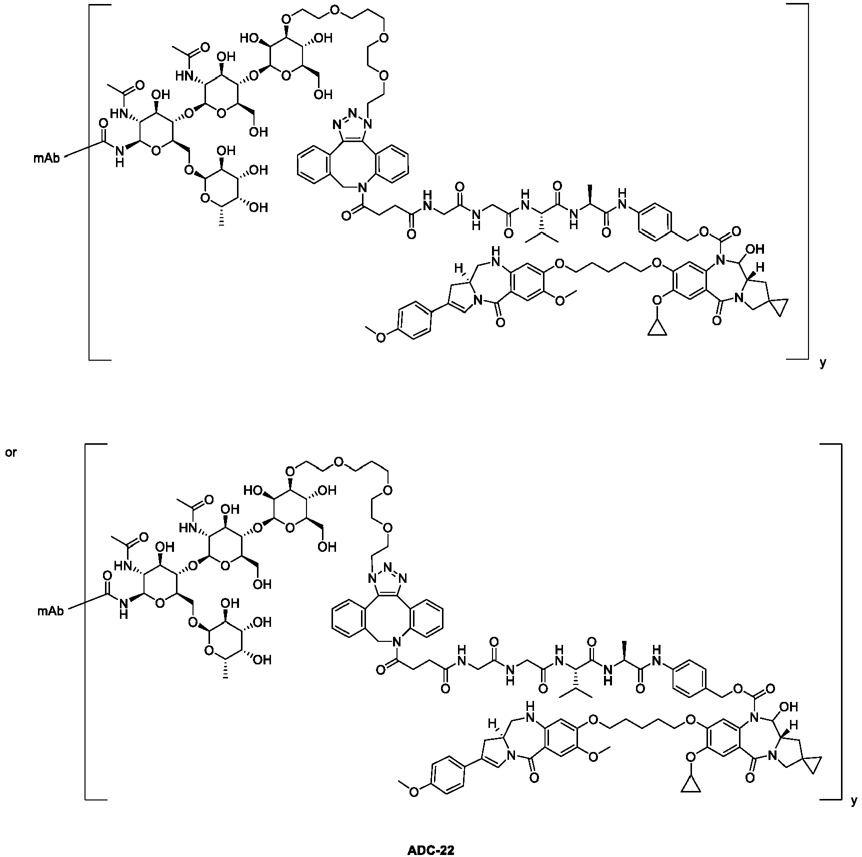

Endo S(WT) (1.16 mg/mL, 135 µL) was added to antibody **Ab2** in an aqueous PBS buffer solution (10.0 mg/mL, 1.57 mL, 108 nmol, a pH 6.5, 0.05 M aqueous PBS buffer solution) at 37 °C. After the addition, the mixture was placed on a 37 °C shaker. After 3 h, the reaction was stopped, and a crude product **22-a** was obtained.

**OLS-4** (1.88 mg, 3.25 µmol) was added to the reaction mixture of the crude product **22-a.** After the addition, the mixture was placed on a 37 °C shaker. After 1 h, the reaction was stopped. The reaction mixture was subjected to centrifugal buffer exchange several times using PBS 7.4 buffer solution to remove excess azide until the final volume was 1.8 mL, and a crude product **22-b** was obtained.

1.42 mL of 1,2-propanediol was added to the treated reaction mixture of the crude product **22-b**. After the mixture was well mixed, a solution of **LD-11** (0.93 mg, 650 nmol, dissolved in 180 µL of dimethyl sulfoxide and 200 µL of 1,2-propanediol) was added. The mixture was left to react on a shaker at room temperature for 12 h. The reaction mixture was centrifuged, and the supernatant was diluted with 4 mL of PBS 7.4 buffer solution and then purified using a Hitrap Protein A HP protein purification column (elution phase: a pH 3.0 aqueous acetic acid buffer solution). The pH of the resulting solution was adjusted to about 5.0 with a Tris hydrochloride solution (1 M, pH = 8.0) to give an affinity eluate of the title product **ADC-22,** and the eluate was refrigerated at 4 °C.

The average value was calculated by MS: y = 1.96.

### Biological Evaluation

### Test Example 1. Inhibition of In Vitro Proliferation of Tumor Cells by PBD Compounds

### I. Objective

The objective of this assay is to measure the activity of the compounds of the present disclosure in inhibiting the proliferation of different tumor cell lines: SK-BR-3 tumor cells (human breast cancer cells, ATCC, Cat. No. HTB-30), NCI-H23 (human non-small cell lung cancer cells, Nanjing Cobioer, Cat. No. CBP60132), and MKN45 (human gastric cancer cells, Nanjing Cobioer, Cat. No. CBP60488). The cells were treated *in vitro* with different concentrations of the compounds. After 6 days of culture, the proliferation of the cells was measured using CTG (CellTiter-Glo^{®} Luminescent Cell Viability Assay, Promega, Cat. No. G7573) reagent, and the *in vitro* activity of the compounds was evaluated based on their IC₅₀ values.

### II. Method

1. Cell culture: SK-BR-3, NCI-H23, and MKN45 cells were separately cultured in McCoy's 5A medium (ThermoFisher, Cat. No. 16600108) and RPMI 1640 medium (ThermoFisher, Cat. No. 11875119) containing 10% FBS (Gibco, 10099-141).
2. Cell plating: After being digested with trypsin (0.25% Trypsin-EDTA (1×), Life Technologies, Cat. No. 25200-072), the SK-BR-3, NCI-H23, and MKN45 were resuspended in their respective media and counted. The density was adjusted to 1.48 × 10⁴ cells/mL, and the cells were plated onto 96-well plates (corning, Cat. No. 3903) at 135 µL/well, i.e., 2000 cells/well, and cultured at 37 °C for 24 h.
3. Drug formulation: PBD drugs were formulated in round-bottom 96-well plates. Plate 1: In the first column, the concentration was 0.05 mM; in the second column through the fourth column, a 3-fold serial dilution was performed in DMSO; in the fifth column through the sixth column, a 5-fold serial dilution was performed in DMSO; in the seventh column through the ninth column, a 10-fold serial dilution was performed in DMSO; and the tenth column contained DMSO. Plate 2: Corresponding media were added to the first column through the tenth column at 196 µL/well; 4 µL of solution was transferred from each well of the first column through the tenth column of plate 1 to plate 2 and well mixed with the medium; 15 µL of the mixture was added to the cells plated the day before, and the cells were cultured at 37 °C for another 6 days.
4. CTG assay: The cell culture plates were taken out and equilibrated to room temperature. CTG was added at 75 µL/well, and the plates were incubated at room temperature in the dark for 10 min. Then luminescence values were measured on a microplate reader (BMG labtech, PHERAstar FS).

### III. Data analysis

Data were processed and analyzed using Graphpad Prism 5, and the results are shown in Table 1 below.

**Table 1. The IC₅₀ values (nM) for the inhibition of the proliferation of the three tumor cell lines by the compounds of the present disclosure**

| Example No. | SK-BR-3 | NCI-H23 | MKN45 |
|---|---|---|---|
| 2-1 | 0.07 | 0.02 | 0.02 |
| 2-2 | 0.05 | 0.02 | 0.09 |
| 2-3 | 0.20 | 0.05 | 0.49 |
| 2-4 | 0.71 | 0.34 | 1.45 |
| 2-5 | 1.84 | 0.41 | 0.91 |
| 2-6 | 0.24 | 0.03 | 0.06 |
| 2-7 | 0.08 | 0.04 | 0.07 |
| 2-9 | 1.25 | 0.23 | 0.19 |
| 2-10 | 0.63 | 0.2 | 0.61 |
| 2-11 | 0.32 | 0.11 | 0.69 |
| 2-13 | 1.64 | 1.42 | 4.35 |
| 2-16 | 0.36 | 0.18 | 0.26 |
| 2-21 | 0.28 | 0.12 | 0.57 |
| 2-22 | 0.26 | 0.08 | 0.17 |
| 2-26 | 2.12 | 2.98 | 2.42 |
| 2-27 | 5 | 7.28 | 7.51 |
| 2-29 | 0.08 | 0.02 | 0.02 |
| 2-30 | 0.21 | 0.02 | 0.04 |
| 2-31 | 0.37 | 0.08 | 0.10 |
| 2-32 | 0.78 | 0.09 | 0.13 |
| 2-33 | 0.73 | 0.30 | 0.38 |
| 2-34 | 1.42 | 0.62 | 1.37 |
| 2-35 | 1.70 | 0.84 | 2.73 |
| 2-39 | 0.58 | 0.39 | 0.84 |

### Test Example 2. In Vitro Efficacy of Caludin18.2-ADCs

### I. Objective:

The *in vitro* killing effects of Claudin18.2-ADCs on the human gastric signet ring cell carcinoma cell strain NUGC4 were measured using CTG (CellTiter-Glo Luminescence Cell Viability Assay^{®}, Promega, G7573).

### II. Method:

1. Cell preparation:
   Human gastric signet ring cell carcinoma cell strain NUGC4 (Nanjing Cobioer, CBP60493);
   A PCDH plasmid containing the full-length sequence of claudin18.2 (SEQ ID NO: 7) was transfected into NUGC4 cells by electroporation. After two weeks of pressure selection, the levels of claudin18.2 expression were measured, and monoclonal cell strains with different expression levels were sorted based on the measured expression levels by flow cytometry. NUGC4/hCLDN18.2 1#, a monoclonal cell strain highly expressing human Claudin18.2, and NUGC4/ hCLDN18.2 11#, a monoclonal cell strain moderately expressing human Claudin18.2, were obtained.
2. On day 1: NUGC4, NUGC4(WT) 11#, and NUGC4/hCLDN18.2 1# cells were harvested, and the density was adjusted to 2.5 × 10⁴/mL; the cells were added to white transparent 96-well plates at 90 µL/well, i.e., about 2500 cells/well, and cultured overnight in a 37 °C, 5% CO₂ incubator.
3. On day 2: Samples were serially diluted 5-fold in 96-well plates from an initial concentration of 400 nM to obtain 9 concentration points, and the diluted samples were added to the cell plates at 10 µL/well; the cells were cultured at 37 °C with 5% CO₂ for 4 days.
4. On day 8: The cell culture plates were taken out, and Cell Titer-Glo Reagent was added at 50 µL/well; the plates were left to stand at room temperature for 2-3 min, and fluorescence values were measured on a PHERAstar FS microplate reader (BMG LABTECH).

### III. Data analysis

Data were analyzed using the software GraphPad Prism.

| ADC | DAR | Cells lowly expressing NUGC4-claudin18.2 | | Cells moderately expressing NUGC4-claudin18.2 | | Cells highly expressing NUGC4-claudin18.2 | |
|---|---|---|---|---|---|---|---|
| | | EC₅₀ (nM) | Emax | EC₅₀ (nM) | Emax | EC₅₀ (nM) | Emax |
| ADC-22 | 1.96 | 11.7 | 88.37 | 9.8 | 97.20 | 2.1 | 94.95 |
| Antibody Ab2 | / | / | -12.83 | / | -17.65 | / | -10.55 |

### IV Conclusion

ADC-22 has good activity in killing the high-expression cell line.

### Test Example 3. In Vitro Efficacy of HER3-ADCs

### I. Objective

The objective of this assay is to measure the killing effects of HER3-ADC samples on cells and evaluate the *in vitro* activity of the HER3-ADCs based on IC₅₀ and Emax values.

### II. Method:

1. HER3-expressing cell strains MCF7 (ATCC, HTB-22), FaDu (ATCC, HTB-43), and SW620 (Nanjing Cobioer, CBP60036) were digested with trypsin, and the digestion was stopped with the respective fresh culture media for these cells. After centrifugation at 1000 rpm, the cells were resuspended in fresh media and counted, and then the cell density was adjusted to 3703 cells/mL. The cells were added to 96-well cell culture plates at 135 µL/well, with the 11th columns containing 135 µL of medium but no cells. The cells were cultured at 37 °C with 5% CO₂ for 16 h.
2. The HER3-ADC samples were diluted with PBS to 15 µM (10× concentration). Five-fold dilutions were performed with PBS from this concentration to obtain 8 concentrations. The 10× solutions were added at 15 µL/well. The cells were cultured at 37 °C with 5% CO₂ for 4 days.
3. CTG was added at 50 µL/well, and the plates were incubated at room temperature in the dark for 10 min. White membranes were affixed to the bottoms of the cell culture plates, and chemiluminescence values were measured on a Victor3 (PE) microplate reader.

### III. Data analysis

The data from this assay was processed using the data processing software GraphPad prism 5.0.

**Table 2. The killing effects of the Her3-ADC drugs on MCF7, FaDu, and SW620**

| | DAR | MCF7++++ | | SW620+++ | | FaDu++ | |
|---|---|---|---|---|---|---|---|
| | | EC₅₀ (nM) | Emax (%) | EC₅₀ (nM) | Emax (%) | EC₅₀ (nM) | Emax (%) |
| ADC-20 | 1.98 | 114.3 | 89.87 | 29.28 | 94.44 | 39.87 | 105.9 |
| Antibody Ab3 | / | / | 10.03 | / | / | / | 28.96 |

### IV Conclusion

ADC-20 has good activity in killing the cell lines with different levels of HER3 expression.

### Test Example 4. In Vitro Efficacy of HER2-ADCs

### I. Objective

The objective of this assay is to measure the killing effects of HER2-ADC samples on cells and evaluate the *in vitro* activity of the HER2-ADCs based on IC₅₀ and Emax values.

### II. Method:

1. HER2-expressing cell strains MCF7 (ATCC, HTB-22) and SK-BR-3 (ATCC, HTB-30) were digested with trypsin, and the digestion was neutralized with fresh culture media. After centrifugation at 1000 rpm, the cells were resuspended in media and counted, and then the density of the cell suspensions was adjusted to 3703 cells/mL. The cell suspensions were added to 96-well cell culture plates (3903) at 135 µL/well, with the 11th columns containing 135 µL of medium but no cells. The cells were cultured at 37 °C with 5% carbon dioxide for 16 h.
2. The HER2-ADC samples were diluted with PBS to 15 µM (10× concentration). Five-fold dilutions were performed with PBS from this concentration to obtain 8 concentrations. The 10× solutions were added at 15 µL/well. The cells were cultured at 37 °C with 5% CO₂ for 4 days.
3. CTG was added at 70 µL/well, and the plates were incubated at room temperature in the dark for 10 min. White membranes were affixed to the bottoms of the cell culture plates, and chemiluminescence values were measured on a Victor3 (PE) microplate reader.

### III. Data analysis

The data from this assay was processed using the data processing software GraphPad prism 5.0.

**Table 3. The killing effects of the Her2-ADC drugs on SK-BR-3 and T47D**

| | DAR | SK-BR-3++++ | | T47D+ | |
|---|---|---|---|---|---|
| | | EC₅₀ (nM) | Emax (%) | EC₅₀ (nM) | Emax (%) |
| ADC-18 | 1.95 | 1.12 | 72.01 | 294.5 | 71.51 |
| Antibody Ab1 | / | / | 41.81 | / | 10.02 |

### IV Conclusion

ADC-18 has good activity in killing the cell lines with different levels of HER2 expression, and the killing activity is correlated with the antigen expression levels-the higher the level of cell surface HER2 expression, the stronger the killing activity of the ADCs.

Although the foregoing invention has been described in detail by using examples for the sake of clear understanding, the description and examples should not be construed as limiting the scope of the present disclosure. The disclosures of all the patents and scientific literature cited herein are clearly incorporated by reference in their entirety.

## Claims

1. An antibody-drug conjugate represented by the following formula or a pharmaceutically acceptable salt thereof: 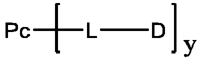
wherein Pc is an antibody, and a glycan chain of the antibody is an unremodeled or remodeled glycan chain;
L is a linker; Pc binds to L through an amino acid thereof or the glycan chain;
y is a drug loading of about 1 to about 10;
D is represented by formula (D): 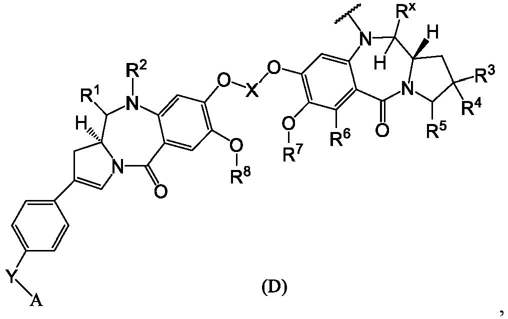
wherein:
R¹ is H, oxo, halogen, or alkyl;
R² is H, alkyl, or 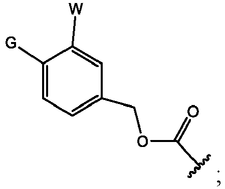 W is H, -C(O)R^{t}, or -C(O)NH-R^{t}; G is a glucuronide group or galactoside group; R^{t} is alkyl or alkoxyalkyl;
R³ is H or alkyl;
R⁴ is H or alkyl;
R⁵ is H, halogen, or alkyl;
or R³ and R⁴, together with the atom to which they are attached, form a ring, or R⁴ and R⁵, together with the atoms to which they are attached, form a ring;
R⁶ is H or alkyl;
R⁷ is alkyl or cycloalkyl; or R⁶ and R⁷, together with the atoms to which they are attached, form a ring;
R⁸ is alkyl or cycloalkyl;
R^{x} is H or hydroxy;
X is alkylene or -(CH₂)ₘ-X₁-(CH₂)ₙ-;
X₁ is a nitrogen atom, an oxygen atom, a sulfur atom, cycloalkyl, heterocyclyl, heteroaryl, or aryl; the cycloalkyl, heterocyclyl, heteroaryl, or aryl is optionally substituted with one or more substituents, wherein each of the substituents is independently selected from the group consisting of amino, hydroxy, hydroxyalkyl, alkyl, and alkoxy;
Y is a bond, a nitrogen atom, an oxygen atom, or a sulfur atom;
A is alkyl, cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R⁹, cycloalkyl, cycloalkylalkyl, haloalkyl, aryl, and heterocyclyl;
R⁹ is H, alkyl, and hydroxyalkyl;
m is 1, 2, or 3; n is 1, 2, or 3;
provided that when X is alkylene, 1) R¹ is oxo, or 2) R^{x} is H, or 3) R⁴ and R⁵, together with the atoms to which they are attached, form a ring, or 4) R⁶ and R⁷, together with the atoms to which they are attached, form a ring, or 5) R⁷ and/or R⁸ are cycloalkyl; or 6) A is not alkyl.

2. The antibody-drug conjugate or the pharmaceutically acceptable salt thereof according to claim 1, wherein the R¹ is H or oxo; preferably, R¹ is H.

3. The antibody-drug conjugate or the pharmaceutically acceptable salt thereof according to claim 1 or 2, wherein the R² is H or C₁₋₆ alkyl; preferably, R² is H.

4. The antibody-drug conjugate or the pharmaceutically acceptable salt thereof according to claim 1 or 2, wherein the R² is 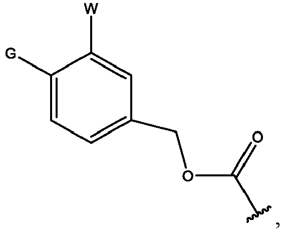 wherein W is H or -C(O)NH-R^{t}, and G is a glucuronide group; R^{t} is C₁₋₆ alkyl or C₁₋₆ alkoxy C₁₋₆ alkyl.

5. The antibody-drug conjugate or the pharmaceutically acceptable salt thereof according to any of claims 1 to 4, wherein the R^{x} is hydroxy.

6. The antibody-drug conjugate or the pharmaceutically acceptable salt thereof according to any of claims 1 to 5, wherein:
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form 3-membered cycloalkyl;
R⁵ is H; R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is alkylene; preferably, X is C₁₋₁₂ alkylene; more preferably, X is pentylene;
Y is selected from the group consisting of a bond, a nitrogen atom, an oxygen atom, and a sulfur atom; preferably, Y is a bond or a sulfur atom; more preferably, Y is a bond;
A is selected from the group consisting of cycloalkyl, heterocyclyl, aryl, and heteroaryl;
the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R⁹, cycloalkyl, cycloalkylalkyl, haloalkyl, aryl, and heterocyclyl; preferably, A is selected from the group consisting of C₃₋₆ cycloalkyl, 5- to 6-membered heterocyclyl, phenyl, and 5- to 6-membered heteroaryl; the C₃₋₆ cycloalkyl, 5- to 6-membered heterocyclyl, phenyl, or 5- to 6-membered heteroaryl is optionally substituted with one or more substituents, wherein each of the substituents is independently selected from the group consisting of C₁₋₆ hydroxyalkyl, C₁₋₆ alkyl, C₃₋₆ cycloalkyl C₁₋₆ alkyl, C₁₋₆ haloalkyl, and -C(O)R⁹;
R⁹ is selected from the group consisting of H, alkyl, and hydroxyalkyl; preferably, R⁹ is C₁₋₆ alkyl or C₁₋₆ hydroxyalkyl.

7. The antibody-drug conjugate or the pharmaceutically acceptable salt thereof according to any of claims 1 to 5, wherein:
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form a 3-membered carbocycle;
R⁵ is H; R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is -(CH₂)ₘ-X₁-(CH₂)ₙ-;
X₁ is an oxygen atom, cycloalkyl, heteroaryl, or aryl; the cycloalkyl, heteroaryl, or aryl is optionally substituted with one or more substituents, wherein each of the substituents is independently selected from the group consisting of amino, hydroxy, hydroxyalkyl, alkyl, and alkoxy; preferably, X₁ is an oxygen atom, C₃₋₆ cycloalkyl, pyridinyl, or phenyl; the C₃₋₆ cycloalkyl or phenyl is optionally substituted with one or more amino or C₁₋₆ alkyl;
m is selected from the group consisting of 1, 2, and 3; n is selected from the group consisting of 1, 2, and 3.

8. The antibody-drug conjugate or the pharmaceutically acceptable salt thereof according to any of claims 1 to 5, wherein:
R¹ is H or alkyl; preferably, R¹ is H or C₁₋₆ alkyl; more preferably, R¹ is H;
R³ and R⁴, together with the atom to which they are attached, form a ring; preferably, R³ and R⁴, together with the atom to which they are attached, form a 3-membered carbocycle;
X is alkylene; preferably, X is C₁₋₆ alkylene; more preferably, X is pentylene;
R⁶ is H or alkyl; preferably, R⁶ is H or C₁₋₆ alkyl;
R⁷ is alkyl or cycloalkyl; preferably, R⁷ is C₁₋₆ alkyl or C₃₋₆ cycloalkyl;
or R⁶ and R⁷, together with the atoms to which they are attached, form a ring;
preferably, R⁶ and R⁷, together with the atoms to which they are attached, form a 4-6-membered ring;
R⁸ is alkyl or cycloalkyl; preferably, R⁸ is C₁₋₆ alkyl or C₃₋₆ cycloalkyl;
provided that R⁷ and R⁸ are not simultaneously alkyl.

9. The antibody-drug conjugate or the pharmaceutically acceptable salt thereof according to any of claims 1 to 5, wherein
R¹ is H or alkyl; preferably, R¹ is H or C₁₋₆ alkyl; more preferably, R¹ is H;
R³ is H;
R⁴ and R⁵, together with the atoms to which they are attached, form a ring; preferably, R⁴ and R⁵, together with the atoms to which they are attached, form a 3-membered carbocycle;
R⁶ is H;
R⁷ is alkyl; preferably, R⁷ is C₁₋₆ alkyl; more preferably, R⁷ is methyl;
R⁸ is alkyl; preferably, R⁸ is C₁₋₆ alkyl; more preferably, R⁸ is methyl;
X is alkylene; preferably, X is C₁₋₁₂ alkylene; more preferably, X is pentylene.

10. The antibody-drug conjugate or the pharmaceutically acceptable salt thereof according to any of claims 1 to 9, wherein L is -L^{a}-L^{b}-L^{c}-L^{d}-;
L^{a} is selected from the group consisting of: 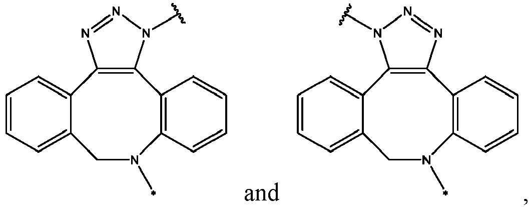 wherein the asterisk * indicates binding to L^{b}, and the wavy line 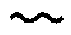 indicates binding to the remodeled glycan chain of Pc;
L^{b} is selected from the group consisting of -C(O)-CH₂CH₂-C(O)-, -C(O)-(CH₂CH₂)₂-C(O)-, -C(O)-CH₂CH₂-C(O)-NH-(CH₂CH₂)₂-C(O)-, -C(O)-CH₂CH₂-C(O)-NH-(CH₂CH₂O)₂-CH₂-C(O)-, -C(O)-CH₂CH₂-NH-C(O)-(CH₂CH₂O)₄-CH₂CH₂-C(O)-, -CH₂-OC(O)-, and -OC(O)-;
L^{c} is a peptide residue consisting of 2 to 7 amino acid residues, wherein the amino acid residues are residues formed from phenylalanine, alanine, proline, isoleucine, glycine, valine, lysine, citrulline, serine, glutamic acid, or aspartic acid; the amino acid residues are unsubstituted or are each independently substituted with one or more substituents, wherein each of the substituents is independently selected from the group consisting of halogen, hydroxy, cyano, amino, C₁₋₆ alkyl, C₁₋₆ haloalkyl, C₁₋₆ alkoxy, and C₃₋₇ cycloalkyl;
L^{d} is -NH-R^{a}-CH₂O-C(O)-, -NH-CH₂O-R^{b}-C(O)-, or a bond;
R^{a} is phenyl or 5-6 membered heterocyclyl; the phenyl and 5-6 membered heterocyclyl are unsubstituted or are each independently substituted with one or more substituents, wherein each of the substituents is independently selected from the group consisting of halogen, oxo, hydroxy, cyano, amino, C₁₋₆ alkyl, C₁₋₆ haloalkyl, and C₁₋₆ alkoxy;
R^{b} is C₁₋₆ alkyl or C₃₋₇ cycloalkyl; the C₁₋₆ alkyl and C₃₋₇ cycloalkyl are unsubstituted or are each independently substituted with one or more substituents, wherein each of the substituents is independently selected from the group consisting of halogen, oxo, hydroxy, cyano, amino, C₁₋₆ alkyl, C₃₋₇ cycloalkyl, 5-6 membered heterocyclyl, C₁₋₆ haloalkyl, and C₁₋₆ alkoxy.

11. The antibody-drug conjugate or the pharmaceutically acceptable salt thereof according to claim 10, wherein L^{b} is
-C(O)-CH₂CH₂-C(O)-.

12. The antibody-drug conjugate or the pharmaceutically acceptable salt thereof according to claim 10, wherein L^{c} is -GGVA-, -VA-, -GGFG-, -GGPI-, -GGVK-, and -GGPL-, preferably -GGVA-.

13. The antibody-drug conjugate or the pharmaceutically acceptable salt thereof according to claim 10, wherein
L^{d} is -NH-R^{a}-CH₂O-C(O)-, and R^{a} is phenyl.

14. The antibody-drug conjugate or the pharmaceutically acceptable salt thereof according to any of claims 1 to 13, wherein the Pc is an antibody that binds to tumor cells and can be taken in by tumor cells; preferably, the Pc is selected from the group consisting of an anti-HER2 (ErbB2) antibody, an anti-EGFR antibody, an anti-B7H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-Claudin18.2 antibody, an anti-ROR1 antibody, an anti-CD19 antibody, an anti-Trop2 antibody, an anti-CD79b antibody, an anti-SLC44A4 antibody, and an anti-Mesothelin antibody.

15. The antibody-drug conjugate or the pharmaceutically acceptable salt thereof according to any of claims 1 to 14, wherein the y is 1 to 8, preferably 1 to 4.

16. The antibody-drug conjugate or the pharmaceutically acceptable salt thereof according to any of claims 1 to 15, wherein the Pc binds to L through an N297 glycan chain thereof, and the N297 glycan chain binds to an Asn at position 297 of a heavy chain of Pc.

17. The antibody-drug conjugate or the pharmaceutically acceptable salt thereof according to any of claims 1 to 16, being the following structure:
Pc-(N297 glycan chain)-[L-D]y
wherein the N297 glycan chain is a remodeled glycan chain and binds to an Asn at position 297 of a heavy chain of Pc;
y is 1 to 4, preferably 1 to 2;
Pc, L, and D are as described in claim 1.

18. The antibody-drug conjugate or the pharmaceutically acceptable salt thereof according to any of claims 1 to 17, wherein the glycan chain is an N297 glycan chain, the structure of which is selected from the group consisting of [N297-(Fuc)SG1], [N297-(Fuc)SG2], [N297-(Fuc)SG3], [N297-(Fuc)SG4], and [N297-(Fuc)SG5]: 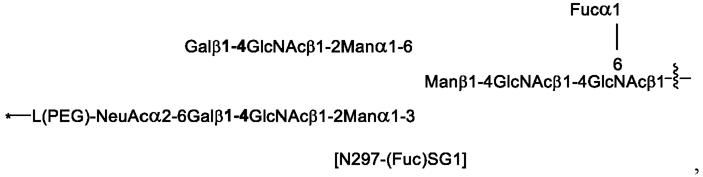 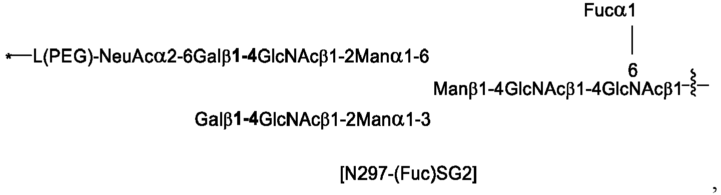 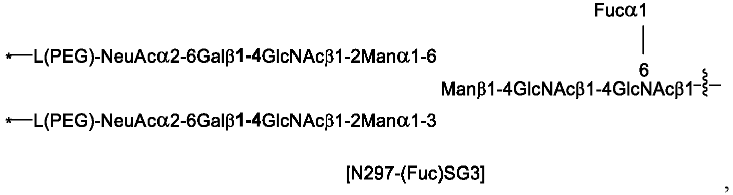 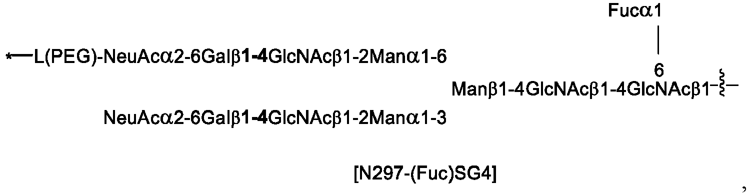 and 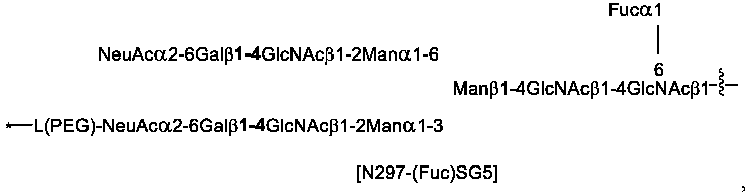
wherein the wavy line 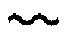 indicates binding to an Asn at position 297 of a heavy chain of Pc;
L(PEG) indicates -(CH₂CH₂O)qCH₂CH₂-NH-, wherein the amino group at the right end indicates amide bonding with the carboxylic acid at locant 2 of the N-acetylneuraminic acid at the non-reducing terminus on the 1-3 chain side or/and 1-6 chain side of a β-Man branched chain of the N297 glycan chain; q is 0 to 20, preferably 1 to 10;
the asterisk * indicates binding to the linker L;
preferably, the glycan chain structure is: 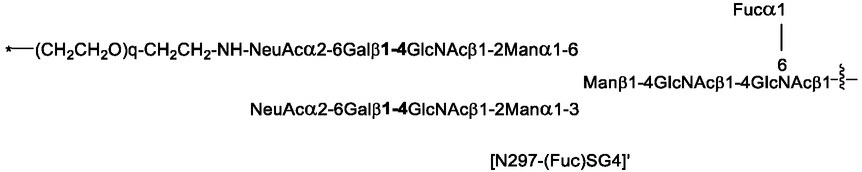 or 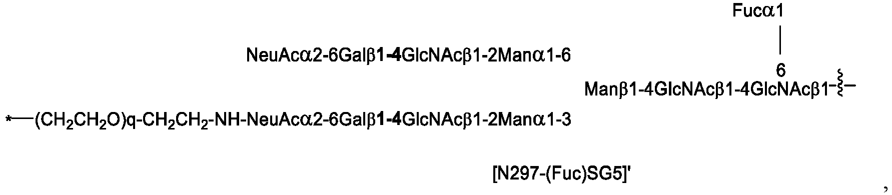
wherein q is 2 or 3.

19. The antibody-drug conjugate or the pharmaceutically acceptable salt thereof according to any of claims 1 to 17, wherein the glycan chain structure is: 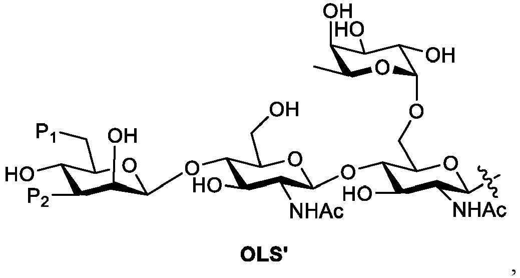
wherein the wavy line 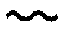 indicates binding to an Asn at position 297 of a heavy chain of Pc;
P₁ and P₂ are each independently selected from the group consisting of hydroxy, *-(CH₂CH₂O)s¹-, and *-(CH₂CH₂O)s²-(CH₂CH₂CH₂O)s³-(CH₂CH₂O)s⁴-; the *-(CH₂CH₂O)s¹- and *-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy; the asterisk * indicates binding to the linker L;
s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5;
provided that P₁ and P₂ are not simultaneously hydroxy or *-(CH₂CH₂O)s¹-;
preferably, the glycan chain structure is selected from the group consisting of: 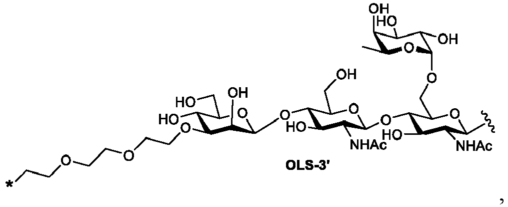 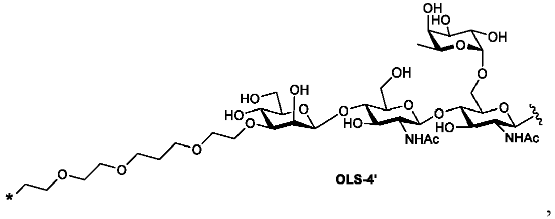 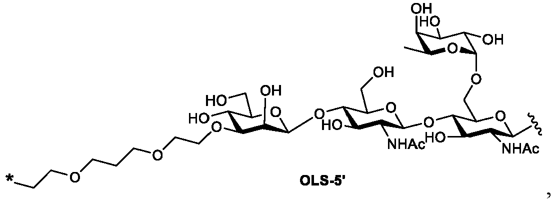 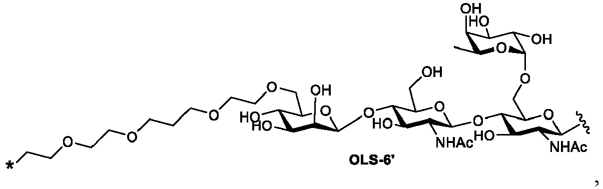 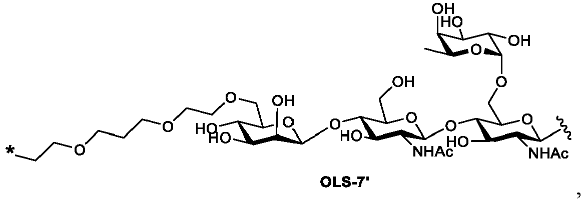 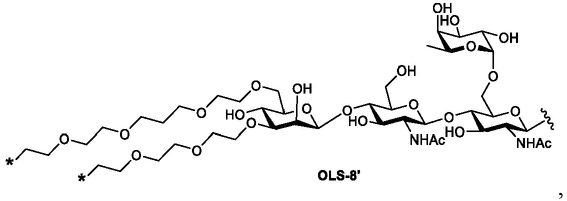 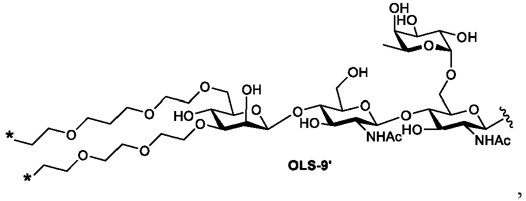 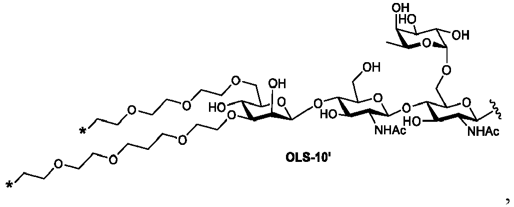 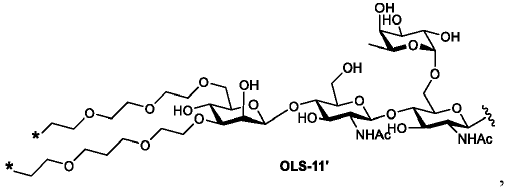 and

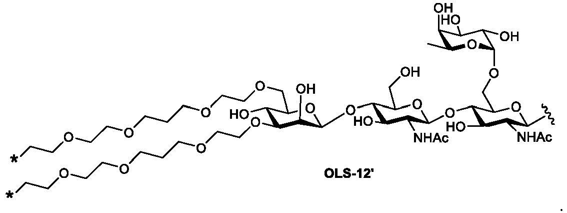

20. The antibody-drug conjugate or the pharmaceutically acceptable salt thereof according to any of claims 1 to 19, being selected from the group consisting of: 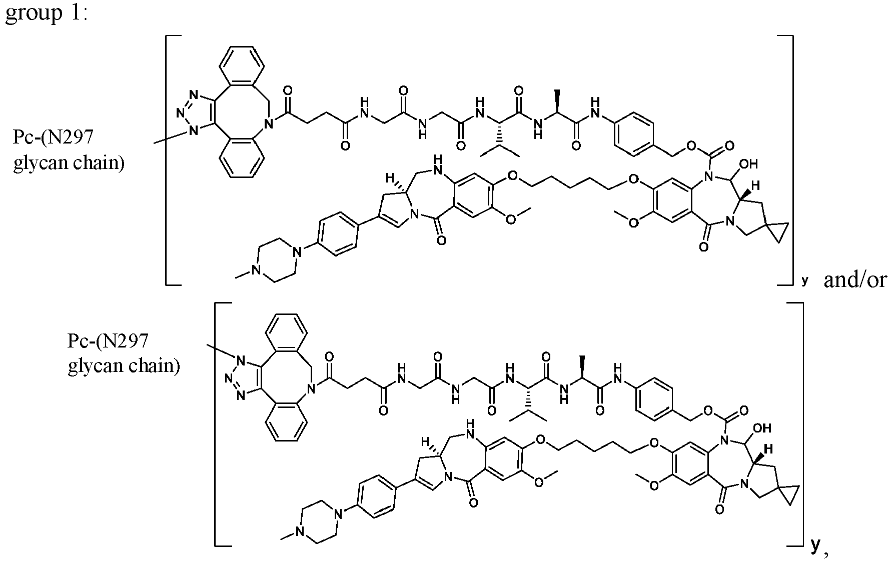 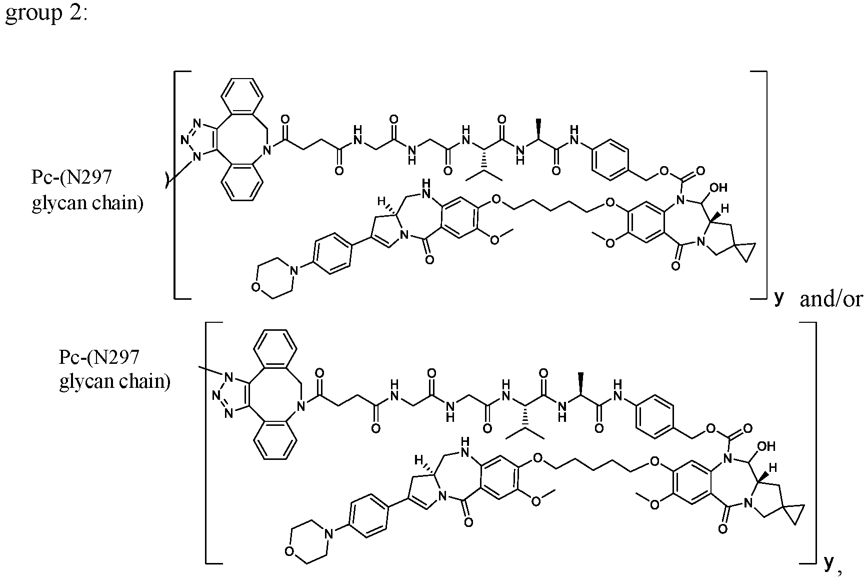 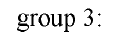 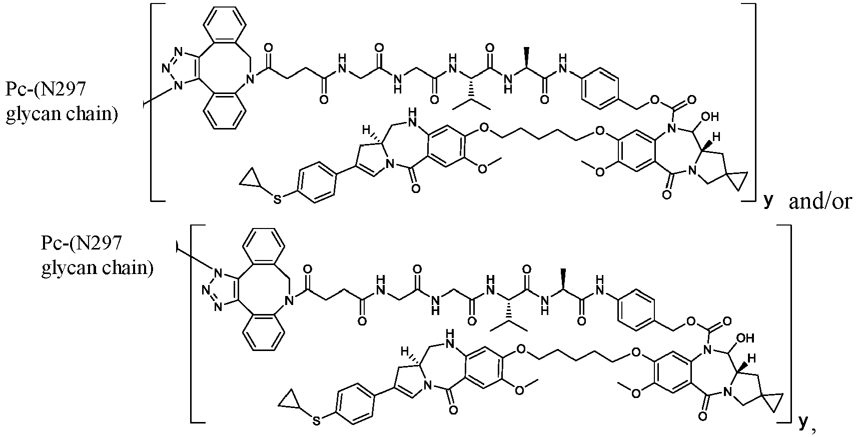 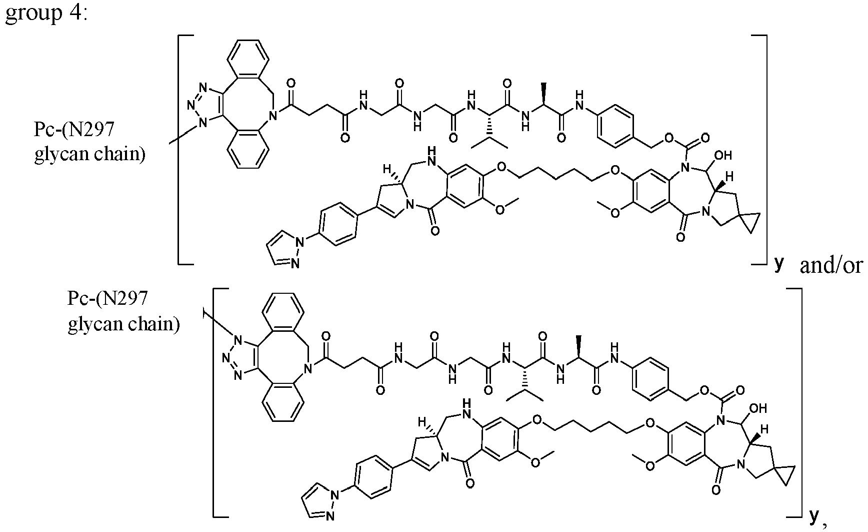 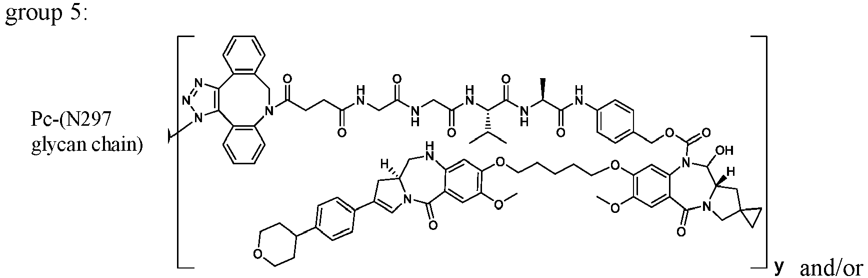 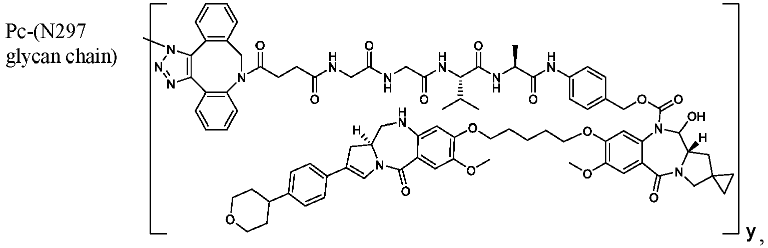 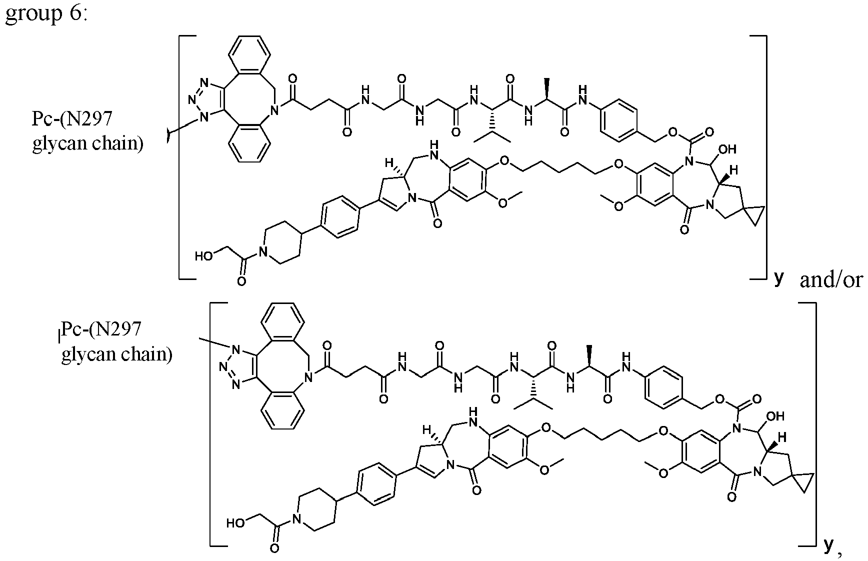 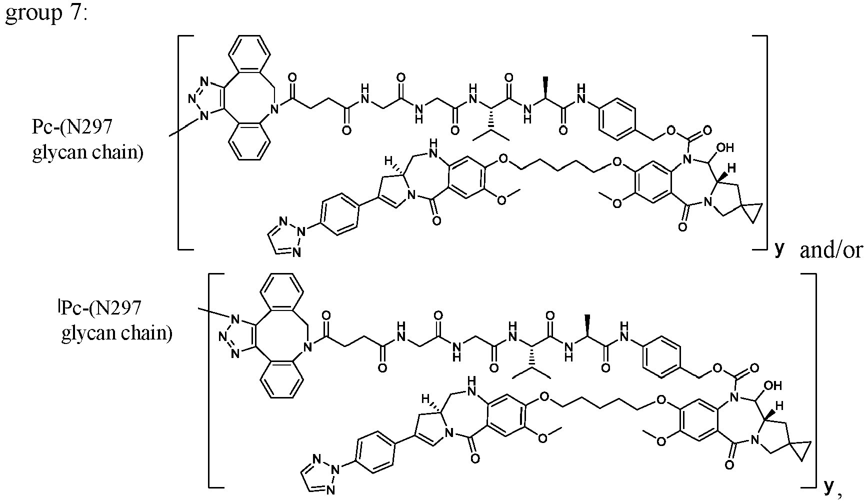 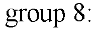 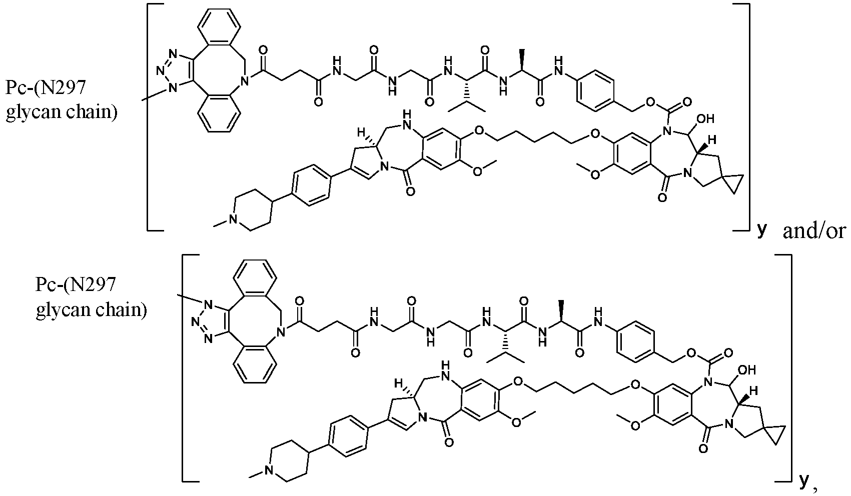 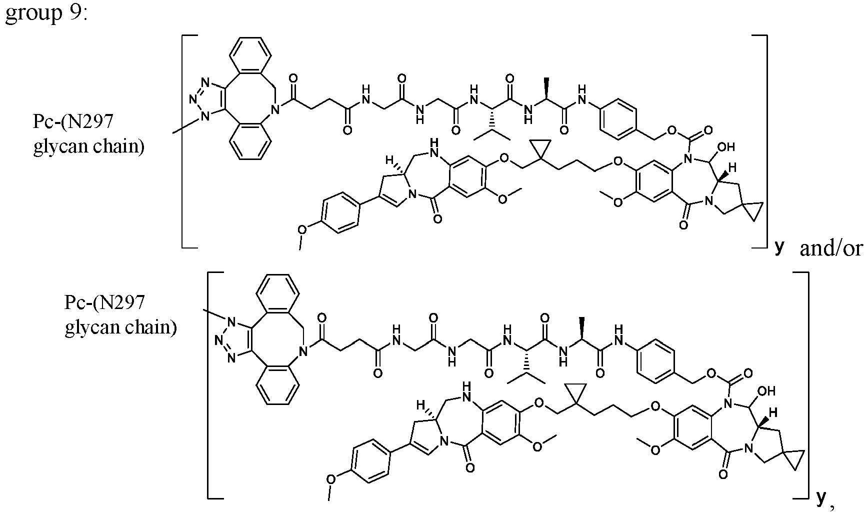 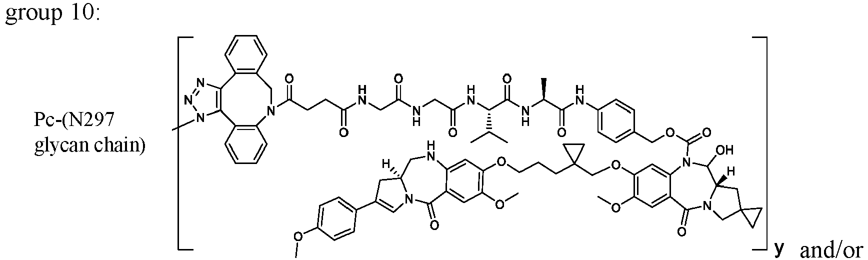 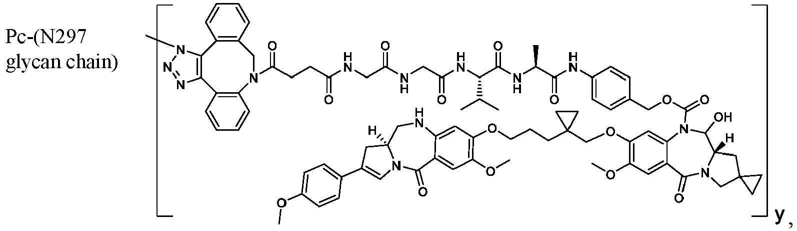 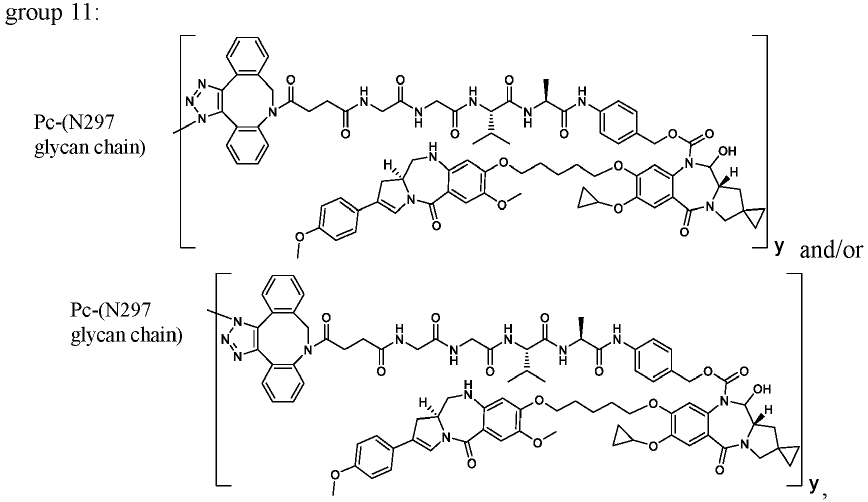 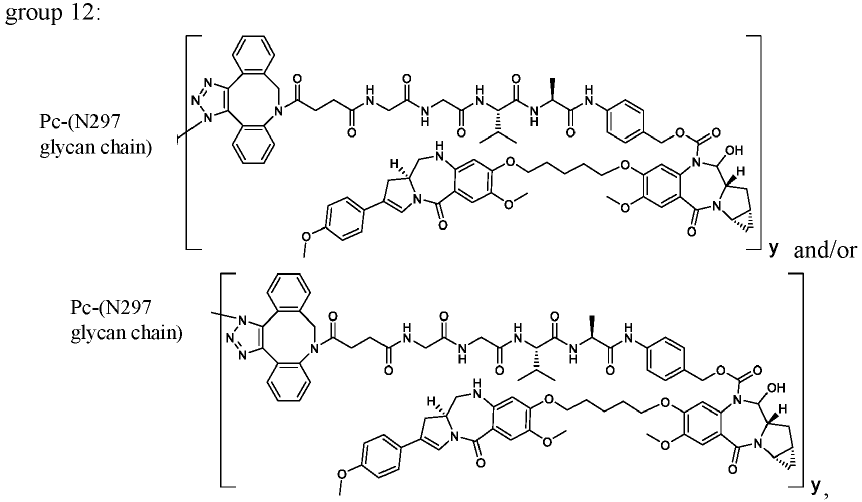 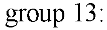 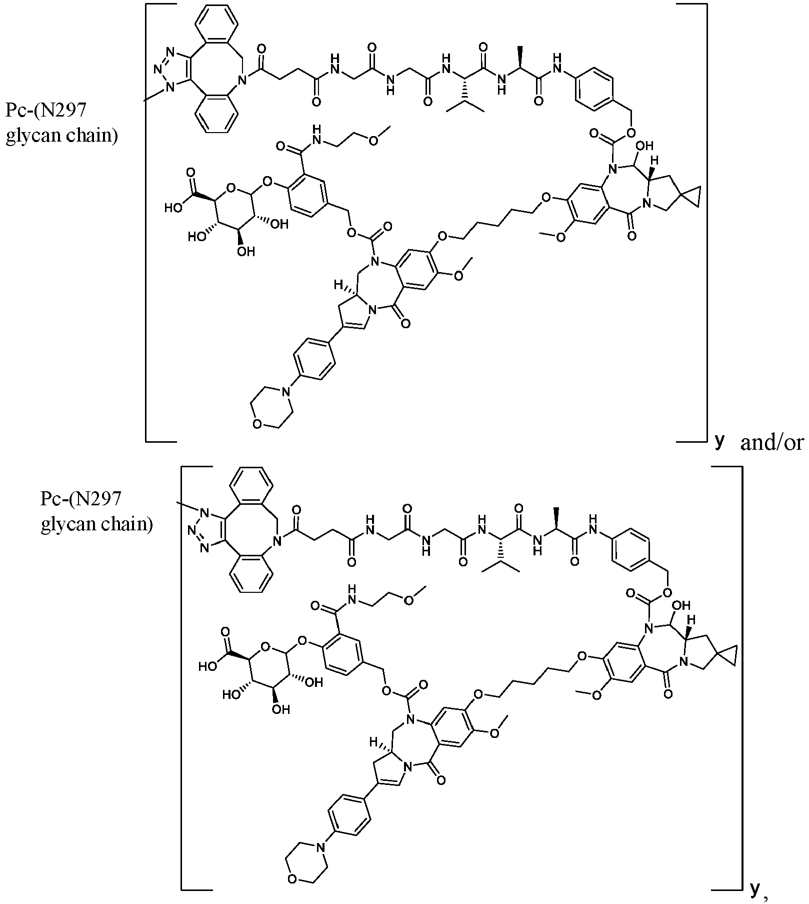 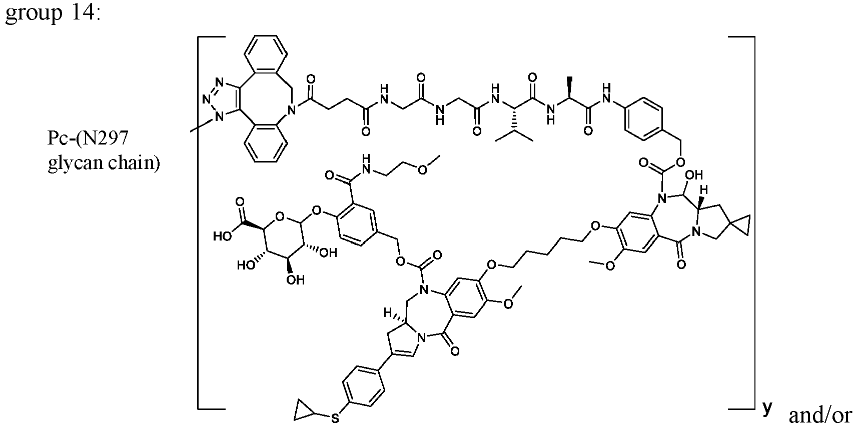 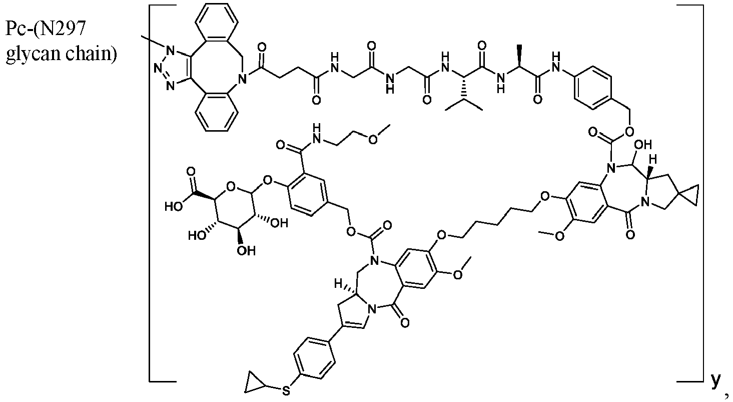 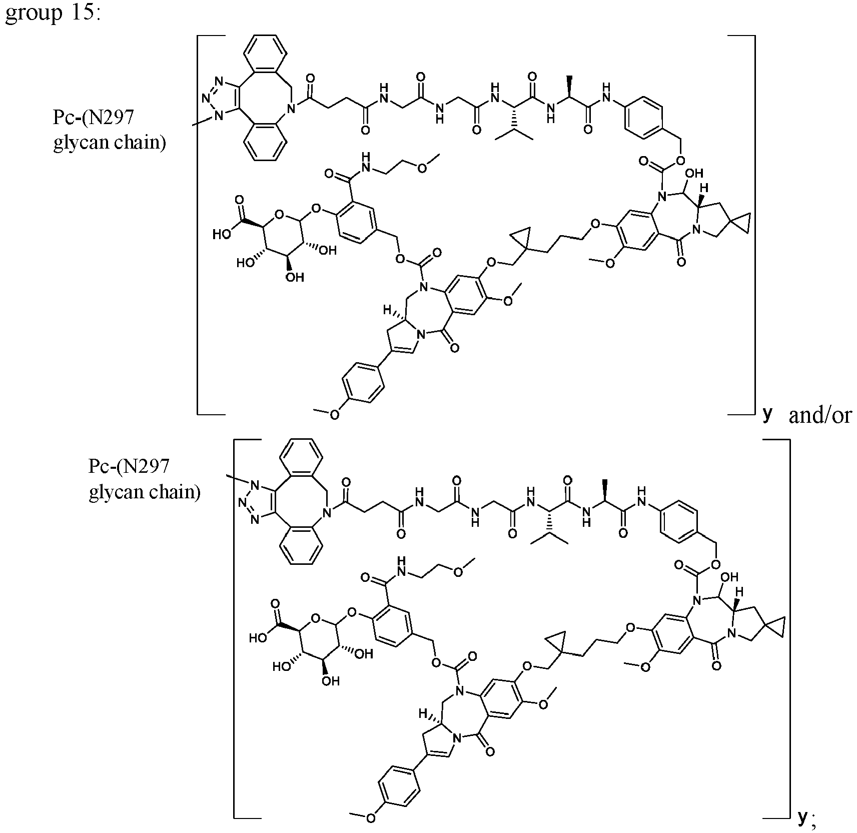 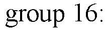 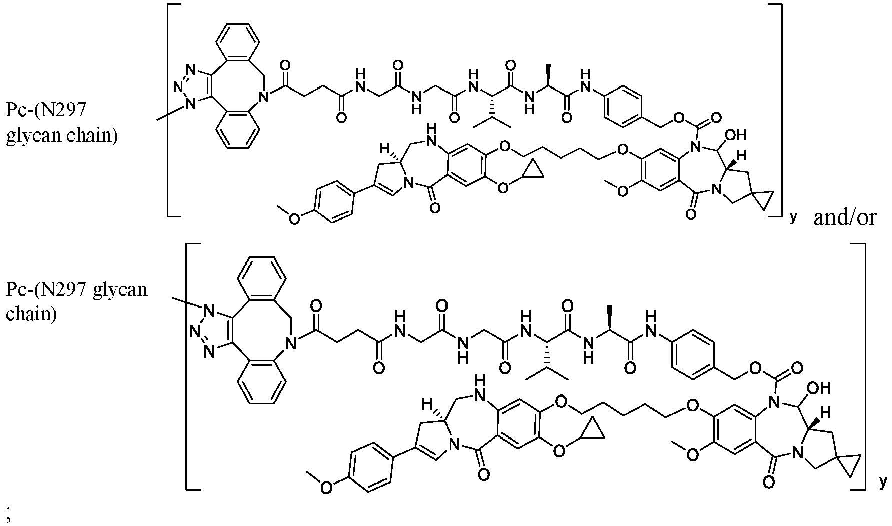 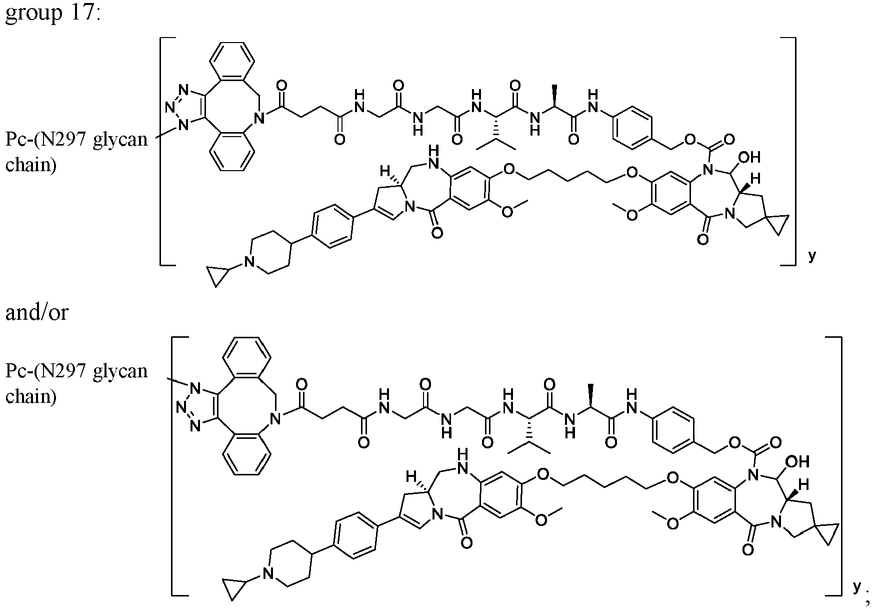 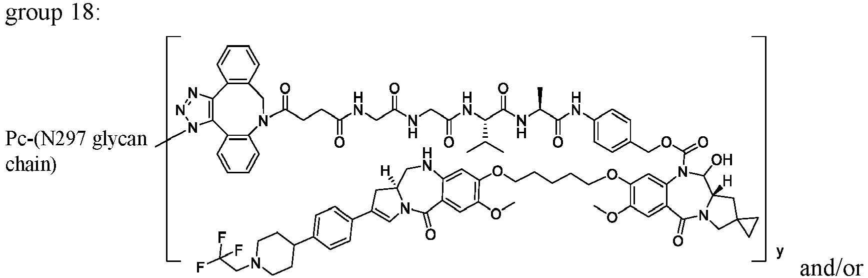 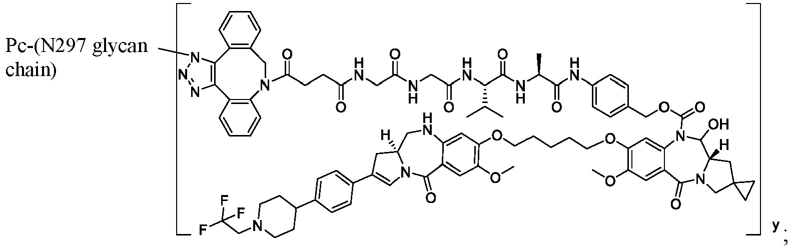 wherein:
y is selected from the group consisting of 1 to 4, preferably 1 to 2;
the N297 glycan chain structure is selected from the group consisting of: 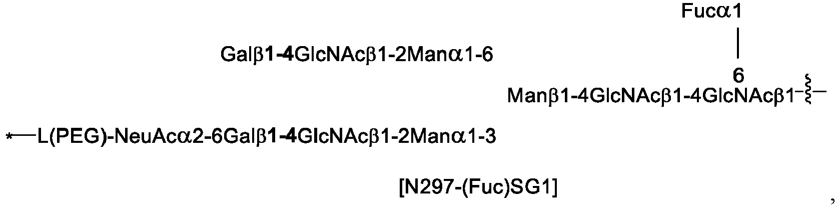 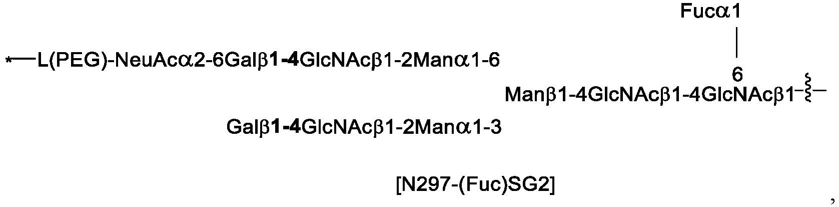 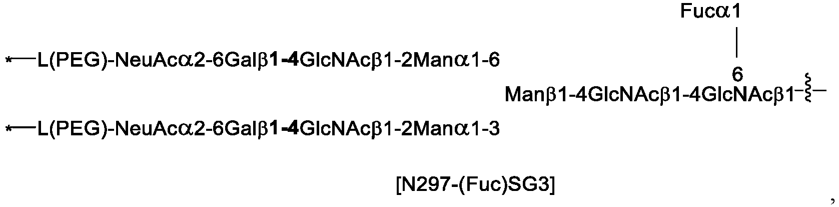 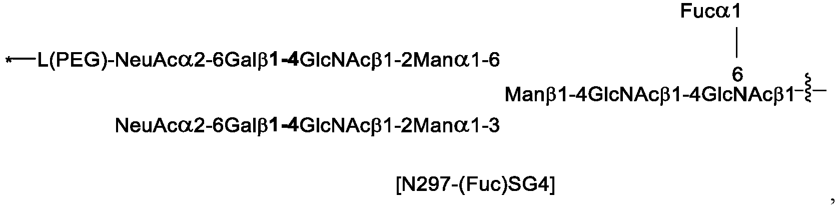 and 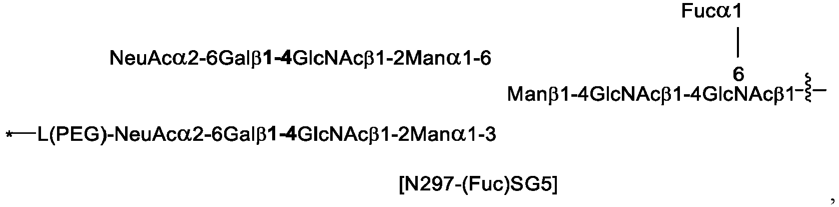
wherein the wavy line 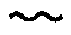 indicates binding to an Asn at position 297 of a heavy chain of Pc, and the asterisk * indicates binding to the linker L;
L(PEG) indicates -(CH₂CH₂O)qCH₂CH₂-NH-, wherein q is selected from the group consisting of 0-20, preferably 1-10; the amino group at the right end indicates amide bonding with the carboxylic acid at locant 2 of the N-acetylneuraminic acid at the non-reducing terminus on the 1-3 chain side or/and 1-6 chain side of a β-Man branched chain of the N297 glycan chain;
or the N297 glycan chain structure is selected from: 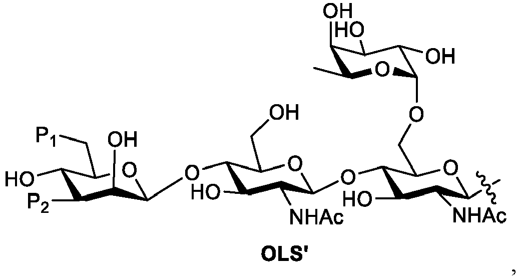
wherein the wavy line 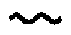 indicates binding to an Asn at position 297 of a heavy chain of Pc;
P₁ and P₂ are each independently selected from the group consisting of hydroxy, *-(CH₂CH₂O)s¹-, and *-(CH₂CH₂O)s²-(CH₂CH₂CH₂O)s³-(CH₂CH₂O)s⁴-; the *-(CH₂CH₂O)s¹- and *-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy; s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5; the asterisk * indicates binding to the linker L;
provided that P₁ and P₂ are not simultaneously hydroxy or *-(CH₂CH₂O)s¹-;
the Pc is as described in claim 14; preferably, the Pc is selected from the group consisting of an anti-HER2 antibody, an anti-HER3 antibody, an anti-Claudin18.2 antibody, and an anti-CD19 antibody;
more preferably, the anti-HER2 antibody comprises a light chain, the sequence of which is set forth in SEQ ID NO: 1, and a heavy chain, the sequence of which is set forth in SEQ ID NO: 2; the anti-Claudin18.2 antibody comprises a light chain, the sequence of which is set forth in SEQ ID NO: 3, and a heavy chain, the sequence of which is set forth in SEQ ID NO: 4; the anti-HER3 antibody comprises a light chain, the sequence of which is set forth in SEQ ID NO: 5, and a heavy chain, the sequence of which is set forth in SEQ ID NO: 6.

21. A compound represented by formula (DL) or a pharmaceutically acceptable salt thereof: 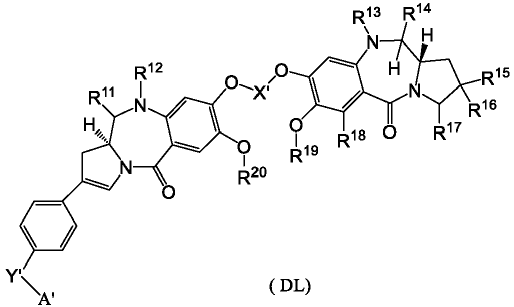 wherein:
R¹¹ is H, oxo, halogen, or alkyl;
R¹² is H, alkyl, or 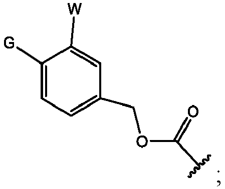 W is H, -C(O)R^{t1}, or -C(O)NH-R^{t1}; G is selected from the group consisting of a glucuronide group and galactoside group; R^{t1} is selected from the group consisting of alkyl and alkoxyalkyl;
R¹³ is L^{a'}-L^{b}-L^{c}-L^{d}-:
L^{a'} is 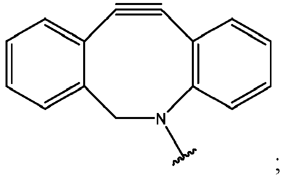
L^{b} is selected from the group consisting of -C(O)-CH₂CH₂-C(O)-, -C(O)-(CH₂CH₂)₂-C(O)-, -C(O)-CH₂CH₂-C(O)-NH-(CH₂CH₂)₂-C(O)-, -C(O)-CH₂CH₂-C(O)-NH-(CH₂CH₂O)₂-CH₂-C(O)-, -C(O)-CH₂CH₂-NH-C(O)-(CH₂CH₂O)₄-CH₂ CH₂-C(O)-, -CH₂-OC(O)-, and -OC(O)-;
L^{c} is a peptide residue consisting of 2 to 7 amino acid residues, wherein the amino acid residues are residues formed from phenylalanine, alanine, proline, isoleucine, glycine, valine, lysine, citrulline, serine, glutamic acid, or aspartic acid; the amino acid residues are unsubstituted or are each independently substituted with one or more substituents, wherein each of the substituents is independently selected from the group consisting of halogen, hydroxy, cyano, amino, C₁₋₆ alkyl, C₁₋₆ haloalkyl, C₁₋₆ alkoxy, and C₃₋₇ cycloalkyl;
L^{d} is -NH-R^{a}-CH₂O-C(O)-, -NH-CH₂O-R^{b}-C(O)-, or a bond;
R^{a} is phenyl or 5-6 membered heterocyclyl; the phenyl and 5-6 membered heterocyclyl are unsubstituted or are each independently substituted with one or more substituents, wherein each of the substituents is independently selected from the group consisting of halogen, oxo, hydroxy, cyano, amino, C₁₋₆ alkyl, C₁₋₆ haloalkyl, and C₁₋₆ alkoxy;
R^{b} is C₁₋₆ alkyl or C₃₋₇ cycloalkyl; the C₁₋₆ alkyl and C₃₋₇ cycloalkyl are unsubstituted or are each independently substituted with one or more substituents, wherein each of the substituents is independently selected from the group consisting of halogen, oxo, hydroxy, cyano, amino, C₁₋₆ alkyl, C₃₋₇ cycloalkyl, 5-6 membered heterocyclyl, C₁₋₆ haloalkyl, and C₁₋₆ alkoxy;
R¹⁴ is H, hydroxy, or alkoxy;
or R¹³ and R¹⁴, together with the atoms to which they are attached, form imine bonds (N=C);
R¹⁵ is H or alkyl;
R¹⁶ is H or alkyl;
R¹⁷ is H, halogen, or alkyl;
or R¹⁵ and R¹⁶, together with the atom to which they are attached, form a ring, or R¹⁶ and R¹⁷, together with the atoms to which they are attached, form a ring;
R¹⁸ is H or alkyl;
R¹⁹ is alkyl or cycloalkyl; or R¹⁸ and R¹⁹, together with the atoms to which they are attached, form a ring;
R²⁰ is alkyl or cycloalkyl;
X' is alkylene or -(CH₂)_{m'}-X'₁-(CH₂)_{n'}-;
X'₁ is a nitrogen atom, an oxygen atom, a sulfur atom, cycloalkyl, heterocyclyl, heteroaryl, or aryl; the cycloalkyl, heterocyclyl, heteroaryl, or aryl is optionally substituted with one or more substituents, wherein each of the substituents is independently selected from the group consisting of amino, hydroxy, hydroxyalkyl, alkyl, and alkoxy;
Y' is a bond, a nitrogen atom, an oxygen atom, or a sulfur atom;
A' is alkyl, cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R²¹, cycloalkyl, cycloalkylalkyl, haloalkyl, aryl, and heterocyclyl;
R²¹ is H, alkyl, and hydroxyalkyl;
m' is 1, 2, or 3; n' is 1, 2, or 3;
provided that when X' is alkylene, 1) R¹¹ is oxo, or 2) R¹⁴ is H, or 3) R¹⁶ and R¹⁷, together with the atoms to which they are attached, form a ring, or 4) R¹⁸ and R¹⁹, together with the atoms to which they are attached, form a ring, or 5) R¹⁹ and/or R²⁰ are cycloalkyl; or 6) A' is not alkyl.

22. The compound or the pharmaceutically acceptable salt thereof according to claim 21, wherein R¹³ and R¹⁴, together with the atoms to which they are attached, form imine bonds (N=C).

23. The compound or the pharmaceutically acceptable salt thereof according to claim 21 or 22, wherein:
R¹³ is L^{a'}-L^{b}-L^{c}-L^{d}-:
L^{a'} is 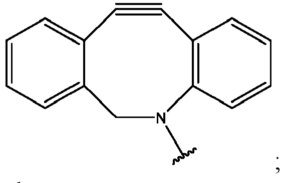
L^{b} is selected from the group consisting of -C(O)-CH₂CH₂-C(O)-, -C(O)-(CH₂CH₂)₂-C(O)-, -C(O)-CH₂CH₂-C(O)-NH-(CH₂CH₂)₂-C(O)-, -C(O)-CH₂CH₂-C(O)-NH-(CH₂CH₂O)₂-CH₂-C(O)-, -C(O)-CH₂CH₂-NH-C(O)-(CH₂CH₂O)₄-CH₂CH₂-C(O)-, -CH₂-OC(O)-, and -OC(O)-;
L^{c} is a peptide residue consisting of 2 to 7 amino acid residues, wherein the amino acid residues are residues formed from phenylalanine, alanine, proline, isoleucine, glycine, valine, lysine, citrulline, serine, glutamic acid, or aspartic acid; the amino acid residues are unsubstituted or are each independently substituted with one or more substituents, wherein each of the substituents is independently selected from the group consisting of halogen, hydroxy, cyano, amino, C₁₋₆ alkyl, C₁₋₆ haloalkyl, C₁₋₆ alkoxy, and C₃₋₇ cycloalkyl;
L^{d} is -NH-R^{a}-CH₂O-C(O)-, -NH-CH₂O-R^{b}-C(O)-, or a bond;
R^{a} is phenyl or 5-6 membered heterocyclyl; the phenyl and 5-6 membered heterocyclyl are unsubstituted or are each independently substituted with one or more substituents, wherein each of the substituents is independently selected from the group consisting of halogen, oxo, hydroxy, cyano, amino, C₁₋₆ alkyl, C₁₋₆ haloalkyl, and C₁₋₆ alkoxy;
R^{b} is C₁₋₆ alkyl or C₃₋₇ cycloalkyl; the C₁₋₆ alkyl and C₃₋₇ cycloalkyl are unsubstituted or are each independently substituted with one or more substituents, wherein each of the substituents is independently selected from the group consisting of halogen, oxo, hydroxy, cyano, amino, C₁₋₆ alkyl, C₃₋₇ cycloalkyl, 5-6 membered heterocyclyl, C₁₋₆ haloalkyl, and C₁₋₆ alkoxy.

24. The compound or the pharmaceutically acceptable salt thereof according to claim 21, wherein R¹⁴ is H or hydroxy; preferably, R¹⁴ is hydroxy.

25. The compound or the pharmaceutically acceptable salt thereof according to any of claims 21 to 24, wherein the R¹¹ is H or oxo; preferably, R¹¹ is H.

26. The compound or the pharmaceutically acceptable salt thereof according to any of claims 21 to 25, wherein the R¹² is H or C₁₋₆ alkyl; preferably, R¹² is H.

27. The compound or the pharmaceutically acceptable salt thereof according to any of claims 21 to 25, wherein the R¹² is represented by the following structure: 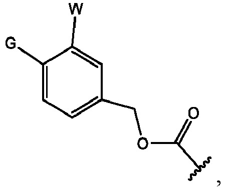 wherein W is H or -C(O)NH-R^{t1}, and G is a glucuronide group; R^{t1} is selected from the group consisting of C₁₋₆ alkyl and C₁₋₆ alkoxy C₁₋₆ alkyl.

28. The compound or the pharmaceutically acceptable salt thereof according to any of claims 21 to 27, wherein:
R¹¹ is H or alkyl; preferably, R¹¹ is H or C₁₋₆ alkyl;
R¹⁵ and R¹⁶, together with the atom to which they are attached, form a 3-6 membered carbocycle;
R¹⁷ is H; R¹⁸ is H;
R¹⁹ is alkyl; preferably, R¹⁹ is C₁₋₆ alkyl;
R²⁰ is alkyl; preferably, R²⁰ is C₁₋₆ alkyl;
X' is alkylene; preferably, X' is C₁₋₆ alkylene; more preferably, X' is pentylene;
Y' is selected from the group consisting of a bond, a nitrogen atom, an oxygen atom, and a sulfur atom; preferably, Y' is a bond or a sulfur atom; more preferably, Y' is a bond;
A' is cycloalkyl, heterocyclyl, aryl, or heteroaryl; the cycloalkyl, heterocyclyl, aryl, or heteroaryl is optionally substituted with one or more substituents, wherein each of the substituents is independently selected from the group consisting of halogen, hydroxy, hydroxyalkyl, alkyl, alkoxy, -C(O)R²¹, cycloalkyl, cycloalkylalkyl, haloalkyl, aryl, and heterocyclyl; preferably, A' is selected from the group consisting of C₃₋₆ cycloalkyl, 5-to 6-membered heterocyclyl, phenyl, and 5- to 6-membered heteroaryl; the C₃₋₆ cycloalkyl, 5- to 6-membered heterocyclyl, phenyl, or 5- to 6-membered heteroaryl is optionally substituted with one or more substituents, wherein each of the substituents is independently selected from the group consisting of C₁₋₆ hydroxyalkyl, C₁₋₆ alkyl, C₃₋₆ cycloalkyl C₁₋₆ alkyl, C₁₋₆ haloalkyl, and -C(O)R²¹;
R²¹ is selected from the group consisting of H, alkyl, and hydroxyalkyl; preferably, R²¹ is C₁₋₆ alkyl or C₁₋₆ hydroxyalkyl.

29. The compound or the pharmaceutically acceptable salt thereof according to any of claims 21 to 27, wherein:
R¹¹ is H or alkyl; preferably, R¹¹ is H or C₁₋₆ alkyl;
R¹⁵ and R¹⁶, together with the atom to which they are attached, form a 3-6 membered carbocycle;
R¹⁷ is H; R¹⁸ is H;
R¹⁹ is alkyl; preferably, R¹⁹ is C₁₋₆ alkyl;
R²⁰ is alkyl; preferably, R²⁰ is C₁₋₆ alkyl;
X' is -(CH₂)_{m'}-X'₁-(CH₂)_{n'}-;
X'₁ is a nitrogen atom, an oxygen atom, a sulfur atom, cycloalkyl, heteroaryl, or aryl; the cycloalkyl, heteroaryl, or aryl is optionally substituted with one or more substituents, wherein each of the substituents is independently selected from the group consisting of amino, hydroxy, hydroxyalkyl, alkyl, and alkoxy; preferably, X'₁ is an oxygen atom, C₃₋₆ cycloalkyl, pyridinyl, or phenyl; the C₃₋₆ cycloalkyl, pyridinyl, or phenyl is optionally substituted with one or more amino or C₁₋₆ alkyl;
Y' is an oxygen atom;
A' is alkyl; preferably, A' is C₁₋₆ alkyl;
m' is selected from the group consisting of 1, 2, and 3; n' is selected from the group consisting of 1, 2, and 3.

30. The compound or the pharmaceutically acceptable salt thereof according to any of claims 21 to 27, wherein:
R¹¹ is H or alkyl; preferably, R¹¹ is H or C₁₋₆ alkyl;
R¹⁵ and R¹⁶, together with the atom to which they are attached, form a 3-6 membered carbocycle;
R¹⁷ is H;
R¹⁸ is H or alkyl; preferably, R¹⁸ is H or C₁₋₆ alkyl;
R¹⁹ is alkyl or cycloalkyl; preferably, R¹⁹ is C₁₋₆ alkyl or C₁₋₆ hydroxyalkyl;
or R¹⁸ and R¹⁹, together with the atoms to which they are attached, form a ring;
preferably, R¹⁸ and R¹⁹, together with the atoms to which they are attached, form a 3-6-membered carbocycle;
R²⁰ is alkyl or cycloalkyl; preferably, R²⁰ is C₁₋₆ alkyl or C₁₋₆ hydroxyalkyl;
X' is alkylene; preferably, X' is C₁₋₆ alkylene; more preferably, X' is pentylene;
Y' is an oxygen atom;
A' is alkyl; preferably, A' is C₁₋₆ alkyl;
provided that R¹⁹ and R²⁰ are not simultaneously alkyl.

31. The compound or the pharmaceutically acceptable salt thereof according to any of claims 21 to 27, wherein:
R¹¹ is H or C₁₋₆ alkyl; preferably, R¹¹ is H;
R¹⁵ is H;
R¹⁶ and R¹⁷, together with the atoms to which they are attached, form a 3-6 membered carbocycle;
R¹⁸ is H;
R¹⁹ is alkyl; preferably, R¹⁹ is C₁₋₆ alkyl;
R²⁰ is alkyl; preferably, R²⁰ is C₁₋₆ alkyl;
X' is alkylene; preferably, X' is C₁₋₆ alkylene; more preferably, X' is pentylene;
Y' is an oxygen atom;
A' is alkyl; preferably, A' is C₁₋₆ alkyl.

32. The compound or the pharmaceutically acceptable salt thereof according to any of claims 21 to 27, being selected from the group consisting of: 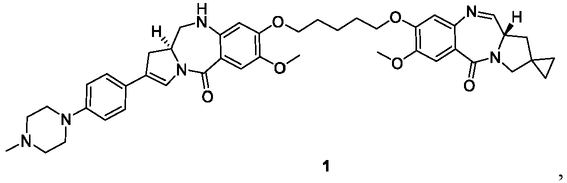 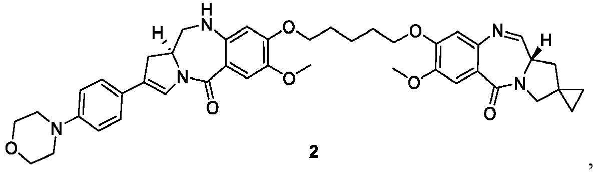 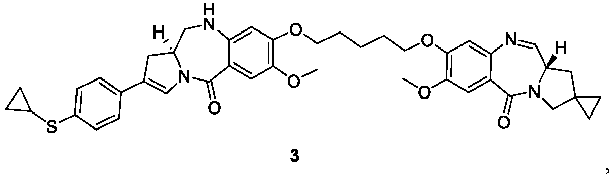 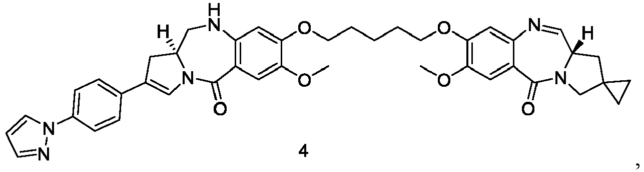 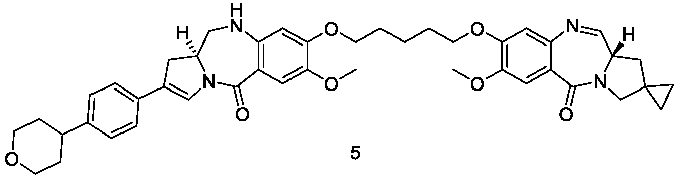 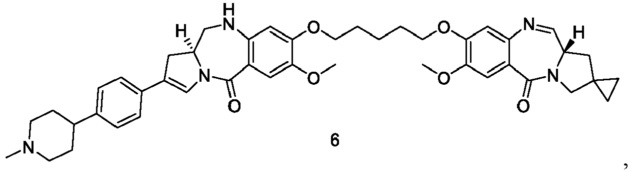 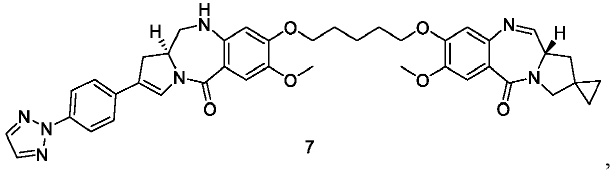 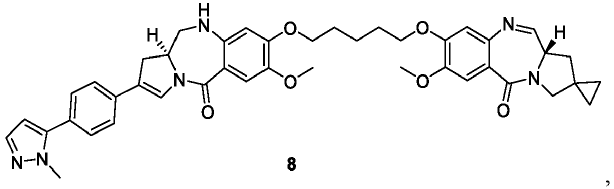 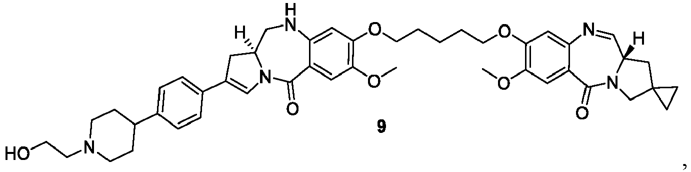 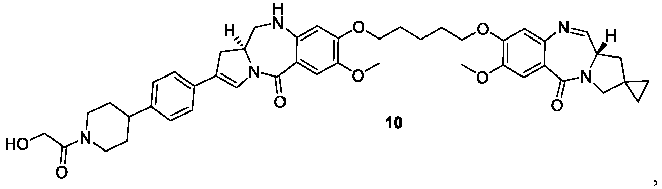 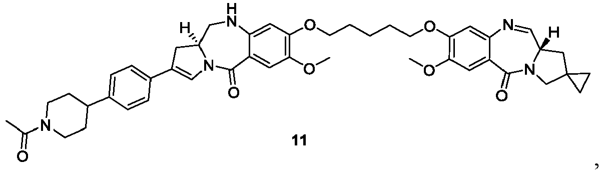 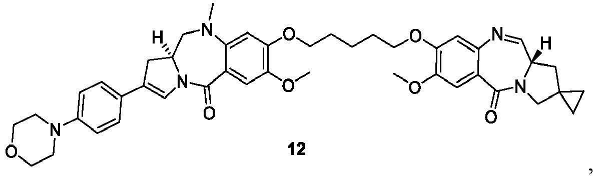 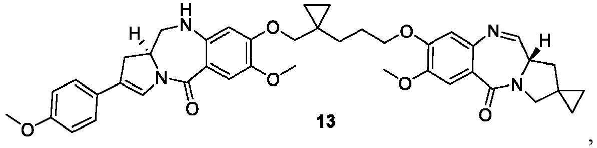 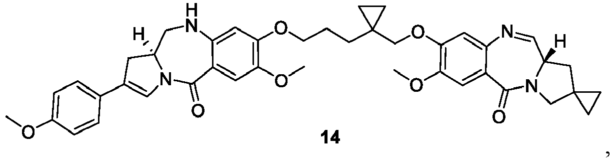 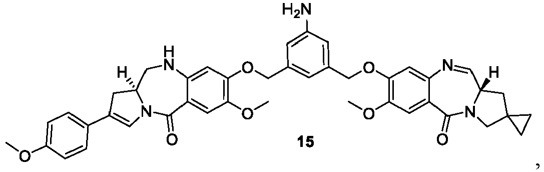 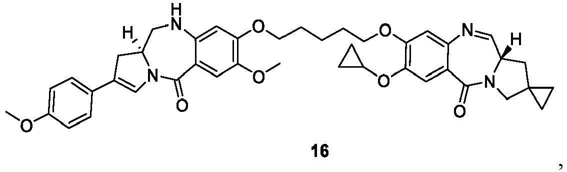 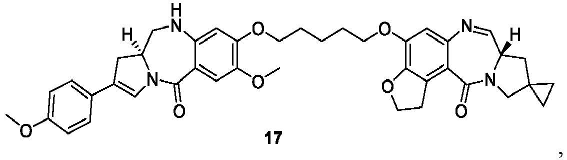 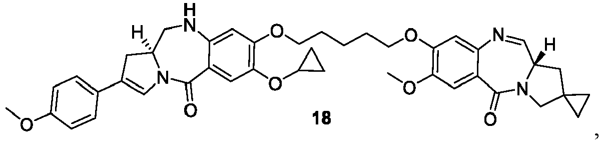 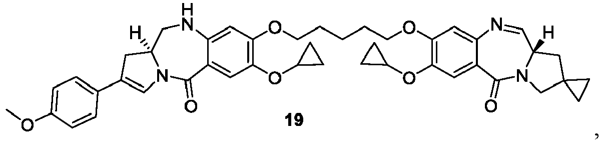 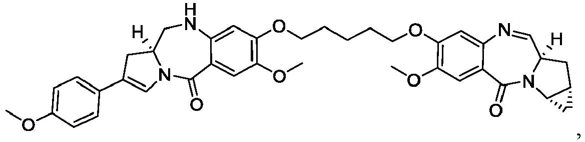 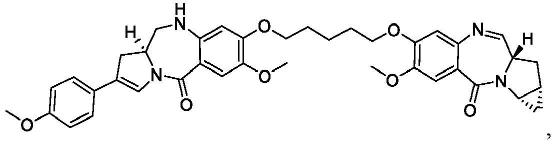 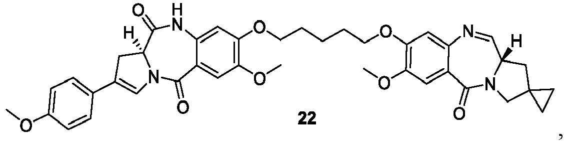 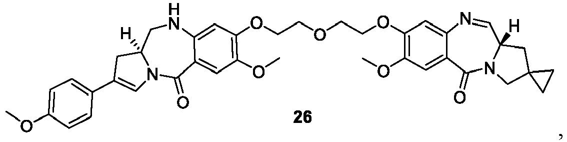 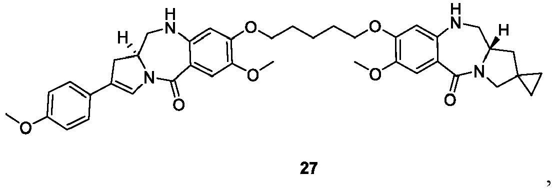 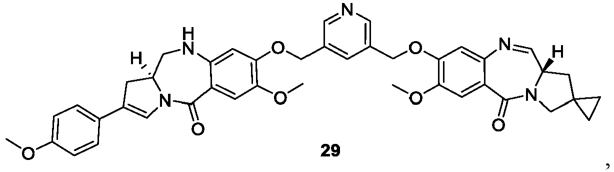 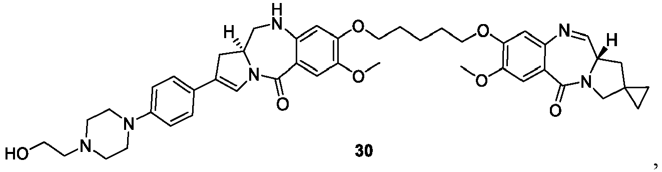 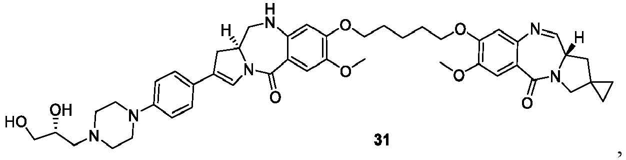 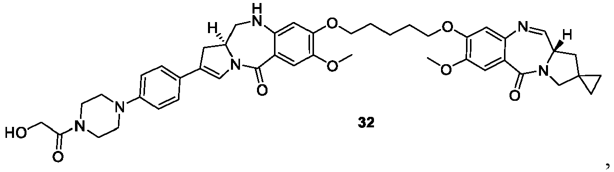 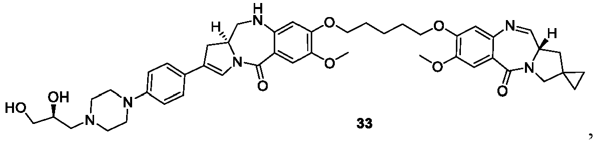 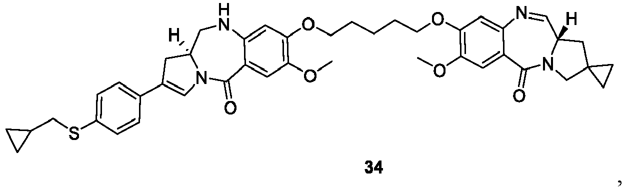 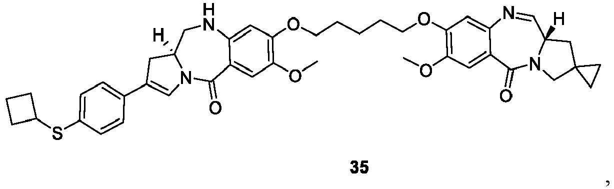 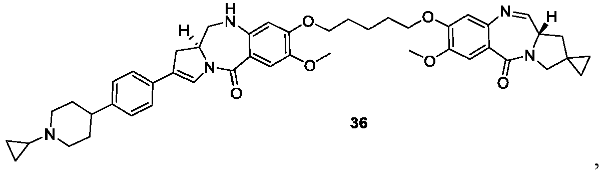 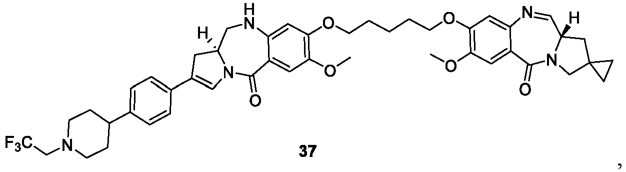 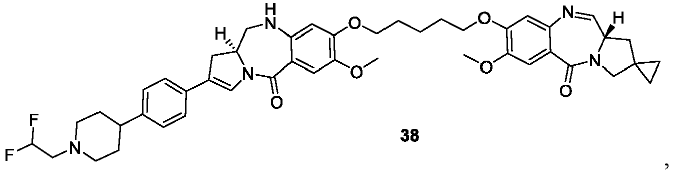 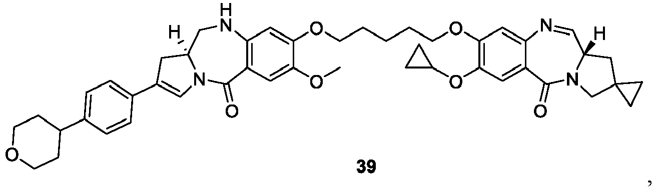 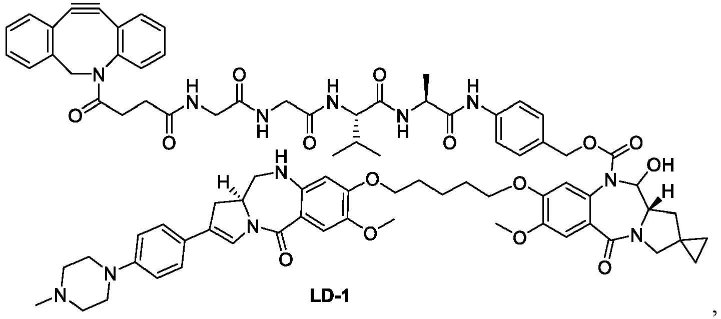 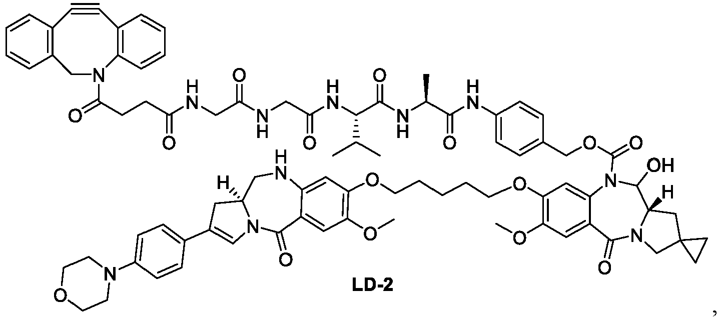 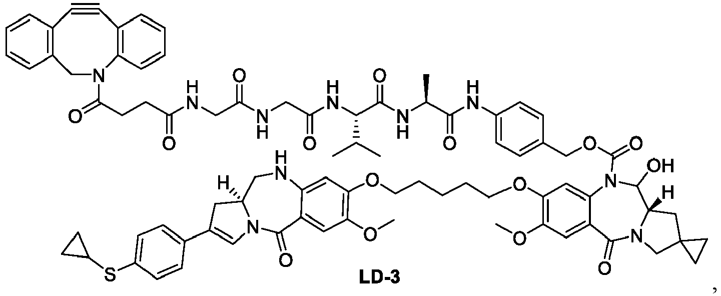 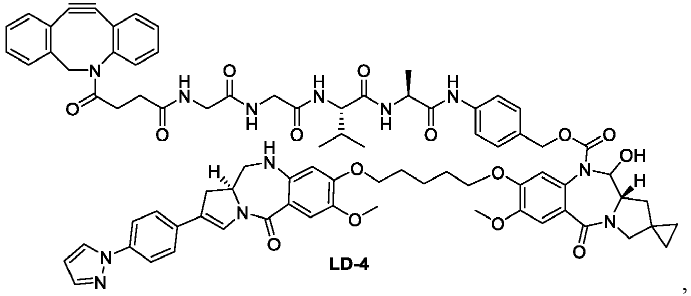 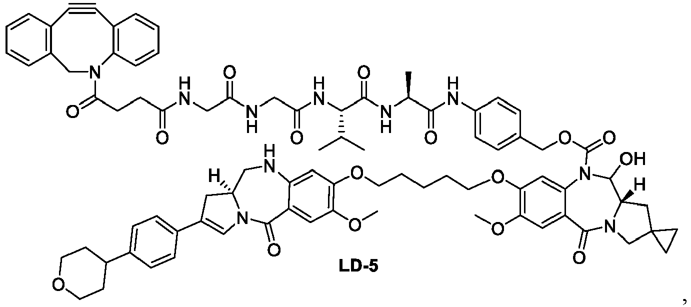 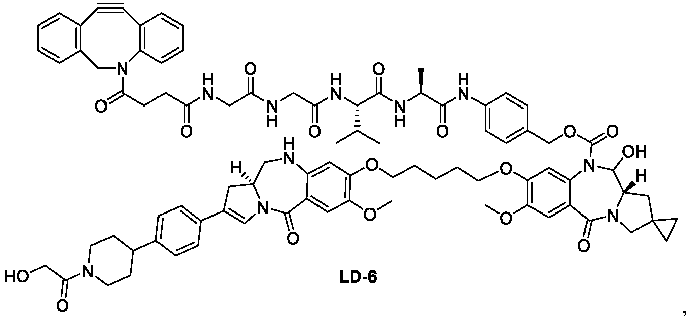 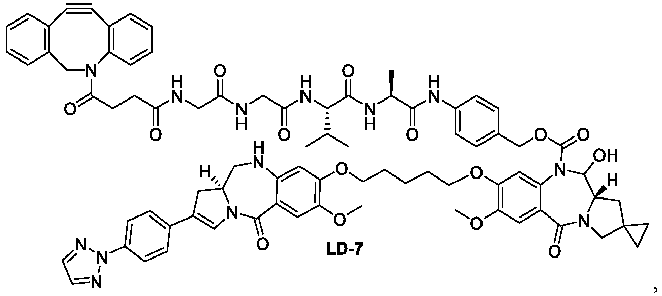 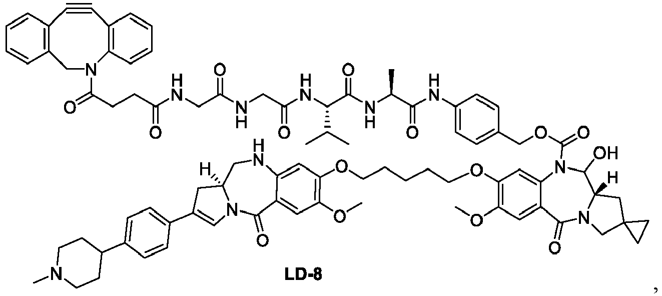 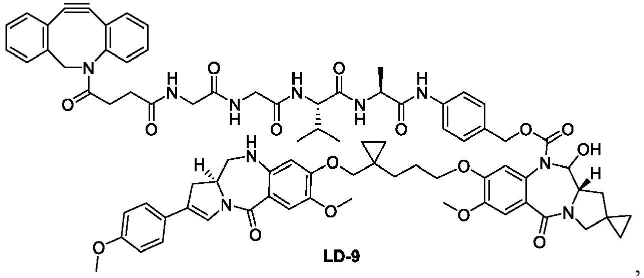 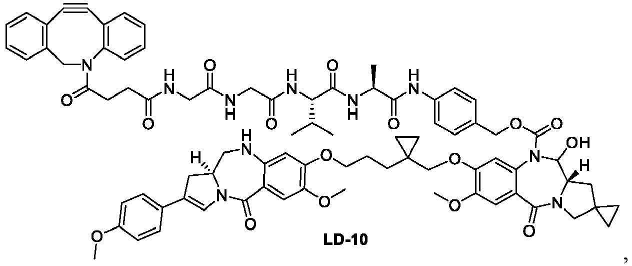 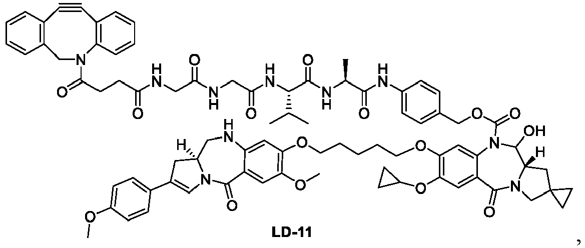 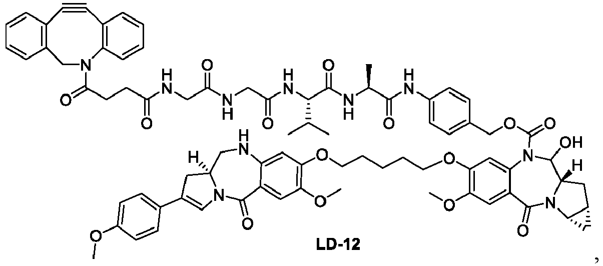 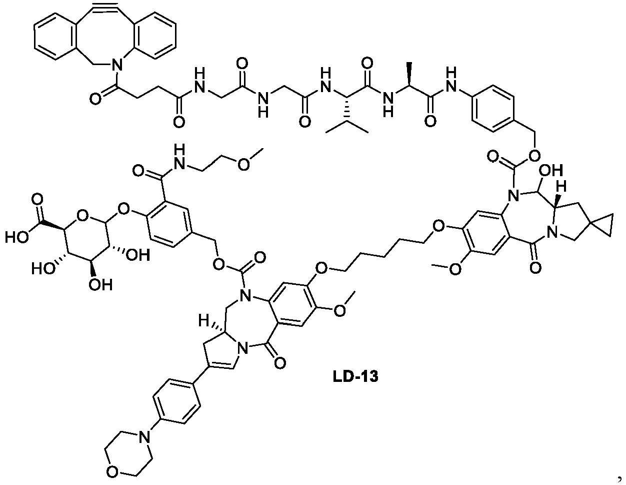 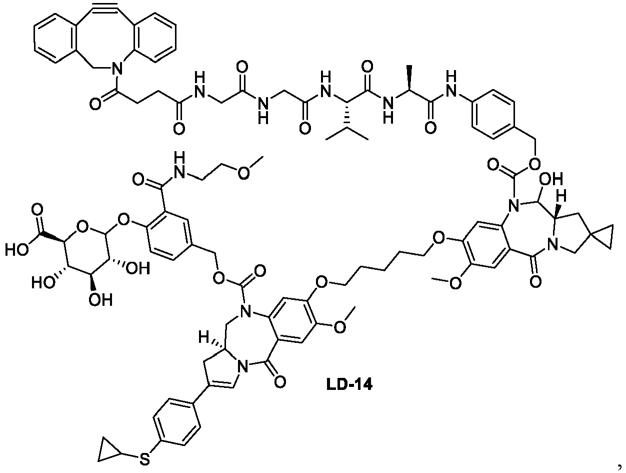 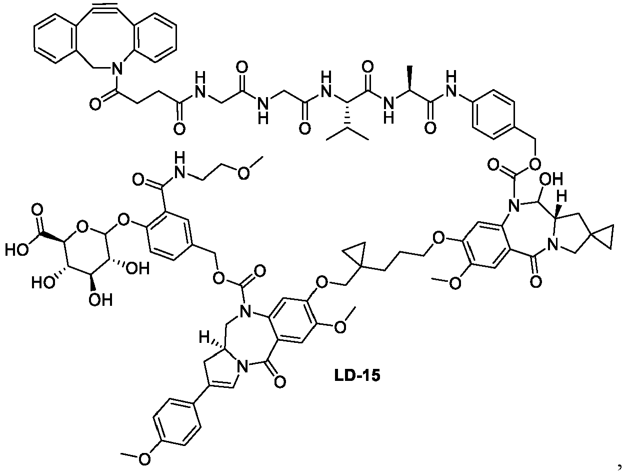 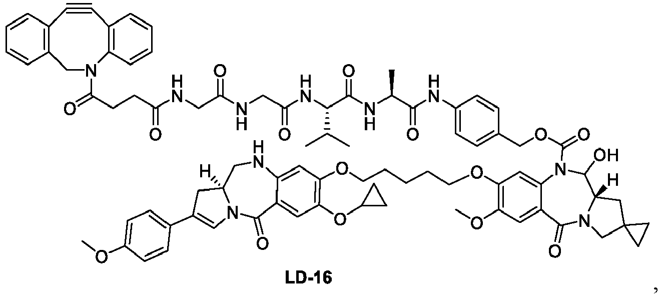 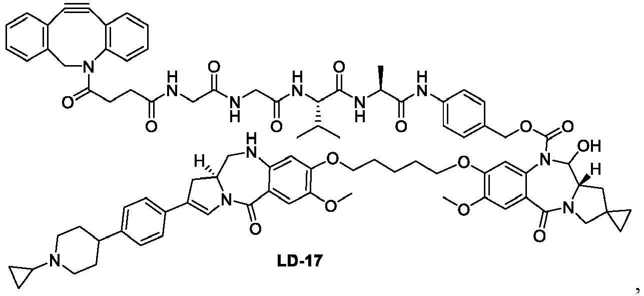 and

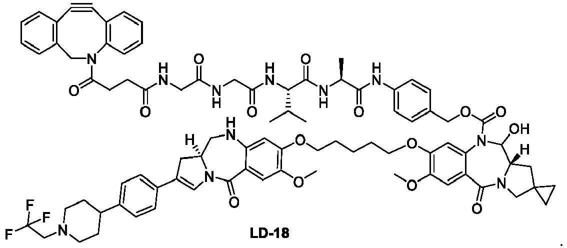

33. A pharmaceutical composition, comprising a therapeutically effective amount of the antibody-drug conjugate or the pharmaceutically acceptable salt thereof according to any of claims 1 to 20, or the compound represented by formula (DL) or the pharmaceutically acceptable salt thereof according to any of claims 21 to 32, and a pharmaceutically acceptable carrier, diluent, or excipient.

34. The antibody-drug conjugate or the pharmaceutically acceptable salt thereof according to any of claims 1 to 20, or the compound represented by formula (DL) or the pharmaceutically acceptable salt thereof according to any of claims 21 to 32, or the pharmaceutical composition according to claim 33 for use as a medicament.

35. Use of the antibody-drug conjugate or the pharmaceutically acceptable salt thereof according to any of claims 1 to 20, or the compound represented by formula (DL) or the pharmaceutically acceptable salt thereof according to any of claims 21 to 32, or the pharmaceutical composition according to claim 33 in the preparation of a medicament for treating a tumor, wherein
preferably, the tumor is ovarian cancer, lung cancer, gastric cancer, endometrial cancer, testicular cancer, cervical cancer, placental choriocarcinoma, kidney cancer, urothelial cancer, colorectal cancer, prostate cancer, glioblastoma multiforme, brain tumor, pancreatic cancer, breast cancer, melanoma, liver cancer, bladder cancer, or esophageal cancer.

36. A disaccharide compound represented by formula (OLS): 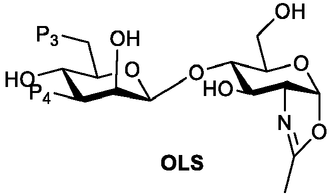
wherein P₃ and P₄ are each independently selected from the group consisting of hydroxy, N₃-(CH₂CH₂O)s¹-, and N₃-(CH₂CH₂O)s²-(CH₂ CH₂CH₂O)s³-(CH₂CH₂O)s⁴-; the N₃-(CH₂CH₂O)s¹- and N₃-(CH₂CH₂O)s²-(CH₂CH₂CH₂O)s³-(CH₂CH₂O)s⁴- are unsubstituted or are each independently substituted with one or more substituents, wherein each of the substituents is independently selected from the group consisting of halogen, C₁₋₆ alkyl, C₃₋₆ cycloalkyl, and C₁₋₆ alkoxy;
s¹ is selected from the group consisting of 1-10, preferably 1-5; s² is selected from the group consisting of 0-10, preferably 1-5; s³ is selected from the group consisting of 1-10, preferably 1-5; s⁴ is selected from the group consisting of 0-10, preferably 1-5;
provided that P₃ and P₄ are not simultaneously hydroxy or N₃-(CH₂CH₂O)s¹-;
preferably, the disaccharide compound represented by formula (OLS) is selected from the group consisting of: 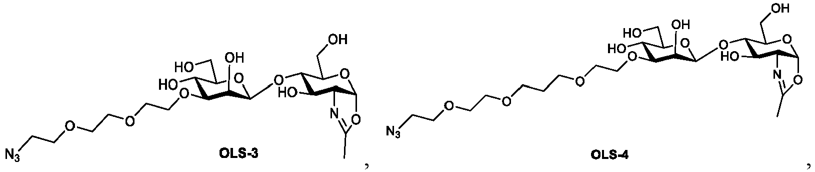 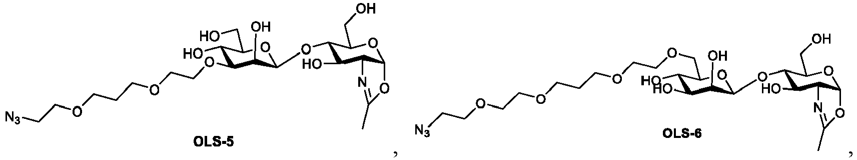 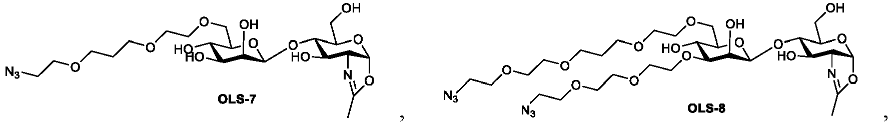 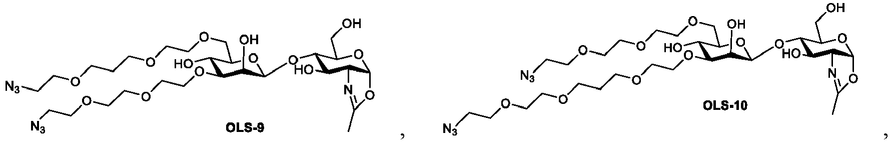 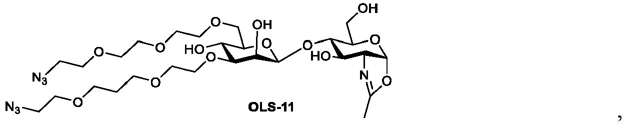 and

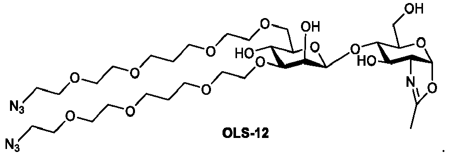

37. A sugar molecule, being: 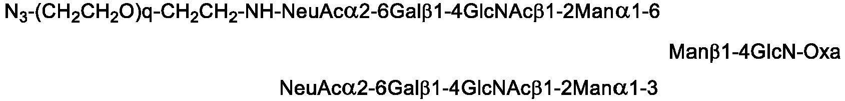 or 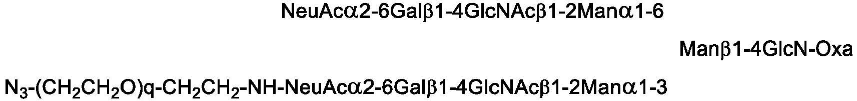 wherein q is 0-20; preferably, q is 1-10; more preferably, q is 2 or 3; wherein Oxa represents an oxazoline ring.
